(19) Europäisches Patentamt / European Patent Office / Office européen des brevets

(11) **EP 4 086 268 A1**

(12) **EUROPEAN PATENT APPLICATION**
published in accordance with Art. 153(4) EPC


(43) Date of publication:
**09.11.2022 Bulletin 2022/45**

(21) Application number: **20908515.8**

(22) Date of filing: **30.12.2020**

(51) International Patent Classification (IPC):
***C07H 15/26*** *(2006.01)* ***A61K 31/7056*** *(2006.01)*
***A61P 35/00*** *(2006.01)*

(52) Cooperative Patent Classification (CPC):
**A61K 31/7056; A61P 35/00; C07H 15/26;**
Y02P 20/55

(86) International application number:
**PCT/KR2020/019466**

(87) International publication number:
**WO 2021/137646 (08.07.2021 Gazette 2021/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.12.2019 KR 20190179748**



(71) Applicant: **LegoChem Biosciences, Inc.**
**Daejeon 34302 (KR)**

(72) Inventors:
- **SONG, Ho Young**
  **Daejeon 34302 (KR)**
- **PARK, Yun-Hee**
  **Daejeon 34302 (KR)**
- **LEE, Kun Jung**
  **Daejeon 34302 (KR)**
- **OH, Ji Hye**
  **Daejeon 34302 (KR)**
- **KIM, Sung Min**
  **Daejeon 34302 (KR)**
- **PARK, Kyung Eun**
  **Daejeon 34302 (KR)**
- **PARK, Yong Gyu**
  **Daejeon 34302 (KR)**
- **RYU, Hyun Min**
  **Daejeon 34302 (KR)**
- **HAN, Na Ra**
  **Daejeon 34302 (KR)**
- **CHUNG, Chul Woong**
  **Daejeon 34302 (KR)**
- **CHAE, Jei Wook**
  **Daejeon 34302 (KR)**
- **KIM, Yong Zu**
  **Daejeon 34302 (KR)**

(74) Representative: **Vossius & Partner**
**Patentanwälte Rechtsanwälte mbB**
**Siebertstraße 3**
**81675 München (DE)**


# (54) PYRROLOBENZODIAZEPINE DERIVATIVE AND LIGAND-LINKER CONJUGATE THEREOF


(57) The present invention relates to a pyrrolobenzodiazepine derivative compound, a conjugate of the same with a ligand, and the use of the same. The pyrrolobenzodiazepine derivative compound is more stable in plasma, is stable even in circulation, and exhibits excellent efficacy. In particular, the conjugate of the pyrrolobenzodiazepine derivative compound with a ligand has a great advantage in that it is possible to target and specifically treat proliferative disease such as cancer, maximize drug efficacy, and minimize the expression of side effects since the conjugate more stably reaches the target cell and effectively exerts drug efficacy while toxicity is greatly reduced as a linker technology that allows drugs to be easily released within cancer cells and maximize drug efficacy is incorporated.


【FIG. 1】

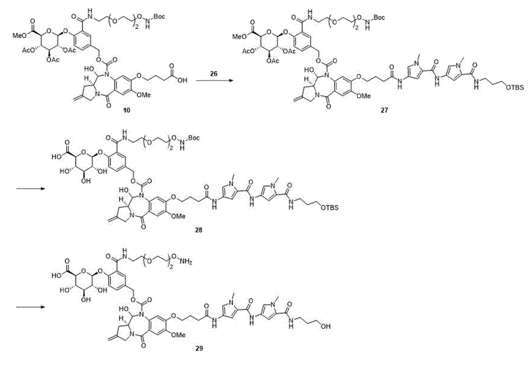


10 27 28 29

EP 4 086 268 A1

## Description

[Technical Field]

The present invention relates to a pyrrolobenzodiazepine derivative and a ligand-linker conjugate compound of the same, pharmaceutically acceptable salts thereof, or solvates thereof. The present invention also relates to a pharmaceutical composition for prevention or treatment of proliferative disease comprising the same.

[Background Art]

DNA refers to a high molecular compound with a double helix structure in which two long strands, which are polymers of nucleotides, wind around each other, and is formed through the polymerization of nucleotides divided into unique nucleobases of cytosine (C), guanine (G), adenine (A), and thymine (T). Such DNA may form chromatin, and the condensed structure of chromatin limits the cell's mechanical access to DNA, and this inhibits essential processes such as transcription, replication, repair and recombination.

Pyrrolobenzodiazepines (PBDs) are natural substances having antibiotic or antitumor properties, and refers to sequence-selective DNA alkylating compounds. Pyrrolobenzodiazepines exhibit biological activity by binding in the minor groove of DNA through selectivity to the 5'-purine-guanine-purine sequence and forming covalent bonds with exocyclic amino groups of guanine bases 3 and 4. It is known that this makes pyrrolobenzodiazepines stronger than systemic chemotherapeutic drugs and block the division of cancer cells without distorting the DNA helix, thereby exhibiting anticancer effects without the general phenomenon of drug resistance. The first PBD, anthramycin, was discovered in 1965. Since then, a number of naturally occurring PBDs have been reported, and over 10 synthesis routes have been developed for a variety of analogues (Hu Y, et al., Benzodiazepine biosynthesis in Streptomyces refuineus. Chem Biol. 2007 Jun;14(6):691-701) .

Other agents belonging to the discovered pyrrolobenzodiazepine antibiotic group include abbeymycin, chicamycin, DC-81, mazethramycin, neothramycin A, porothramycin, prothracarcin and the like, and analogs of pyrrolobenzodiazepines have been steadily studied.

Pyrrolobenzodiazepines have the following general formula.

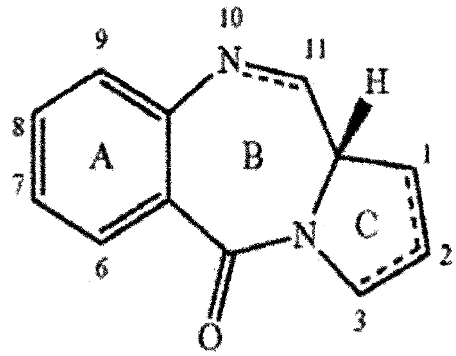

The pyrrolobenzodiazepines differ from one another in the number, type and position of substituents on the aromatic ring A and the pyrrolo C ring, and the degree of saturation of the C ring. An imine (N=C), carbinolamine (NH-CH(OH)) or carbinolamine methyl ether (NH-CH(OMe)) exists at the N10-C11 position, the electrophilic center responsible for the alkylation of DNA, of the B ring.

It has been known through recent studies that pyrrolobenzodiazepines may be linked with other monomers through the C8 position to form dimers and the dimers may be used as antibody-drug conjugates (ADCs). For example, there are SG3249 (tesirine) with SG3199 as a payload and SGD-1910 (talirine) with dimer SGD-1882 having endo-exo unsaturation as a payload. Recently, ADCT-301, ADCT-402, ADCT-601 and ADCT-602 including tesirine have also been in clinical trials.

In this regard, there are a registered patent for a pyrrolobenzodiazepine (Medimmune, KR 10-1960130), a registered patent for a pyrrolobenzodiazepine and a conjugate of the same (Medimmune, KR 10-1891859), a registered patent for a pyrrolobenzodiazepine (Spirogen, KR 10-1059183), a registered patent for a pyrrolobenzodiazepine-anti-CD22-antibody conjugate (ADC Therapeutics SA, KR 10-1995620), a published patent for a pyrrolobenzodiazepine-antibody conjugate (ADC Therapeutics SA, KR 10-2019-0100413), a published patent for a pyrrolobenzodiazepine and a conjugate of the same (Mersana Therapeutics, US 2017-0369453), a published patent for pyrrolobenzodiazepine prodrug (ENDOCYTE, WO 2016-148674), and the like.

Meanwhile, there are a paper, which discloses that monomeric pyrrolobenzodiazepine compounds used as a precursor are stable and exhibit little cytotoxicity (Masterson, Luke A. et al., Synthesis and biological evaluation of novel pyrrolo[2,1-c][1,4]benzodiazepine prodrugs for use in antibody directed enzyme prodrug therapy, Bioorganic & Medicinal Chemistry Letters, 16(2), 252-256 (2006)), a technology related to an antibody-drug conjugate having a form linked to the form of a pyrrolobenzodiazepine dimer as a carbamate (Zhang, Donglu et al, Linker Immolation Determines Cell Killing Activity of Disulfide-Linked Pyrrolobenzodiazepine Antibody-Drug Conjugates, ACS Medicinal Chemistry Letters, 7(11), 988-993 (2016)), and the like.

However, in the case of the above technologies, there are limitations in that it is not easy to scale up the compounds because of a low yield at the time of pyrrolobenzodiazepine synthesis, it is difficult to satisfactorily solve the problem of poor stability in blood after administration, and the compounds cannot be applied clinically because of the toxicity even if the drug efficacy is excellent.

DNA minor groove binders are family compounds of lexitropsins, the first discovered compounds are distamycin A and netropsin, and distamycin A was identified as an antibiotic isolated from a culture of *Strptomyces distallicus* in 1962. In addition, there are anthracycline-based anticancer drugs such as actinomycin A and daunorubicin, and these anticancer drugs have a mechanism of inhibiting the synthesis of DNA and RNA by intervening in the G-C base pairing site or by binding to the DNA minor groove to form hydrogen bonds with the base pairs.

Studies on the anticancer effect of these agents are being conducted, but these agents have the side effect in common that they are highly toxic to the heart and cause life-threatening heart damage.

Therefore, there is a demand for the development of more selective and effective drugs with greatly few side effects.

Accordingly, the inventors of the present invention have prepared new compounds by conjugating various substituents to the conventional pyrrolobenzodiazepine parent nucleus structures, and confirmed that the synthesized compounds have significantly reduced side effects such as toxicity while exhibiting an equivalent level of efficacy compared to conventionally known pyrrolobenzodiazepine compounds, thereby completing the present invention.

[Summary of Invention]

[Technical Problem]

An object of the present invention is to provide a novel pyrrolobenzodiazepine derivative compound with significantly reduced side effects such as toxicity while exhibiting an equivalent level of efficacy compared to a conventional pyrrolobenzodiazepine compound, and a conjugate of the same with a ligand.

Another object of the present invention is to provide a pharmaceutical composition for prevention or treatment of proliferative disease comprising the compound or a conjugate of the compound with a ligand as an active ingredient.

Another object of the present invention is to provide a method for preventing or treating proliferative disease by administering the compound or a conjugate of the compound with a ligand to an individual.

[Solution to Problem]

The present invention relates to a pyrrolobenzodiazepine derivative, a pharmaceutically acceptable salt or solvate thereof.

In the present invention, the pyrrolobenzodiazepine derivative is represented by the following Chemical Formula I, and a pharmaceutically acceptable salt or solvate thereof is provided:

$$P-(B)_a-(B')_b-D \quad (I)$$

where,

P is represented by the following Chemical Formula II, where P is bonded or not bonded to Linker I;

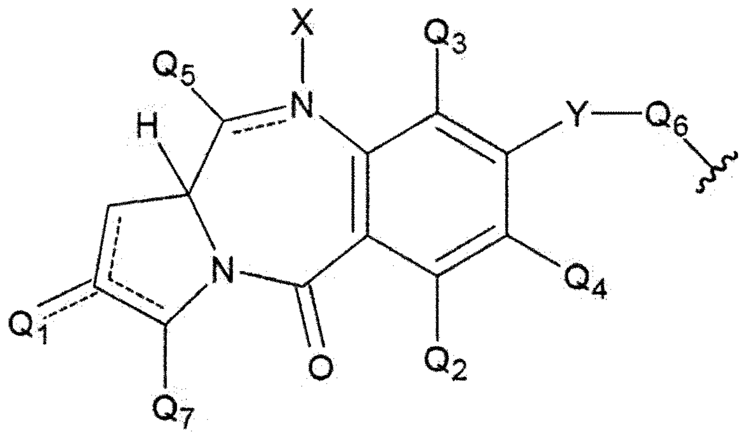

(II)

where,

a dotted line indicates arbitrary presence of a double bond between C1 and C2 or C2 and C3 and arbitrary presence of a double bond between N10 and C11;

$Q_1$ is selected from the group consisting of H, OH, O, $CH_2$, CN, $R^m$, $OR^m$, CH-$R^{m'}$, C($R^{m'}$)$_2$, O-$SO_2$-$R^m$, $CO_2R^m$, $COR^m$, halo and dihalo,

where $R^{m'}$ is selected from the group consisting of $R^m$, $CO_2R^m$, $COR^m$, CHO, $CO_2H$, and halo,

where $R^m$ is selected from the group consisting of substituted or unsubstituted $C_{1-12}$ alkyl, substituted or unsubstituted $C_{2-12}$ alkenyl, substituted or unsubstituted $C_{2-12}$ alkynyl, substituted or unsubstituted $C_{5-20}$ aryl, substituted or unsubstituted $C_{5-20}$ heteroaryl, substituted or unsubstituted $C_{3-6}$ cycloalkyl, substituted or unsubstituted 3- to 7-membered heterocyclyl, substituted or unsubstituted 3- to 7-membered heterocycloalkyl, and substituted or unsubstituted 5- to 7-membered heteroaryl,

wherein, when substituted, respective hydrogen atoms of $C_{1-12}$ alkyl, $C_{1-12}$ alkoxy, $C_{2-12}$ alkenyl, $C_{2-12}$ alkynyl, $C_{5-20}$ aryl, $C_{5-20}$ heteroaryl, $C_{3-6}$ cycloalkyl, 3- to 7-membered heterocyclyl, 3- to 7-membered heterocycloalkyl, or 5- to 7-membered heteroaryl are each independently substituted with any one or more selected from the group consisting of $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3- to 7-membered heterocyclyl, 3- to 7-membered heterocycloalkyl, and 5-to 7-membered heteroaryl;

$Q_2$, $Q_3$, $Q_5$, and $Q_7$ are each independently selected from the group consisting of -H, -$R^m$, -OH, -$OR^m$, -SH, - $SR^m$, -$NH_2$, -$NHR^m$, -$NR^mR^{m'}$, -$NO_2$, and halo,

where $R^m$ and $R^{m'}$ are as defined above;

$Q_4$ is selected from the group consisting of -H, -$R^m$, -OH, -$OR^m$, -SH, -$SR^m$, -$NH_2$, -$NHR^m$, -$NR^mR^{m'}$, -$NO_2$, halo, substituted or unsubstituted $C_{1-6}$ alkyl, substituted or unsubstituted $C_{1-6}$ alkoxy, substituted or unsubstituted $C_{2-6}$ alkenyl, substituted or unsubstituted $C_{2-6}$ alkynyl, substituted or unsubstituted $C_{3-6}$ cycloalkyl, substituted or unsubstituted 3- to 7-membered heterocycloalkyl, substituted or unsubstituted $C_{5-12}$ aryl, substituted or unsubstituted 5- to 7-membered heteroaryl, -CN, -NCO,-$OR^n$, -OC(=O)$R^n$, -OC(=O)$NR^nR^{n'}$, -OS(=O)$R^n$, -OS(=O)$_2R^n$, -$SR^n$, -S(=O)$R^n$, -S(=O)$_2R^n$, -S(=O)$NR^nR^{n'}$, -S(=O)$_2NR^nR^{n'}$,-OS(=O)$NR^nR^{n'}$, -OS(=O)$_2NR^nR^{n'}$, -$NR^nR^{n'}$, -$NR^n$C(=O)$R^o$,-$NR^n$C(=O)$OR^o$, -$NR^n$C(=O)$NR^oR^{o'}$, -NR"S(=O)$R^o$, -$NR^n$S(=O)$_2R^o$,-$NR^n$S(=O)$NR^oR^{o'}$, -$NR^n$S(=O)$_2NR^oR^{o'}$, -C(=O)$R^n$, -C(=O)$OR^n$ and-C(=O)$NR^nR^{n'}$,

wherein, when substituted, one or more hydrogen atoms may be each independently substituted with $C_{1-6}$ alkyl, $C_{1-6}$ alkoxy, $C_{2-6}$ alkenyl, $C_{2-6}$ alkynyl, $C_{3-6}$ cycloalkyl, 3- to 7-membered heterocycloalkyl, $C_{5-10}$ aryl, 5- to 7-membered heteroaryl, -ORP, -OC(=O)$R^p$, - OC(=O)$NR^pR^{p'}$, -OS(=O)$R^p$, -OS(=O)$_2$RP, -SRP, -S(=O)$R^p$, - S(=O)$_2$RP, -S(=O)$NR^pR^{p'}$, -S(=O)$_2NR^pR^{p'}$, -OS(=O)$NR^pR^{p'}$, - OS(=O)$_2NR^pR^{p'}$, -$NR^pR^{p'}$, -$NR^p$C(=O)$R^q$, -$NR^p$C(=O)$OR^q$, - $NR^p$C(=O)$NR^qR^{q'}$, -$NR^p$S(=O)$R^q$, -$NR^p$S(=O)$_2R^q$, -$NR^p$S(=O)$NR^qR^{q'}$, - $NR^p$S(=O)$_2NR^qR^{q'}$, -C(=O)$R^p$, -C(=O)$OR^p$ or -C(=O)$NR^pR^p$,

where, $R^n$, $R^{n'}$, $R^o$, $R^{o'}$, $R^p$, $R^{p'}$, $R^q$ and $R^{q'}$ are each independently selected from the group consisting of hydrogen, $C_{1-7}$ alkyl, $C_{2-7}$ alkenyl, $C_{2-7}$ alkynyl, $C_{3-13}$ cycloalkyl, 3- to 7-membered heterocycloalkyl, $C_{6-10}$ aryl, and 5- to 7-membered heteroaryl;

X is a moiety bonded to Linker I, and is absent when not bonded to Linker I,

Y is selected from the group consisting of -O-, -S-, and -NH-; and

$Q_6$ is absent, or is a substituted or unsubstituted and saturated or unsaturated $C_{3-12}$ hydrocarbon chain,

B and B' are each independently a bond, -O-, -NH-, - S-, -C(=O)-, -S(=O)-, -O-$(CH_2)_n$-, -$(CH_2)_n$-, -$(CH_2)_n$(C=O)-, -$(CH_2)_n$(C=O)NH-, -$C_{6-10}$ arylene-, -O-(-$C_{6-10}$ arylene)-, - $(CH_2)_n$-$C_{6-10}$ arylene-, 5- to 20-membered heteroarylene

containing a heteroatom independently selected from N, O or S, -0-(5- to 20-membered heteroarylene containing a heteroatom independently selected from N, 0 or S)- or - $(CH_2CH_2O)_n$-,
where n is each independently an integer of 1 or more and 10 or less, and
a and b are each independently an integer of 0 or 1, and
D is represented by the following Chemical Formula III,

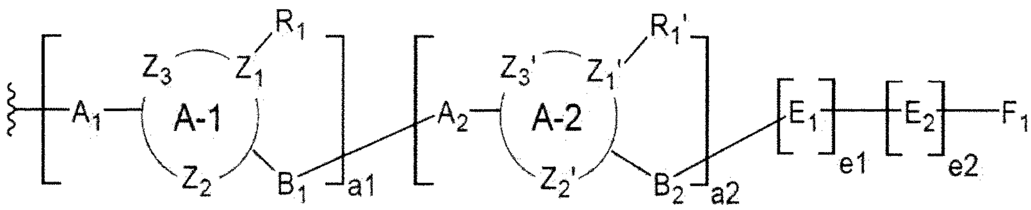

(III)

where,

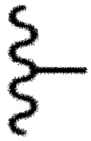

means a site bonded to P, B or B',
$A_1$ and $A_2$ are each independently a bond, -NH-, -O-, -C(=O)-, -C(=O)NH-, -NHC(=O)-, $C_{6-10}$ arylene or 6- to 10-membered heteroarylene containing a heteroatom independently selected from N, O or S;
A-1 and A-2 are each independently selected from 5-to 20-membered heterocycloalkylene, 5- to 20-membered heterocycloalkenylene, or 5- to 20-membered heteroarylene, in which one or more ring atoms are heteroatoms independently selected from N, O or S,
$Z_1$, $Z_2$, $Z_3$, $Z_1'$, $Z_2'$ and $Z_3'$ are each independently C, N or S,
$R_1$ and $R_1'$ are each independently $C_{1-10}$ heteroalkyl in which one or more atoms include heteroatoms independently selected from N, O or S, $C_{1-10}$ alkyl, $-(CH_2)_mOH$, $-(CH_2)_mNH_2$, $-(CH_2)_mNHCH_3$, hydrogen, halo, or -C(=O)OH,
m is an integer from 0 to 10,
$B_1$ and $B_2$ are each independently a bond, -NH-, -O-, -C(=O)-, or -S-,
$a_1$ and $a_2$ are each independently an integer from 0 to 2; wherein $a_1 + a_2$ is an integer of 2 or less,
$E_1$ is a bond, -NH-, -C(=O)-, -C(=O)O-, -O-, -S-, - $OCH_2$-, $C_{1-20}$ alkylene, or $-((CH_2)_p(CH_2E_{11}))_q-$, and $e_1$ is an integer from 0 to 10;
$E_2$ is a bond, $C_{1-20}$ alkylene, or $-((CH_2)_p(CH_2E_{11}))_q-$, and $e_2$ is an integer from 0 to 20,
where $E_{11}$ is a bond, O, or S, p is an integer from 0 to 10, and q is an integer from 1 to 20; and
$F_1$ is hydrogen, -OH, $-OCH_3$, -SH, $-SCH_3$,

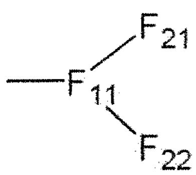 , 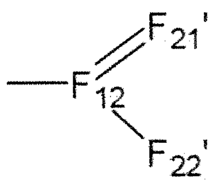 , or 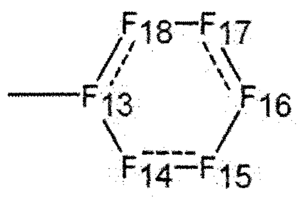 ,

where,
$F_{11}$, $F_{12}$, $F_{13}$, $F_{14}$, $F_{15}$, $F_{16}$, $F_{17}$, and $F_{18}$ are each independently C, N, 0 or S,
a dotted line means a double bond or a single bond,
$F_{21}$, $F_{22}$ and $F_{22}'$ are each independently selected from -H, $-NH_2$, $-CH_3$, $-(CH_2)_rCH_3$, $-(CH_2)_rOH$, $-(CH_2)_rNH_2$, $-F_{33}(CH_2)_rCH_3$, $-F_{33}(CH_2)_rOH$, $-F_{33}(CH_2)_rNH_2$, or Linker II, where $F_{33}$ is selected from -O-, -S-, $-CH_2$-, -C(=O)-, -NH-, $-C(NH_2)$- or -C(=NH)-, and
$F_{21}'$ is selected from $=CH_2$, =NH, $=F_{33}'(CH_2)_rCH_3$, $=F_{33}'(CH_2)_rOH$, or $=F_{33}'(CH_2)_rNH_2$, where $F_{33}'$ is selected from =N-, =CH-, =C(OH)- or $=C(NH_2)$- and r is an integer from 0 to 10;

wherein, Linker I and Linker II are represented by the following Chemical Formula IV, and are each independently selected,

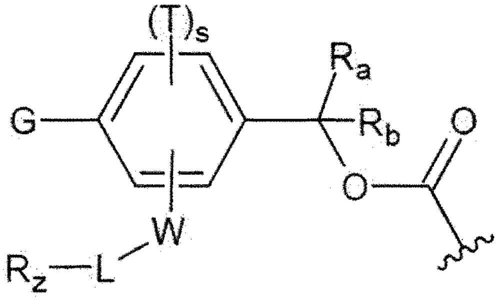

(IV)

where,
G is a glucuronic acid) moiety or

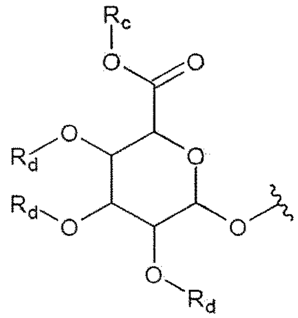

;

where $R_c$ is hydrogen or a carboxyl protecting group,
each $R_d$ is independently hydrogen or a hydroxyl protecting group;
$R_a$ and $R_b$ are each independently hydrogen, $C_{1-8}$ alkyl or $C_{3-8}$ cycloalkyl;
W is -C(=O)-, -C(=O)NR'-, -C(=O)O-, $-S(=O)_2NR'-$, - P(=O)R"NR'-, -S(=O)NR'- or $-P(=O)_2NR'-$, and C, S or P is directly bonded to a phenyl ring,
where R' and R" are each independently hydrogen, $C_{1-8}$ alkyl, $C_{3-8}$-membered cycloalkyl, $C_{1-8}$ alkoxy, $C_{1-8}$ alkylthio, mono- or di- $C_{1-8}$ alkylamino, 3- to 20-membered heteroaryl or a compound of $C_{6-20}$-membered aryl;
T is independently $C_{1-8}$ alkyl, halogen, cyano or nitro;
s is an integer from 0 to 3;
L is one or more units selected from the group consisting of a connection unit and a branching unit, or a combination of these units,
wherein the connection unit connects W and $R_z$, W and a branching unit, or a branching unit and another branching unit, wherein the branching unit connects a connection unit and W or a connection unit and another connection unit, the connection unit is

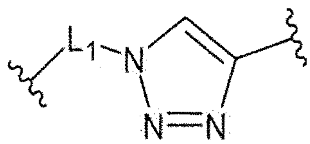

,

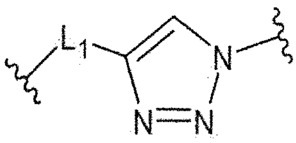
,
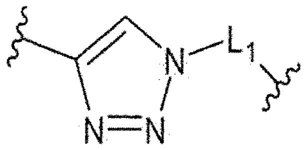
,
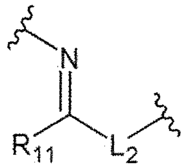
,

$(CH_2CH_2V)_t$-, $-(CH_2)_t(V(CH_2)_u)_v$-, or a combination thereof;

where $L_1$ is a single bond or $C_{2-30}$ alkenyl, $R_{11}$ is H or $C_{1-10}$ alkyl, and $L_2$ is $C_{2-30}$ alkenyl;

where t is an integer from 0 to 10, u is an integer from 0 to 12, v is an integer from 1 to 20, V is a single bond, -O-, or -S-,

the branching unit is selected from the group consisting of $C_{2-100}$ alkenyl, a hydrophilic amino acid, - C(=O)-, $-C(=O)NR^v$-, -C(=O)O-, $-(CH_2)_{n1}-NHC(=O)-(CH_2)_{n2}$-, - $(CH_2)_{n3}-C(=O)NH-(CH_2)_{n4}$-, $-(CH_2)_{n1}-NHC(=O)-(CH_2)_{n2}-C(=O)$-,$-(CH_2)_{n3}-C(=O)NH-(CH_2)_{n4}-C(=O)$-, $-S(=O)_2NR^v$-, $-P(=O)R^wNR^v$-,$-S(=O)NR^v$-, and $-P(=O)_2NR^v$-,

wherein, when the branching unit is $C_{2-100}$ alkenyl, a carbon atom of the alkenyl may be substituted with one or more heteroatoms selected from the group consisting of N, O and S, and the alkenyl may be further substituted with one or more $C_{1-20}$ alkyl,

where $R^v$ and $R^w$ are each independently H, $C_{1-8}$ alkyl, $C_{3-8}$ cycloalkyl, $C_{1-8}$ alkoxy, $C_{1-8}$ alkylthio, mono- or di-$C_{1-8}$ alkylamino, $C_{3-20}$ heteroaryl or $C_{5-20}$ aryl,

n1, n2, n3 and n4 are each independently an integer from 0 to 10, and

$R_z$ is $-O-NH_2$, $-NH_2$, $N_3$, substituted or unsubstituted $C_{1-12}$ alkyl, $C_{1-12}$ alkynyl, $C_{1-3}$ alkoxy, substituted or unsubstituted 3- to 20-membered heteroaryl, 3- to 20-membered heterocyclyl, substituted or unsubstituted $C_{5-20}$ aryl, or

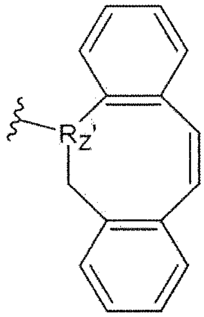
,

where $R_z$' is N or C,

wherein, when substituted, one or more hydrogen atoms are each independently substituted with OH, =0, halo, $C_{1-6}$ alkyl, $C_{1-6}$ alkoxy, $C_{2-6}$ alkenyl, oxy, carboxy, $C_{1-6}$ alkoxycarbonyl, $C_{1-6}$ alkylcarbonyl, formyl, $C_{3-8}$ aryl, $C_{5-12}$ aryloxy, $C_{5-12}$ arylcarbonyl or $C_{3-6}$ heteroaryl.

In an aspect of the present invention, the linker may further contain a binding unit formed by a 1,3-dipolar cycloaddition reaction, a hetero-Diels-Alder reaction, a nucleophilic substitution reaction, a non-aldol-type carbonyl reaction, an addition to a carbon-carbon multiple bond, an oxidation reaction or a click reaction.

In an aspect of the present invention, the binding unit is formed by a reaction between acetylene and an azide, or between an aldehyde or ketone group and a hydrazine or alkoxyamine.

In an aspect of the present invention, the linker may further contain at least one or more isoprenyl units having the structure of

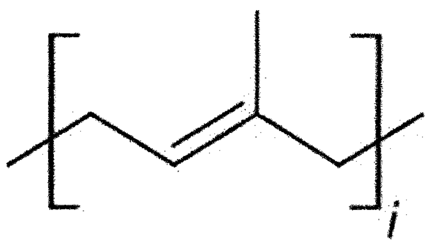

, where i is at least 2 or more.

In an aspect of the present invention, at least one isoprenyl unit is a substrate of an isoprenoid transferase or a product of an isoprenoid transferase.

In an aspect of the present invention, the isoprenyl unit of the linker is covalently bound to an antibody by a thioether bond, and the thioether bond contains a sulfur atom of cysteine of the antibody.

In an aspect of the present invention, the antibody contains an amino acid motif recognized by an isoprenoid transferase, and the thioether bond contains a sulfur atom of cysteine of the amino acid motif.

In an aspect of the present invention, the dotted line may indicate the presence of a double bond between C2 and C3.

In an aspect of the present invention, $Q_1$ may be selected from the group consisting of substituted or unsubstituted $C_{1-6}$ alkyl, substituted or unsubstituted $C_{2-6}$ alkenyl, substituted or unsubstituted $C_{5-7}$ aryl and substituted or unsubstituted $C_{3-6}$ heteroaryl.

In an aspect of the present invention, $Q_2$, $Q_3$ $Q_5$, and $Q_7$ may each independently be -H or -OH.

In an aspect of the present invention, $Q_4$ may be methoxy, ethoxy or butoxy.

In an aspect of the present invention, X may be bonded to the

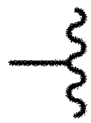

moiety of Linker I.

In an aspect of the present invention, Y may be -O-.

In an aspect of the present invention, $Q_6$ may be a substituted or unsubstituted and saturated or unsaturated $C_{3-8}$ hydrocarbon chain.

In an aspect of the present invention, G may be

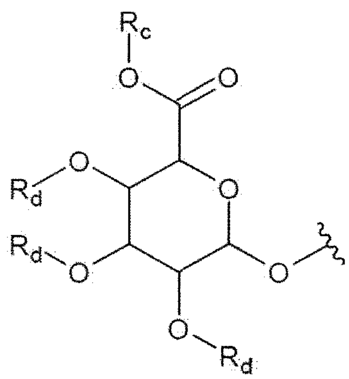

,

where $R_c$ may be hydrogen or a carboxyl protecting group, and each $R_d$ may be independently hydrogen or a hydroxyl protecting group.

In a specific aspect of the present invention, $R_c$ may be hydrogen and each $R_d$ may be hydrogen.

In an aspect of the present invention, T may independently be $C_{1-8}$ alkyl, halogen, cyano or nitro.

In an aspect of the present invention, s may be 0.

In an aspect of the present invention, W may be - C(=O)-, -C(=O)NR'- or -C(=O)O-.

In an aspect of the present invention, W may be - C(=O)NR'-, where C(=0) may be bonded to a phenyl ring and NR' may be bonded to L.

In a specific aspect of the present invention, G may be

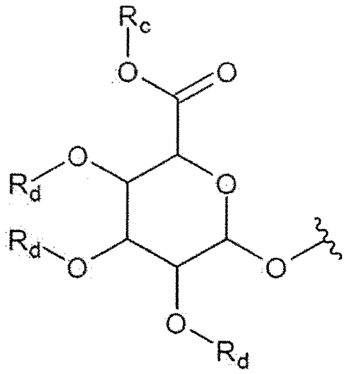

, and W may be -C(O)NR'-, where C(0) may be bonded to a phenyl ring, NR' may be bonded to L, and $R_c$ and $R_d$ may be hydrogen.

In an aspect of the present invention, the branching unit may be selected from the group consisting of $C_{2-8}$ alkenyl, a hydrophilic amino acid, -C(O)-, -C(O)NR$^v$-, - C(O)O-, $-(CH_2)_{n1}$-NHC(=O)-$(CH_2)_{n2}$-, $-(CH_2)_{n3}$-C(O)NH-$(CH_2)_{n4}$-, $-(CH_2)_{n1}$-NHC(O)-$(CH_2)_{n2}$-C(O)-, and $-(CH_2)_{n3}$-H-$(CH_2)_{n4}$-C(O)-,

wherein, when the branching unit is $C_{2-8}$ alkenyl, a carbon atom of the alkenyl may be substituted with one or more heteroatoms selected from the group consisting of N, O and S, and the alkenyl may be further substituted with one or more $C_{1-20}$ alkyl,
where $R^v$ may be H, $C_{1-8}$ alkyl, $C_{3-8}$ cycloalkyl, $C_{1-8}$ alkoxy, $C_{1-8}$ alkylthio, mono- or di- $C_{1-8}$ alkylamino, $C_{3-20}$ heteroaryl or $C_{5-20}$ aryl, and
n1, n2, n3 and n4 may each independently be an integer from 0 to 5.

In an aspect of the present invention, the connection unit may be $-(CH_2)_t(V(CH_2)_u)_v$-, where t may be an integer from 0 to 8, u may be an integer from 0 to 12, v may be an integer from 1 to 10, and V may be a single bond or -0-.

In an aspect of the present invention, the connection unit may be $-(CH_2)_t(V(CH_2)_u)_v$-, where t may be an integer of 2, u may be an integer of 2, v may be an integer of 2, and V may be -0-.

In an aspect of the present invention, the connection unit may include

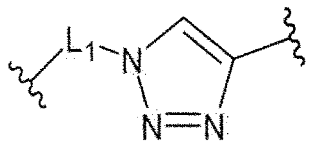

,

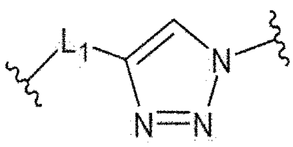

,

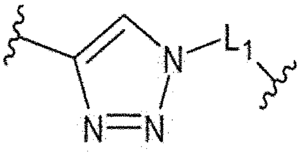

or

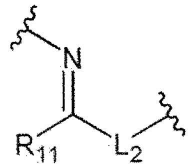

,

where $L_1$ may be a single bond or $C_{2-8}$ alkenyl, $R_{11}$ may be H or $C_{1-6}$ alkyl, and $L_2$ may be $C_{2-8}$ alkenyl.

In an aspect of the present invention, $R_z$ may be $-O-NH_2$, $-NH_2$, $N_3$, $-OCH_3$, $-OCH_2CH_3$, $-O(CH_2)_2CH_3$, or

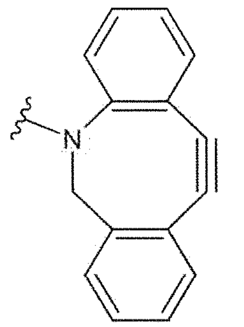

.

In an aspect of the present invention, at least one or more of B or B' may be -C(=O)-.

In an aspect of the present invention, $A_1$ and $A_2$ may each independently be -C(=O)- or -NH-.

In an aspect of the present invention, at least one or more of $Z_1$, $Z_2$, $Z_3$, $Z_1'$, $Z_2'$, or $Z_3'$ may be N.

In an aspect of the present invention, A-1 and A-2 may be selected from

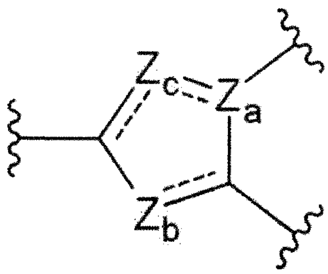

,

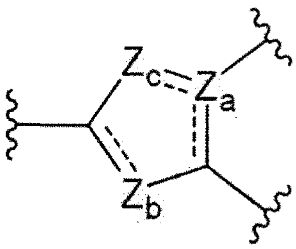

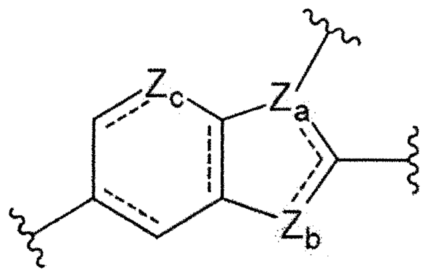

,

, or

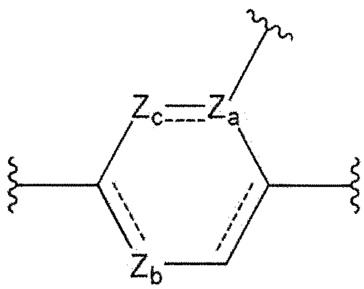

, where a dotted line may mean a double bond or a single bond, $Z_a$ may be $Z_1$ or $Z_1'$, $Z_b$ may be $Z_2$ or $Z_2'$, $Z_c$ may be $Z_3$ or $Z_3'$,

$Z_1$, $Z_2$, $Z_3$, $Z_1'$, $Z_2'$ and $Z_3'$ may be as defined above, and
$R_1$ or $R_1'$ may be connected to $Z_a$.

In a specific aspect of the present invention, A-1 and A-2 may be

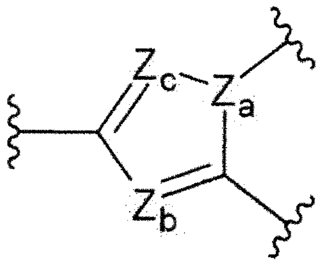

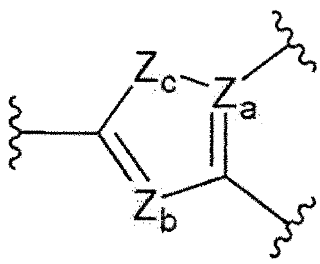

,

or

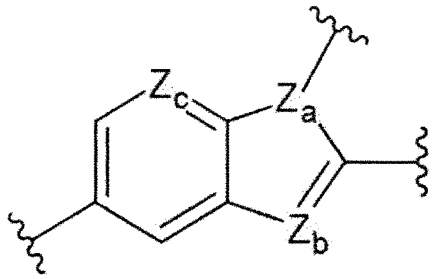

,

where a dotted line may mean a double bond or a single bond, $Z_a$ may be $Z_1$ or $Z_1$', $Z_b$ may be $Z_2$ or $Z_2$', $Z_c$ may be $Z_3$ or $Z_3$',

at least one or more of $Z_1$, $Z_2$, $Z_3$, $Z_1$', $Z_2$' or $Z_3$' may be N or S, and
$R_1$ or $R_1$' may be connected to $Z_a$.

In an aspect of the present invention, $a_1$ and $a_2$ may be an integer of 1.

In an aspect of the present invention, $E_1$ may be a bond, -NH-, or -C(=O)- and $e_1$ may be an integer from 1 to 5.

In an aspect of the present invention, $E_2$ may be a bond, $C_{1-5}$ alkylene, or $-((CH_2)_p(CH_2E_{11}))_q-$,

when $E_2$ is $-((CH_2)_p(CH_2E_{11}))_q-$, $E_{11}$ may be O, p may be an integer from 1 to 3,
q may be an integer from 1 to 15, and $e_2$ may be an integer from 1 to 5.

In an aspect of the present invention, $R_1$ and $R_1$' may each independently be hydrogen, $C_{1-5}$ alkyl, $(CH_2)_mOH$ or $(CH_2)_mNH_2$, where m may be an integer from 1 to 7.

In an aspect of the present invention, $F_1$ may be - OH, $-OCH_3$, -SH, $-SCH_3$,

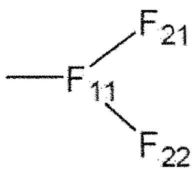

or

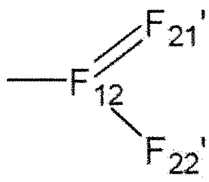

,

where $F_{11}$ and $F_{12}$ may each independently be C or N,
$F_{21}$, $F_{22}$ and $F_{22}$' may each independently be selected from -H, $-(CH_2)_rCH_3$, $-(CH_2)_rOH$, $-(CH_2)_rNH_2$, $-F_{33}(CH_2)_rCH_3$, - $F_{33}(CH_2)_rOH$, $-F_{33}(CH_2)_rNH_2$ or Linker II, where $F_{33}$ may be selected from --C(=O)-, -NH-, or -C(=NH)-,
$F_{21}$' may be selected from $=CH_2$, =NH, $=F_{33}$' $(CH_2)_rCH_3$, =F33'$(CH_2)_rOH$, =F33' $(CH_2)_r$NH2 or Linker II, where $F_{33}$' may be selected from =N-, =CH-, =C(OH)- or $=C(NH_2)$-, and
at least one or more of $F_{21}$, $F_{22}$ or $F_{22}$' may be $-CH_3$.

In an aspect of the present invention, the pyrrolobenzodiazepine derivative may further comprises the following General Formula V:

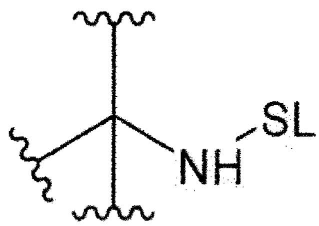

(V)

where,

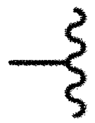

moieties are each independently bonded to a first linker or hydrogen, wherein the first linker is Linker I or Linker II; and SL means a second linker, wherein the second linker is represented by the following Formula VI,

$L'-R_{zz}'$ (VI)

where L' and $R_{zz}'$ are the same as L and $R_z$ in Chemical Formula (IV), respectively.

In a specific aspect of the present invention, the first linker may contain at least one or more branching units, wherein the branching unit may be selected from the group consisting of -C(O)-, -C(O)NR$^v$-, -C(O)O-, -$(CH_2)_{n1}$-NHC(O)-$(CH_2)_{n2}$-, -$(CH_2)_{n3}$-H-$(CH_2)_{n4}$-, -$(CH_2)_{n1}$-NHC(O)-$(CH_2)_{N2}$-C(O)-, -$(CH_2)_{n3}$-H-$(CH_2)_{n4}$-$S(O)_2$NR$^v$-, -P(O)R$^w$NR$^v$-, -S(O)NR$^v$-, and -$PO_2$NR$^v$-,

$R^v$ and $R^w$ may each independently be H, $C_{1-8}$ alkyl, $C_{3-8}$ cycloalkyl, $C_{1-8}$ alkoxy, $C_{1-8}$ alkylthio, mono- or di- $C_{1-8}$ alkylamino, $C_{3-20}$ heteroaryl or $C_{5-20}$ aryl,
n1, n2, n3 and n4 may each independently be an integer from 0 to 5.

In an aspect of the present invention, L' may include one or more branching units and connection units,

wherein the branching unit may be selected from the group consisting of $C_{2-8}$ alkenyl, a hydrophilic amino acid, -C(O)-, and -C(O)O-, and
the connection unit may be - $(CH_2)_t(V(CH_2)_u)_v$-,
where t may be an integer from 0 to 5, u may be an integer of 2, v may be an integer from 1 to 10, and V may be -0-; and $R_{zz}'$ may be -O-$NH_2$, -$NH_2$, $N_3$, -$OCH_3$, or -$OCH_2CH_3$.

In a specific aspect of the present invention, the pyrrolobenzodiazepine derivative represented by Chemical Formula I may be selected from the group consisting of

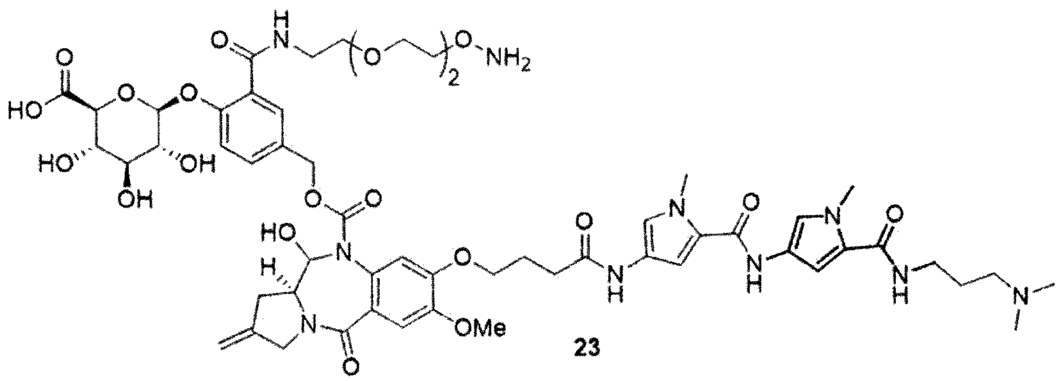

23

,

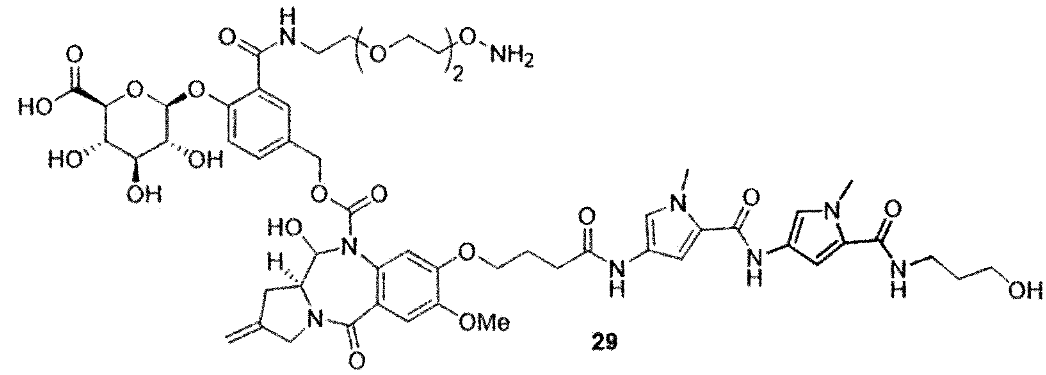

29

,

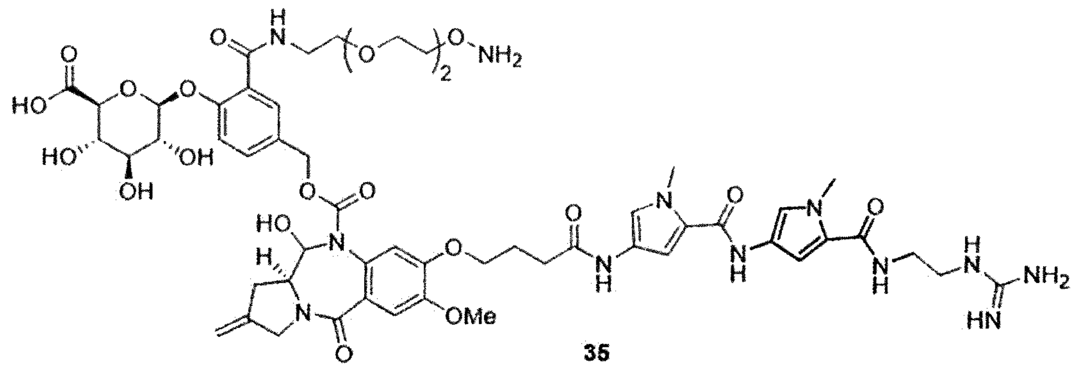

35

,

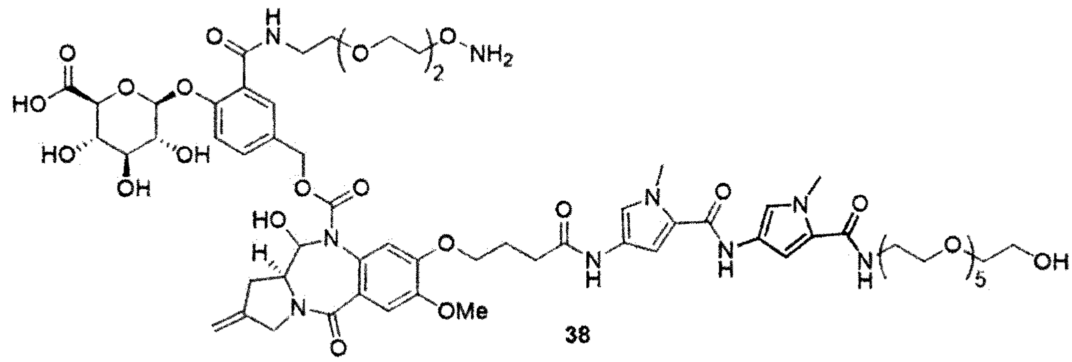

38

,

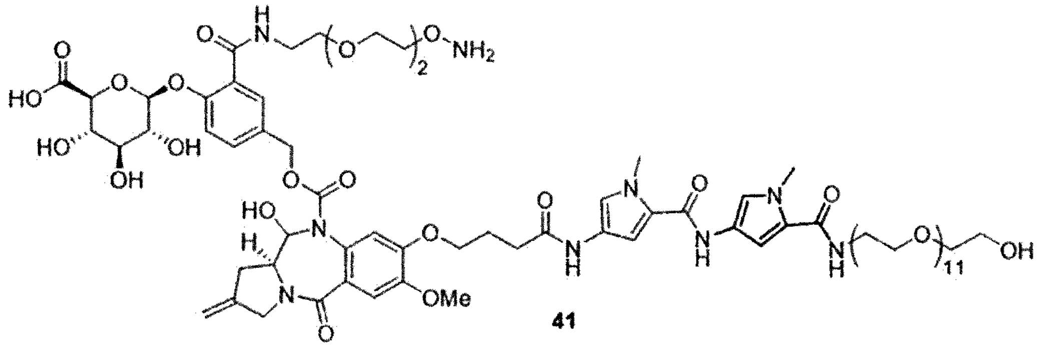

41

,

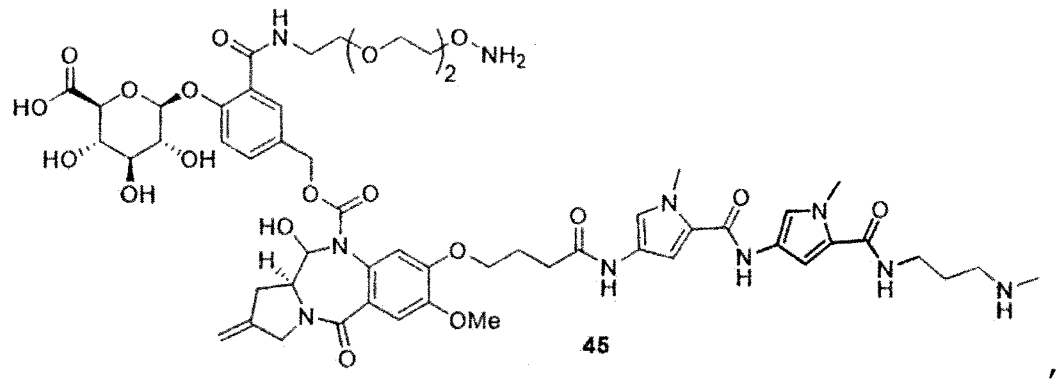

45

,

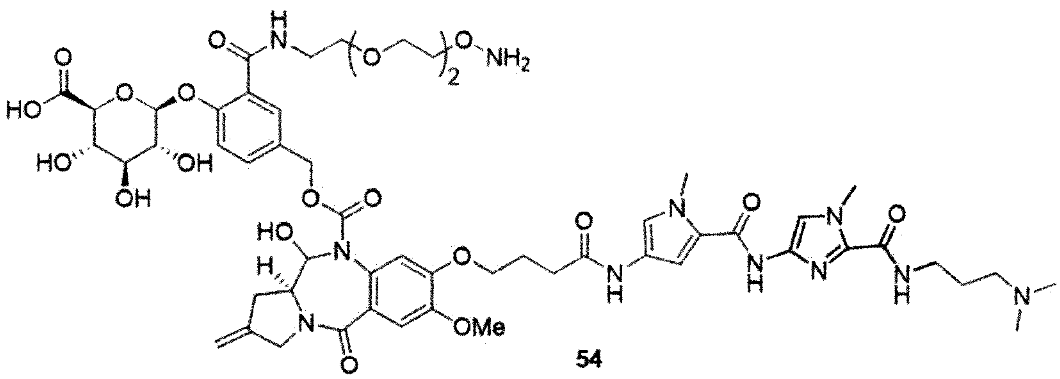

54

,

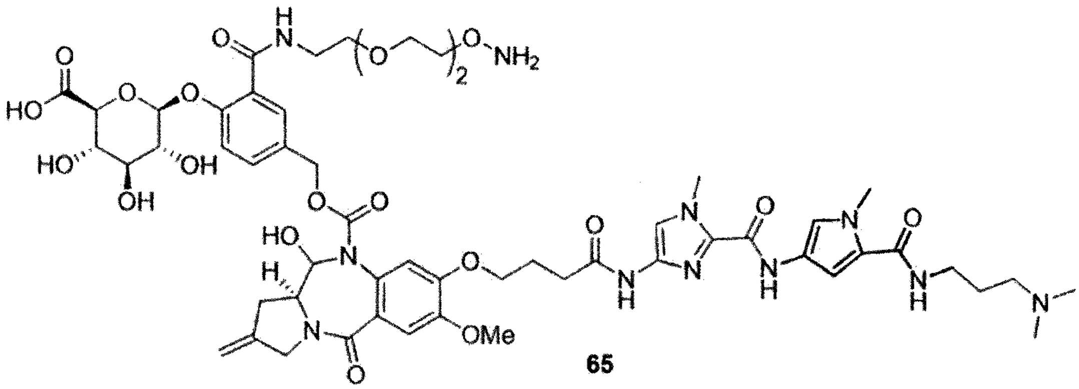

65

,

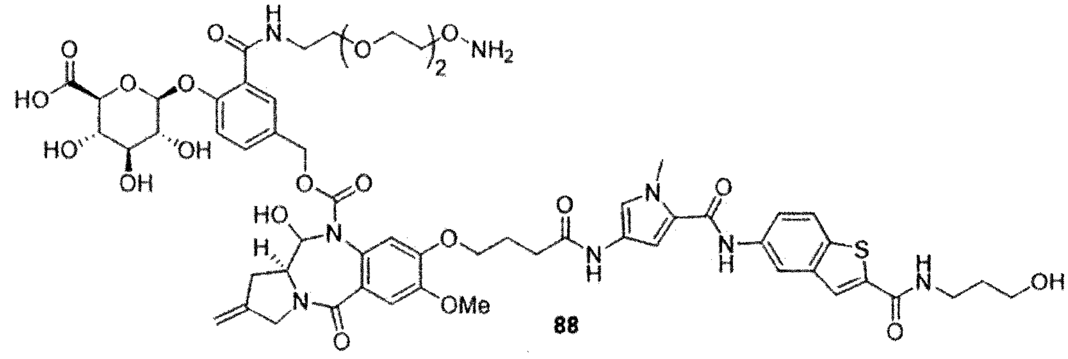

88

,

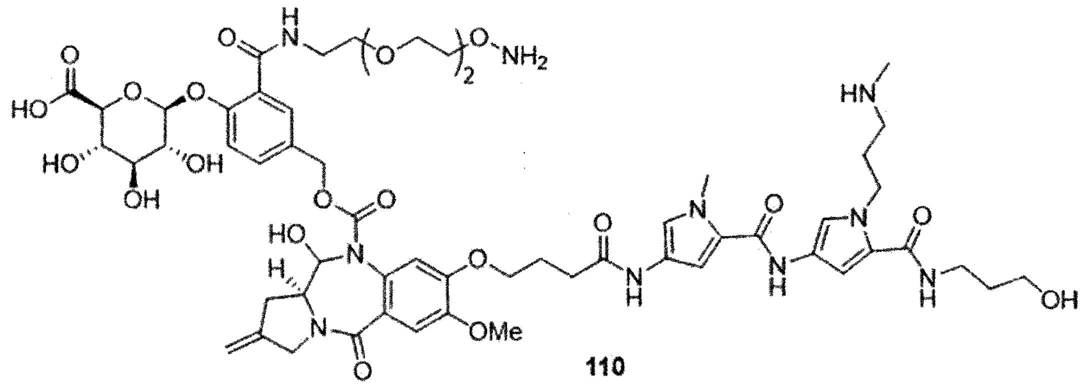

110

,

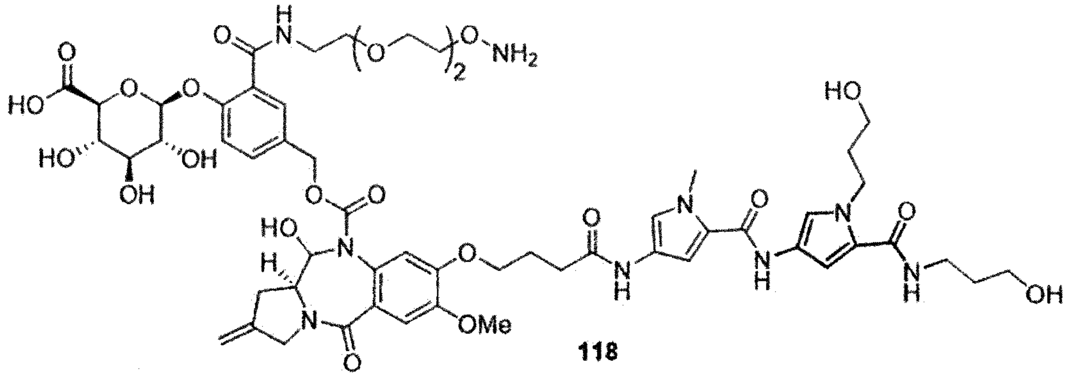

118

,

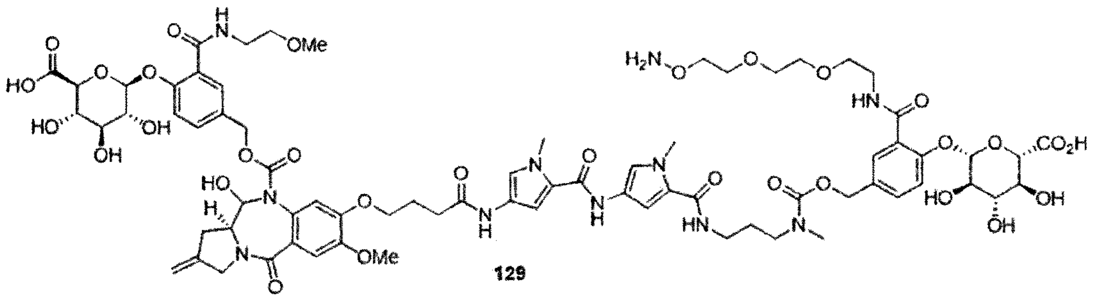

129

,

and

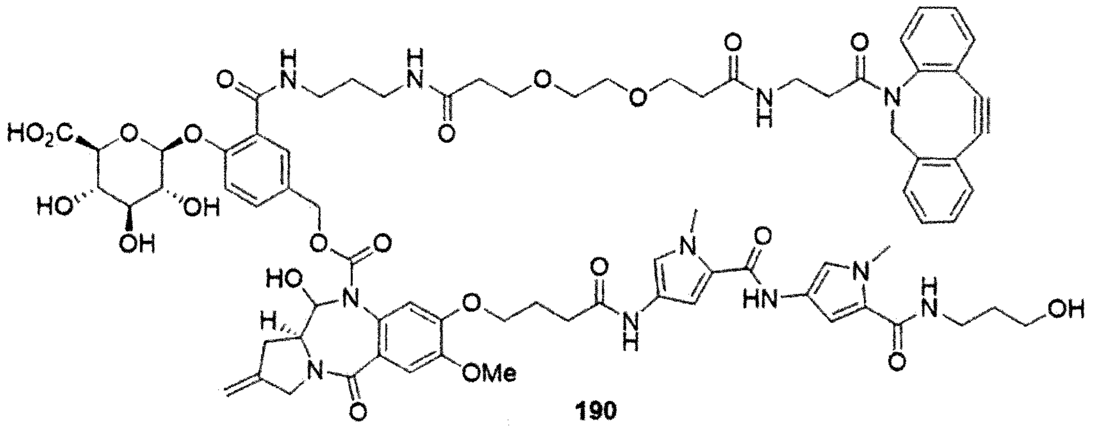

190

.

In an aspect of the present invention, the pyrrolobenzodiazepine derivative represented by Chemical Formula I may be selected from the group consisting of

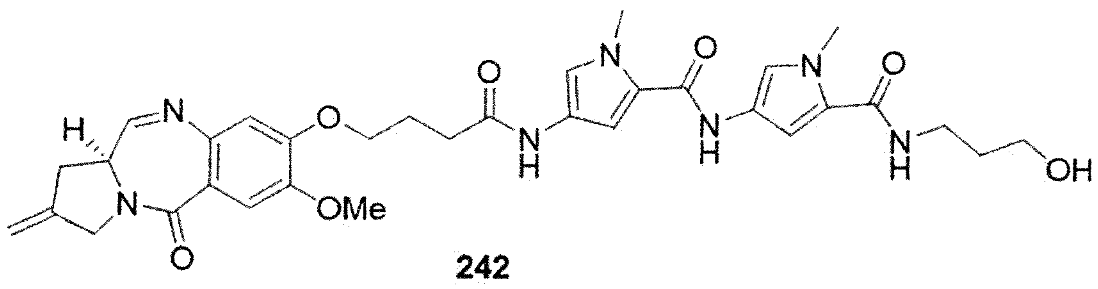

242

,

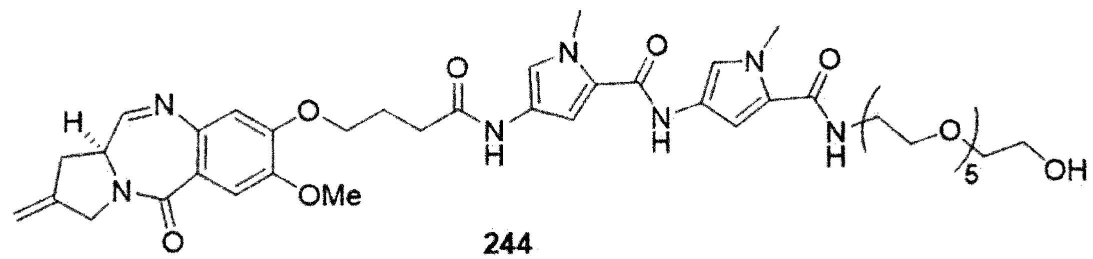

244

,

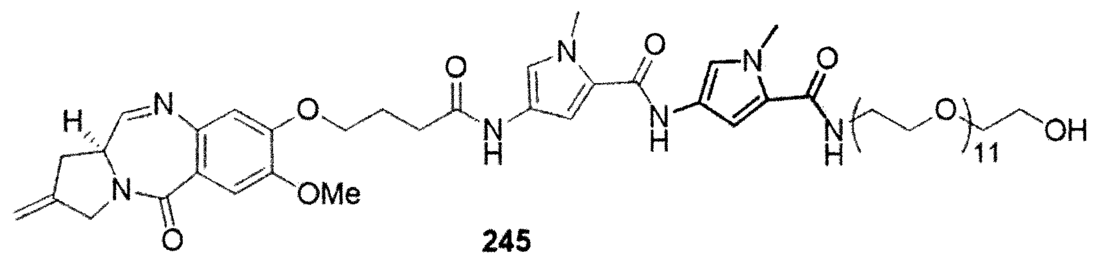

245

, and

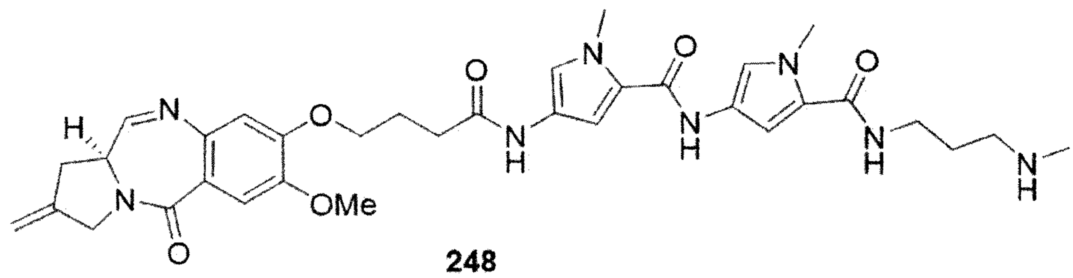

248

.

In an aspect of the present invention, the pyrrolobenzodiazepine derivative further comprising a linker including a tris structure may be

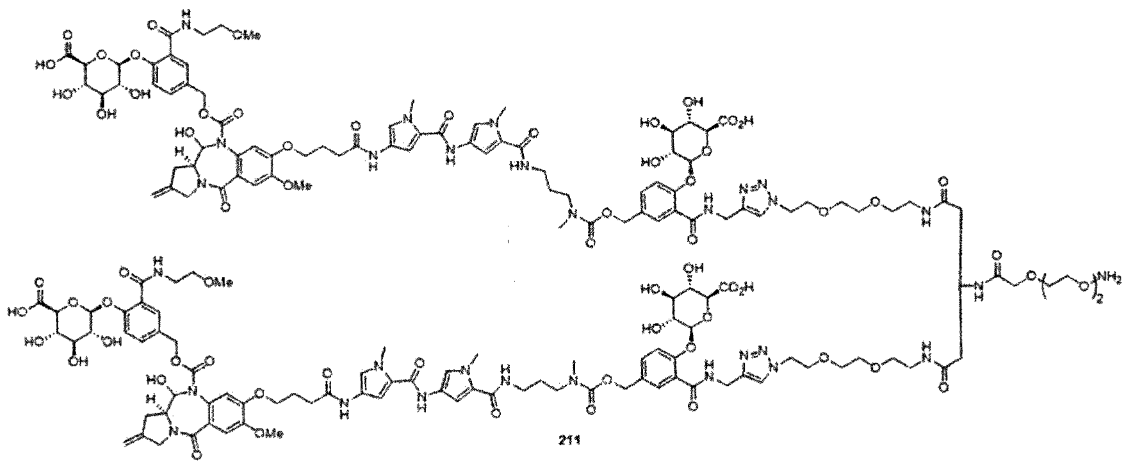

211

In the present invention, the pyrrolobenzodiazepine derivative is represented by the following Chemical Formula VII, and a pharmaceutically acceptable salt or solvate thereof is provided:

P'-B'-D' (VII)

where,

P' is the same as P described above;
B' is $B_a$'-$B_b$'-$B_c$',
where $B_a$' is -O-, -NH-, -S-, -C(=O)-, -S(=O)-, - $(CH_2)_{n'}(C{=}O)$-, $-(CH_2)_{n'}-$, $C_{6-10}$ arylene, 5 to 20-membered heteroarylene containing a heteroatom independently selected from N, O or S, or $-((CH_2)_{n'}B_1)_{m'}B_2-$, where $B_1$ and $B_2$ are each $CH_2$, NH, O, or S,
$B_b$' is a bond, $C_{5-20}$ arylene, $C_{5-20}$ cycloalkylene, or 5- to 20-membered heterocycloalkylene, 5- to 20-membered heterocycloalkenylene, or 5- to 20-membered heteroarylene, in which one or more ring atoms are heteroatoms independently selected from N, 0 or S, and
$B_c$' is a bond, -O-, -NH-, -S-, -C(=O)-, -S(=O)-, - $CH_2(C{=}O)$-, $-(CH_2)_{n'}-$ or $-(CH_2CH_2O)_{n'}-$,
where n' and m' are each independently an integer from 1 to 10, and
D' is the same as D described above.

In an aspect of the present invention, $B_a$' may be a bond, $-(CH_2)_{n'}-$ or $-(CH_2)_{n'}B_2-$, where $B_2$ is 0 and n' may be an integer from 1 to 10.

In an aspect of the present invention, $B_b$' may be a bond,

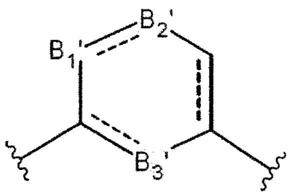

,

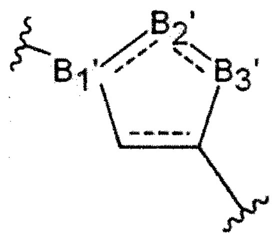

,

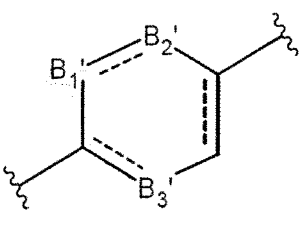

, or

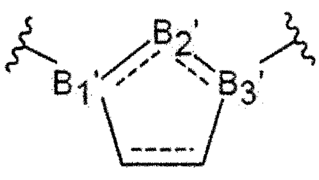

,

where the dotted line may mean a double bond or a single bond, and
$B_1$', $B_2$' and $B_3$' may each independently be C, N, or S.

In an aspect of the present invention, $B_c$' may be a bond, -C(=O)-, -NH-, or $-(CH_2CH_2O)_n-$, where n may be an integer from 1 to 10.

In an aspect of the present invention, D' may be the same as D, and $a_1 + a_2$ may be an integer from 1 to 10 except 2.

In a specific aspect of the present invention,

the pyrrolobenzodiazepine derivative represented by Chemical Formula VII may be selected from the group consisting of

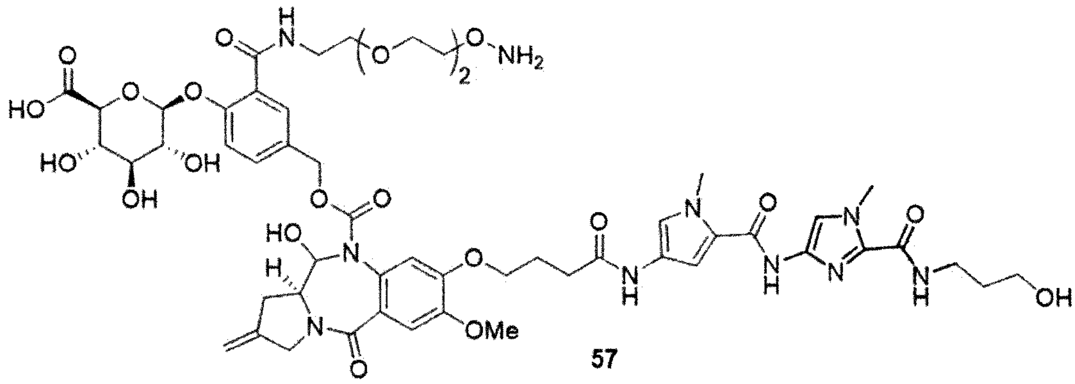

57

,

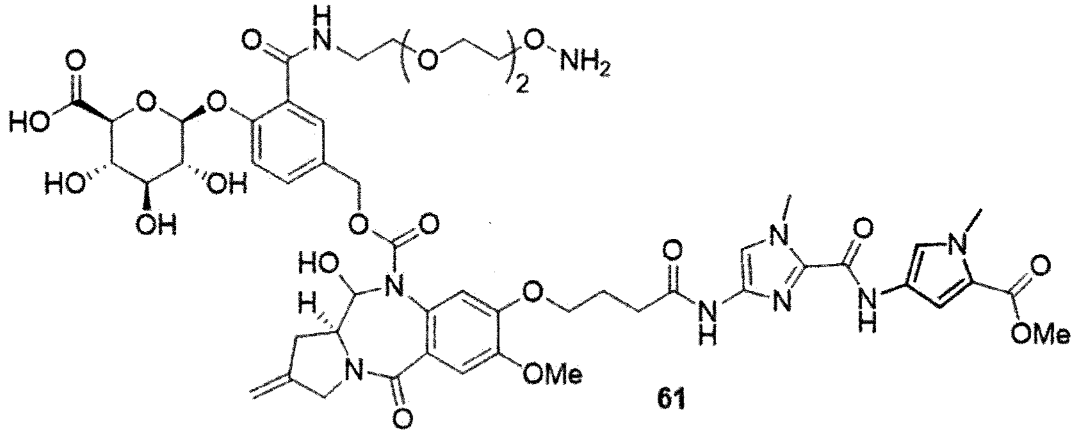

61

,

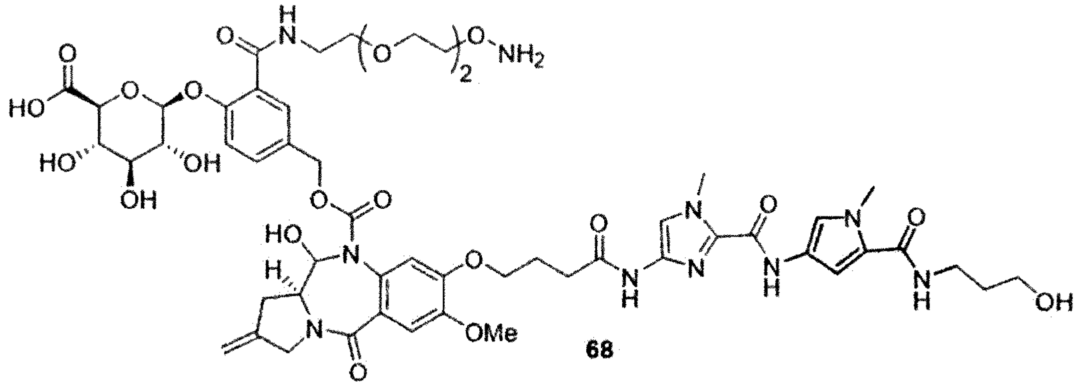

68

,

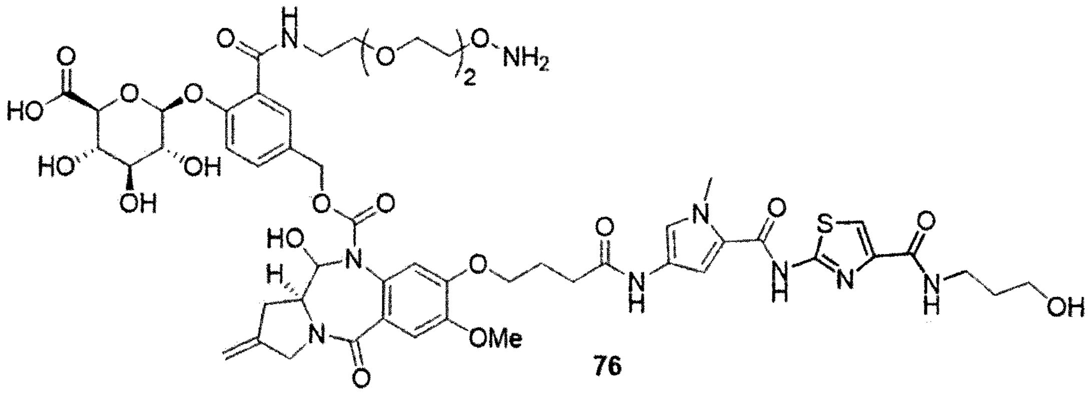

76

,

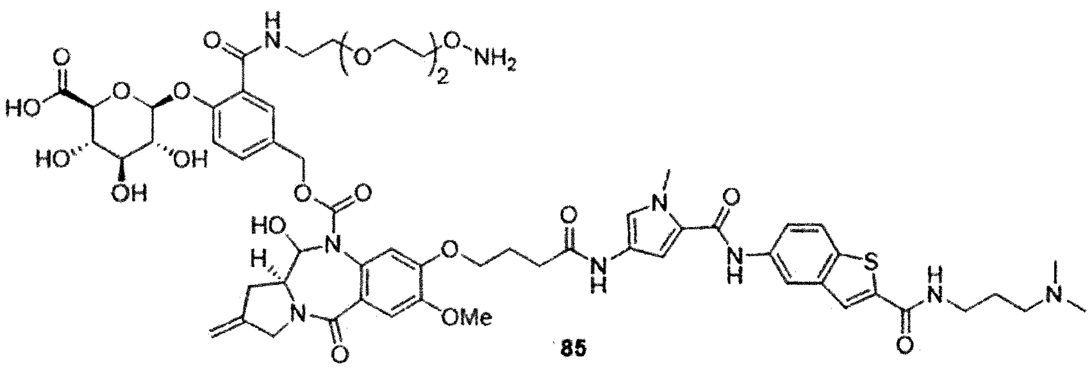

85

,

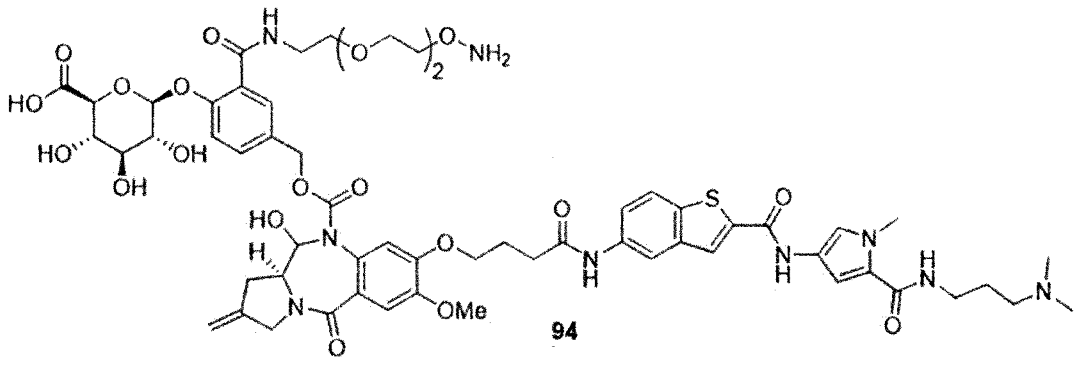

94

,

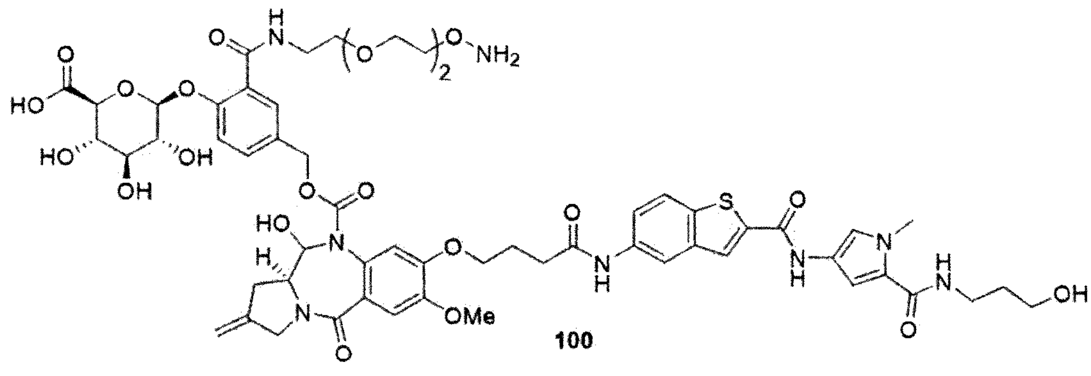

100

,

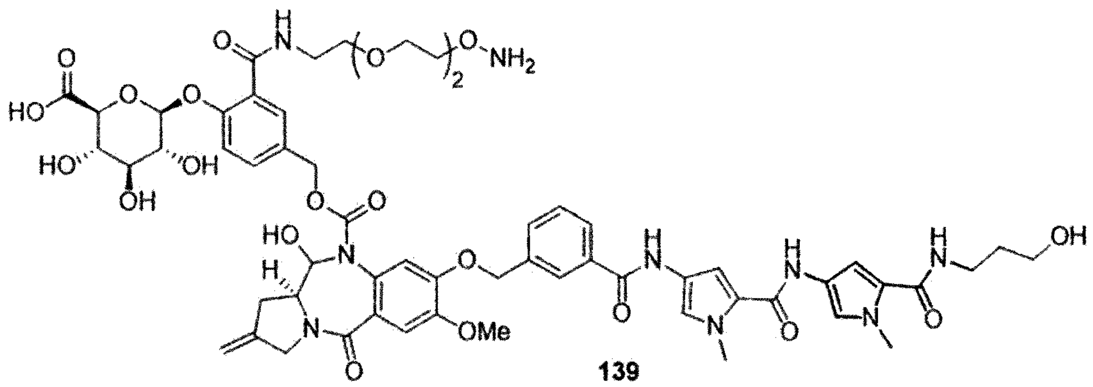

139

,

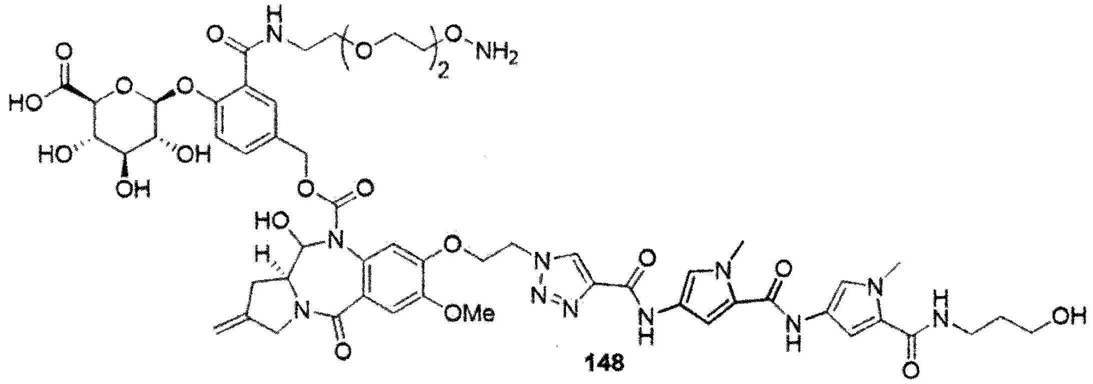

148

,

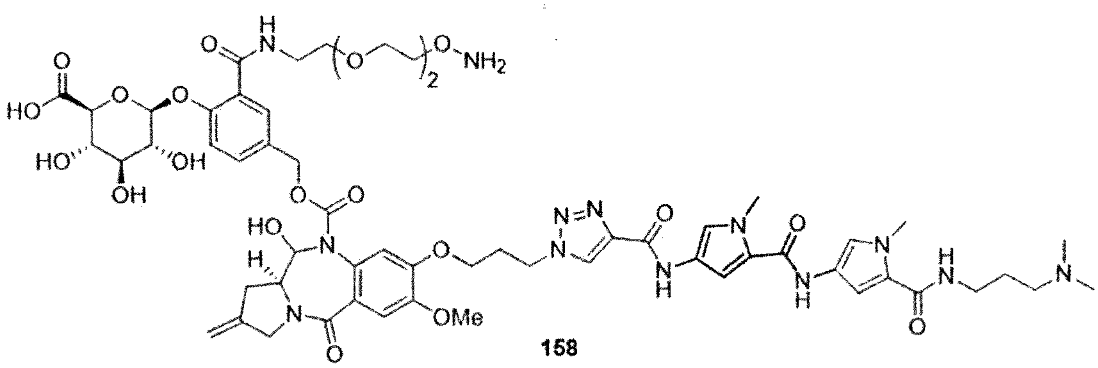

158

,

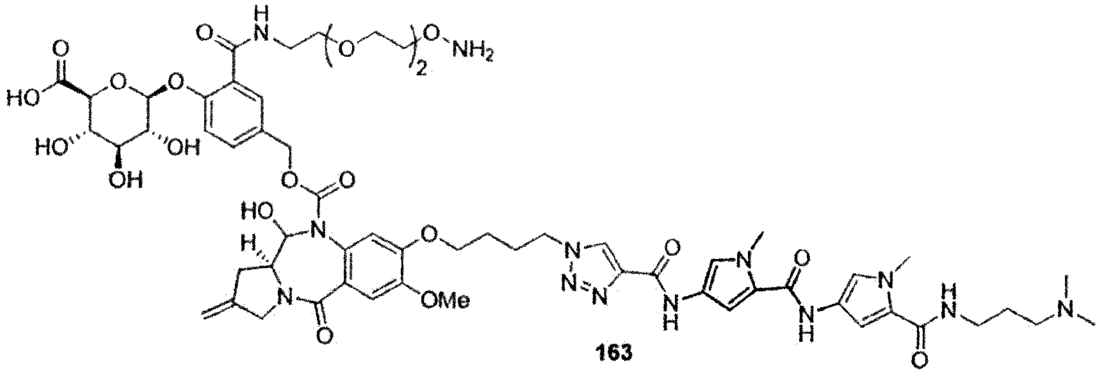

163

,

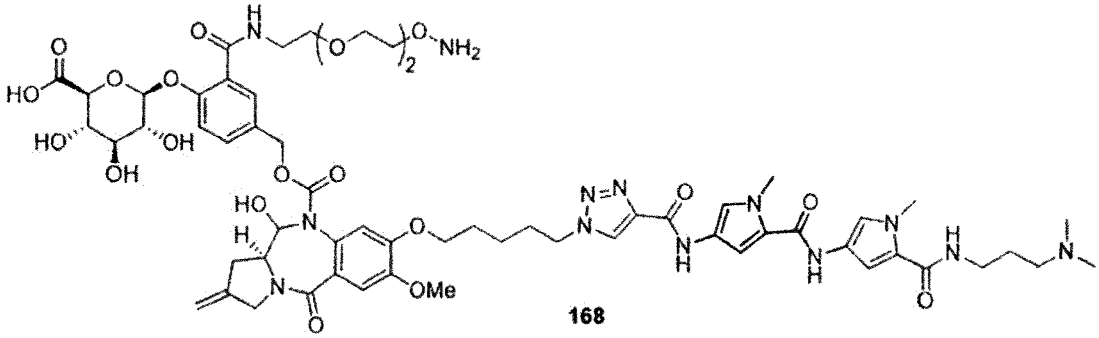

168

,

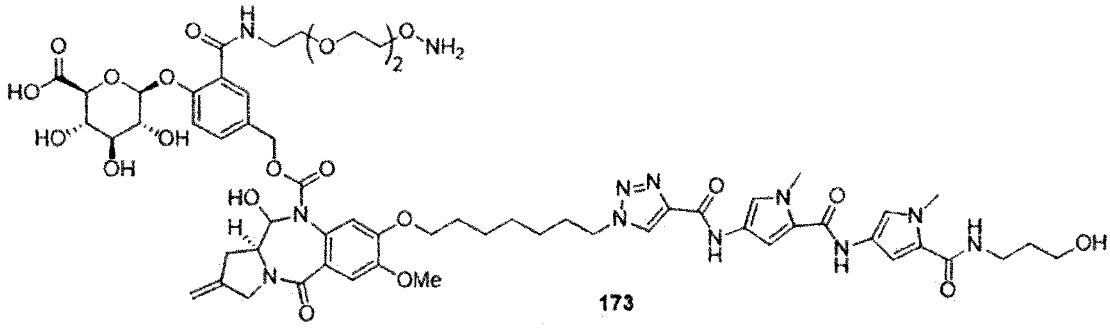

173

,

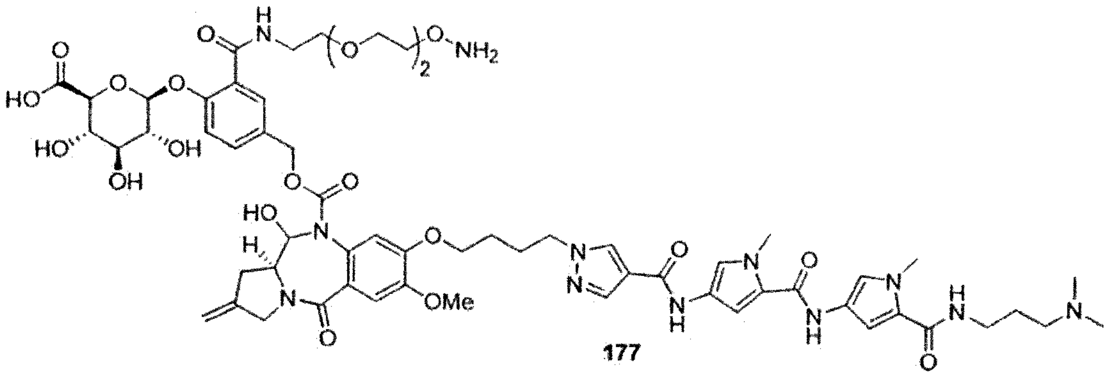

177

,

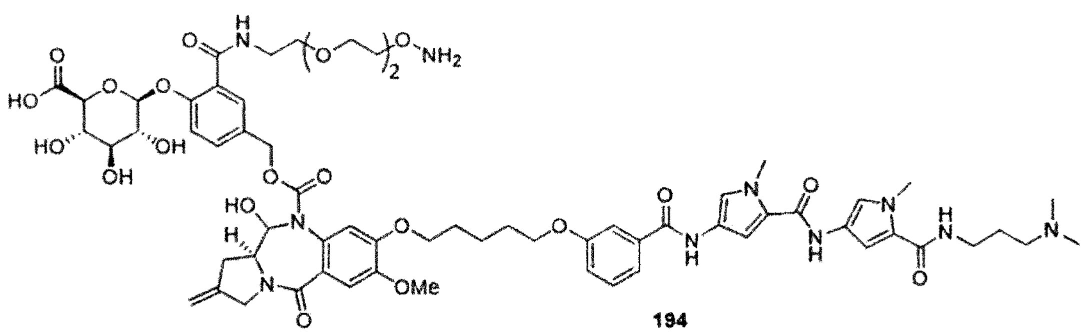

194

,

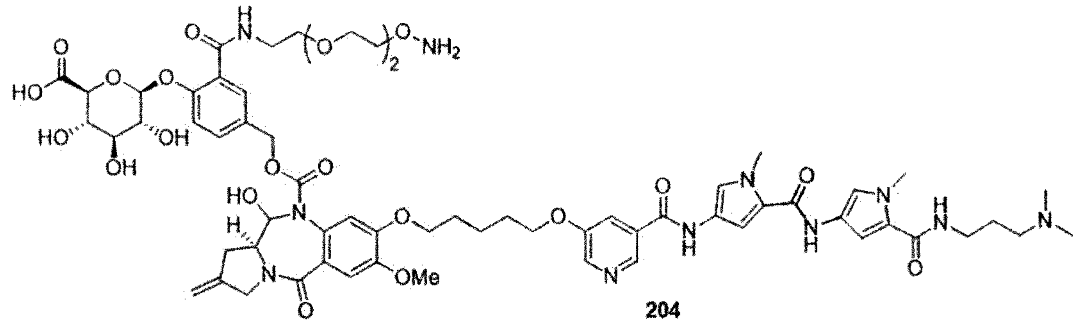

204

,

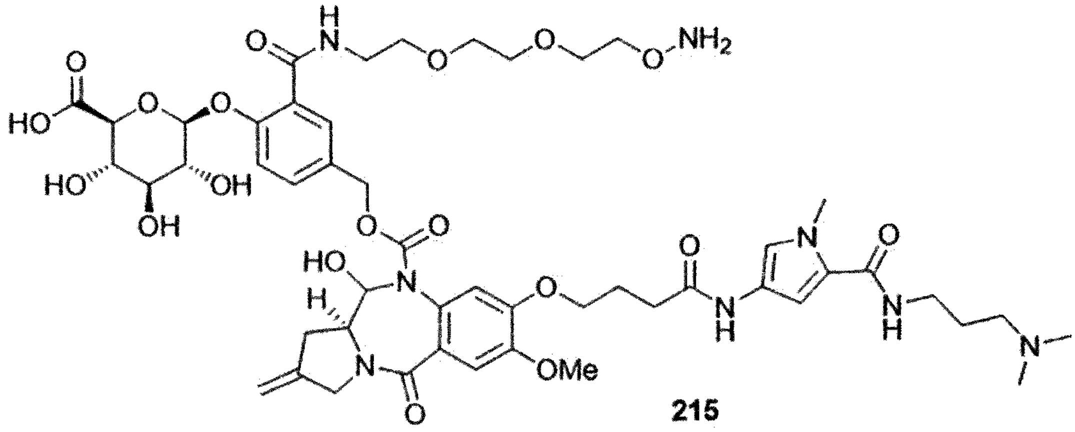

215

,

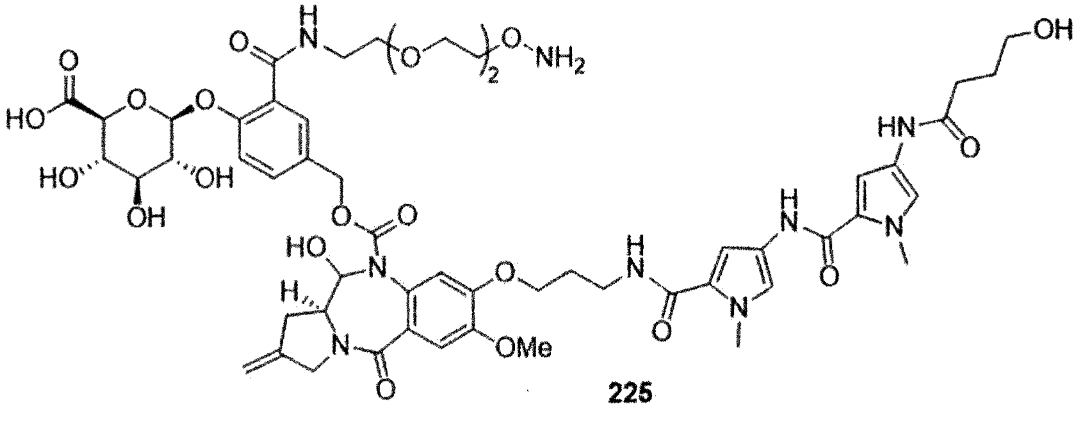

225

,

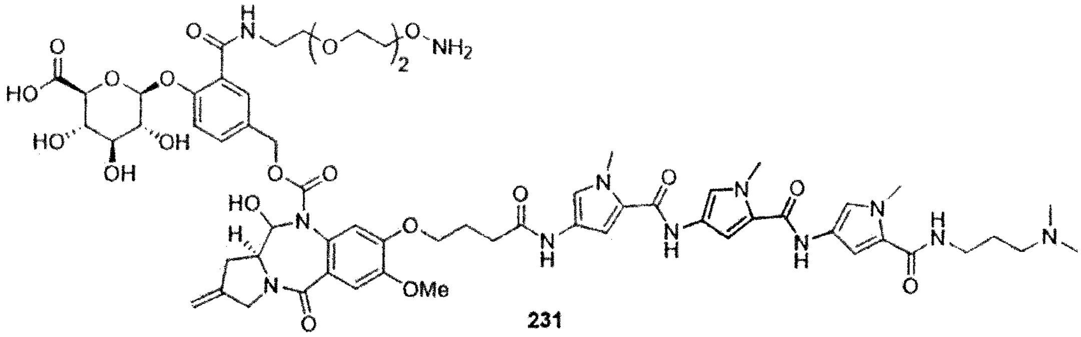

231

,

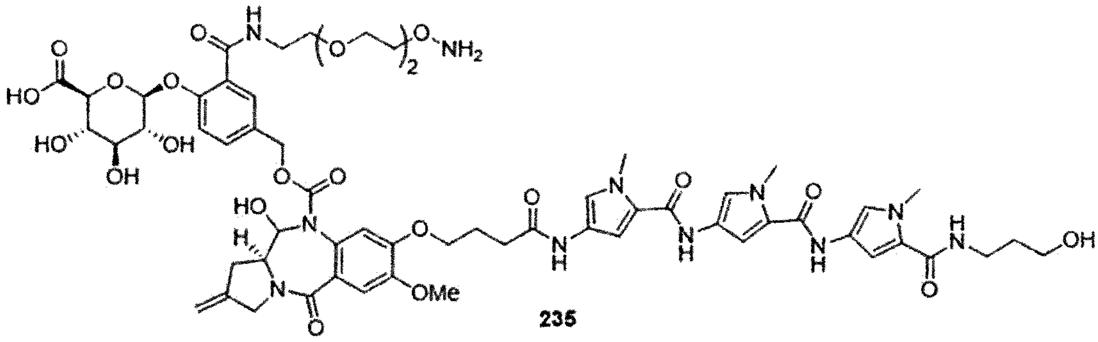

235

,

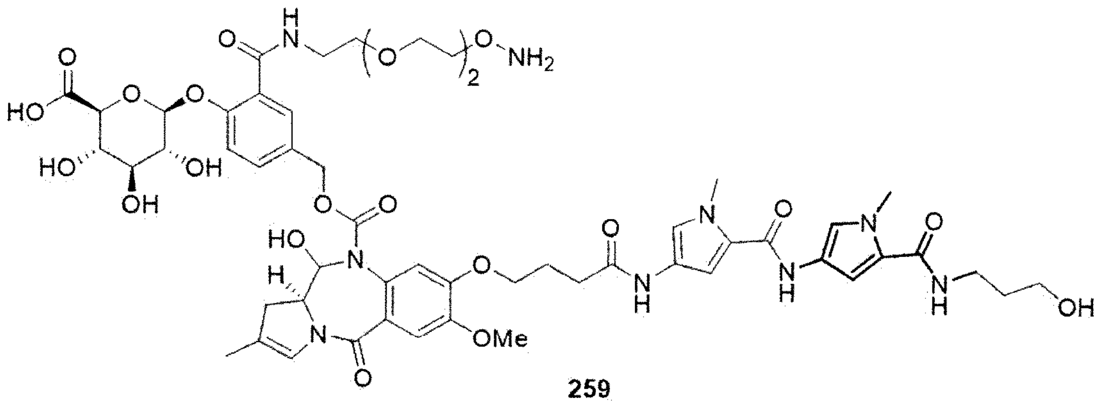

259

, and

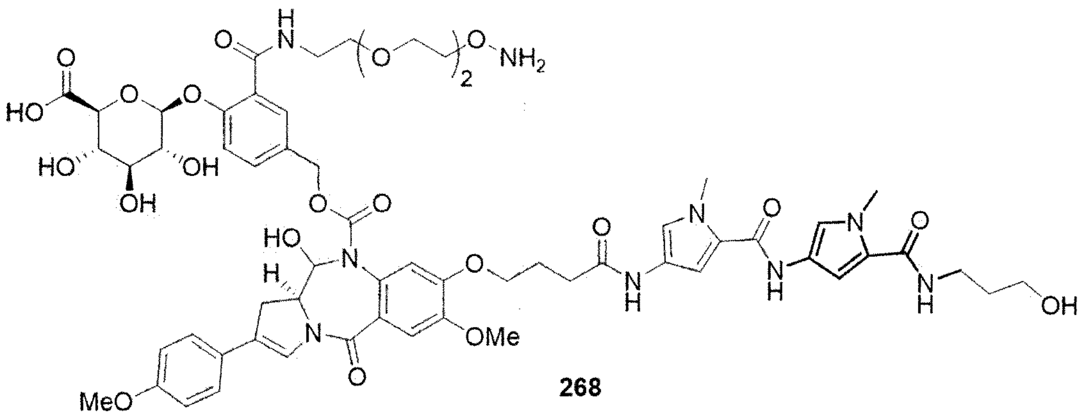

268

.

The present invention also provides a pyrrolobenzodiazepine derivative-ligand conjugate comprising the pyrrolobenzodiazepine derivative described above, or a pharmaceutically acceptable salt or solvate of the derivative-ligand conjugate.

In an aspect of the present invention, the ligand may be an antibody.

In a specific aspect of the present invention, the antibody may have one or more amino acid motifs that may be recognized by an isoprenoid transferase.

In a specific aspect of the present invention, the isoprenoid transferase may be FTase (farnesyl protein transferase) or GGTase (geranylgeranyl transferase).

In a specific aspect of the present invention, the amino acid motif may be CYYX, XXCC, XCXC or CXX, where C may be cysteine, Y may be an aliphatic amino acid, and X may be an amino acid that determines substrate specificity of an isoprenoid transferase.

Specifically, X in each occurrence independently represents glutamine, glutamate, serine, cysteine, methionine, alanine, or leucine; and the thioether bond contains a sulfur atom of cysteine of the amino acid motif.

In a specific aspect of the present invention, the amino acid motif may be CYYX, and Y in each occurrence may independently be alanine, isoleucine, leucine, methionine or valine.

In an aspect of the present invention, the protein may be an antibody, and the antibody may include an amino acid sequence of GGGGGGGCVIM.

In an embodiment of the invention, the protein having the amino acid motif may be selected from the group consisting of A-HC-$(G)_z$CVIM, A-HC-$(G)_z$CVLL, A-LC-(G)zCVIM and A-LC-$(G)_z$CVLL, where A may represent an antibody, HC may represent a heavy chain, LC may represent a light chain, G may represent a glycine unit, and z may be an integer from 0 to 20.

Isoprenoid transferases can recognize substrates as well as isosubstrates. An isosubstrate refers to a substrate analog having modifications to the substrate. Isoprenoid transferases alkylate specific amino acid motifs (for example, CAAX motifs) at the C-terminus of proteins (see: Benjamin P. Duckworth et al, ChemBioChem 2007, 8,98; Uyen T. T. Nguyen et al, ChemBioChem 2007, 8, 408; Guillermo R. Labadie et al, J. Org. Chem. 2007, 72(24), 9291; James W. Wollack et al, ChemBioChem 2009, 10, 2934). Functionalized proteins may be produced using isoprenoid transferases and isosubstrates via alkylation at the C-terminal cysteine(s).

For example, the cysteine residue of the C-terminal CAAX motif may be reacted with an isosubstrate using an isoprenoid transferase. In a certain occurrence, AAX may then be removed by a protease. The obtained cysteine may then be methylated at the carboxy terminus by an enzyme (see: Iran M. Bell, J. Med. Chem. 2004, 47(8), 1869).

Proteins of the present invention may be prepared by any molecule or cell biology technique well known in the art. For example, transient transfection may be used. The genetic sequence encoding a certain amino acid motif recognizable by an isoprenoid transferase may be inserted into a known plasmid vector using standard PCR techniques so as to express a protein (fragment or analog thereof) having the certain amino acid motif at its C-terminus. In this way, a protein having one or more amino acid motifs recognizable by isoprenoid transferases may be expressed.

In an aspect of the present invention, when the protein is a monoclonal antibody, one or more light chains of a monoclonal antibody, one or more heavy chains of a monoclonal antibody, or both may include an amino acid moiety having an amino acid motif recognizable by an isoprenoid transferase, and those skilled in the art can readily select a protein (for example, a target cell of a subject) that selectively binds a target of interest.

In an aspect of the present invention, a fragment of an antibody or antigen that specifically binds to a target of interest may be included.

The present invention also provides a pharmaceutical composition for prevention or treatment of proliferative disease comprising the pyrrolobenzodiazepine derivative-ligand conjugate described above or a pharmaceutically acceptable salt or solvate thereof.

In an aspect of the present invention, the proliferative disease may be one selected from the group consisting of neoplasia, tumor, cancer, leukemia, psoriasis, bone disease, fibroproliferative disorder, and atherosclerosis.

In a specific aspect of the present invention, the cancer may be one selected from the group consisting of lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma.

In the present invention, the pharmaceutical composition may comprise the pyrrolobenzodiazepine derivative-ligand conjugate described above or a pharmaceutically acceptable salt or solvate thereof; and further a pharmaceutically acceptable excipient.

The present invention also provides a pharmaceutical composition for prevention or treatment of proliferative disease comprising the pyrrolobenzodiazepine derivative-ligand conjugate described above or a pharmaceutically acceptable salt or solvate thereof; one or more therapeutic co-agents; and a pharmaceutically acceptable excipient.

The present invention also provides a method for preventing or treating proliferative disease, which comprises administering the pyrrolobenzodiazepine derivative-ligand conjugate described above or a pharmaceutically acceptable salt or solvate thereof to an individual. Herein, the method for preventing or treating proliferative disease may further comprise a pharmaceutically acceptable excipient and/or one or more therapeutic co-agents.

The present invention also provides use of the pyrrolobenzodiazepine derivative-ligand conjugate described above or a pharmaceutically acceptable salt or solvate thereof in a pharmaceutical composition for prevention or treatment of proliferative disease. Herein, the composition may further comprise a pharmaceutically acceptable excipient and/or one or more therapeutic co-agents.

[Advantageous Effects of Invention]

The present invention relates to a pyrrolobenzodiazepine derivative compound, a conjugate of the same with a ligand, and the use of the same. The pyrrolobenzodiazepine derivative compound is more stable in plasma, is stable even in circulation, and exhibits excellent efficacy.

In particular, the conjugate of the pyrrolobenzodiazepine derivative compound with a ligand has a great advantage in that it is possible to target and specifically treat proliferative disease such as cancer, maximize drug efficacy, and minimize the expression of side effects since the conjugate more stably reaches the target cell and effectively exerts drug efficacy while toxicity is greatly reduced as a linker technology that allows drugs to be easily released within cancer cells and maximize drug efficacy is incorporated.

[Brief Description of Drawings]

FIG. 1 is a scheme illustrating a preparation example of a pyrrolobenzodiazepine derivative.
FIG. 2 is a scheme illustrating a synthesis example of ADC (antibody-drug conjugate) through a pyrrolobenzodiazepine derivative.

[Description of Embodiments]

In the present invention, the pyrrolobenzodiazepine derivative is represented by the following Chemical Formula I, and a pharmaceutically acceptable salt or solvate thereof is provided. A pyrrolobenzodiazepine derivative-ligand conjugate comprising the pyrrolobenzodiazepine derivative, or a pharmaceutically acceptable salt or solvate of the derivative-ligand conjugate is also provided.

[Embodiments]

Hereinafter, embodiments of the present invention will be described in detail so that those of ordinary skill in the art to which the present invention pertains can easily carry out the present invention. Embodiments of the present invention are provided in order to more completely explain the present invention to those of ordinary skill in the art. Embodiments of the present invention may be modified in various other forms, and the scope of the present invention is not limited to the embodiments described below.
Unless defined otherwise in the present invention, scientific and technical terms used herein have the meanings commonly understood by those skilled in the art. Furthermore, unless the context specifically requires otherwise, the singular includes the plural and the plural includes the singular.
When it is said that a certain part "includes" a certain element throughout the specification of the present invention, this means that other elements are not excluded but may be further included unless otherwise stated.

**[Definition]**

The following definitions apply herein:
In the present invention, "conjugates" refers to cell binding agents that are covalently bound to one or more molecules of a cytotoxic compound. Here, the "cell binding agent" is a molecule having affinity for a biological target, and may be, for example, a ligand, a protein, an antibody, specifically a monoclonal antibody, a protein or antibody fragment, a peptide, an oligonucleotide, or an oligosaccharide, and the binding agent functions to direct the biologically active compound to the biological target. In an aspect of the present invention, the conjugate may be designed to target tumor cells via cell surface antigens. The antigen may be a cell surface antigen that is overexpressed or expressed in an abnormal cell type. Specifically, the target antigen may be expressed only on proliferative cells (for example, tumor cells). The target antigen may usually be selected based on different expression between proliferative and normal tissues. In the present invention, the ligand is bound to a linker.
In the present invention, an "antibody" is an immunoglobulin molecule capable of specifically binding to a target, for example, a carbohydrate, a polynucleotide, a lipid, or a polypeptide via at least one antigen recognition site located in the variable region of the immunoglobulin molecule.
In the present invention, an "antibody" encompasses intact polyclonal or monoclonal antibodies as well as an arbitrary antigen-binding moiety (for example, "antigen-binding fragment") of an intact antibody that retains the ability to specifically bind to a given antigen or single chain thereof, a fusion protein containing the antibody, and another arbitrary modified arrangement of an immunoglobulin molecule containing an antigen recognition site, for example, but not limited to, Fab; Fab'; F(ab')2 Fd fragment; Fv fragment; a single domain antibody (dAb) fragments; an isolated complementarity determining region (CDR); single chain (scFv) and single domain antibodies (for example, shark and camelid antibodies), maxibodies, minibodies, intrabodies, diabodies, triabodies, tetrabodies, v-NARs and bis-scFvs (see, for example, Hollinger and Hudson, 2005, Nature Biotechnology 23(9): 1126-1136). The antibody includes antibodies of an arbitrary class, such as IgG, IgA or IgM (or subclasses thereof), and the antibody is not required to be of an arbitrary particular class. Depending on the amino acid sequence of the constant region of the heavy chain of an antibody, immunoglobulins

may be assigned to different classes. There are five main classes of immunoglobulins: IgA, IgD, IgE, IgG and IgM, and some of these may be further divided into subclasses (isotypes), for example, IgG1, IgG2, IgG3, IgG4, IgA1 and IgA2. The heavy chain (HC) constant domains corresponding to the different classes of immunoglobulins are called alpha, delta, epsilon, gamma and mu, respectively. The subunit structures and three-dimensional configurations of different classes of immunoglobulins are well known. The antibody of the present invention may be prepared using techniques well known in the art, for example, recombinant techniques, phage display techniques, synthetic techniques or combinations of these techniques or other techniques readily known in the art.

In the present invention, an "isolated antibody" refers to an antibody that is substantially free of other antibodies with different antigenic specificities, and may be substantially free of other cell substances and/or chemical substances.

In the present invention, a "biological target" refers to an antigen located on the surface of a tumor, cancer cell, or extracellular matrix.

In the present invention, being "unsubstituted or substituted" means a parent group that may be unsubstituted or substituted, being "substituted" means a parent group having one or more substituents, and a substituent refers to a chemical moiety covalently bound to a parent group or fused to a parent group.

In the present invention, "halo" refers to fluorine, chlorine, bromine, iodine, and the like.

In the present invention, "alkyl" refers to a monovalent moiety obtained by removing a hydrogen atom from a carbon atom of an aliphatic or alicyclic saturated or unsaturated (unsaturated, completely unsaturated) hydrocarbon compound. Examples of saturated alkyl include methyl, ethyl, propyl, butyl, pentyl, hexyl, heptyl, and the like, examples of saturated straight chain alkyl include methyl, ethyl, n-propyl, n-butyl, n-pentyl (amyl), n-hexyl, n-heptyl, and the like, and examples of saturated branched chain alkyl include isopropyl, isobutyl, sec-butyl, tert-butyl, isopentyl, neopentyl, and the like.

In the present invention, "alkoxy" means -OR [where R is an alkyl group], and examples thereof include methoxy, ethoxy, n-propoxy, isopropoxy, n-butoxy, secbutoxy, isobutoxy, tert-butoxy and the like.

In the present invention, "aryl" means a monovalent moiety obtained by removing a hydrogen atom from an aromatic ring atom of an aromatic compound having a ring atom.

In the present invention, "alkenyl" is alkyl having one or more carbon-carbon double bonds, and examples of unsaturated alkenyl groups include ethenyl (vinyl, - $CH=CH_2$), 1-propenyl (-$CH=CHCH_3$), 2-propenyl, isopropenyl, butenyl, pentenyl, hexenyl, and the like.

In the present invention, "alkynyl" is an alkyl group having one or more carbon-carbon triple bonds, and examples of unsaturated alkynyl groups include ethynyl, 2-propynyl and the like.

In the present invention, "carboxy" refers to - C(=O)OH.

In the present invention, "formyl" refers to - C(=O)H.

In the present invention, "aryl" relates to a monovalent moiety obtained by removing a hydrogen atom from an aromatic ring atom of an aromatic compound. For example, "$C_{5-7}$ aryl" means a monovalent moiety, which has 5 to 7 ring atoms and is obtained by removing a hydrogen atom from an aromatic ring atom of an aromatic compound, and "$C_{5-10}$ aryl" means a monovalent moiety, which has 5 to 10 ring atoms and is obtained by removing a hydrogen atom from an aromatic ring atom of an aromatic compound. A prefix ($C_{5-7}$, $C_{5-10}$, and the like) herein refers to the number of ring atoms or the range of the number of ring atoms, whether carbon atoms or heteroatoms. For example, "$C_{5-6}$ aryl" relates to an aryl group having 5 or 6 ring atoms. Here, the ring atoms may be all carbon atoms as in a "carboaryl group". Examples of carboaryl groups include, but are not limited to, those derived from benzene, naphthalene, azulene, anthracene, phenanthrene, naphthacene, and pyrene. Examples of an aryl group containing fused rings of which at least one is an aromatic ring include, but are not limited to, groups derived from indane, indene, isoindene, tetralin, acenaphthene, fluorene, phenalene, acephenanthrene and aceanthrene. Alternatively, the ring atom may include one or more heteroatoms as in a "heteroaryl group".

In the present invention, "heteroaryl" is aryl containing one or more heteroatoms, and examples thereof include pyridine, pyrimidine, benzothiophene, furyl, dioxalanyl, pyrrolyl, oxazolyl, pyridyl, pyridazinyl, pyrimidinyl, and the like, more specifically, $C_9$ with two fused rings derived from benzofuran, isobenzofuran, indole, isoindole, indolizine, indoline, isoindoline, purine (adenine or guanine), benzimidazole, indazole, benzoxazole, benzisoxazole, benzodioxole, benzofuran, benzotriazole, benzothiofuran, benzothiazole, and benzothiadiazole; $C_{10}$ with two fused rings derived from chromene, isochromene, chromane, isochromane, benzodioxane, quinoline, isoquinoline, quinolizine, benzoxazine, benzodiazine, pyridopyridine, quinoxaline, quinazoline, cinnoline, phthalazine, naphthyridine, and pteridine; $C_{11}$ with two fused rings derived from benzodiazepine; $C_{13}$ with three fused rings derived from carbazole, dibenzofuran, dibenzothiophene, carboline, perimidine, and pyridoindole; and $C_{14}$ with three fused rings derived from acridine, xanthene, thioxanthene, oxantrene, phenoxathiin, phenazine, phenoxazine, phenothiazine, thianthrene, phenanthridine, phenanthroline, and phenazine.

In the present invention, "cycloalkyl" is an alkyl group that is a cyclyl group, and relates to a monovalent moiety obtained by removing a hydrogen atom from an alicyclic ring atom of a cyclic hydrocarbon compound. Examples of cycloalkyl groups include, but are not limited to, those derived from:

saturated monocyclic hydrocarbon compounds: cyclopropane, cyclobutane, cyclopentane, cyclohexane, cycloheptane, methylcyclopropane, dimethylcyclopropane, methylcyclobutane, dimethylcyclobutane, methylcyclopentane, dimethylcyclopentane and methylcyclohexane;
unsaturated monocyclic hydrocarbon compounds: cyclopropene, cyclobutene, cyclopentene, cyclohexene, methylcyclopropene, dimethylcyclopropene, methylcyclobutene, dimethylcyclobutene, methylcyclopentene, dimethylcyclopentene and methylcyclohexene; and saturated heterocyclic hydrocarbon compounds: norcarane, norpinane, and norbornane.

In the present invention, "heterocyclyl" relates to a monovalent moiety obtained by removing a hydrogen atom from a ring atom of a heterocyclic compound. In the heterocyclic compound, the bond in the cyclic structure is not limited to a single bond, and includes both a double bond and a triple bond. The heterocyclic compound also includes compounds having two or more fused rings.
In the present invention, a prefix (for example, $C_1$-$_{12}$, $C_{3-8}$, and the like) refers to the number of ring atoms or the range of the number of ring atoms, whether carbon atoms or heteroatoms. For example, the term "$C_{3-6}$ heterocyclyl" as used herein relates to a heterocyclyl group having 3 to 6 ring atoms.
Examples of monocyclic heterocyclyl groups include, but are not limited to, those derived from:

$N_1$: aziridine, azetidine, pyrrolidine, pyrroline, 2H- or 3H-pyrrole, piperidine, dihydropyridine, tetrahydropyridine, and azepine;
$N_2$: imidazolidine, pyrazolidine, imidazoline, pyrazoline, and piperazine;
$O_1$: oxirane, oxetane, oxolane, oxole, oxane, dihydropyran, pyran, and oxepin;
$O_2$: dioxolane, dioxane and dioxepane;
$O_3$: trioxane;
$N_1O_1$: tetrahydrooxazole, dihydrooxazole, tetrahydroisoxazole, dihydroisoxazole, morpholine, tetrahydrooxazine, dihydrooxazine, and oxazine
$S_1$: thiirane, thietane, thiolane, thiane, and thiepan;
$N_1S_1$: thiazoline, thiazolidine, and thiomorpholine;
$N_2O_1$: oxadiazine;
$O_1S_1$: oxathiole and oxathiane; and
$N_1O_1S_1$: oxathiazine.

In the present invention, as the "pharmaceutically acceptable salt", an acid addition salt formed by a pharmaceutically acceptable free acid may be used, and an organic acid or an inorganic acid may be used as the free acid.
The organic acid includes, but is not limited to, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid and aspartic acid. The inorganic acid includes, but is not limited to, hydrochloric acid, hydrobromic acid, sulfuric acid and phosphoric acid.
For example, when the compound is an anion or has a functional group that may be an anion (for example, -COOH may be $-COO^-$), a salt may be formed using an appropriate cation. Examples of appropriate inorganic cations include, but are not limited to, alkali metal ions such as $Na^+$ and $K^+$, alkaline earth metal cations such as $Ca^{2+}$ and $Mg^{2+}$, and other cations such as $Al^{3+}$. Examples of appropriate organic cations include, but are not limited to, ammonium ions (namely, $NH_4^+$) and substituted ammonium ions (for example, $NH_3R^+$, $NH_2R_2^+$, $NHR_3^+$, and $NR_4^+$).
Examples of some appropriate substituted ammonium ions include those derived from: ethylamine, diethylamine, dicyclohexylamine, triethylamine, butylamine, ethylenediamine, ethanolamine, diethanolamine, piperazine, benzylamine, phenylbenzylamine, choline, meglumine and tromethamine, as well as amino acids, for example, lysine and arginine. Examples of a common quaternary ammonium ion include $N(CH_3)^{4+}$.
For example, when the compound is a cation or has a functional group that may be a cation (for example, $-NH_2$ may be $NH_3^+$), a salt may be formed using an appropriate anion. Examples of appropriate inorganic anions include, but are not limited to, those derived from the following inorganic acids: hydrochloric acid, hydrobromic acid, hydroiodic acid, sulfuric acid, sulfurous acid, nitric acid, nitrous acid, phosphoric acid and phosphorous acid.
Examples of appropriate organic anions include, but are not limited to, those derived from the following organic acids: 2-acetoxybenzoic acid, acetic acid, ascorbic acid, aspartic acid, benzoic acid, camphor sulfonic acid, cinnamic acid, citric acid, edetic acid, ethanedisulfonic acid, ethanesulfonic acid, fumaric acid, glucheptonic acid, gluconic acid, glutamic acid, glycolic acid, hydroxymaleic acid, hydroxynaphthalene carboxylic acid, isethionic acid, lactic acid, lactobionic acid, lauric acid, maleic acid, malic acid, methanesulfonic acid, mucous acid, oleic acid, oxalic acid, palmitic acid, pamic acid, pantothenic acid, phenylacetic acid, phenylsulfonic acid, propionic acid, pyruvic acid, salicylic acid, stearic acid, succinic acid, sulfanilic acid, tartaric acid, toluenesulfonic acid and valeric acid. Examples of appropriate organic polymer anions include, but are not limited to, those derived from the following polymer acids: tannic acid and carboxymethyl cellulose.

In the present invention, a "solvate" refers to a molecular complex between the compound according to the present invention and solvent molecules, and examples of the solvate include, but are not limited to, compounds according to the present invention bound to water, isopropanol, ethanol, methanol, dimethyl sulfoxide, ethyl acetate, acetic acid, ethanolamine or a mixed solvent thereof.

It may be convenient or desirable to prepare, purify and/or handle corresponding solvates of the active compounds. The term "solvate" is used herein in its conventional sense to refer to a complex of a solute (for example, active compounds, salts of active compounds) and a solvent. When the solvent is water, the solvate may conveniently be referred to as a hydrate, for example, a monohydrate, dihydrate, or trihydrate.

The pharmaceutical composition of the present invention may comprise a pharmaceutically acceptable excipient. The pharmaceutically acceptable excipient may include macromolecules that are usually slowly metabolized, for example, proteins, polysaccharides, polylactic acid, polyglycolic acid, polymeric amino acids, amino acid copolymers, lipid aggregates, and the like. Such pharmaceutically acceptable excipients may be appropriately selected and used by those skilled in the art.

The composition comprising a pharmaceutically acceptable excipient may be various oral or parenteral formulations. In the case of being formed into a preparation, the composition is prepared using commonly used diluents or excipients such as fillers, extenders, binders, wetting agents, disintegrants, and surfactants.

Solid preparations for oral administration include tablets, pills, powders, granules, capsules, and the like. Such solid preparations are prepared by mixing one or more compounds with at least one or more excipients, for example, starch, calcium carbonate, sucrose or lactose, gelatin, and the like. In addition to simple excipients, lubricants such as magnesium stearate and talc may also be used.

Liquid preparations for oral administration include suspensions, oral liquids, emulsions, syrups, and the like. In addition to water and liquid paraffin, which are commonly used simple diluents, various excipients such as wetting agents, sweetening agents, fragrances, preservatives, and the like may be contained.

Preparations for parenteral administration include sterile aqueous solutions, non-aqueous solutions, suspensions, emulsions, freeze-dried preparations, and suppositories. As non-aqueous solvents and suspension solvents, propylene glycol, polyethylene glycol, vegetable oils such as olive oil, injectable esters such as ethyl oleate, and the like may be used. As the base of suppositories, witepsol, macrogol, tween 61, cacao butter, laurin oil, glycerogelatin, and the like may be used.

The pharmaceutical composition may have any one formulation selected from the group consisting of injections, tablets, pills, powders, granules, capsules, suspensions, oral liquids, emulsions, syrups, sterilized aqueous solutions, non-aqueous solutions, suspensions, emulsions, freeze-dried preparations and suppositories.

For intravenous, dermal or subcutaneous injection, and the like, the active ingredient may be in the form of an acceptable aqueous solution for parenteral administration, which is pyrogen-free and has appropriate pH, isotonicity and stability. Those skilled in the art can prepare appropriate solutions using isotonic vehicles, for example, aqueous sodium chloride solution, Ringer's solution, and lactate Ringer's solution, and the solutions may be contained as preservatives, stabilizers, buffers, antioxidants or other additives if necessary. Solid forms suitable for injection may also be prepared as emulsions or in the form of polypeptides encapsulated in liposomes.

The phrase "effective amount" or "therapeutically effective amount" as used herein refers to an amount necessary (with respect to the dosage and duration and means of administration) to achieve a desired therapeutic result. An effective amount is at least the minimum amount of an active agent necessary to confer a therapeutic benefit to a subject and is less than a toxic amount. For example, the dosage ranges from about 100 ng/kg to about 100 mg/kg per patient, and may more typically be in the range of about 1 μg/kg to about 10 mg/kg. When the active compound is a salt, ester, amide, prodrug or the like, the dosage is calculated on the basis of the parent compound, so the actual weight used increases proportionally. The pyrrolobenzodiazepine derivative compound according to the present invention may be formulated to contain 0.1 mg to 3000 mg, 1 mg to 2000 mg, or 10 mg to 1000 mg of active ingredient per dosage form, but is not limited thereto. The active ingredient may be administered to obtain a peak plasma concentration of the active compound of about 0.05 μM to 100 μM, 1 μM to 50 μM, or 5 μM to 30 μM. For example, the active ingredient may arbitrarily be administered by intravenous injection of a 0.1 w/v% to 5 w/v% solution in saline.

The concentration of the active compound in the pharmaceutical composition may be determined by the absorption, inactivation and excretion rates of the drug and other factors known to those skilled in the art. The dosage may vary depending on the severity of the symptoms/disease. The dosage and administration method for a certain patient may be adjusted according to the professional judgment of the administration supervisor, taking into account the patient's severity of symptoms/diseases, needs, age, responsiveness to drugs, and the like, and the concentration ranges presented herein are exemplary only and are not intended to limit the embodiments of the claimed composition thereto. The active ingredient may be administered in a single dose, or may be administered in smaller dosages divided into several doses.

In the present invention, "proliferative disease" refers to unwanted or uncontrolled proliferation of undesirable

excessive or abnormal cells such as neoplastic or hyperplastic growth of cells in vitro or in vivo. The proliferative disease includes, for example, neoplasms, tumors, cancer, leukemia, psoriasis, bone diseases, fibroproliferative disorders, atherosclerosis, and the like, and may include benign, premalignant or malignant cell proliferation, but is not limited thereto. The cancer may be lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, or melanoma, but is not limited thereto.

In the present invention, a "therapeutic agent" is an agent that exerts cytotoxic, cytostatic and/or immunomodulatory effects on proliferative disease, for example, cancer cells or activated immune cells. Examples of therapeutic agents include cytotoxic agents, chemotherapeutic agents, cytostatic agents and immunomodulators.

The pyrrolobenzodiazepine derivative, its precursor, and pyrrolobenzodiazepine derivative-ligand conjugate according to the present invention may be synthesized according to the following procedure.

However, the following Examples and Experimental Examples are merely illustrative of the present invention, and the contents of the present invention are not limited to the following Examples and Experimental Examples.

[Examples]

**<Example 1> Preparation of Compound 7**

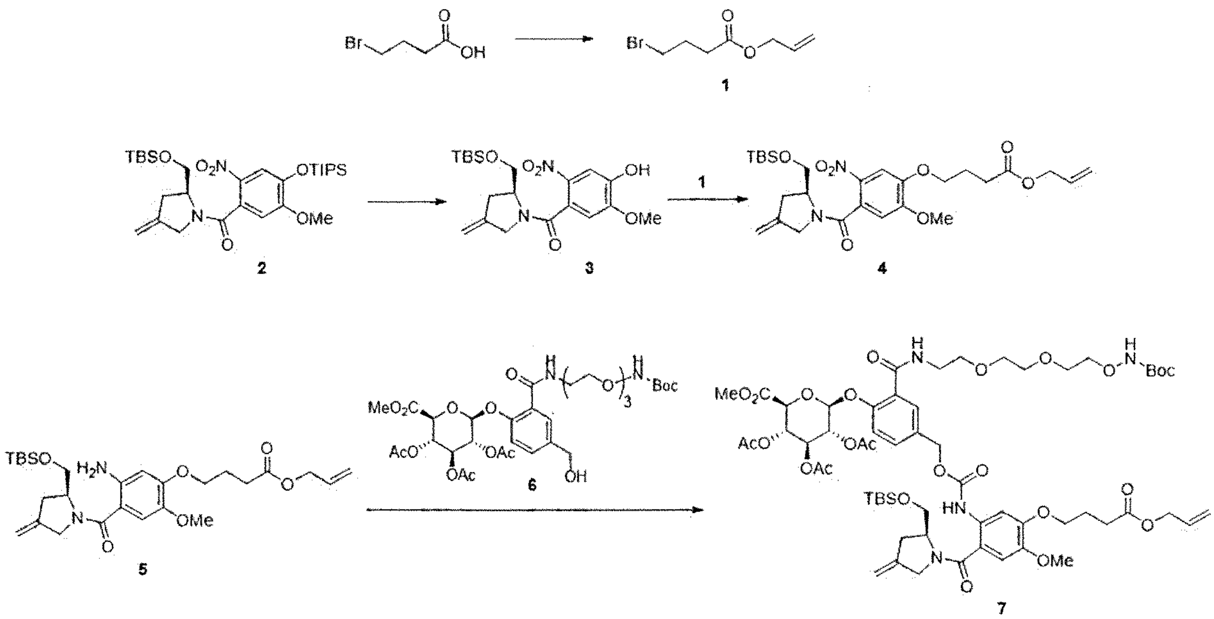


**Preparation of Compound 1**

After 4-bromobutyric acid (10.0 g, 59.9 mmol) and allyl alcohol (40.7 mL, 598.7 mmol) were dissolved in toluene (240 mL), then p-toluenesulfonic acid (1.1 g, 6.0 mmol) was added, and the mixture was stirred for 17 hours under a nitrogen atmosphere while being heated under reflux. The reaction mixture was diluted with ethyl acetate (300 mL) and washed with 0.1 N aqueous sodium hydroxide solution (200 mL). The washed organic layer was washed with brine (200 mL) and dried over anhydrous sodium sulfate. After filtration and concentration under reduced pressure, the product was purified by column chromatography, thereby obtaining Compound 1 (11.5 g, 92%). $^1$H-NMR(400 MHz, $CDCl_3$) $\delta$ 5.93 (m, 1H), 5.30 (d, 1H), 5.26 (d, 1H), 4.59 (d, 2H), 3.47 (t, 2H), 2.54 (t, 2H), 2.19 (q, 2H).

**Preparation of Compound 3**

Compound 2 (36 g, 62.1 mmol, Compound 2 was prepared by the method described in Korean Patent Publication No. 10-2020-0084802) was dissolved in *N,N*-dimethylformamide/distilled water (300 mL/6 mL), then sodium acetate (6.1 g, 74.6 mmol) was added, and the reaction solution was stirred at room temperature under a nitrogen atmosphere. After 3 hours, distilled water (500 mL) was added, followed by extraction with ethyl acetate (2 × 200 mL). The collected organic layers were washed with distilled water (200 mL) and brine (200 mL) in that order, and then dried over anhydrous

sodium sulfate. Filtration, concentration, and purification by column chromatography were performed, thereby obtaining Compound 3 (26 g, 99%). $^1$H-NMR(400 MHz, $CDCl_3$) δ7.77-7.76 (m, 1H), 6.81-6.76 (m, 1H), 6.05-6.01 (m, 1H), 5.10-4.83 (m, 2H), 4.58-4.54 (m, 1H), 3.99 (s, 3H), 3.89-3.56 (m, 3H), 3.34-3.26 (m, 1H), 2.81-2.67 (m, 2H), 0.89 (s, 9H), 0.09 (s, 6H) .

### Preparation of Compound 4

Compound 3 (9.1 g, 21.7 mmol) and Compound 1 (6.8 g, 32.6 mmol) were dissolved in N,N-dimethylformamide (50 mL), then potassium carbonate (6.0 g, 43.5 mmol) and sodium iodide (1.6 g, 10.8 mmol) were added, and the mixture was stirred at 100°C for 4 hours under a nitrogen atmosphere. The reaction mixture was diluted with ethyl acetate (300 mL) and washed with distilled water (2 × 100 mL). The washed organic layer was washed with brine (100 mL) and dried over anhydrous sodium sulfate. After filtration and concentration under reduced pressure, the product was purified by column chromatography, thereby obtaining Compound 4 (11.0 g, 92%). $^1$H-NMR(400 MHz, $CDCl_3$) δ7.69 (s, 1H), 6.73 (s, 1H), 5.93 (m, 1H), 5.30 (d, 1H), 5.26 (d, 1H), 4.98 (s, 1H), 4.83 (s, 1H), 4.59 (d, 2H), 4.58 (brs, 1H), 4.13 (t, 2H), 3.89 (s, 4H), 3.75 (brs, 1H), 3.71 (s, 1H), 3.31-3.27 (m, 1H), 2.73 (m, 2H), 2.57 (t, 2H), 2.20 (q, 2H), 0.91 (s, 9H), 0.09 (s, 6H).

### Preparation of Compound 5

Compound 4 (11.0 g, 20.0 mmol) was dissolved in ethyl acetate (80 mL) and ethanol (120 mL), zinc dust (26.1 g, 400.2 mmol) was added at 0°C, and formic acid (15.1 mL, 400.2 mmol) was added. After stirring at room temperature for 2.5 hours, the reaction mixture was diluted with ethyl acetate (30 mL) and filtered through Celite, and ethyl acetate (70 mL) was added. The organic layer was washed with saturated aqueous sodium hydrogen carbonate solution (2 × 100 mL) and brine (50 mL) in that order, and then dried over anhydrous sodium sulfate. Filtration, concentration, and purification by column chromatography were performed, thereby obtaining Compound 5 (9.9 g, 95%). $^1$H-NMR(400 MHz, $CDCl_3$) δ 6.74 (s, 1H), 6.25 (s, 1H), 5.92 (m, 1H), 5.30 (d, 1H), 5.24 (d, 1H), 4.97 (s, 1H), 4.90 (s, 1H), 4.59 (d, 2H), 4.33 (brs, 1H), 4.18 (m, 1H), 4.09 (m, 1H), 4.03 (t, 2H), 3.76 (s, 3H), 3.61 (brs, 1H), 2.68 (m, 2H), 2.57 (t, 2H), 2.16 (q, 2H), 0.87 (s, 9H), 0.09 (s, 6H).

### Preparation of Compound 7

Compound 5 (9.9 g, 19.1 mmol) was dissolved in dry tetrahydrofuran (250 mL), and then triphosgene (2.0 g, 6.8 mmol) and triethylamine (10.7 mL, 76.3 mmol) were added at 0°C, and Compound 6 (16.4 g, 21.0 mmol, Compound 6 was prepared by the method described in Korean Patent Publication No. 10-2018-0110645) dissolved in dry tetrahydrofuran (50 mL) was added. After stirring at room temperature for 17 hours, the reaction mixture was diluted with ethyl acetate (200 mL), washed with distilled water (150 mL), and then dried over anhydrous sodium sulfate. After filtration and concentration under reduced pressure, the product was purified by column chromatography, thereby obtaining Compound 7 (14.4 g, 60%). $^1$H-NMR(400 MHz, $CDCl_3$) δ 8.95 (brs, 1H), 8.02 (s, 1H), 7.83 (s, 1H), 7.72 (s, 1H), 7.49 (d, 1H), 7.43 (t, 1H), 7.03 (d, 1H), 6.67 (s, 1H), 5.9 1(m, 1H), 5.40-5.22 (m, 6H), 5.12 (s, 2H), 5.00 (s, 1H), 4.97 (s, 1H), 4.59 (d, 2H), 4.19 (d, 2H), 3.98 (t, 2H), 3.86 (m, 2H), 3.80 (s, 1H), 3.77 (s, 3H), 3.70-3.65 (m, 12H), 3.53 (m, 1H), 2.68 (m, 2H), 2.56 (t, 2H), 2.15 (q, 2H), 2.05 (s, 9H), 1.46 (s, 9H), 0.87 (s, 9H), 0.09 (s, 6H). EI-MSm/z: $[M+H]^+$1275.8.

### <Example 2> Preparation of Compound 10

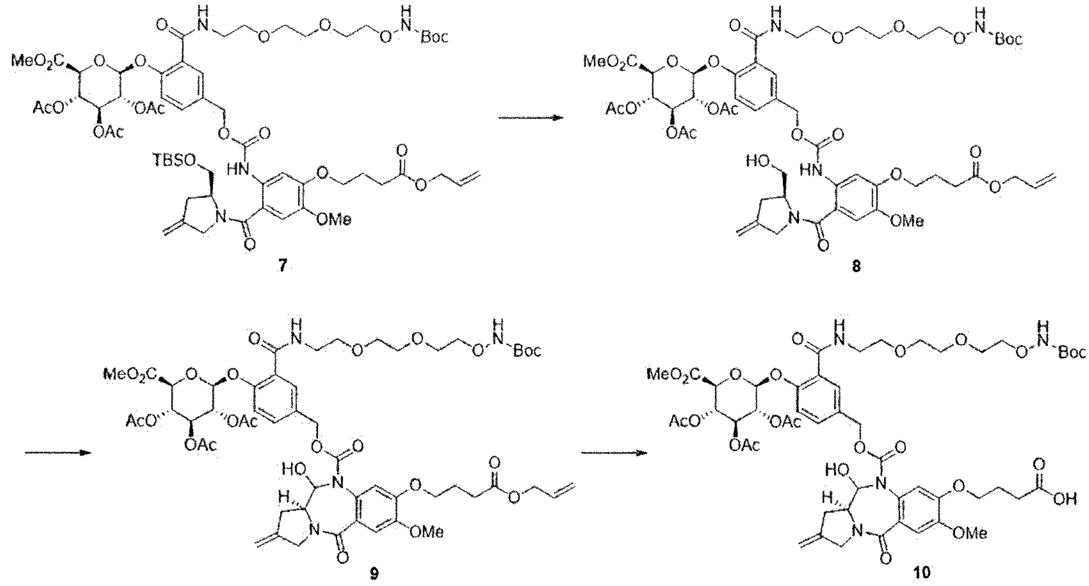

7 8 9 10

## Preparation of Compound 8

Compound 7 (14.4 g, 11.3 mmol) was dissolved in tetrahydrofuran/distilled water (50 mL/50 mL), and acetic acid (50 mL) was added at 0°C, and the mixture was stirred at room temperature for 17 hours. The reaction mixture was diluted with ethyl acetate (100 mL), washed with distilled water (50 mL) and saturated sodium hydrogen carbonate solution (2 × 100 mL) in that order, and dried over anhydrous sodium sulfate. After filtration and concentration under reduced pressure, the product was purified by column chromatography, thereby obtaining Compound 8 (12.6 g, 96%). $^1$H-NMR(400 MHz, $CDCl_3$) δ 8.37 (s, 1H), 8.03 (s, 1H), 7.74 (s, 1H), 7.72 (s, 1H),7.51 (t, 1H), 7.43 (d, 1H), 7.02 (d, 1H), 6.81 (s, 1H), 5.92 (m, 1H), 5.40-5.22 (m, 6H), 5.13 (s, 2H), 5.00 (s, 1H), 4.91 (s, 1H), 4.60 (d, 2H), 4.19 (d, 1H), 4.16 (s, 1H), 4.11 (m, 3H), 3.98 (t, 2H), 3.78 (s, 3H), 3.73-3.65 (m, 12H), 3.53 (m, 1H), 2.76 (m, 1H), 2.57 (t, 2H), 2.50 (m, 1H), 2.17 (q, 2H), 2.05 (s, 9H), 1.46 (s, 9H). EI-MSm/z:$[M+H]^+$1161.6.

## Preparation of Compound 9

Compound 8 (12.6 g, 10.9 mmol) was dissolved in dichloromethane (200 mL), Dess-Martin periodinane (5.6 g, 13.1 mmol) was added, and the mixture was stirred at room temperature for 5 hours under a nitrogen atmosphere. The reaction mixture was diluted with dichloromethane (200 mL), washed with saturated aqueous sodium hydrogen carbonate solution (150 mL) and aqueous sodium thiosulfate solution (100 mL), and then dried over anhydrous sodium sulfate. After filtration and concentration under reduced pressure, the product was purified by column chromatography, thereby obtaining Compound 9 (6.6 g, 52%). $^1$H-NMR(400 MHz, $CDCl_3$) δ 7.97 (s, 1H), 7.80(s, 1H), 7.45(s, 1H), 7.23 (d, 1H), 7.19 (s, 1H), 7.00 (d, 1H), 6.61 (s, 3H), 5.92 (m, 1H), 5.58 (d, 1H), 5.41-5.22 (m, 8H), 5.13 (s, 2H), 4.83 (d, 1H), 4.59 (d, 2H), 4.27 (m, 2H), 4.11 (m, 2H), 3.96 (m, 2H), 3.88 (s, 3H), 3.74-3.65 (m, 15H), 3.52 (m, 1H), 2.90 (m, 1H), 2.70 (d, 1H), 2.57 (t, 2H), 2.13 (q, 2H), 2.05 (s, 9H), 1.46 (s, 9H). EI-MSm/z:$[M+H]^+$1159.6.

## Preparation of Compound 10

Compound 9 (600 mg, 0.52 mmol) was dissolved in *N,N*-dimethylformamide (10 mL), then tetrakis(triphenylphosphine)palladium(0) (238 mg, 0.21 mmol) and pyrrolidine (0.05 mL, 0.57 mmol) were added, and the mixture was stirred at room temperature for 1 hour under a nitrogen atmosphere. The reaction mixture was diluted with ethyl acetate (50 mL), washed with saturated aqueous ammonium chloride solution (50 mL) and distilled water (50 mL), and dried over anhydrous sodium sulfate. Filtration and concentration under reduced pressure were performed, thereby obtaining Compound 10 (570 mg, 98%) as a yellow solid. EI-MS m/z : $[M+H]^+$1119.6, $[M/2+H]^+$510.3.

## <Example 3> Preparation of Compound 14

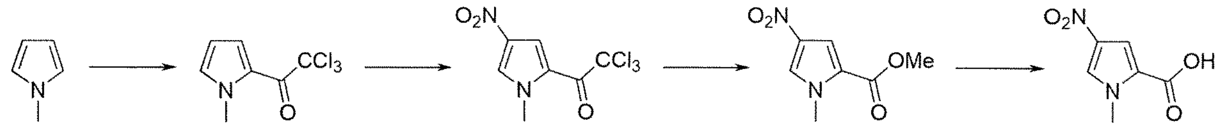

11 12 13 14

**Preparation of Compound 11**

After 1-methylpyrrole (20.0 g, 246.5 mmol) was dissolved in tetrahydrofuran (100 mL), trichloroacetyl chloride (27.5 ml, 246.5 mmol) dissolved in tetrahydrofuran (100 mL) was gradually added using a dropping funnel at 0°C under a nitrogen atmosphere. The reaction solution was heated to room temperature and stirred for 3 hours. The reaction mixture was concentrated, diluted with ethyl acetate (100 mL), and then washed with distilled water (100 mL). The collected organic layers were dried over anhydrous sodium sulfate, thereby obtaining Compound 11 (55.7 g, 99%). $^1$H-NMR(400 MHz, $CDCl_3$) δ7.51-7.49 (m, 1H), 6.96 (s, 1H), 6.23-6.21 (m, 1H), 3.97 (s, 3H).

**Preparation of Compound 12**

Compound 11 (15.8 g, 69.7 mmol) was dissolved in acetic anhydride (170 mL), copper (II) nitrate hydrate (17 g, 73.2 mmol) was added at 0°C under a nitrogen atmosphere, and the mixture was stirred for 2 hours. Saturated aqueous sodium hydrogen carbonate solution (200 mL) was gradually added to the reaction mixture. The aqueous layer was extracted with ethyl acetate (200 mL), followed by washing with distilled water (200 mL). The collected organic layers were dried over anhydrous sodium sulfate, then filtered and concentrated. Recrystallization from dichloromethane/hexane (50 mL/50 mL) was performed, and the remaining filtrate was purified by column chromatography, thereby obtaining Compound 12 (0.8 g, 72%) as a white solid. $^1$H-NMR(400 MHz, $CDCl_3$) δ 7.94 (d, *J* = 1.6 Hz, 1H), 7.75 (s, 1H), 4.05 (s, 1H).

**Preparation of Compound 13**

After Compound 12 (45.7 g, 168.4 mmol) was diluted with methanol (300 mL), sodium methoxide (1.8 g, 33.6 mmol) was added at 0°C under a nitrogen atmosphere, and the mixture was stirred at room temperature for 2 hours. The reaction mixture was neutralized by adding 6 N aqueous hydrochloric acid solution, and then concentrated under reduced pressure, distilled water (200 mL) was added, followed by extraction with ethyl acetate (2 × 200 mL). The collected organic layers were dried over anhydrous sodium sulfate, then filtered and concentrated. After addition of acetone (50 mL), a white solid produced was filtered, thereby obtaining Compound 13 (29.6 g, 95%). $^1$H-NMR(400 MHz, $CDCl_3$) δ 7.59 (d, *J* = 1.6 Hz, 1H), 7.41 (d, J = 1.6 Hz, 1H), 3.99 (s, 3H), 3.86 (s, 3H).

**Preparation of Compound 14**

Compound 13 (15.1 g, 81.9 mmol) was diluted with methanol (200 mL), sodium hydroxide (9.8 g, 245.9 mmol) dissolved in distilled water (200 mL) was added at 0°C under a nitrogen atmosphere, and the mixture was stirred at room temperature for 12 hours. The reaction mixture was adjusted to have a pH of about 2 by adding 6 N aqueous hydrochloric acid solution, and then concentrated under reduced pressure, distilled water (200 mL) was added, followed by extraction with ethyl acetate (2 × 100 mL). The collected organic layers were dried over anhydrous sodium sulfate, then filtered and concentrated. Recrystallization from ethyl acetate/hexane (50 mL/50 mL) was performed, thereby obtaining Compound 14 (13.4 g, 96%). $^1$H-NMR(400 MHz, DMSO-$d_6$) δ13.14 (brs, 1H), 8.23 (d, J = 1.6 Hz, 1H), 7.25 (d, J = 2 Hz, 1H), 3.91 (s, 3H).

**<Example 4> Preparation of Compound 18**

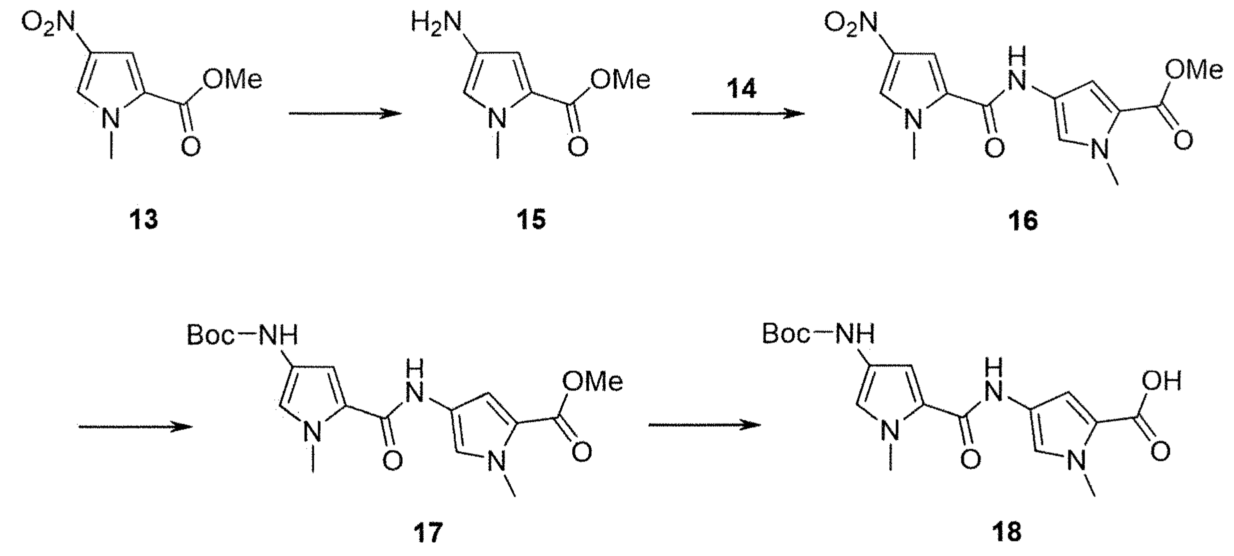


13 15 16

17 18

**Preparation of Compound 15**

Compound 13 (7.5 g, 40.8 mmol) was dissolved in methanol (200 mL) and ethyl acetate (200 mL), and then palladium/charcoal (10% w/w, 1.5 g) was added at 0°C under a nitrogen atmosphere. Sodium borohydride (4.6 g, 122.5 mmol) dissolved in distilled water (100 mL) was gradually added using a dropping funnel, and then the mixture was stirred at room temperature for 1 hour. The reaction mixture was filtered through Celite, then diluted with ethyl acetate (100 mL), and washed with distilled water (100 mL). The collected organic layers were dried over anhydrous sodium sulfate, then filtered, concentrated, and purified by column chromatography, thereby obtaining Compound 15 (6.2 g, 99%). $^1$H-NMR(400 MHz, $CDCl_3$) δ 6.45 (d, *J* = 2.4 Hz, 1H), 6.36 (d, *J* = 2 Hz, 1H), 3.81 (s, 3H), 3.77 (s, 3H).

**Preparation of Compound 16**

Compound 14 (4.8 g, 28.2 mmol) was dissolved in *N,N*-dimethylformamide (70 mL), then *N,N,N',N'*-tetramethyl-*O*-(1*H*-benzotriazol-1-yl)uronium hexafluorophosphate (HBTU, 16.8 g, 42.3 mmol) and *N,N'*-diisopropylethylamine (14.7 mL, 84.6 mmol) were added sequentially at 0°C under a nitrogen atmosphere, and the mixture was stirred at room temperature for 30 minutes. Compound 15 (6.2 g, 40.8 mmol) dissolved in *N,N*-dimethylformamide (70 mL) was added to the reaction solution, and the mixture was stirred at room temperature for 12 hours. The reaction mixture was concentrated, diluted with dichloromethane (100 mL), and the produced solid was filtered, thereby obtaining Compound 16 (8.5 g, 99%). $^1$H-NMR (400 MHz, DMSO-$d_6$) δ 10.25 (s, 1H), 8.19 (s, 1H), 7.55 (d, J = 1.6 Hz, 1H), 7.45 (s, 1H), 6.88 (d, *J* = 1.6 Hz, 1H), 3.94 (s, 3H), 3.84 (s, 3H), 3.74 (s, 3H).

**Preparation of Compound 17**

Compound 16 (6.0 g, 19.58 mmol) was dissolved in methanol (60 mL) and N,N-dimethylformamide (60 mL), and then palladium/charcoal (10% w/w, 1.0 g) and di-t-butyl dicarbonate (13.5 mL, 58.7 mmol) were added. The reaction solution was stirred at room temperature for 18 hours under a hydrogen atmosphere. The reaction mixture was filtered through Celite and then concentrated. The concentrated filtrate was diluted with ethyl acetate (200 mL), washed with distilled water (150 mL), and dried over anhydrous sodium sulfate. After filtration and concentration under reduced pressure, the product was purified by column chromatography, thereby obtaining Compound 17 (5.7 g, 78%). $^1$H-NMR(400 MHz, $CDCl_3$) δ 7.41 (s, 2H), 6.82 (s, 1H), 6.72 (s, 1H), 6.56 (s, 1H), 6.20 (s, 1H), 3.90 (s, 6H), 3.81 (s, 3H), 1.50 (s, 9H).

**Preparation of Compound 18**

After 1,4-dioxane (15 mL) was added to Compound 17 (651 mg, 1.73 mmol), sodium hydroxide (345 mg, 8.64 mmol) dissolved in distilled water (5 mL) was added at 0°C under a nitrogen atmosphere. The solution was heated at 70°C and stirred for 5 hours. After 1,4-dioxane was concentrated, the pH was adjusted to about 2 with 6 N aqueous hydrochloric acid solution, followed by dilution with ethyl acetate (50 mL), washing with distilled water (50 mL), and drying over anhydrous sodium sulfate. Filtration and concentration were performed, thereby obtaining Compound 18 (630 mg,

98%). $^1$H-NMR (400 MHz, $CDCl_3$) δ 7.64 (s, 1H), 7.49 (s, 1H), 6.90 (s, 1H), 6.78 (s, 1H), 6.73 (s, 1H), 6.24 (s, 1H), 3.90 (s, 6H), 1.44 (s, 9H).

**<Example 5> Preparation of Compound 20**

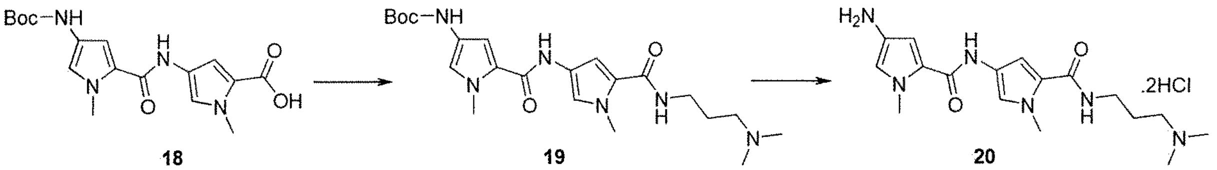

18 19 20

## Preparation of Compound 19

Compound 18 (1.3 g, 3.72 mmol) was dissolved in *N,N*-dimethylformamide (30 mL), then 3-(dimethylamino)-1-propylamine (0.56 mL, 4.46 mmol), *N,N,N',N'*-tetramethyl-*O*-(1*H*-benzotriazol-1-yl)uronium hexafluorophosphate (HBTU, 2.2 g, 5.58 mmol) and *N,N'*-diisopropylethylamine (2.0 mL, 11.16 mmol) were sequentially added at 0°C under a nitrogen atmosphere, and the mixture was stirred at room temperature for 19 hours. The reaction mixture was diluted with ethyl acetate (150 mL), then washed with saturated aqueous ammonium chloride solution (100 mL), saturated sodium hydrogen carbonate solution (100 mL) and distilled water (100 mL), and dried over anhydrous sodium sulfate. Filtration, concentration, and purification by column chromatography were performed, thereby obtaining Compound 19 (1.2 g, 72%). $^1$H-NMR(400 MHz, $CDCl_3$) δ 7.64 (s, 1H), 7.57 (s, 1H), 7.20 (s, 1H), 6.80 (s, 1H), 6.62 (s, 1H), 6.44 (s, 1H), 6.31 (s, 1H), 3.90 (s, 6H), 3.48-3.43 (m, 2H), 2.56-2.53 (m, 2H), 2.38 (s, 6H), 1.81-1.76 (m, 2H), 1.49 (s, 9H).

## Preparation of Compound 20

Compound 19 (300 mg, 0.67 mmol) was dissolved in dichloromethane (4 mL), then hydrochloric acid (4 M 1,4-dioxane solution, 2 mL) was added at 0°C under a nitrogen atmosphere, and the mixture was stirred for 2 hours. The reaction mixture was concentrated under reduced pressure, thereby obtaining Compound 20 (278 mg). EI-MS m/z : $[M+H]^+$347.53, 1/2$[M+H]^+$174.32.

**<Example 6> Preparation of Compound 23**

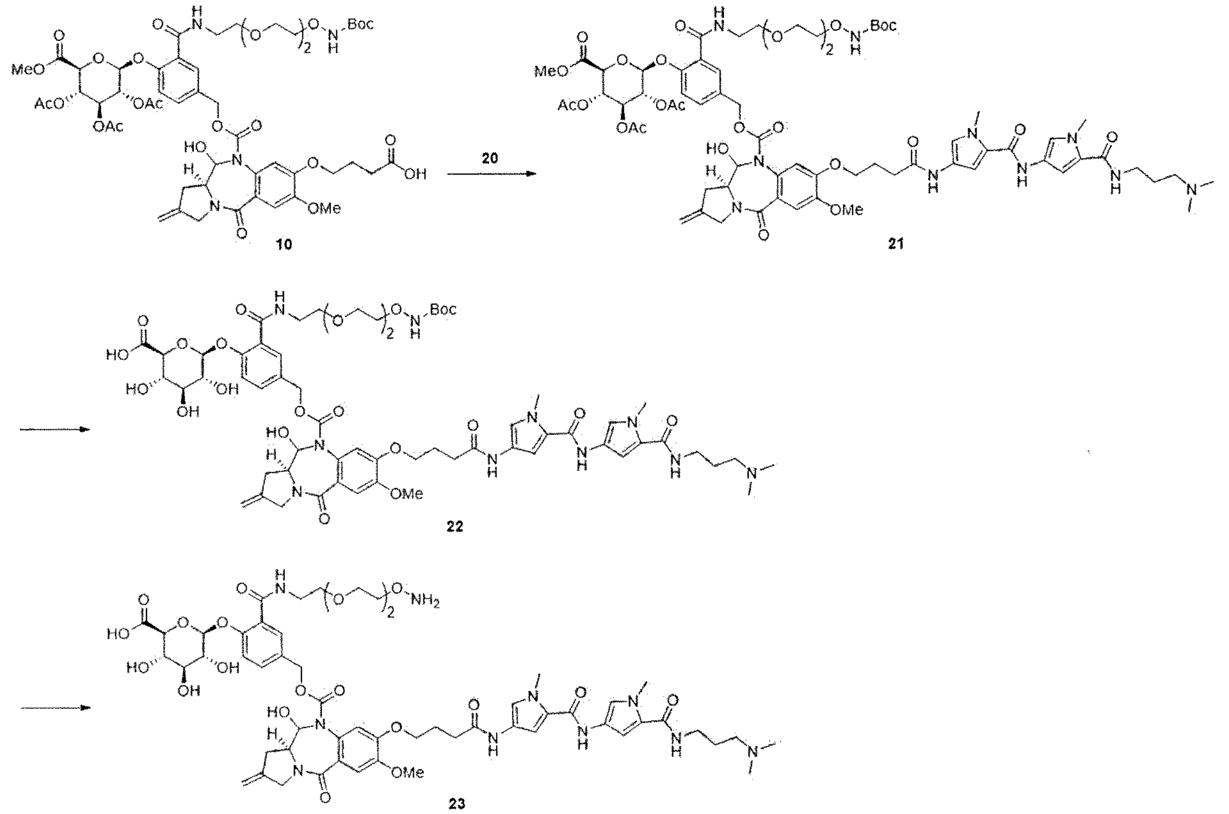


10

21

22

23

## Preparation of Compound 21

Compound 10 (144 mg, 0.128 mmol) was dissolved in*N,N*-dimethylformamide (2 mL), then Compound 20 (70 mg, 0.167 mmol), *N,N,N',N'*-tetramethyl-*O*-(1*H*-benzotriazol-1-yl)uronium hexafluorophosphate (HBTU, 77 mg, 0.193 mmol) and *N,N'*-diisopropylethylamine (0.11 mL, 0.645 mmol) were sequentially added at 0°C under a nitrogen atmosphere, and the mixture was stirred at room temperature for 12 hours. After addition of ethyl acetate (50 mL), the reaction mixture was washed with saturated aqueous ammonium chloride solution (50 mL), saturated aqueous sodium hydrogen carbonate solution (50 mL) and distilled water (50 mL). The collected organic layers were dried over anhydrous sodium sulfate, filtered and then concentrated. The product was purified by column chromatography, thereby obtaining Compound 21 (163 mg, 87%). EI-MS m/z : $[M+H]^+$1448.25, 1/2$[M+H]^+$724.57.

## Preparation of Compound 22

Compound 21 (163 mg, 0.126 mmol) was dissolved in methanol/tetrahydrofuran (2 mL/2 mL), and then a solution of lithium hydroxide (14 mg, 0.337 mmol) in distilled water (2 mL) was gradually added at -40°C under a nitrogen atmosphere. The mixture was stirred for 2 hours while the reaction temperature was gradually raised to 0°C. The reaction mixture was neutralized with acetic acid, then concentrated under reduced pressure, and freeze-dried, thereby obtaining Compound 22 (150 mg, crude). EI-MS m/z : $[M+H]^+$1308.03, $[M/2+H]^+$664.60.

## Preparation of Compound 23

Compound 22 (150 mg, 0.1 mmol) was diluted with dichloromethane (16 mL), then trifluoroacetic acid (4 mL) was added at 0°C under a nitrogen atmosphere, and the mixture was stirred for 2 hours. The reaction mixture was concentrated under reduced pressure, purified by HPLC, and freeze-dried, thereby obtaining Compound 23 as a white solid (12 mg). EI-MS m/z : $[M+H]^+$1207.93, $[M/2+H]^+$604.59.

## <Example 7> Preparation of Compound 26

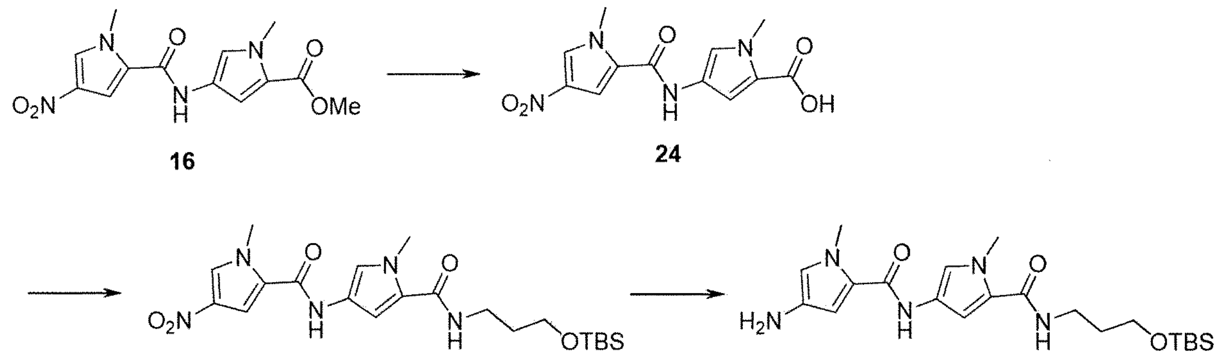

16 24

25 26

## Preparation of Compound 24

After 1,4-dioxane (100 mL) was added to Compound 16 (5.46 g, 17.8 mmol), sodium hydroxide (3.56 g, 89.1 mmol) dissolved in distilled water (40 mL) was added at 0°C under a nitrogen atmosphere. The solution was heated at 80°C and stirred for 6 hours. After 1,4-dioxane was concentrated, the pH was adjusted to about 2 with 6 N aqueous hydrochloric acid solution, followed by dilution with ethyl acetate (150 mL), washing with distilled water (100 mL), and drying over anhydrous sodium sulfate. Filtration and concentration were performed, thereby obtaining Compound 24 (4.63 g, 89%). $^1$H-NMR(400 MHz, DMSO-$d_6$) δ 8.19 (s, 1H), 7.56 (s, 1H), 7.42 (s, 1H), 6.83 (s, 1H), 3.95 (s, 3H), 3.83 (s, 3H).

## Preparation of Compound 25

Compound 24 (217 mg, 0.74 mmol) was dissolved in N,N-dimethylformamide (3 mL), then 3-amino-1-propanol (0.07 mL, 0.97 mmol), *N,N,N',N'*-tetramethyl-*O*-(1*H*-benzotriazol-1-yl)uronium hexafluorophosphate (HBTU, 443 mg, 1.12 mmol), and *N,N'*-diisopropylethylamine (0.26 mL, 1.49 mmol) were sequentially added at 0°C under a nitrogen atmosphere, and the mixture was stirred at room temperature for 4 hours. The reaction mixture was diluted with ethyl acetate (100 mL), washed with saturated sodium hydrogen carbonate solution (100 mL) and distilled water (50 mL), then dried over anhydrous sodium sulfate, filtered and concentrated under reduced pressure. The obtained compound was dissolved in *N,N*-dimethylformamide/dichloromethane (5 mL/10 mL), and imidazole (101.3 mg, 1.49 mmol) and *t*-butyldimethylsilyl chloride (168 mg, 1.12 mmol) were added at 0°C. The reaction solution was stirred at room temperature for 17 hours under a nitrogen atmosphere, the reaction mixture was concentrated, then diluted with ethyl acetate (50 mL), and washed with a saturated aqueous ammonium chloride solution (50 mL) and distilled water (50 mL), and the organic layer was dried over anhydrous sodium sulfate. After filtration and concentration under reduced pressure, the product was purified by column chromatography, thereby obtaining Compound 25 (310 mg, 90%). $^1$H-NMR (400 MHz, DMSO-$d_6$) δ 10.22 (s, 1H), 8.17 (s, 1H), 8.01 (t, 1H), 7.57 (s, 1H), 7.19 (s, 1H), 6.82 (s, 1H), 3.95 (s, 3H), 3.80 (s, 3H), 3.63 (t, 2H), 3.21 (q, 2H), 1.72-1.65 (m, 2H), 0.87 (s, 9H), 0.03 (s, 6H).

## Preparation of Compound 26

Compound 25 (158 mg, 0.34 mmol) was dissolved in methanol (5 mL), and then palladium/charcoal (10% w/w, 80 mg) was added. The reaction solution was stirred at room temperature for 3 hours under a hydrogen atmosphere. The reaction mixture was filtered through Celite and then concentrated, thereby obtaining Compound 26 (142 mg, 96%). $^1$H-NMR(400 MHz, DMSO-$d_6$) δ 9.55 (s, 1H), 7.93 (t, 1H), 7.13 (s, 1H), 6.79 (s, 1H), 6.35 (s, 1H), 6.25 (s, 1H), 3.77 (s, 3H), 3.71 (s, 3H), 3.63 (t, 2H), 3.21 (q, 2H), 1.71-1.65 (m, 2H), 0.87 (s, 9H), 0.03 (s, 6H).

## <Example 8> Preparation of Compound 29

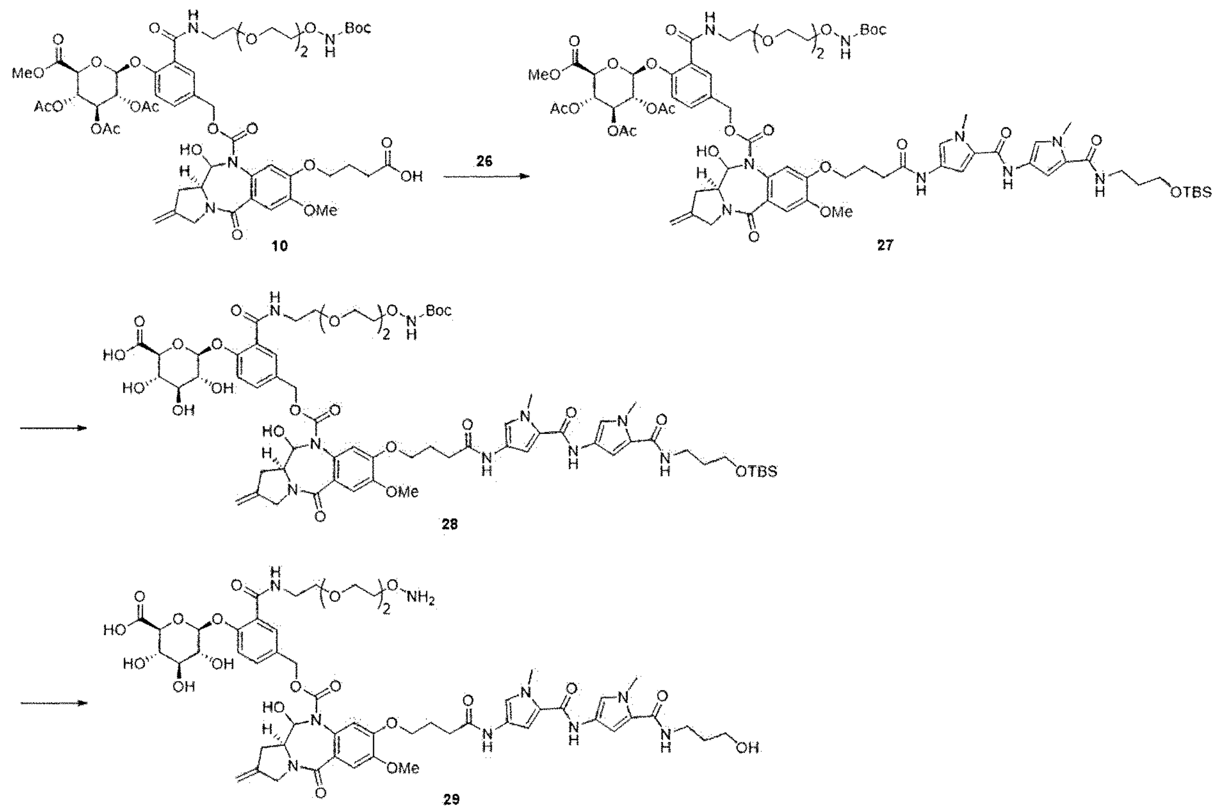


10 27 28 29

## Preparation of Compound 27

Compound 10 (crude 146 mg, 0.13 mmol) was dissolved in *N,N*-dimethylformamide (3 mL), then 1-hydroxybenzotriazole (27 mg, 0.20 mmol) and *N*-(3-dimethylaminopropyl)-*N'*-ethylcarbodiimide hydrochloride (38 mg, 0.20 mmol) were sequentially added at 0°C under a nitrogen atmosphere, and Compound 26 (114 mg, 0.26 mmol) dissolved in *N,N*-dimethylformamide (1 mL) was added. After stirring at room temperature for 5 hours, the reaction mixture was diluted with ethyl acetate (50 mL), then washed with saturated aqueous ammonium chloride solution (50 mL), saturated sodium hydrogen carbonate solution (50 mL), and distilled water (50 mL) in that order, and then dried over anhydrous sodium sulfate. After filtration and concentration under reduced pressure, the product was purified by column chromatography, thereby obtaining Compound 27 (85 mg, 42%). EI-MS m/z : [M+H]$^+$1534.88, 1/2[M+H]$^+$768.49, 1/2[M-BOC+H]$^+$718.36.

## Preparation of Compound 28

Compound 27 (85 mg, 0.05 mmol) was dissolved in methanol/tetrahydrofuran (1.0 mL/1.0 mL), and then a solution of lithium hydroxide (7 mg, 0.16 mmol) in distilled water (1.0 mL) was gradually added at -40°C under a nitrogen atmosphere. The mixture was stirred for 5 hours while the reaction temperature was gradually raised to 0°C. The reaction mixture was neutralized with acetic acid, concentrated under reduced pressure, purified by HPLC, and freeze-dried, thereby obtaining Compound 28 (18 mg). EI-MS m/z : [M-TBS+H]$^+$1280.82, 1/2[M-BOC+H]$^+$591.04.

## Preparation of Compound 29

Compound 28 (18 mg) was diluted with dichloromethane (1.5 mL), then trifluoroacetic acid (0.5 mL) was added at 0°C under a nitrogen atmosphere, and the mixture was stirred for 1 hour. The reaction mixture was concentrated under reduced pressure, purified by HPLC, and freeze-dried, thereby obtaining Compound 29 as a pale yellow solid (6.1 mg). EI-MS m/z : [M+H]$^+$1180.59, [M/2+H]$^+$591.88.

## <Example 9> Preparation of Compound 34

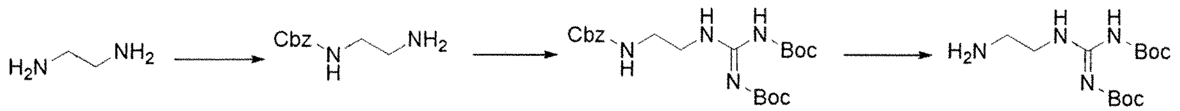

30 31 32

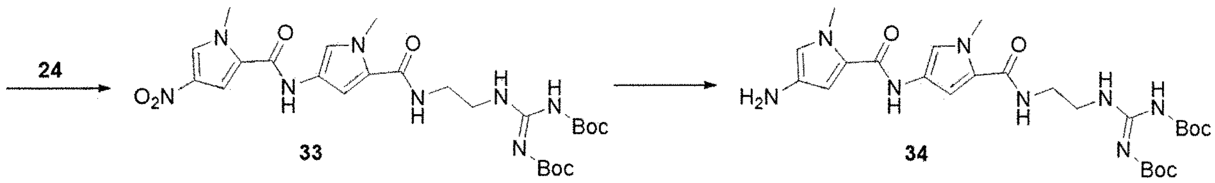


33 34

### Preparation of Compound 30

Ethylenediamine (6 mL, 90 mmol) was dissolved in dichloromethane (90 mL), then benzyl chloroformate (1.5 g, 9 mmol) dissolved in dichloromethane (25 mL) was gradually added at 0°C under a nitrogen atmosphere, and the mixture was stirred at room temperature for 2 hours. The reaction mixture was diluted with dichloromethane (100 mL) and washed with brine (100 mL). The organic layer was dried over anhydrous sodium sulfate, filtered and concentrated under reduced pressure, thereby obtaining Compound 30 (1.75 g, 99%). $^1$H-NMR(400 MHz, $CDCl_3$) δ 7.32-7.28 (m, 5H), 5.07 (s, 2H), 3.21 (t, *J* = 6.4 Hz, 2H), 2.73 (t, *J* = 2 Hz, 2H).

### Preparation of Compound 31

Compound 30 (880 mg, 4.5 mmol) was dissolved in dichloromethane (15 mL), and then *N,N*'-Di-Boc-1*H*-pyrazole-1-carboxamidine (1.68 g, 5.4 mmol) and triethylamine (0.8 mL, 5.4 mmol) were sequentially added at 0°C under a nitrogen atmosphere, and the mixture was stirred at room temperature for 2 hours. The reaction mixture was concentrated and purified by column chromatography, thereby obtaining Compound 31 (1.96 g, 99%). $^1$H-NMR(400 MHz, $CDCl_3$) δ 11.43 (s, 1H), 8.53 (s, 1H), 7.35-7.29 (m, 5H), 5.09 (s, 1H), 3.62-3.54 (m, 2H), 3.40-3.36 (m, 2H), 1.48 (s, 9H), 1.44 (s, 9H).

### Preparation of Compound 32

Compound 31 (1.9 g, 4.3 mmol) was dissolved in ethyl acetate (40 mL), and then palladium/charcoal (10% w/w, 190 mg) was added. The reaction solution was stirred at room temperature for 3 hours under a hydrogen atmosphere. The reaction mixture was filtered through Celite, then concentrated, and purified by column chromatography, thereby obtaining Compound 32 (0.92 g, 67%). $^1$H-NMR(400 MHz, $CDCl_3$) δ 11.51 (s, 1H), 8.66 (s, 1H), 3.51-3.46 (m, 2H), 2.91-2.88 (m, 2H), 1.50 (s, 18H).

### Preparation of Compound 33

Compound 24 (150 mg, 0.51 mmol) was dissolved in *N,N*-dimethylformamide (5 mL), then Compound 32 (186 mg, 0.62 mmol), *N,N,N',N'*-tetramethyl-*O*-(1*H*-benzotriazol-1-yl)uronium hexafluorophosphate (HBTU, 305 mg, 0.77 mmol), and *N,N*'-diisopropylethylamine (0.18 mL, 1.02 mmol) were sequentially added at 0°C under a nitrogen atmosphere, and the mixture was stirred at room temperature for 16 hours. The reaction mixture was concentrated under reduced pressure and purified by column chromatography, thereby obtaining Compound 33 (174 mg, 59%). $^1$H-NMR (400 MHz, $CDCl_3$) δ 11.55 (s, 1H), 8.69 (s, 1H), 7.59 (s, 1H), 7.50 (s, 1H), 7.22 (s, 1H), 7.12 (s, 1H), 6.46 (s, 1H), 4.02 (s, 3H), 3.92 (s, 3H), 3.74-3.62 (m, 2H), 3.56-3.52 (m, 2H), 1.53 (s, 9H), 1.50 (s, 9H).

### Preparation of Compound 34

Compound 33 (174 mg, 0.3 mmol) was dissolved in methanol (5 mL), and then palladium/charcoal (10% w/w, 70 mg) was added. The reaction solution was stirred at room temperature for 12 hours under a hydrogen atmosphere. The reaction mixture was filtered through Celite and then concentrated, thereby obtaining Compound 34 (158 mg, 96%). EI-MS m/z : $[M+H]^+$547.30.

**<Example 10> Preparation of Compound 35**

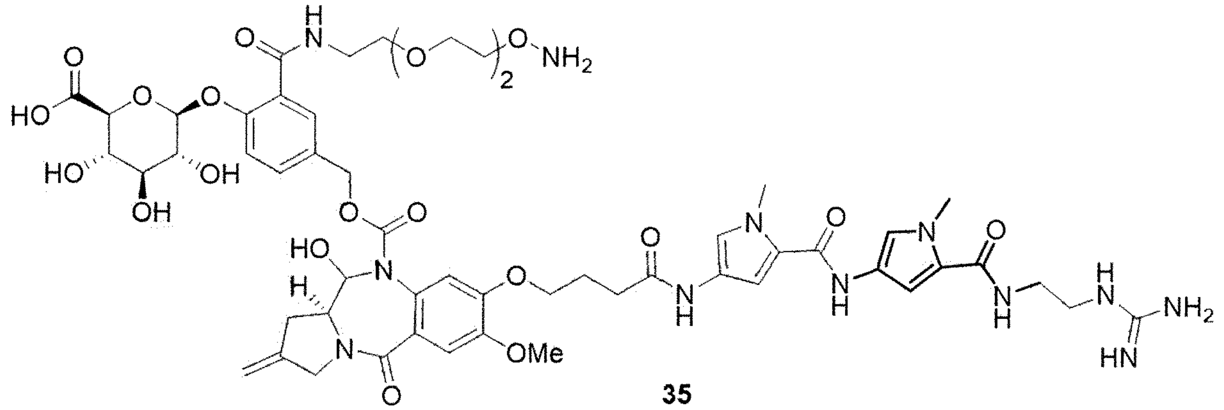

**35**

Compound 35 was synthesized from Compound 10 and Compound 34 in a similar manner to the synthesis of Compound 23. EI-MS m/z : $[M+H]^+$1207.44, $[M/2+H]^+$604.50.

**<Example 11> Preparation of Compound 37**

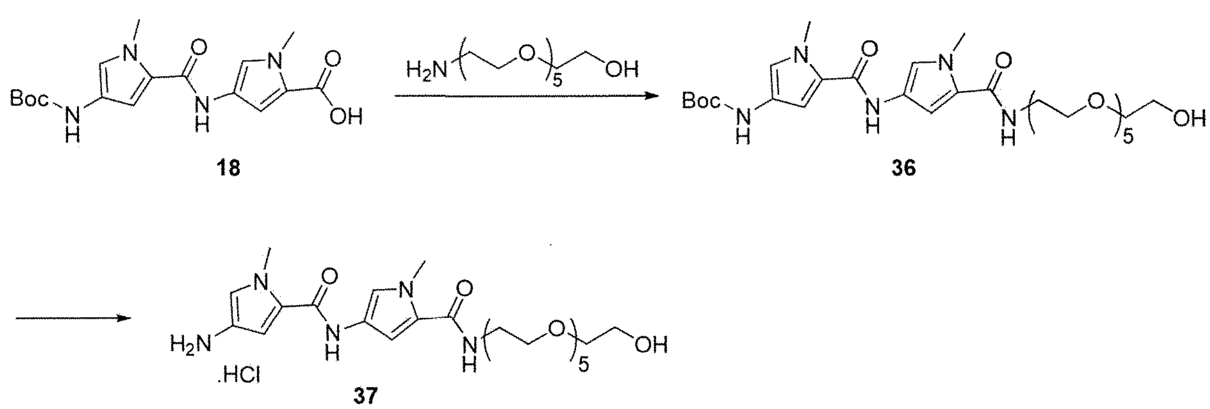

**18** **36**

**37**

**Preparation of Compound 36**

Compound 18 (200 mg, 0.55 mmol) was dissolved in *N,N*-dimethylformamide (5 mL), then $NH_2$-PEG6-OH(232mg, 0.83 mmol), *N,N,N',N'*-tetramethyl-*O*-(1*H*-benzotriazol-1-yl)uronium hexafluorophosphate (HBTU, 328 mg, 0.83 mmol), and *N,N'*-diisopropylethylamine (0.3 mL, 1.66 mmol) were sequentially added at 0°C under a nitrogen atmosphere, and the mixture was stirred at room temperature for 3 hours. The reaction mixture was diluted with ethyl acetate (50 mL), then washed with saturated aqueous ammonium chloride solution (50 mL), saturated sodium hydrogen carbonate solution (50 mL) and distilled water (50 mL), and dried over anhydrous sodium sulfate. Filtration, concentration, and purification by column chromatography were performed, thereby obtaining Compound 36 (255 mg, 74%). $^1H$-NMR(400 MHz, $CDCl_3$) 58.35(s, 1H), 7.36(s, 1H), 6.95(s, 1H), 6.88(s, 1H), 6.65(s, 3H), 3.91(s, 6H), 3.72-3.54(m, 24H), 1.49(s, 9H).

**Preparation of Compound 37**

Compound 36 (139 mg, 0.22 mmol) was dissolved in dichloromethane (2 mL), then hydrochloric acid (4 M 1,4-dioxane solution, 1 mL) dissolved in 1,4-dioxane was added at 0°C under a nitrogen atmosphere, and the mixture was stirred for 2 hours. The reaction mixture was concentrated under reduced pressure, thereby obtaining Compound 37 (124 mg). EI-MS m/z : [M+H]+ 526.33, 1/2 [M+H]+ 282.37.

**<Example 12> Preparation of Compound 38**

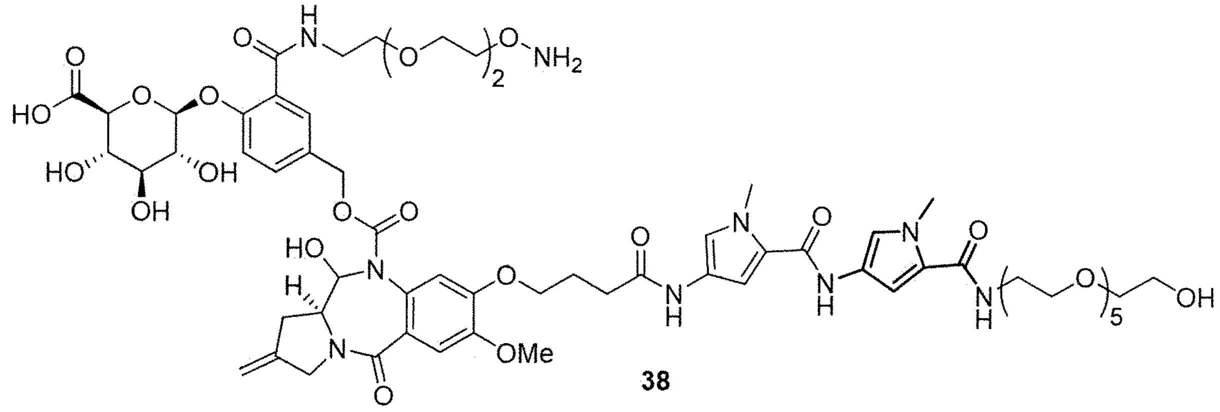

38

Compound 38 was synthesized from Compound 10 and Compound 37 in a similar manner to the synthesis of Compound 23. EI-MS m/z : $[M+H]^+$1386.33, $[M/2+H]^+$694.09.

**<Example 13> Preparation of Compound 40**

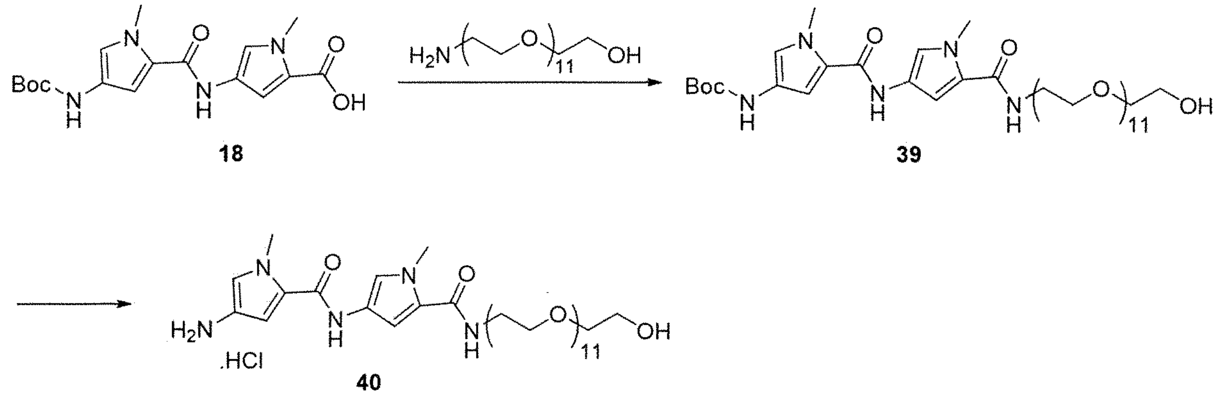

18 39 40

**Preparation of Compound 39**

Compound 18 (100 mg, 0.27 mmol) was dissolved in *N,N*-dimethylformamide (5 mL), then Compound $NH_2$-PEG12-OH (238 mg, 0.4 mmol), *N,N,N',N'*-tetramethyl-*O*-(1*H*-benzotriazol-1-yl)uronium hexafluorophosphate (HBTU, 164 mg, 0.4 mmol), and *N,N'*-diisopropylethylamine (0.14 mL, 0.8 mmol) were sequentially added at 0°C under a nitrogen atmosphere, and the mixture was stirred at room temperature for 12 hours. The reaction mixture was concentrated under reduced pressure, thereby obtaining Compound 39 (245 mg, crude). $^1$H-NMR(400 MHz, $CDCl_3$) δ 8.47 (s, 1H), 7.33 (s, 1H), 6.66 (s, 2H), 3.99 (s, 3H), 3.89 (s, 3H), 3.75-3.42 (m, 48H), 1.49 (s, 9H). EI-MS m/z: $[M+Na]^+$912.42, $[M+H]^+$890.45, 1/2$[M-BOC+H]^+$395.99.

**Preparation of Compound 40**

Compound 39 (245 mg, 0.27 mmol) was diluted with dichloromethane (8 mL), then hydrochloric acid (4 M 1,4-dioxane solution, 2 mL) was added at 0°C under a nitrogen atmosphere, and the mixture was stirred for 2 hours. The reaction mixture was concentrated under reduced pressure, thereby obtaining Compound 40 (213 mg). EI-MS m/z : $[M+Na]^+$813.47, $[M/2+H]^+$395.94.

**<Example 14> Preparation of Compound 41**

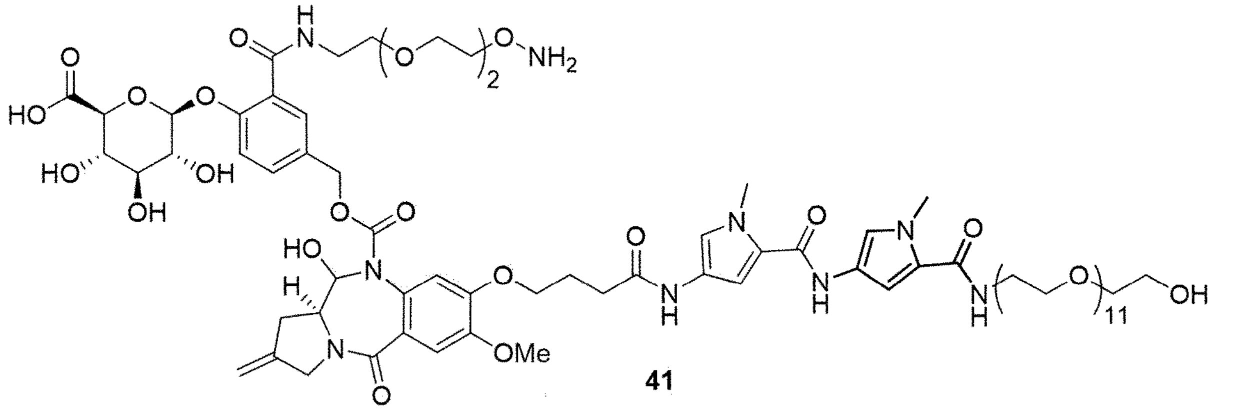

41

Compound 41 was synthesized from Compound 10 and Compound 40 in a similar manner to the synthesis of Compound 23. EI-MS m/z : $[M+H]^+$1651.57, $[M/2+H]^+$826.17.

**<Example 15> Preparation of Compound 44**

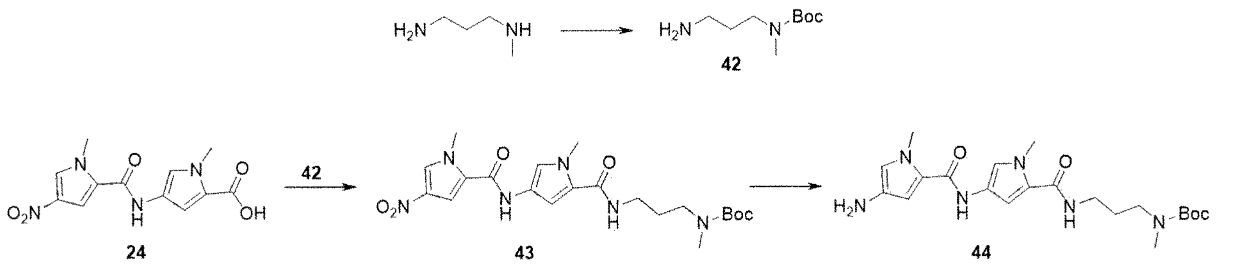


42

24

43

44

**Preparation of Compound 42**

After 3-(methylamino)propylamine (20.0 g, 228.02 mmol) was dissolved in tetrahydrofuran (300 mL), di-t-butyl dicarbonate (9.8 g, 44.9 mmol) dissolved in tetrahydrofuran (200 mL) was gradually added using a dropping funnel at 0°C under a nitrogen atmosphere. The reaction solution was heated to room temperature and stirred for 5 hours. The reaction mixture was concentrated, diluted with dichloromethane (200 mL), washed with distilled water (100 mL), and dried over anhydrous sodium sulfate. After filtration and concentration under reduced pressure, the product was purified by column chromatography, thereby obtaining Compound 42 (5.1 g, 60%). $^1$H-NMR(400 MHz, $CDCl_3$) δ 3.29 (s, 2H), 2.83 (s, 3H), 2.70-2.67 (m, 2H), 1.68-1.61 (m, 2H), 1.43 (s, 9H) .

**Preparation of Compound 43**

Compound 24 (400 mg, 1.36 mmol) was dissolved in *N,N*-dimethylformamide (5 mL), then *N,N,N',N'*-tetramethyl-*O*-(1*H*-benzotriazol-1-yl)uronium hexafluorophosphate (HBTU, 778 mg, 2.05 mmol) and *N,N'*-diisopropylethylamine (0.47 mL, 2.73 mmol) were added at 0°C under a nitrogen atmosphere, and the mixture was stirred for 3 minutes. Compound 42 (309 mg, 1.64 mmol) was dissolved in *N,N*-dimethylformamide (3 mL) and then added to the reaction solution at 0°C, the temperature was gradually raised, and the mixture was stirred at room temperature for 16 hours. Saturated aqueous ammonium chloride solution (20 mL) was added to the reaction mixture, followed by extraction with ethyl acetate (3 × 20 mL). The extracted solution was washed with saturated sodium hydrogen carbonate solution (15 mL) and distilled water (15 mL), and then dried over anhydrous sodium sulfate. Filtration, concentration, and purification by column chromatography were performed, thereby obtaining Compound 43 (582 mg, 92%). $^1$H-NMR (400 MHz, $CDCl_3$) δ 7.69 (brs, 1H), 7.59 (s, 1H), 7.36 (brs, 1H), 7.29 (brs, 1H), 7.19 (s, 1H), 6.58 (brs, 1H), 4.02 (s, 3H), 3.93 (s, 3H), 3.37

(m, 4H), 2.85 (s, 3H), 1.71 (m, 2H), 1.49 (s, 9H), EI-MS m/z : $[M+H]^+$463.39.

**Preparation of Compound 44**

Compound 43 (160 mg, 0.34 mmol) was dissolved in methanol (5 mL), and then palladium/charcoal (10% w/w, 80 mg) was added. The reaction solution was stirred at room temperature for 2 hours under a hydrogen atmosphere. The reaction mixture was filtered through Celite and then concentrated, thereby obtaining Compound 44 (140 mg). $^1H$-NMR(400 MHz, $CDCl_3$) $\delta$ 7.36 (brs, 1H), 7.30 (brs, 1H), 6.53 (s, 1H), 6.33 (s, 1H), 6.19 (s, 1H), 3.92 (s, 3H), 3.86 (s, 3H), 3.37-3.32 (m, 4H), 2.84 (s, 3H), 1.71 (m, 2H), 1.48 (s, 9H), EI-MS m/z : $[M+H]^+$433.41.

**<Example 16> Preparation of Compound 45**

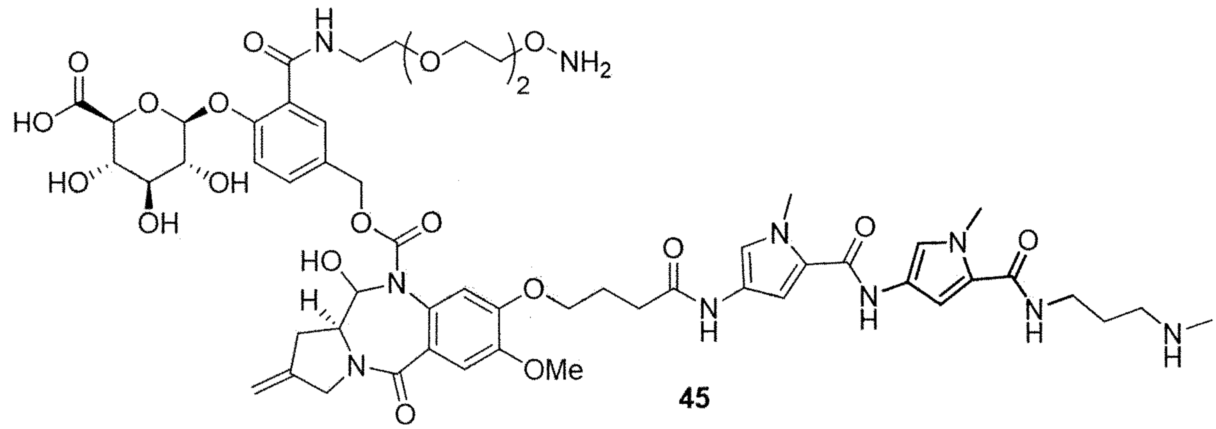

45

Compound 45 was synthesized from Compound 10 and Compound 44 in a similar manner to the synthesis of Compound 23. $^1H$-NMR (400 MHz, Methanol-$d_4$) $\delta$ 7.76 (brs, 1H), 7.30 (m, 2H), 7.14 (s, 3H), 6.87 (s, 1H), 6.79 (s, 1H), 6.76 (brs, 1H), 5.57 (d, *J* =8.8 Hz, 1H), 5.23-5.18 (m, 3H), 5.11 (d, *J* =7.6 Hz, 1H), 4.25-4.21 (m, 1H), 4.15 (m, 2H), 4.08-4.03 (m, 3H), 3.88-3.80 (m, 12H), 3.69-3.52 (m, 13H), 3.41 (t, *J* =6 Hz, 2H), 3.03 (t, *J* =7.2 Hz, 2H), 2.94 (m, 1H), 2.50 (m, 2H), 2.11 (m, 2H), 1.93(m, 2H), EI-MS m/z : $[M+H]^+$1193.44, $[M/2+H]^+$597.49.

**<Example 17> Preparation of Compound 51**

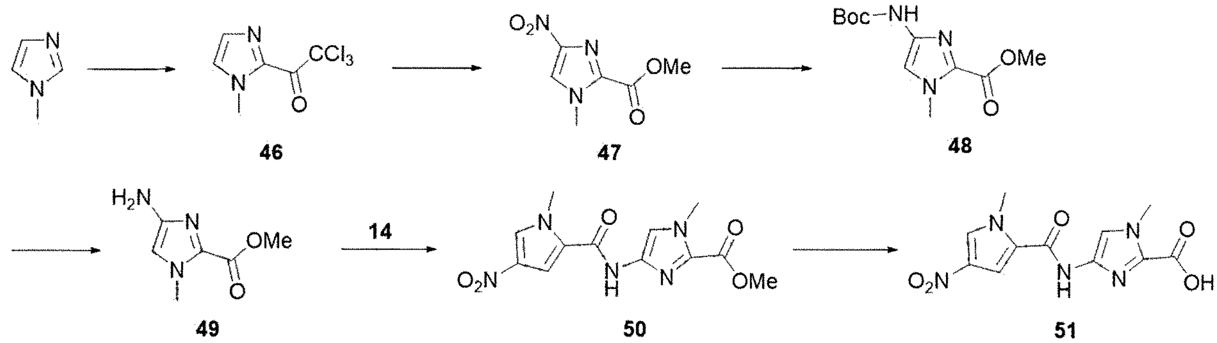


46 47 48 49 50 51

**Preparation of Compound 46**

After 1-methylimidazole (5 g, 95.9 mmol) was dissolved in dichloromethane (40 mL), trichloroacetyl chloride (6.79 ml, 95.9 mmol) dissolved in dichloromethane (40 mL) was gradually added using a dropping funnel at 0°C over 1 hour under a nitrogen atmosphere. The reaction solution was heated to room temperature and stirred for 6 hours. Triethylamine (13.3 mL, 95.9 mmol) was added at room temperature, and the reaction mixture was stirred for 30 minutes. The reaction mixture was concentrated and recrystallized from chloroform and hexane, thereby obtaining Compound

46 (12.6 g, 96%). $^1$H-NMR(400 MHz, $CDCl_3$) δ 7.35 (s, 1H), 7.16 (s, 1H), 4.06 (s, 3H).

**Preparation of Compound 47**

After nitric acid (8 mL, 193.9 mmol) and sulfuric acid (0.35 mL, 6.6 mmol) were added to acetic anhydride (110 mL) at -10°C under a nitrogen atmosphere, Compound 46 (12.6 g, 55.3 mmol) was added. After stirring at 0°C for 3 hours, the reaction mixture was diluted with chloroform (200 mL), and then saturated aqueous sodium hydrogen carbonate solution (200 mL) was gradually added. The aqueous layer was extracted with chloroform (100 mL), and the collected organic layers were dried over anhydrous sodium sulfate. After filtration and concentration, methanol (200 mL) was added, and the mixture was stirred at room temperature for 2 hours. Methanol was concentrated, the resultant was diluted with dichloromethane (100 mL), and the produced solid was filtered, thereby obtaining Compound 47 (6.3 g, 61%). $^1$H-NMR(400 MHz, $CDCl_3$) δ 7.84 (s, 1H), 4.12 (s, 3H), 3.98 (s, 3H).

**Preparation of Compound 48**

After methanol (300 mL) was added to Compound 47 (6.3 g, 34.1 mmol), di-*t*-butyl dicarbonate (20 mL, 102.4 mmol) and palladium/charcoal (10% w/w, 630 mg) were added in that order, and the mixture was stirred at room temperature for 3 hours under a hydrogen atmosphere. The reaction mixture was further stirred for 12 hours under a nitrogen atmosphere. The reaction mixture was filtered through Celite, then concentrated, and purified by column chromatography, thereby obtaining Compound 48 (7.9 g, 91%). $^1$H-NMR(400 MHz, $CDCl_3$) δ 9.69 (s, 1H), 7.32 (s, 1H), 3.88 (s, 3H), 3.78 (s, 3H), 1.46 (s, 9H).

**Preparation of Compound 49**

Compound 48 (300 mg, 1.17 mmol) was dissolved in dichloromethane (1 mL), then trifluoroacetic acid (1 mL) was added at 0°C under a nitrogen atmosphere, and the mixture was stirred for 2 hours. The reaction mixture was concentrated under reduced pressure, then diluted with dichloromethane (30 mL), and washed with saturated aqueous sodium hydrogen carbonate solution (30 mL). The aqueous layer was extracted with dichloromethane (30 mL), and the collected organic layers were dried over anhydrous sodium sulfate. Filtration and concentration were performed, thereby obtaining Compound 49 (160 mg). $^1$H-NMR (400 MHz, $CDCl_3$) δ 6.37 (s, 1H), 3.92 (s, 3H). 3.90 (s, 3H), 3.56 (brs, 2H) EI-MS m/z : $[M+H]^+$156.17.

**Preparation of Compound 50**

Compound 14 (300 mg, 1.76 mmol) was dissolved in thionyl chloride (3 mL), and then the reaction solution was heated at 70°C for 1 hour and concentrated under reduced pressure. After dichloromethane (4 mL) and *N,N'*-diisopropylethylamine (0.92 mL, 5.29 mmol) were added at -50°C under a nitrogen atmosphere, Compound 49 (329 mg, 2.12 mmol) diluted with dichloromethane (4 mL) was added to the reaction solution. After the temperature was raised to room temperature, the mixture was stirred for 18 hours. Saturated aqueous ammonium chloride solution (15 mL) was added to the reaction mixture, followed by extraction with ethyl acetate (20 mL × 3), and washing with saturated aqueous sodium hydrogen carbonate solution (20 mL), distilled water (20 mL), and aqueous sodium chloride solution (20 mL) was performed. Drying over anhydrous sodium sulfate, filtration, concentration, and purification by column chromatography were performed, thereby obtaining Compound 50 (140 mg, 25%). $^1$H-NMR (400 MHz, $CDCl_3$) δ 8.27 (brs, 1H), 7.61 (s, 1H), 7.53 (s, 1H), 7.13 (d, *J* =1.6 Hz, 1H), 4.11 (s, 6H), 3.95 (s, 3H), EI-MS m/z : $[M+H]^+$308.20.

**Preparation of Compound 51**

Compound 50 (140 mg, 0.45 mmol) was dissolved in methanol (2.5 mL), then a solution of lithium hydroxide (25.8 mg, 0.61 mmol) in distilled water (2.5 mL) was gradually added at room temperature under a nitrogen atmosphere, and the mixture was heated to 70°C and stirred for 1 hour. The reaction mixture was acidified with 1 N aqueous hydrochloric acid (pH of about 4) and concentrated under reduced pressure, thereby obtaining Compound 51 (133 mg). $^1$H-NMR (400 MHz, DMSO-$d_6$) δ 11.11 (s, 1H), 8.19 (s, 1H), 7.80 (d, *J* =2 Hz, 1H), 7.64 (s, 1H), 3.96 (s, 3H), 3.93 (s, 3H), EI-MS m/z : $[M+H]^+$294.09.

**<Example 18> Preparation of Compound 53**

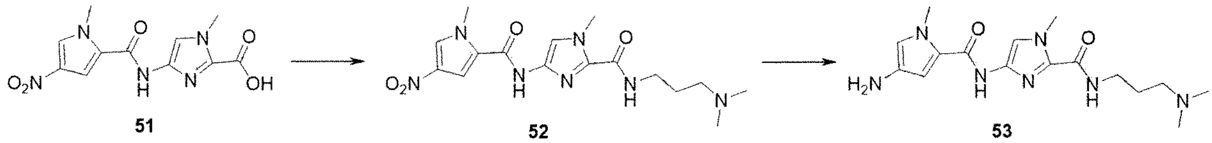

51 52 53

Preparation of Compound 52

Compound 51 (133 mg, 0.45 mmol) was dissolved in *N,N*-dimethylformamide (3 mL), and then *N,N,N',N'*-tetramethyl-*O*-(1*H*-benzotriazol-1-yl)uronium hexafluorophosphate (HBTU, 258 mg, 0.68 mmol) and *N,N'*-diisopropylethylamine (0.16 mL, 0.91 mmol) were added at 0°C under a nitrogen atmosphere. After *N,N'*-dimethyl-1,3-propanediamine (0.11 mL, 0.91 mmol) was added to the reaction solution, the temperature was raised to room temperature, and the mixture was stirred for 17 hours. Saturated aqueous ammonium solution (15 mL) was added to the reaction mixture, followed by extraction with ethyl acetate (20 mL × 3), and washing with saturated aqueous sodium hydrogen carbonate solution (20 mL), distilled water (20 mL), and aqueous sodium chloride solution (20 mL) was performed in that order. Drying over anhydrous sodium sulfate, filtration, concentration, and purification by column chromatography were performed, thereby obtaining Compound 52 (70 mg, 40%). $^1$H-NMR(400 MHz, DMSO-$d_6$) δ10.78(s, 1H), 8.19(s, 1H), 8.02(t, *J* =6.4 Hz, 1H), 7.75 (d, *J* =0.8 Hz, 1H), 7.51 (s, 1H), 3.95 (s, 3H), 3.93 (s, 3H), 3.25 (m, 2H), 2.24 (m, 2H), 2.21 (m, 6H), 1.62 (m, 2H), EI-MS m/z : [M+H]$^+$378.30.

Preparation of Compound 53

Compound 52 (70 mg, 0.18 mmol) was dissolved in methanol (3 mL), and then palladium/charcoal (10% w/w, 35 mg) was added at room temperature. The reaction solution was stirred at room temperature for 2 hours under a hydrogen atmosphere. The reaction mixture was filtered through Celite and then concentrated under reduced pressure, thereby obtaining Compound 53 (58 mg). EI-MS m/z : [M+H]$^+$348.34, [M/2+H]$^+$174.75.

**<Example 19> Preparation of Compound 54**

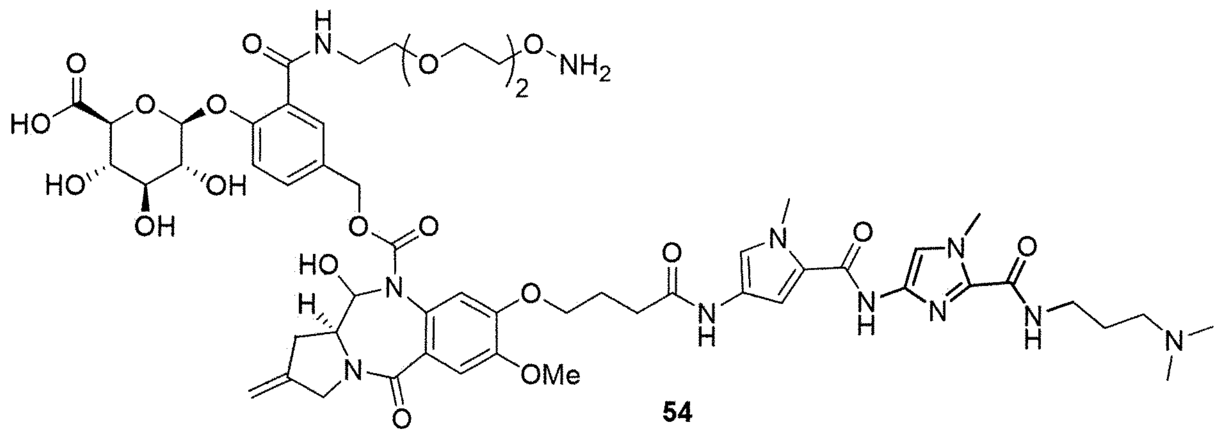

54

Compound 54 was synthesized from Compound 10 and Compound 53 in a similar manner to the synthesis of Compound 23. $^1$H-NMR (400 MHz, Methanol-$d_4$) δ 7.76 (brs, 1H), 7.40 (brs, 1H), 7.29-7.26 (m, 2H), 7.15 (m, 2H), 6.79 (m, 2H), 5.58 (d, *J* =10 Hz, 1H), 5.25 (d, *J* =12 Hz, 1H), 5.17-5.12 (m, 4H), 4.24-4.15 (m, 4H), 4.11 (d, *J* =9.2 Hz, 2H), 4.09-3.99 (m, 6H), 3.87-3.80 (m, 11H), 3.69-3.55 (m, 17H), 3.46 (t, *J* =6.4 Hz, 2H), 3.21 (m, 3H), 2.92 (m, 10H), 2.67-2.62 (m, 2H), 2.52 (m, 2H), 2.12 (m, 2H), 2.00 (m, 2H), EI-MS m/z : [M+H]$^+$1208.43, [M/2+H]$^+$605.08.

**<Example 20> Preparation of Compound 56**

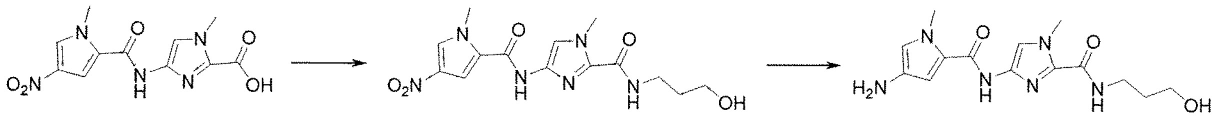

51 55 56

### Preparation of Compound 55

Compound 51 (156 mg, 0.53 mmol) was dissolved in *N,N*-dimethylformamide (3 mL), and then *N,N,N',N'*-tetramethyl-*O*-(1*H*-benzotriazol-1-yl)uronium hexafluorophosphate (HBTU, 303 mg, 0.79 mmol) and *N,N'*-diisopropylethylamine (0.28 mL, 1.59 mmol) were added at 0°C under a nitrogen atmosphere. After 3-amino-1-propanol (0.12 mL, 1.59 mmol) was added to the reaction solution, the temperature was raised to room temperature, and the mixture was stirred for 18 hours. Saturated aqueous ammonium chloride solution (15 mL) was added to the reaction mixture, followed by extraction with chloroform (20 mL × 3), and washing with saturated aqueous sodium hydrogen carbonate solution (20 mL), distilled water (20 mL), and aqueous sodium chloride solution (20 mL) was performed in that order. Drying over anhydrous sodium sulfate, filtration, concentration, and purification by column chromatography were performed, thereby obtaining Compound 55 (115 mg, 61%). $^1$H-NMR(400 MHz, DMSO-$d_6$) δ 10.79 (s, 1H), 8.17 (s, 1H), 7.92 (t, *J* =6 Hz, 1H), 7.74 (d, *J* =1.2 Hz, 1H), 7.50 (s, 1H), 4.54 (brs, 1H), 3.93 (s, 3H), 3.92 (s, 3H), 3.46 (m, 2H), 3.27 (m, 2H), 1.62 (m, 2H), EI-MS m/z : $[M+H]^+$351.26.

### Preparation of Compound 56

Compound 55 (115 mg, 0.32 mmol) was dissolved in methanol (5 mL), and palladium/charcoal (10% w/w, 50 mg) was added at room temperature. The reaction solution was stirred at room temperature for 4 hours under a hydrogen atmosphere. The reaction mixture was filtered through Celite and then concentrated under reduced pressure, thereby obtaining Compound 56 (100 mg). $^1$H-NMR(400 MHz, DMSO-$d_6$) δ 9.92 (s, 1H), 7.91 (t, *J* =6 Hz, 1H), 7.42 (s, 1H), 6.47 (d, *J* =1.6 Hz, 1H), 6.28 (d, *J* =2 Hz, 1H), 4.52 (brs, 1H), 3.90 (s, 3H), 3.78 (brs, 2H), 3.71 (s, 3H), 3.44 (m, 2H), 3.27 (m, 2H), 1.66 (m, 2H), EI-MS m/z : $[M+H]^+$321.29.

### <Example 21> Preparation of Compound 57

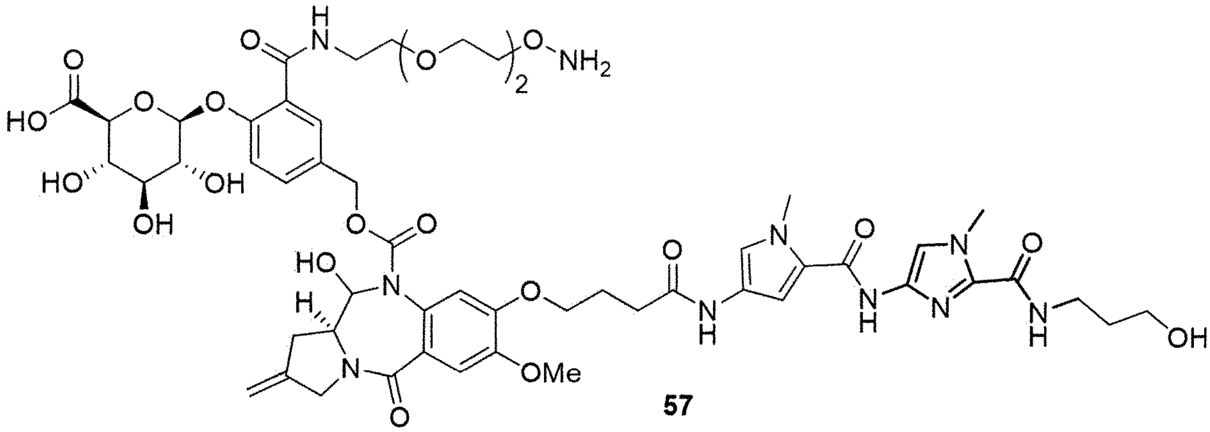

57

Compound 57 was synthesized from Compound 10 and Compound 56 in a similar manner to the synthesis of Compound 23. $^1$H-NMR (400 MHz, Methanol-$d_4$) δ 7.76 (brs, 1H), 7.38 (s, 1H), 7.29-7.26 (m, 2H), 7.16 (d, *J* =10 Hz, 2H), 6.81 (brs, 1H), 6.75 (brs, 1H), 5.58 (m, 1H), 5.27 (d, *J* =11.6 Hz, 1H), 5.15 (m, 3H), 4.24-4.09 (m, 4H), 4.09 (s, 1H), 4.00 (m, 4H), 3.87-3.79 (m, 9H), 3.68-3.55 (m, 14H), 3.44 (t, *J* =6.8 Hz, 2H), 2.98 (m, 1H), 2.63 (m, 1H), 2.50 (m, 2H), 2.11 (m, 2H), 1.80 (m, 2H), EI-MS m/z : $[M+H]^+$1181.44, $[M/2+H]^+$591.51.

### <Example 22> Preparation of Compound 60

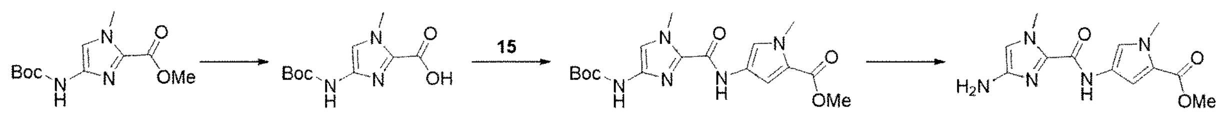


48 58 59 60

### Preparation of Compound 58

After tetrahydrofuran (150 mL) was added to Compound 48 (7.9 g, 31.2 mmol), sodium hydroxide (12.5 g, 312 mmol) dissolved in distilled water (150 mL) was added at 0°C under a nitrogen atmosphere, and the mixture was stirred at room temperature for 12 hours. After the reaction solvent was concentrated, the reaction mixture was acidified (pH of about 2) with 6 N aqueous hydrochloric acid solution, followed by extraction with ethyl acetate (2 × 500 mL). The collected organic layers were dried over anhydrous sodium sulfate, then filtered and concentrated. Acetone (100 mL) was added, followed by stirring, and the produced solid was filtered, thereby obtaining Compound 58 (5.6 g, 74%). $^1$H-NMR(400 MHz,

$CDCl_3$) δ 9.63 (s, 1H), 7.25 (s, 1H), 3.86 (s, 3H), 1.46 (s, 9H).

### Preparation of Compound 59

Compound 58 (686 mg, 2.84 mmol) and Compound 15 (570 mg, 3.69 mmol) were dissolved in *N,N*-dimethylformamide (9 mL), and then *N,N,N',N'*-tetramethyl-*O*-(1*H*-benzotriazol-1-yl)uronium hexafluorophosphate (HBTU, 1.61 g, 4.26 mmol) and *N,N'*-diisopropylethylamine (0.99 mL, 5.68 mmol) were gradually added at 0°C under a nitrogen atmosphere. After the reaction temperature was raised to room temperature, the mixture was stirred for 3 hours. Saturated aqueous ammonium chloride solution (10 mL) was added to the reaction mixture, followed by extraction with ethyl acetate (20 mL × 3), and the collected organic layers were washed with saturated sodium hydrogen carbonate solution (10 mL) and distilled water (10 mL) and then dried over anhydrous sodium sulfate. After filtration and concentration, the product was purified by column chromatography, thereby obtaining Compound 59 (1.0 g, 93%). $^1$H-NMR(400 MHz, $CDCl_3$) δ 8.77 (brs, 1H), 7.41 (d, *J* =1.6 Hz, 1H), 7.14 (brs, 1H), 6.90 (brs, 1H), 6.78 (brs, 1H), 4.04 (s, 3H), 3.91 (s, 3H), 3.81 (s, 3H), 1.51 (s, 9H), EI-MS m/z : $[M+H]^+$378.16.

### Preparation of Compound 60

Compound 59 (260 mg, 0.68 mmol) was dissolved in dichloromethane (4.8 mL), and then hydrochloric acid (4 M in 1,4-dioxane, 2.4 mL, 9.63 mmol) was gradually added at 0°C under a nitrogen atmosphere. After the temperature was raised to room temperature, the mixture was stirred for 24 hours, and the reaction mixture was basified (pH of about 12) with saturated aqueous sodium carbonate solution. This solution was subjected to extraction with dichloromethane (20 mL × 3), followed by drying over anhydrous sodium sulfate. After filtration and concentration, the product was purified by column chromatography, thereby obtaining Compound 60 (70 mg, 36%). $^1$H-NMR(400 MHz, $CDCl_3$) δ 8.11 (s, 1H), 7.40 (d, *J* =1.6 Hz, 1H), 6.78 (d, *J* =2 Hz, 1H), 6.31 (s, 1H), 3.99 (s, 3H), 3.90 (s, 3H), 3.80 (s, 3H), EI-MS m/z : $[M+H]^+$278.23.

### <Example 23> Preparation of Compound 61

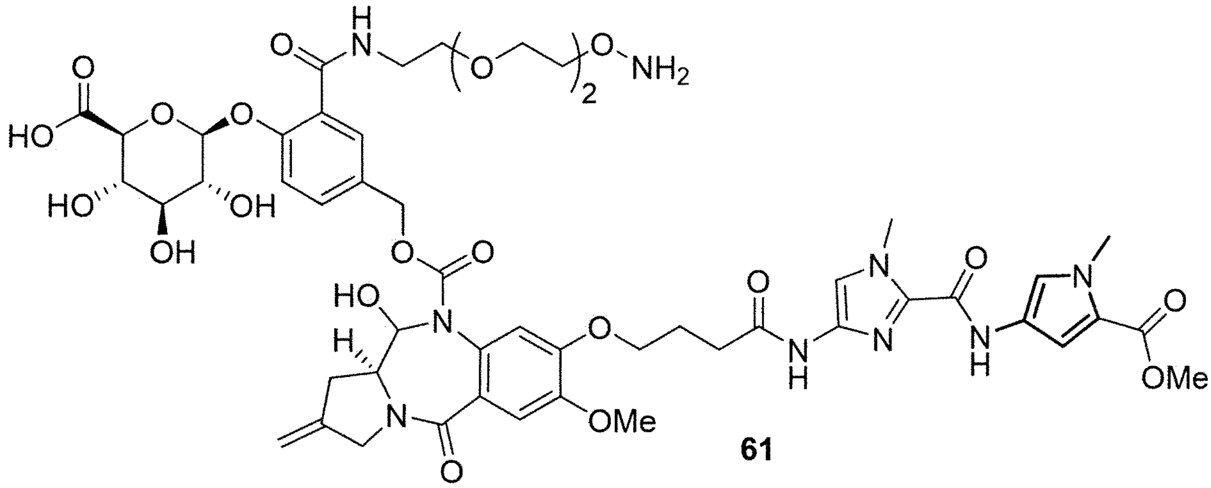

61

Compound 61 was synthesized from Compound 10 and Compound 60 in a similar manner to the synthesis of Compound 23. $^1$H-NMR (400 MHz, DMSO-$d_6$) $\delta$ 10.22 (brs, 1H), 10.07 (brs, 1H), 8.28 (brs, 1H), 7.74 (brs, 1H), 7.50 (s, 1H), 7.45 (s, 1H), 7.37 (m, 1H), 7.23 (m, 1H), 7.04 (s, 1H), 6.98 (s, 1H), 6.71 (brs, 1H), 5.35 (m, 1H), 5.19-5.13 (m, 4H), 4.84 (m, 1H), 4.10-3.73 (m, 18H), 3.62 (brs, 3H), 3.54-3.51 (m, 6H), 2.00 (m, 1H), EI-MS m/z : $[M+H]^+$1138.36, $[M/2+H]^+$569.85.

**<Example 24> Preparation of Compound 64**

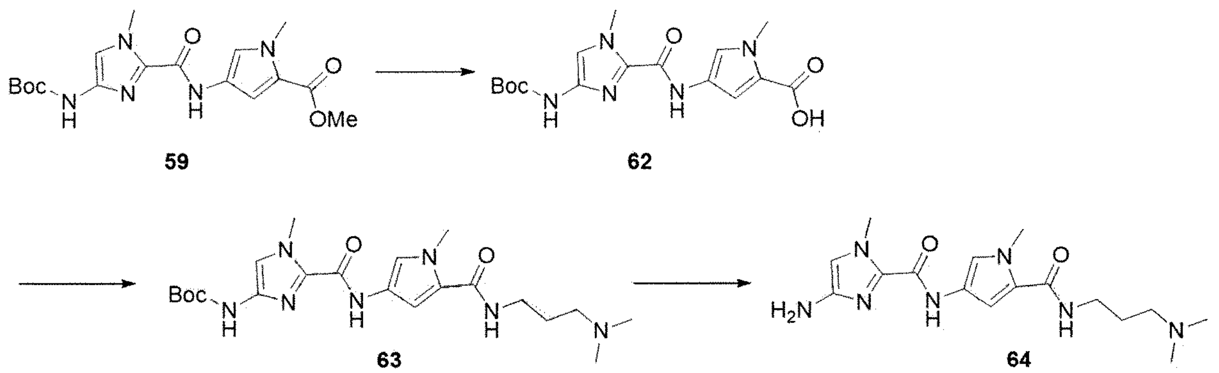

59 62 63 64

**Preparation of Compound 62**

Compound 59 (200 mg, 0.52 mmol) was dissolved in methanol (3 mL), then a solution of lithium hydroxide (63.5 mg, 1.58 mmol) in distilled water (3 mL) was gradually added at room temperature under a nitrogen atmosphere, and the mixture was heated to 80°C and stirred for 1 hour. The reaction mixture was diluted with ethyl acetate (20 mL) and then acidified with 1 N aqueous hydrochloric acid (pH of about 4), followed by extraction with ethyl acetate (20 mL × 3). The collected organic layers were dried over anhydrous sodium sulfate, filtered and concentrated under reduced pressure, thereby obtaining Compound 62 (166 mg). $^1$H-NMR (400 MHz, DMSO-$d_6$) $\delta$ 10.06 (brs, 1H), 9.34 (br s, 1H), 7.43 (s, 1H), 7.20 (br s, 1H), 6.91 (br s, 1H), 3.92 (s, 3H), 3.82 (s, 3H), 1.45 (s, 9H), EI-MS m/z : $[M+H]^+$364.28.

**Preparation of Compound 63**

Compound 62 (166 mg, 0.45 mmol) was dissolved in *N,N*-dimethylformamide (5 mL), and then *N,N,N',N'*-tetramethyl-*O*-(1*H*-benzotriazol-1-yl)uronium hexafluorophosphate (HBTU, 259 mg, 0.68 mmol) and *N,N'*-diisopropylethylamine (0.16 mL, 0.91 mmol) were added at 0°C under a nitrogen atmosphere. After *N,N'*-dimethyl-1,3-propanediamine (0.11 mL, 0.91 mmol) was added to the reaction solution, the temperature was raised to room temperature, and the mixture was stirred for 18 hours. The reaction mixture was diluted with saturated aqueous **ammonium chloride solution** (15 mL), followed by extraction with ethyl acetate (20 mL × 3), and the collected organic layers were washed

with saturated aqueous sodium hydrogen carbonate solution (20 mL), distilled water (20 mL), and aqueous sodium chloride solution (20 mL) in that order. Drying over anhydrous sodium sulfate, filtration, concentration, and purification by column chromatography were performed, thereby obtaining Compound 63 (188 mg, 92%). $^1$H-NMR(400 MHz, $CDCl_3$) δ 8.72 (s, 1H), 7.73 (br s, 1H), 7.19 (d, *J* =1.2 Hz, 1H), 7.13 (br s, 1H), 6.85 (br s, 1H), 6.43 (d, *J* =1.6 Hz, 1H), 4.04 (s, 3H), 3.92 (s, 3H), 3.46 (m, 2H), 2.46 (t, *J* =5.6 Hz, 2H), 1.71 (m, 2H), EI-MS m/z : $[M+H]^+$448.27, $[M/2+H]^+$224.93.

**Preparation of Compound 64**

Compound 63 (188 mg, 0.42 mmol) was diluted with dichloromethane (3.2 mL), then trifluoroacetic acid (1.6 mL) was added at 0°C under a nitrogen atmosphere, and the mixture was stirred for 3 hours. The reaction mixture was concentrated under nitrogen gas, thereby obtaining Compound 64 (145 mg). $^1$H-NMR (400 MHz, DMSO-$d_6$) δ 10.32 (s, 1H), 9.29 (br s, 1H), 8.18 (t, *J* =5.2 Hz, 1H), 7.23 (s, 2H), 7.03 (s, 1H), 3.96 (s, 3H), 3.81 (s, 3H), 3.24 (m, 2H), 3.06 (m, 2H), 2.79 (m, 6H), 1.83 (m, 2H), EI-MS m/z : $[M+H]^+$348.34, $[M/2+H]^+$174.75.

**<Example 25> Preparation of Compound 65**

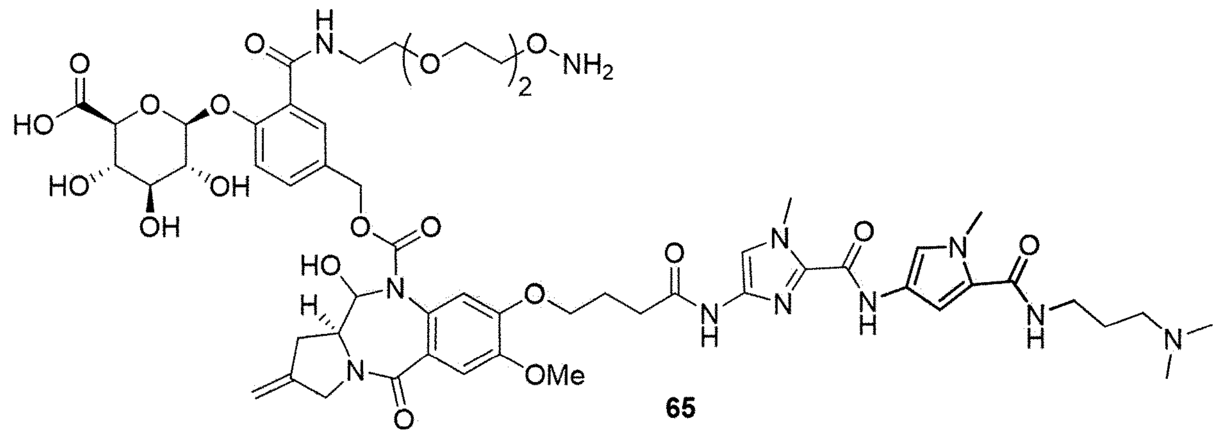

**65**

Compound 65 was synthesized from Compound 10 and Compound 64 in a similar manner to the synthesis of Compound 23. $^1$H-NMR (400 MHz, Methanol-$d_4$) δ 7.77 (br s, 1H), 7.40 (s, 1H), 7.35-7.28 (m, 2H), 7.24 (s, 1H), 7.14 (s, 1H), 6.92 (s, 1H), 6.70 (s, 1H), 5.56 (d, *J* =9.6 Hz, 1H), 5.30 (d, J =9.6 Hz, 1H), 5.17 (m, 3H), 4.80 (d, *J* =11.6 Hz, 1H), 4.25-4.01 (m, 6H), 4.03-3.95 (m, 5H), 3.89 (s, 3H), 3.83 (s, 3H), 3.74 (m, 2H), 3.67-3.54 (m, 14H), 3.41 (t, *J* =6 Hz, 2H), 3.16 (t, *J* =7.6 Hz, 2H), 2.66 (m, 1H), 2.53 (m, 2H), 2.11 (m, 2H), 1.99 (m, 2H), EI-MS m/z : $[M+H]^+$1208.51, $[M/2+H]^+$605.05.

**<Example 26> Preparation of Compound 67**

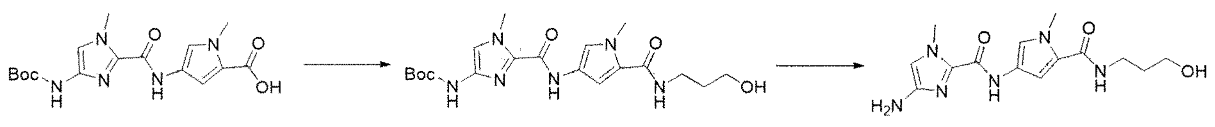

**62** **66** **67**

**Preparation of Compound 66**

Compound 62 (240 mg, 0.66 mmol) was dissolved in *N,N*-dimethylformamide (3 mL), and then *N,N,N',N'*-tetramethyl-*O*-(1*H*-benzotriazol-1-yl)uronium hexafluorophosphate (HBTU, 376 mg, 0.99 mmol) and *N,N'*-diisopropylethylamine (0.34 mL, 1.98 mmol) were added at 0°C under a nitrogen atmosphere. After 3-amino-1-propanol (0.15 mL, 1.98 mmol) was added to the reaction solution, the temperature was raised to room temperature, and the mixture was stirred for 18 hours. The reaction mixture was diluted with saturated aqueous ammonium chloride solution (15 mL), followed by extraction with ethyl acetate (20 mL × 3), and the collected organic layers were washed with saturated aqueous sodium hydrogen carbonate solution (20 mL), distilled water (20 mL), and aqueous sodium chloride solution

(20 mL) in that order. Drying over anhydrous sodium sulfate, filtration, concentration, and purification by column chromatography were performed, thereby obtaining Compound 66 (242 mg, 87%). $^1$H-NMR(400 MHz, $CDCl_3$) δ 8.80 (br s, 1H), 7.14 (br s, 2H), 7.06 (br s, 1H), 6.56 (s, 1H), 6.27 (br s, 1H), 4.03 (s, 3H), 3.89 (s, 3H), 3.69 (m, 2H), 3.53 (m, 2H), 3.26 (br s, 1H), 1.51 (s, 9H), EI-MS m/z : $[M+H]^+$421.33.

**Preparation of Compound 67**

Compound 66 (100 mg, 0.23 mmol) was diluted with dichloromethane (1 mL), then trifluoroacetic acid (1 mL) and distilled water (0.2 mL) were added at 0°C under a nitrogen atmosphere, and the mixture was stirred for 1 hour. The reaction mixture was basified with sodium carbonate (pH of about 8) and subjected to extraction with dichloromethane (20 mL × 3). The collected organic layers were dried over anhydrous sodium sulfate, filtered and concentrated, thereby obtaining Compound 67 (46 mg). $^1$H-NMR (400 MHz, DMSO-$d_6$) δ 9.90 (s, 1H), 7.95 (t, $J$ =5.6 Hz, 1H), 7.16 (s, 1H), 6.90 (s, 1H), 6.37 (s, 1H), 4.47 (m, 1H), 4.38 (br s, 2H), 3.83 (s, 3H), 3.76 (s, 3H), 3.36 (m, 2H), 3.21 (m, 2H), 1.60 (m, 2H), EI-MS m/z : $[M+H]^+$321.30, $[M/2+H]^+$159.08.

**<Example 27> Preparation of Compound 68**

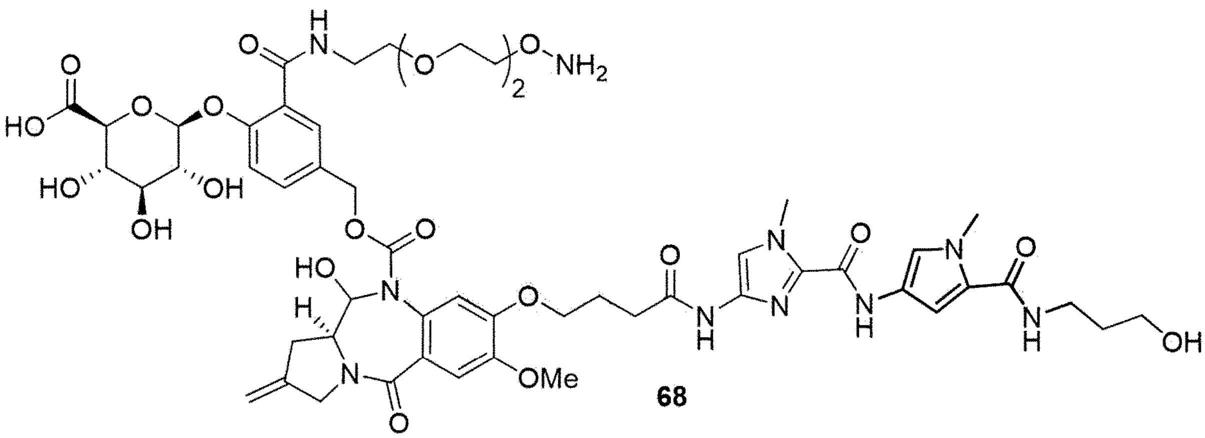

68

Compound 68 was synthesized from Compound 10 and Compound 67 in a similar manner to the synthesis of Compound 23. EI-MS m/z : $[M+H]^+$1181.37, $[M/2+H]^+$591.49.

**<Example 28> Preparation of Compound 75**

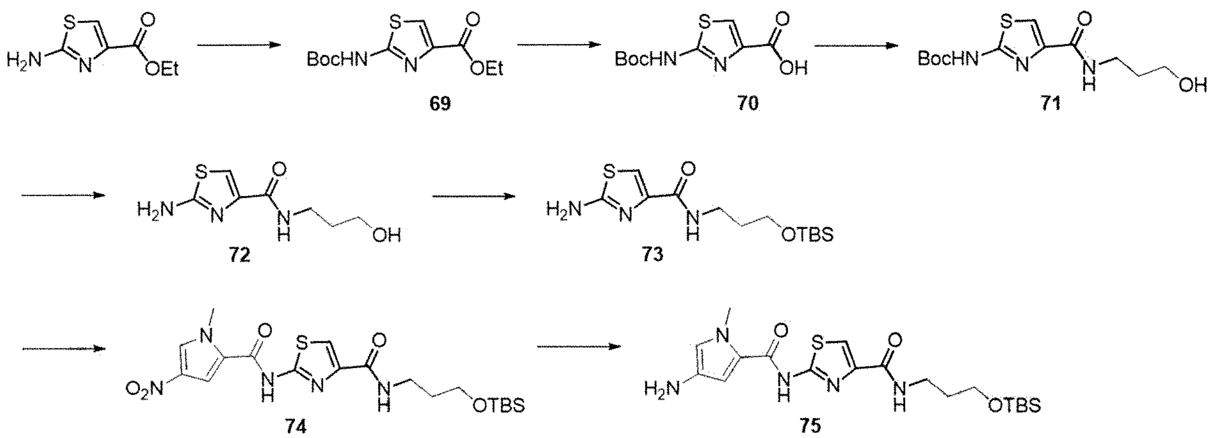

69 70 71

72 73

74 75

**Preparation of Compound 69**

After ethyl 2-aminothiazole-4-carboxylate (1.0 g, 5.81 mmol) was dissolved in dichloromethane (7 mL), 4-dimethylaminopyridine (14.0 mg, 0.12 mmol) and di-*t*-butyl dicarbonate (1.76 mL, 7.55 mmol) were added under a nitrogen atmosphere, and the mixture was stirred at room temperature for 18 hours. The reaction mixture was diluted with dichloromethane (50 mL), washed with distilled water (50 mL) and brine (50 mL), and dried over anhydrous sodium sulfate. The collected organic layers were dried over anhydrous sodium sulfate, filtered and concentrated, thereby obtaining Compound 69 (1.7 g, crude). EI-MS m/z : $[M+H]^+$273.21.2, $[M+H]^+$545.17.

**Preparation of Compound 70**

Compound 69 (1.7 g, 5.81 mmol) was dissolved in tetrahydrofuran (6 mL) and methanol (6 mL), then a solution of lithium hydroxide (697 mg, 17.3 mmol) in distilled water (1 mL) was added under a nitrogen atmosphere, and the mixture was stirred for 3 hours. The organic solvent was concentrated under reduced pressure, then the resultant was diluted with distilled water (50 mL) and adjusted to have a pH of about 4 with 6 N aqueous hydrochloric acid solution, and the produced solid was filtered, thereby obtaining Compound 70 (1.4 g, 99%). $^1$H-NMR(400 MHz, DMSO-$d_6$) $\delta$ 12.80 (br s, 1H), 11.70 (s, 1H), 7.91 (s, 1H), 1.48 (s, 9H).

**Preparation of Compound 71**

Compound 70 (700 mg, 2.87 mmol) was dissolved in dichloromethane (20 mL), then 1-ethyl-3-(3-dimethylaminopropyl)carbodiimide (668 mg, 4.30 mmol) and 1-hydroxybenzotriazole (581 mg, 4.30 mmol) were added under a nitrogen atmosphere, and the mixture was stirred at room temperature for 1 hour. After triethylamine (0.59 mL, 4.30 mmol) and 3-amino-1-propanol (0.65 mL, 8.60 mmol) were added to the reaction solution, the mixture was stirred at room temperature for 18 hours. The reaction mixture was washed with saturated aqueous sodium hydrogen carbonate solution (20 mL) and brine (20 mL). The collected organic layers were dried over anhydrous sodium sulfate, then filtered, concentrated, and purified by column chromatography, thereby obtaining Compound 71 (520 mg, 60%). $^1$H-NMR(400 MHz, DMSO-$d_6$) $\delta$ 11.59 (s, 1H), 7.75 (t, *J* = 6 Hz, 1H), 7.69 (s, 1H), 4.52 (s, 1H), 3.45 (s, 2H), 3.30 (s, 2H), 1.64 (m, 2H), 1.48 (s, 9H).

**Preparation of Compound 72**

Compound 71 (260.0 mg, 0.86 mmol) was diluted with dichloromethane (12 mL), then trifluoroacetic acid (3 mL) was added under a nitrogen atmosphere, and the mixture was stirred at room temperature for 2 hours. After addition of distilled water (10 mL), the reaction mixture was adjusted to have a pH of about 11 with 2 N aqueous sodium hydroxide solution, followed by extraction with dichloromethane (10 mL $\times$ 5). The collected organic layers were dried over anhydrous sodium sulfate, filtered and concentrated, thereby obtaining Compound 72 (170 mg, 98%). EI-MS m/z : $[M+H]^+$202.19.

**Preparation of Compound 73**

Compound 72 (490 mg, 0.86 mmol) was dissolved in *N,N*-dimethylformamide (6 mL), then imidazole (250 mg, 3.66 mmol) and t-butyldimethylsilyl chloride (440 mg, 2.93 mmol) were added at 0°C under a nitrogen atmosphere, and the mixture was stirred at room temperature for 2 hours. After addition of ethyl acetate (20 mL), the reaction mixture was washed with aqueous sodium hydroxide solution (20 mL) having a pH of about 11. The collected organic layers were dried over anhydrous sodium sulfate, then filtered, concentrated, and purified by column chromatography, thereby obtaining Compound 73 (430 mg, 80%). $^1$H-NMR(400 MHz, $CDCl_3$) $\delta$ 7.42 (s, 1H), 7.33 (s, 1H), 4.91 (s, 2H), 3.75 (t, *J* = 5.6 Hz, 2H), 3.50 (q, *J* = 6.4 Hz, 2H), 1.80 (m, 2H), 0.91 (s, 9H), 0.08 (s, 6H).

**Preparation of Compound 74**

Compound 73 (210.0 mg, 1.24 mmol) was dissolved in thionyl chloride (6 mL) and heated under reflux for 2 hours. The reaction solution was concentrated under reduced pressure, dissolved again in dichloromethane, and then concentrated under reduced pressure several times. The produced compound was dissolved in *N,N*-dimethylformamide (2 mL), then *N,N'*-diisopropylethylamine (1.0 mL, 6.20 mmol) and Compound 14 (430 mg, 1.36 mmol) were sequentially added at 0°C under a nitrogen atmosphere, and the mixture was heated at 100°C for 3 hours. After addition of ethyl acetate (20 mL), the reaction mixture was washed with distilled water (20 mL). The collected organic layers were dried over anhydrous sodium sulfate, then filtered, concentrated, and purified by column chromatography, thereby obtaining Compound 74 (130 mg, 23%). $^1$H-NMR(400 MHz, $CDCl_3$) $\delta$ 9.05 (s, 1H) 7.79 (s, 1H), 7.70 (s, 1H), 7.55 (s, 1H), 7.36 (s,

1H), 4.09 (s, 3H), 3.79 (t, *J* = 5.6 Hz, 2H), 3.57 (m, 2H), 1.83 (m, 2H), 0.95 (s, 9H), 0.13 (s, 6H).

**Preparation of Compound 75**

Compound 74 (130 mg, 0.27 mmol) was dissolved in methanol (3 mL) and ethyl acetate (3 mL), and then palladium/charcoal (10% w/w, 60 mg) was added. The reaction solution was stirred at room temperature for 18 hours under a hydrogen atmosphere. The reaction mixture was filtered through Celite and then concentrated, thereby obtaining Compound 75 (115 mg, 97%). EI-MS m/z : $[M+H]^+$438.37.

**<Example 29> Preparation of Compound 76**

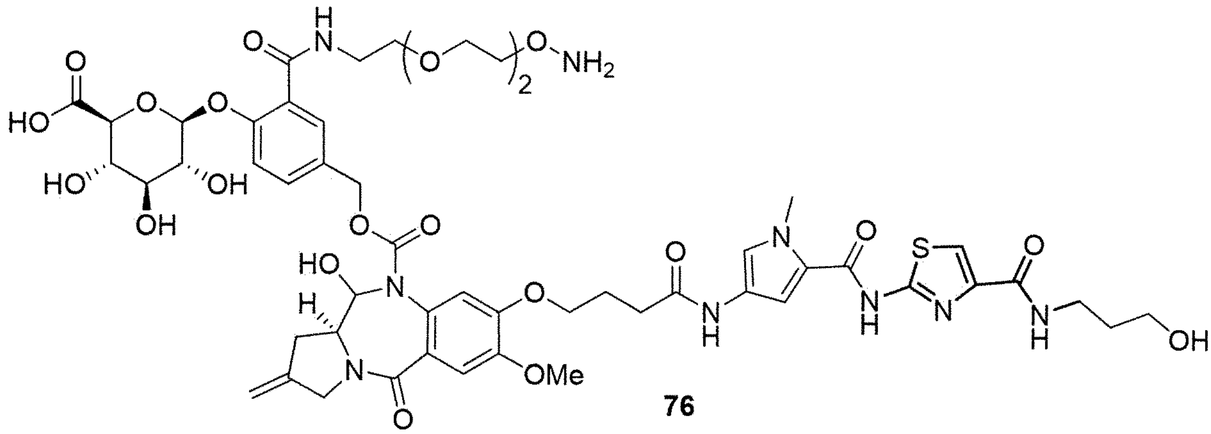

**76**

Compound 76 was synthesized from Compound 10 and Compound 75 in a similar manner to the synthesis of Compound 29. EI-MS m/z : $[M+H]^+$1184.49, $[M/2+H]^+$592.85.

**<Example 30> Preparation of Compound 84**

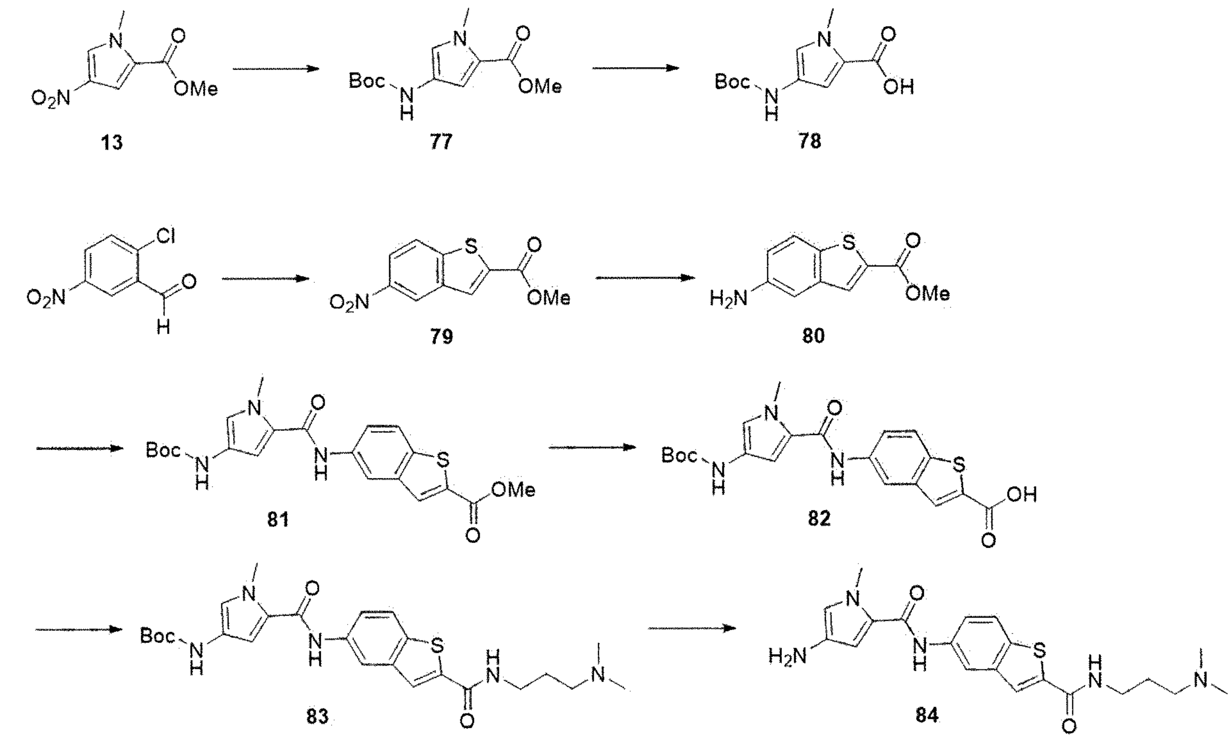

13 77 78 79 80 81 82 83 84

## Preparation of Compound 77

Compound 13 (5.5 g, 29.8 mmol) was dissolved in methanol (100 mL) and ethyl acetate (100 mL), and then palladium/charcoal (10% w/w, 1.1 g) was added at 0°C under a nitrogen atmosphere. After sodium borohydride ($NaBH_4$, 3.4 g, 89.6 mmol) was added to distilled water (100 mL), then this solution was gradually added to the reaction solution using a dropping funnel, and the mixture was stirred at room temperature for 3 hours. The reaction mixture was filtered through Celite, followed by dilution with dichloromethane (100 mL), washing with distilled water (100 mL), and drying over anhydrous sodium sulfate. Filtration, concentration and dilution with dichloromethane (450 mL) were performed. To this solution, di-*t*-butyl dicarbonate (9.4 g, 43.1 mmol) dissolved in dichloromethane (300 mL) was added. The reaction solution was heated under reflux and stirred for 18 hours. The reaction mixture was washed with distilled water (300 mL) and dried over anhydrous sodium sulfate. Filtration, concentration, and purification by column chromatography were performed, thereby obtaining Compound 77 (6.0 g, 80%). $^1$H-NMR(400 MHz, $CDCl_3$) δ 7.08 (s, 1H), δ 6.60 (s, 1H), δ 6.21 (s, 1H), 3.86 (s, 3H), 3.79 (s, 3H), 1.49 (s, 9H).

## Preparation of Compound 78

Compound 77 (3.7 g, 14.51 mmol) was diluted with 1,4-dioxane (30 mL), and then sodium hydroxide (1.1 g, 29.0 mmol) dissolved in distilled water (15 mL) was added at 0°C under a nitrogen atmosphere. The solution was stirred for 3 hours while being heated at 70°C. After the reaction solvent was concentrated, the pH was adjusted to about 2 with 6 N aqueous hydrochloric acid solution, followed by dilution with ethyl acetate (100 mL), washing with distilled water (50 mL), and drying over anhydrous sodium sulfate. Filtration and concentration were performed, thereby obtaining Compound 78 (3.4 g, 97%). $^1$H-NMR (400 MHz, $CDCl_3$) δ 7.18 (s, 1H), δ 6.72 (s, 1H), δ 6.28 (s, 1H), 3.86 (s, 3H), 1.50 (s, 9H).

## Preparation of Compound 79

After 2-chloro-5-nitrobenzaldehyde (3.7 g, 19.9 mmol) was dissolved in *N,N*-dimethylformamide (40 mL), methyl mercaptoacetate (1.82 mL, 19.9 mmol) and potassium carbonate (3.3 g, 24 mmol) were added, and the mixture was stirred at room temperature for 18 hours. Distilled water (50 mL) was added to the reaction mixture, and the produced solid was filtered, thereby obtaining Compound 79 (4.65 g, 95%). $^1$H-NMR(400 MHz, DMSO-$d_6$) δ 9.00 (d, *J* = 1.6 Hz, 1H), 8.45 (s, 1H), 8.38-8.30 (m, 2H), 3.93 (s, 3H) .

**Preparation of Compound 80**

Compound 79 (1.6 g, 6.74 mmol) was dissolved in methanol (50 mL), and then palladium/charcoal (10% w/w, 312 mg) was added. The reaction solution was stirred at room temperature for 1.5 hours under a hydrogen atmosphere. The reaction mixture was filtered through Celite and then concentrated, thereby obtaining Compound 80 (1.23 g, 81%). $^1$H-NMR (400 MHz, DMSO-$d_6$) δ 7.92 (s, 1H), 7.65 (d, *J* = 8.8 Hz, 1H), 7.04 (s, 1H), 6.88 (d, *J* = 8.8 Hz, 1H), 5.28 (s, 2H), 3.84 (s, 3H).

**Preparation of Compound 81**

Compound 78 (564 mg, 2.35 mmol) and Compound 80 (444 mg, 2.14 mmol) were dissolved in *N,N*-dimethylformamide (20 mL), then 1-ethyl-3-(3-dimethylaminopropyl)carbodiimide (821 mg, 4.28 mmol) and 4-dimethylaminopyridine (645 mg, 5.35 mmol) were added at 0°C under a nitrogen atmosphere, and the mixture was stirred at room temperature for 16 hours. After addition of ethyl acetate (30 mL), the reaction mixture was washed with saturated aqueous ammonium chloride solution (30 mL), saturated aqueous sodium hydrogen carbonate solution (30 mL) and distilled water (30 mL) in that order. The collected organic layers were dried over anhydrous sodium sulfate, filtered and then concentrated. Recrystallization from diethyl ether and hexane was performed, thereby obtaining Compound 81 (770 mg, 84%). $^1$H-NMR(400 MHz, DMSO-$d_6$) δ 9.99 (s, 1H), 9.15 (s, 1H), 8.96 (d, *J* = 1.6 Hz, 1H), 8.18 (s, 1H), 7.98-7.96 (m, 1H), 7.80-7.78 (m, 1H), 6.98 (d, *J* = 7.6 Hz, 2H), 3.88 (s, 3H), 3.81 (s, 3H), 1.46 (s, 9H). EI-MS m/z : $[M+H]^+$430.1.

**Preparation of Compound 82**

Compound 81 (270 mg, 0.627 mmol) was dissolved in methanol (10 mL), and then a solution of sodium hydroxide (250 mg, 6.26 mmol) in distilled water (10 mL) was gradually added under a nitrogen atmosphere. The mixture was stirred for 4 hours under reflux. The reaction mixture was adjusted to have a pH of about 3 with 1 N aqueous hydrochloric acid solution, followed by extraction with dichloromethane, drying over anhydrous sodium sulfate, and concentration under reduced pressure, thereby obtaining Compound 82 (227 mg, 87%). $^1$H-NMR (400 MHz, DMSO-$d_6$) δ 9.96 (s, 1H), 9.14 (s, 1H), 8.45 (s, 1H), 8.06 (s, 1H), 7.95-7.93 (m, 1H), 7.77-7.75 (m, 1H), 6.98 (d, *J* = 7.6 Hz, 2H), 3.83 (s, 3H), 1.46 (s, 9H).

**Preparation of Compound 83**

Compound 82 (227 mg, 0.54 mmol) and 3-(dimethylamino)-1-propylamine (0.75 mL, 0.59 mmol) were dissolved in *N,N*-dimethylformamide (10 mL), then 1-ethyl-3-(3-dimethylaminopropyl)carbodiimide (209 mg, 1.09 mmol) and 4-dimethylaminopyridine (167 mg, 1.36 mmol) were added at 0°C under a nitrogen atmosphere, and the mixture was stirred at room temperature for 16 hours. After addition of ethyl acetate (10 mL), the reaction mixture was washed with saturated aqueous ammonium chloride solution (10 mL), saturated aqueous sodium hydrogen carbonate solution (10 mL) and distilled water (10 mL) in that order. The collected organic layers were dried over anhydrous sodium sulfate, filtered and concentrated, thereby obtaining Compound 83 (280 mg). EI-MS m/z : $[M+H]^+$500.23.

**Preparation of Compound 84**

Compound 83 (170 mg, 0.34 mmol) was dissolved in dichloromethane (2 mL), hydrochloric acid (4 M 1,4-dioxane solution, 2 mL) was added, and the mixture was stirred at room temperature for 1 hour. The reaction mixture was concentrated under reduced pressure, thereby obtaining Compound 84 (160 mg). EI-MS m/z : $[M+H]^+$400.3.

**<Example 31> Preparation of Compound 85**

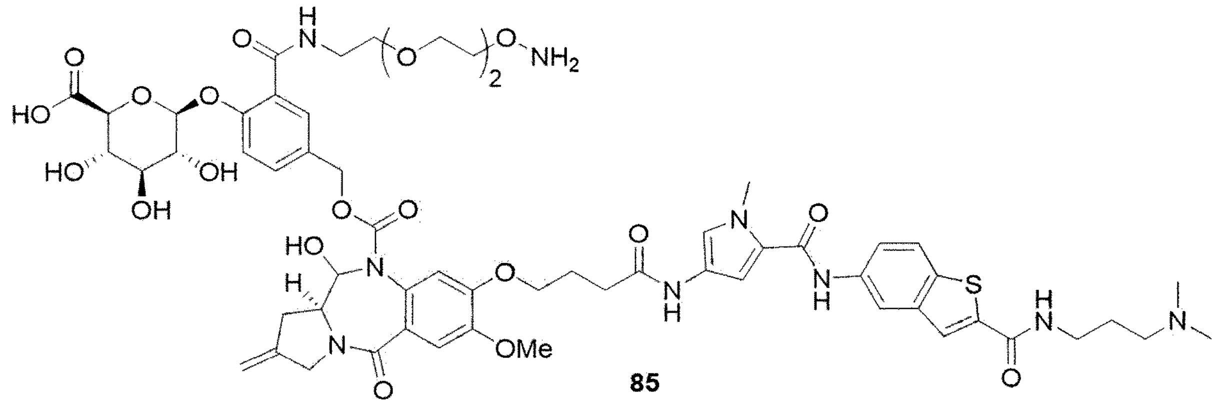

85

Compound 85 was synthesized from Compound 10 and Compound 84 in a similar manner to the synthesis of Compound 23. EI-MS m/z : [M+H]+ 1260.41, [M/2+H]+ 631.1.

**<Example 32> Preparation of Compound 87**

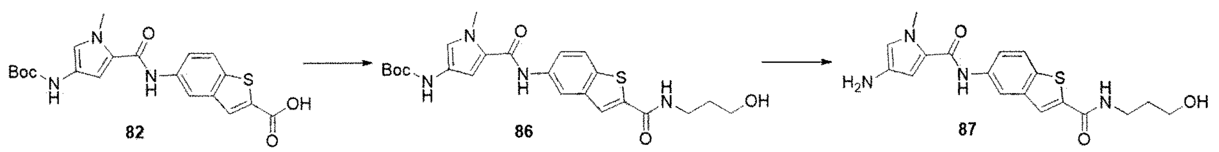

82 86 87

**Preparation of Compound 86**

Compound 82 (253 mg, 0.609 mmol) and 3-amino-1-propanol (0.07 mL, 0.913 mmol) were dissolved in *N,N*-dimethylformamide (5 mL), then 1-ethyl-3-(3-dimethylaminopropyl)carbodiimide (233 mg, 1.21 mmol) and 4-dimethylaminopyridine (186 mg, 1.52 mmol) were added at 0°C under a nitrogen atmosphere, and the mixture was stirred at room temperature for 16 hours. After addition of ethyl acetate (10 mL), the reaction mixture was washed with saturated aqueous ammonium chloride solution (10 mL), saturated aqueous sodium hydrogen carbonate solution (10 mL) and distilled water (10 mL) in that order. The collected organic layers were dried over anhydrous sodium sulfate, filtered, and diluted with dichloromethane (10 mL) and diethyl ether (20 mL), and then the produced solid was filtered, thereby obtaining Compound 86 (123 mg, 43%). $^1$H-NMR(400 MHz, DMSO-$d_6$) $\delta$ 9.92(s, 1H), 9.14 (s, 1H), 8.68 (t, *J* = 5.6 Hz, 1H), 8.32 (s, 1H), 7.98-7.89 (m, 2H), 7.75-7.73 (m, 1H), 6.96 (s, 1H), 4.49 (t, *J* = 5.2 Hz, 1H) 3.82 (s, 3H), 3.60-3.45 (m, 2H), 3.35-3.30 (m, 2H), 1.73-1.66 (m, 2H), 1.46 (s, 9H).

**Preparation of Compound 87**

Compound 86 (123 mg, 0.26 mmol) was dissolved in dichloromethane (2 mL), hydrochloric acid (4 M 1,4-dioxane solution, 2 mL) was added, and the mixture was stirred at room temperature for 3 hours, followed by concentration under reduced pressure, washing with ethyl acetate (10 mL) and aqueous sodium carbonate solution (10 mL), and concentration to dryness over anhydrous sodium sulfate, thereby obtaining Compound 87 (97 mg). $^1$H-NMR(400 MHz, DMSO-$d_6$) $\delta$ 9.92(s, 1H), 9.14 (s, 1H), 8.79(br, 2H), 8.68 (t, *J* = 5.6 Hz, 1H), 8.32 (s, 1H), 7.98-7.89 (m, 2H), 7.75-7.73 (m, 1H), 6.96 (s, 1H), 4.49 (t, *J* = 5.2 Hz, 1H) 3.82 (s, 3H), 3.60-3.45 (m, 2H), 3.35-3.30 (m, 2H), 1.73-1.66 (m, 2H), 1.46 (s, 9H).

**<Example 33> Preparation of Compound 88**

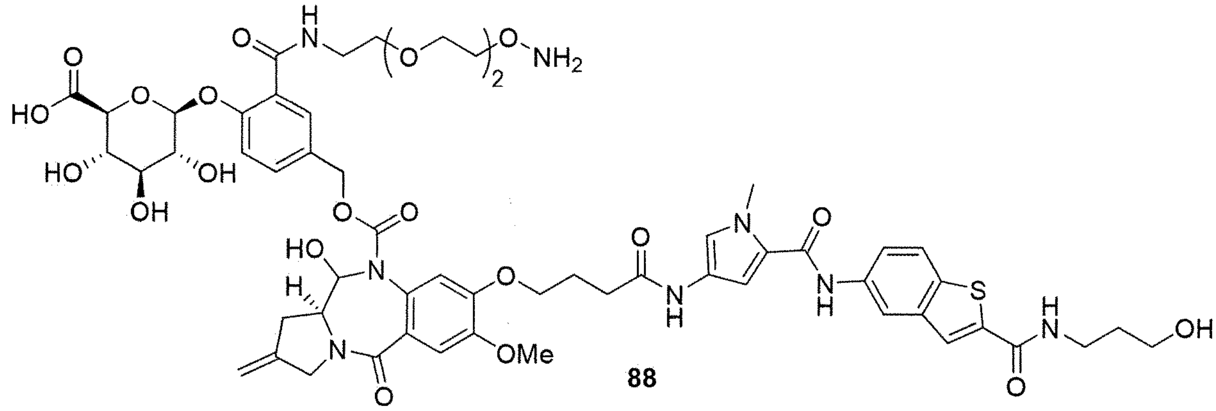

88

Compound 88 was synthesized from Compound 10 and Compound 87 in a similar manner to the synthesis of Compound 23. EI-MS m/z : $[M+H]^+$1234.41, $[M/2+H]^+$617.56.

**<Example 34> Preparation of Compound 93**

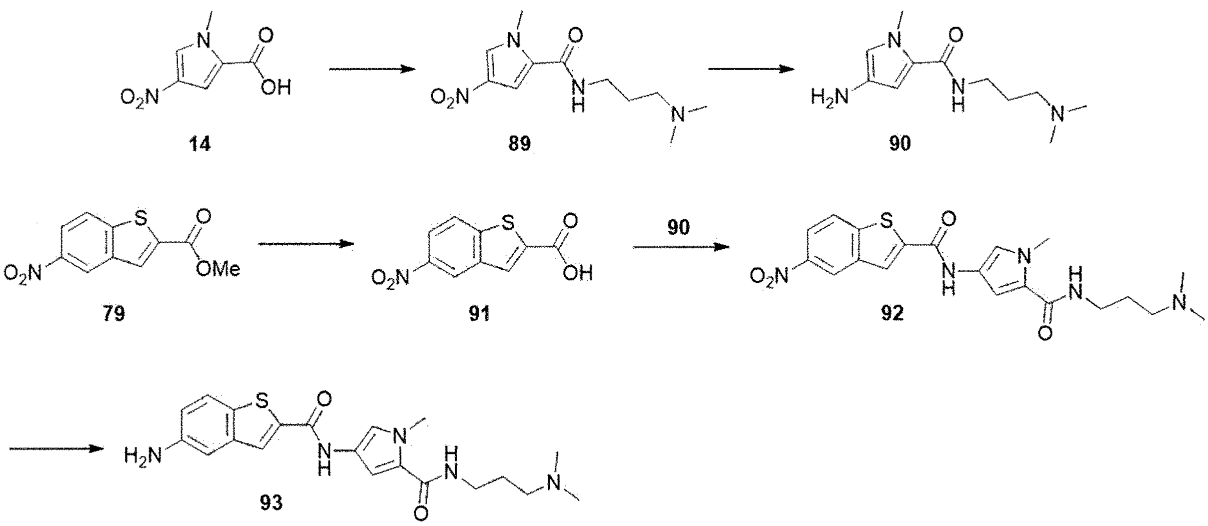


14 89 90 79 91 92 93

__Preparation of Compound 89__

To Compound 14 (300 mg, 1.72 mmol) was added thionyl chloride (2 mL), and the mixture was stirred for 30 minutes under reflux. The reaction solution was concentrated under reduced pressure, dried, and dissolved in dichloromethane (8 mL), and this solution was added to a solution of 3-(dimethylamino)-1-propylamine (466 mg, 5.29 mmol) and triethylamine (0.74 mL, 5.29 mmol) in dichloromethane (5 mL) at -50°C under a nitrogen atmosphere. The mixture was stirred for 2 hours while the reaction temperature was gradually raised to 0°C. The reaction mixture was diluted with dichloromethane (30 mL), washed with distilled water (20 mL), and dried over anhydrous sodium sulfate. Filtration and concentration were performed, thereby obtaining Compound 89 (396 mg, 94%). $^1$H-NMR(400 MHz, DMSO-$d_6$) $\delta$ 8.40 (t, 1H), 8.11 (d, 1H), 7.40 (d, 1H), 3.90 (s, 3H), 3.20 (m, 2H), 2.23 (t, 2H), 1.61 (m, 2H).

__Preparation of Compound 90__

Compound 89 (150 mg, 0.59 mmol) was dissolved in methanol (6 mL), and then palladium/charcoal (10% w/w, 80 mg) was added. The reaction solution was stirred at room temperature for 2 hours under a hydrogen atmosphere. The reaction mixture was filtered through Celite and then concentrated, thereby obtaining Compound 90 (132 mg, 100%).

**Preparation of Compound 91**

After methanol (5.5 mL) was added to Compound 79 (300 mg, 1.26 mmol), 1 M aqueous sodium hydroxide solution (5 mL)) was added at 0°C under a nitrogen atmosphere, and the mixture was heated under reflux for 3 hours. After concentration of methanol, the pH was adjusted to about 2 with 1 N aqueous hydrochloric acid solution, and the produced solid was filtered, thereby obtaining Compound 91 (248 mg, 88%). $^1$H-NMR(400 MHz, DMSO-$d_6$) δ 8.98 (d, *J* = 1.6 Hz, 1H), 8.35-8.28 (m, 3H).

**Preparation of Compound 92**

After thionyl chloride (3 mL) was added to Compound 91 (166 mg, 0.744 mmol), the mixture was stirred at 70°C for 1 hour and concentrated under reduced pressure. After dichloromethane (5 mL) was added and dissolved, triethylamine (0.3 mL, 2.23 mmol) and Compound 90 (217 mg, 0.967 mmol) were added at 0° under a nitrogen atmosphere, and the mixture was stirred at room temperature for 3 hours. Concentration under reduced pressure, and purification by column chromatography were performed, thereby obtaining Compound 92 (189 mg, 60%). $^1$H-NMR(400 MHz, DMSO-$d_6$) δ 10.57(s, 1H), 8.96 (d, *J* = 1.6 Hz, 1H), 8.41 (s, 1H), 8.35-8.32 (m, 1H), 8.28-8.25 (m, 1H), 8.16 (t, *J* = 5.6 Hz, 1H), 7.28 (s, 1H), 6.88 (s, 1H), 3.83 (s, 3H), 3.22-3.18 (m, 2H), 2.32-2.26 (m, 2H), 2.16 (s, 6H), 1.66-1.59 (m, 2H), 1.24-1.21 (m, 1H). EI-MS m/z : $[M+H]^+$430.2.

**Preparation of Compound 93**

Compound 92 (110 mg, 0.25 mmol) was dissolved in methanol (20 mL), then palladium/charcoal (10% w/w, 110 mg) was added, and the mixture was stirred at room temperature for 1 hour under a hydrogen atmosphere. The reaction mixture was filtered through Celite and then concentrated under reduced pressure, thereby obtaining Compound 93 (102 mg, 99%). EI-MS m/z : $[M+H]^+$400.2.

**<Example 35> Preparation of Compound 94**

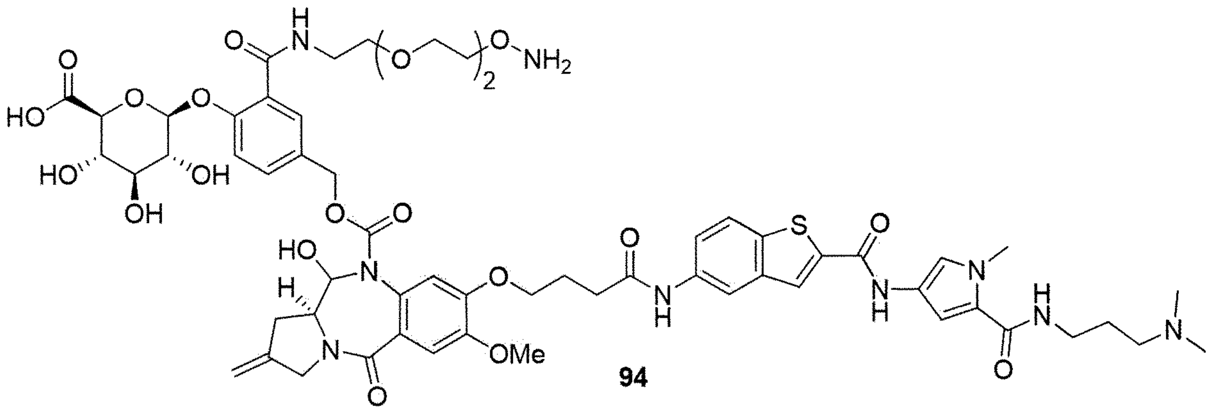

94

Compound 94 was synthesized from Compound 10 and Compound 93 in a similar manner to the synthesis of Compound 23. EI-MS m/z : $[M+H]^+$1260.61, $[M/2+H]^+$630.90.

**<Example 36> Preparation of Compound 99**

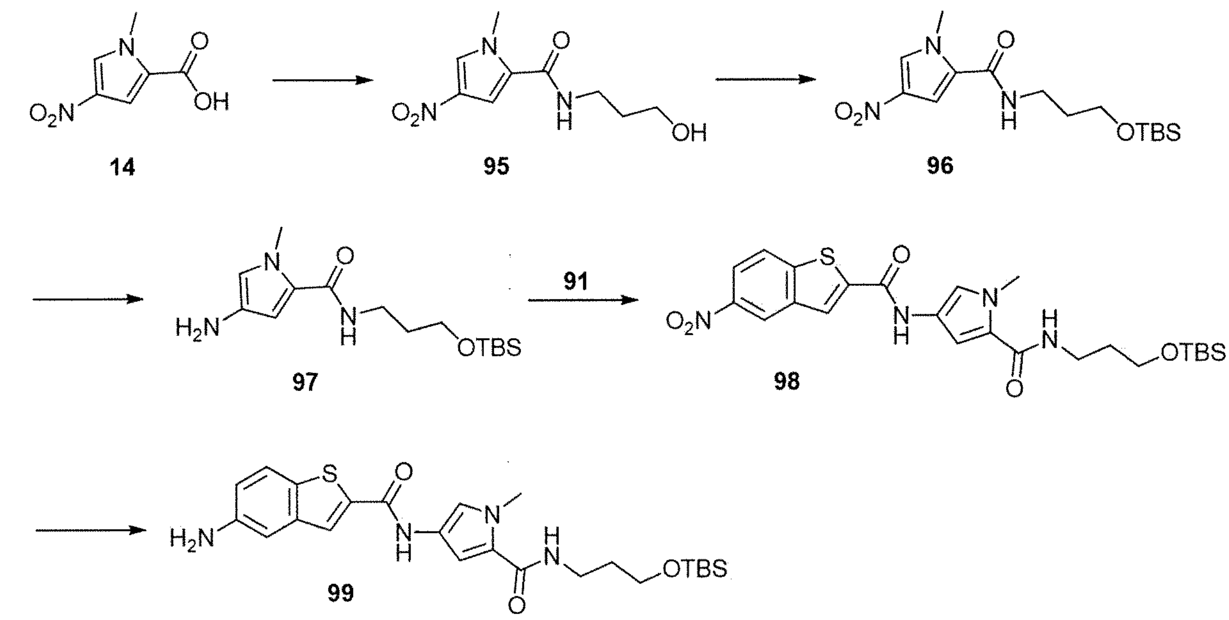


14 95 96 97 98 99

**Preparation of Compound** 95

After thionyl chloride (10 mL) was added to Compound 14 (600 mg, 3.526 mmol), the mixture was stirred at 70°C for 1 hour and concentrated under reduced pressure. After dichloromethane (15 mL) was added and dissolved, triethylamine (1.47 mL, 10.58 mmol) and 3-amino-1-propanol (0.8 mL, 10.6 mmol) were added at 0° under a nitrogen atmosphere, and the mixture was stirred at room temperature for 1 hour. After addition of dichloromethane (200 mL), the reaction mixture was washed with saturated aqueous sodium hydrogen carbonate solution (20 mL). The collected organic layers were dried over anhydrous sodium sulfate, filtered and concentrated under reduced pressure, thereby obtaining Compound 95 (527 mg, crude). $^1$H-NMR(400 MHz, $CDCl_3$) δ 7.55 (s, 1H), 7.08 (s, 1H), 6.72 (br, 1H), 3.99(s, 3H), 3.78-3.75 (m, 2H), 3.59-3.54 (m, 2H), 3.03-2.98 (m, 1H), 1.88-1.78(m, 2H).

**Preparation of Compound 96**

Compound 95 (527 mg, 2.32 mmol) was dissolved in dichloromethane (30 mL), t-butyldimethylsilyl chloride (524 mg, 3.48 mmol) and imidazole (315 mg, 4.64 mmol) were added at 0°C, and the mixture was stirred at room temperature for 16 hours. The reaction mixture was washed with saturated aqueous ammonium chloride solution (30 mL), and the organic layer was dried over anhydrous sodium sulfate. Filtration, concentration under reduced pressure, and purification by column chromatography were performed, thereby obtaining Compound 96 (660 mg, 83%). $^1$H-NMR(400 MHz, $CDCl_3$) δ 7.53 (s, 1H), 7.07 (s, 1H), 6.88 (br, 1H), 3.98 (s, 3H), 3.84-3.81 (m, 2H), 3.54-3.50 (m, 2H), 1.84-1.20 (m, 2H), 0.91 (s, 9H), 0.10 (s, 6H).

**Preparation of Compound 97**

Compound 96 (400 mg, 1.17 mmol) was dissolved in methanol (30 mL), then palladium/charcoal (10% w/w, 200 mg) was added, and the mixture was stirred at room temperature for 1 hour under a hydrogen atmosphere. The reaction mixture was filtered through Celite and then concentrated under reduced pressure, thereby obtaining Compound 97 (293 mg, 76%). $^1$H-NMR(400 MHz, $CDCl_3$) δ 7.53 (s, 1H), 7.07 (s, 1H), 6.88 (br, 1H), 6.66 (br, 2H), 3.98 (s, 3H), 3.84-3.81 (m, 2H), 3.54-3.50 (m, 2H), 1.84-1.20 (m, 2H), 0.91 (s, 9H), 0.10 (s, 6H).

**Preparation of Compound 98**

Compound 91 (100 mg, 0.446 mmol) and Compound 97 (145 mg, 0.490 mmol) were dissolved in *N,N*-dimethylformamide (5 mL), then 1-ethyl-3-(3-dimethylaminopropyl)carbodiimide (171 mg, 0.892 mmol) and 4-dimethylaminopyridine (136 mg, 1.11 mmol) were added at 0°C under a nitrogen atmosphere, and the mixture was stirred at room temperature for 16 hours. After addition of ethyl acetate (10 mL), the reaction mixture was washed with saturated aqueous ammonium chloride solution (10 mL), saturated aqueous sodium hydrogen carbonate solution (10 mL) and distilled water (10 mL) in that order. The collected organic layers were dried over anhydrous sodium sulfate, filtered, then

concentrated, and purified by column chromatography, thereby obtaining Compound 98 (117 mg, 51%). $^1$H-NMR(400 MHz, DMSO-$d_6$) δ 10.85 (2, 1H), 8.96 (s, 1H), 8.41 (s, 1H), 8.35-8.31 (m, 1H), 8.28-8.25 (m, 1H), 8.05 (t, $J$ = 5.2 Hz, 1H), 7.28 (s, 1H), 6.89 (s, 1H), 3.82 (s, 3H), 3.67-3.62 (m, 2H), 3.24-3.21 (m, 2H), 1.73-1.68 (m, 2H), 0.87 (s, 9H), 0.06 (s, 6H).

**Preparation of Compound 99**

Compound 98 (117 mg, 0.226 mmol) was dissolved in methanol (20 mL), then palladium/charcoal (10% w/w, 100 mg) was added, and the mixture was stirred at room temperature for 16 hours under a hydrogen atmosphere. The reaction mixture was filtered through Celite and then concentrated under reduced pressure, thereby obtaining Compound 99 (110 mg, crude). EI-MS m/z : [M+H]$^+$487.33.

**<Example 37> Preparation of Compound 100**

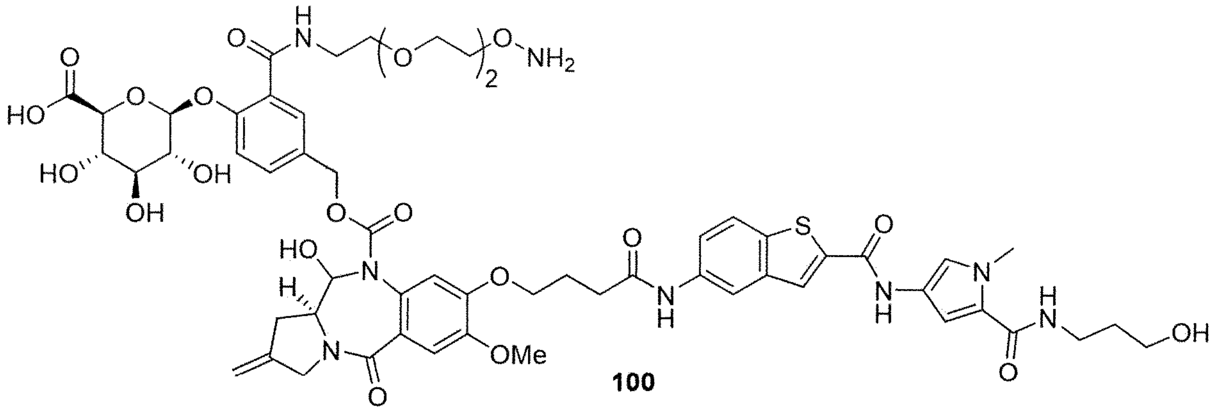

100

Compound 100 was synthesized from Compound 10 and Compound 99 in a similar manner to the synthesis of Compound 29. EI-MS m/z : [M+H]$^+$1234.41, [M/2+H]$^+$617.60.

**<Example 38> Preparation of Compound 109**

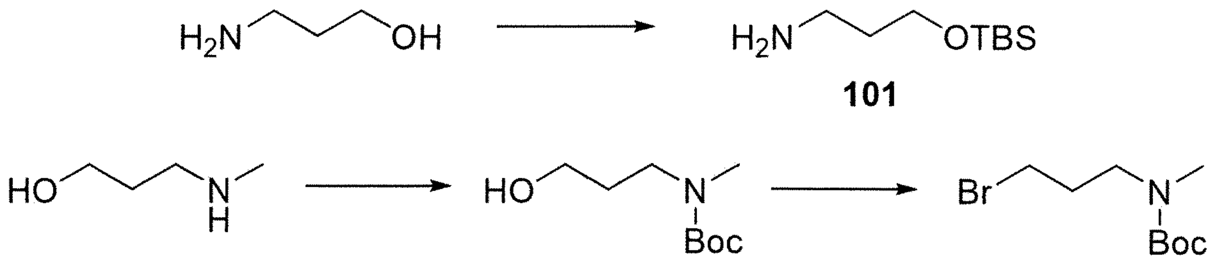

101

102

103

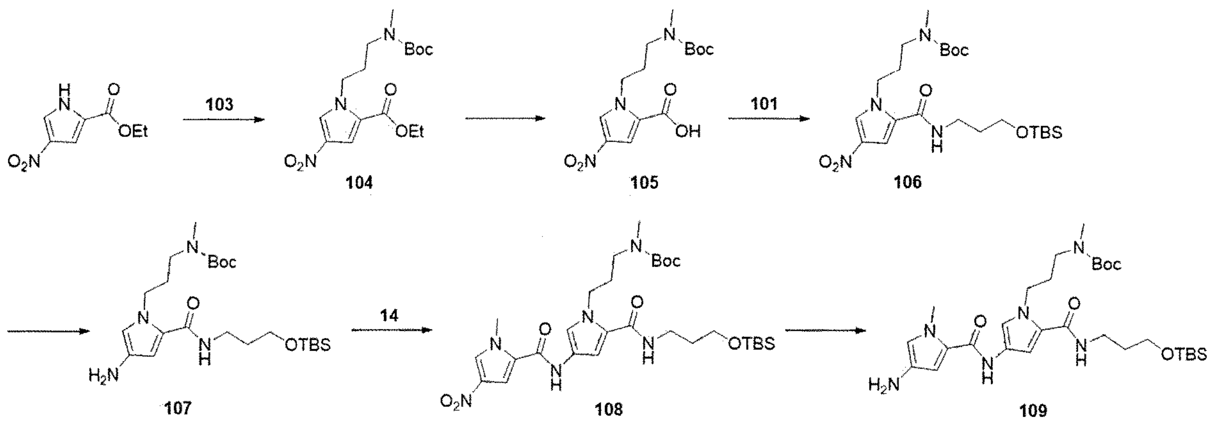


## Preparation of Compound 101

In dichloromethane (30 mL) was dissolved 3-amino-1-propanol (1.5 g, 20.0 mmol), t-butyldimethylsilyl chloride (3.15 g, 21.0 mmol) and imidazole (2.72 g, 40.0 mmol) were added at 0°C, and the mixture was stirred at room temperature for 16 hours. Concentration under reduced pressure and purification by column chromatography were performed, thereby obtaining Compound 101 (3.65 g, 96%). $^1$H-NMR (400 MHz, $CDCl_3$) δ 3.71-3.67 (m, 2H), 2.81-2.78 (m, 2H), 2.06 (br, 2H), 1.69-1.62 (m, 2H), 0.88 (s, 9H), 0.04 (s, 6H).

## Preparation of Compound 102

After 3-(methylamino)-1-propanol (1.2 g, 13.4 mmol) was dissolved in dichloromethane (15 mL), di-*t*-butyl dicarbonate (3.2 g, 14.74 mmol) dissolved in dichloromethane (5 mL) was gradually added at 0°C under a nitrogen atmosphere. After stirring at room temperature for 1 hour, 0.1 N aqueous hydrochloric acid solution (10 mL) was added at 0°C. The organic layer was washed with distilled water (10 mL) and brine (10 mL), dried over anhydrous sodium sulfate, concentrated under reduced pressure, and then purified by column chromatography, thereby obtaining Compound 102 (2.0 g, 78%). $^1$H-NMR (400 MHz, $CDCl_3$) δ 3.54-3.48 (m, 2H), 3.42-3.39 (m, 2H), 2.83 (s, 3H), 1.78-1.68 (m, 2H), 1.46 (s, 9H).

## Preparation of Compound 103

Compound 102 (1 g, 5.28 mmol) was dissolved in dichloromethane (16 mL), and then triphenylphosphine (2.12 g, 8.08 mmol) was added. At 0°C under a nitrogen atmosphere, carbon tetrabromide (2.6 g, 8.08 mmol) dissolved in dichloromethane (10 mL) was added, and the mixture was stirred for 30 minutes, then concentrated under reduced pressure, and purified by column chromatography, thereby obtaining Compound 103 (0.6 g, 45%). $^1$H-NMR (400 MHz, $CDCl_3$) δ 3.41-3.33 (m, 4H), 2.87 (s, 3H), 2.10-2.06 (m, 2H), 1.46 (s, 9H).

## Preparation of Compound 104

In acetone (20 mL) was dissolved ethyl 4-nitro-1*H*-pyrrole-2-carboxylate (292 mg, 1.56 mmol), Compound 103 (0.6 g, 2.38 mmol) and potassium carbonate (493 mg, 3.57 mmol) were added, and the mixture was stirred at 60°C for 12 hours under a nitrogen atmosphere. The reaction mixture was concentrated under reduced pressure and subjected to extraction with dichloromethane (20 mL) and distilled water (20 mL), and drying over anhydrous sodium sulfate was performed. Filtration, concentration, and purification by column chromatography were performed, thereby obtaining Compound 104 (0.53 g, 98%). $^1$H-NMR (400 MHz, $CDCl_3$) δ 7.74 (br s, 1H), 7.52 (s, 1H), 4.37-4.28 (m, 4H), 3.42 (s, 2H), 2.86 (s, 3H), 2.06-1.99 (m, 2H), 1.46 (s, 9H), 1.38-1.30 (t, *J* = 7.2 Hz, 3H).

## Preparation of Compound 105

Compound 104 (0.53 g, 1.55 mmol) was dissolved in ethanol (10 mL), 1 N aqueous sodium hydroxide solution (10 mL)) was added under a nitrogen atmosphere, and the mixture was stirred at 60°C for 2 hours. After concentration of ethanol, the pH was adjusted to about 5 with 1 N aqueous hydrochloric acid solution, extraction with ethyl acetate (20 mL) was performed, and the organic layer was dried over anhydrous sodium sulfate. Filtration and concentration were performed, thereby obtaining Compound 105 (493 mg, 96%). $^1$H-NMR (400 MHz, $CDCl_3$) δ 7.80 (br s, 1H), 7.55 (s, 1H),

4.39-4.35 (m, 2H), 3.32 (s, 2H), 2.87 (s, 3H), 2.17-2.03 (m, 2H), 1.46 (s, 9H).

**Preparation of Compound 106**

Compound 105 (350 mg, 1.07 mmol) and Compound 101 (303 mg, 1.6 mmol) were dissolved in *N,N*-dimethylformamide (10 mL), *N,N,N',N'*-tetramethyl-*O*-(1*H*-benzotriazol-1-yl)uronium hexafluorophosphate (HBTU, 637 mg, 1.60 mmol) and *N,N'*-diisopropylethylamine (0.55 mL, 3.20 mmol) were sequentially added at 0°C under a nitrogen atmosphere, and the mixture was stirred at room temperature for 12 hours. The reaction mixture was diluted with ethyl acetate (20 mL) and washed with distilled water (20 mL) and brine (20 mL), and the organic layer was dried over anhydrous sodium sulfate. After filtration and concentration under reduced pressure, the product was purified by column chromatography, thereby obtaining Compound 106 (290 mg, 54%). $^1$H-NMR (400 MHz, $CDCl_3$) δ 7.65 (br s, 1H), 7.01 (s, 1H), 6.89 (br s, 1H), 4.37-4.30 (m, 2H), 3.83-3.81 (m, 2H), 3.53-3.49 (m, 2H), 3.28-3.26 (m, 2H), 2.84 (s, 3H), 2.07-2.00 (m, 2H), 1.83-1.78 (m, 2H), 1.45 (s, 9H), 0.90 (s, 9H), 0.10 (s, 6H).

**Preparation of Compound 107**

Compound 106 (290 mg, 0.58 mmol) was dissolved in methanol (30 mL), then palladium/charcoal (10% w/w, 100 mg) was added under a hydrogen atmosphere, and the mixture was stirred at room temperature for 2 hours. The reaction mixture was filtered through Celite and then concentrated under reduced pressure, thereby obtaining Compound 107 (250 mg, 92%). $^1$H-NMR (400 MHz, $CDCl_3$) δ 6.99 (br s, 1H), 6.83 (br s, 1H), 6.61 (br s, 2H), 4.28-4.24 (m, 2H), 3.71-3.70 (m, 2H), 3.45-3.40 (m, 2H), 3.23-3.19 (m, 2H), 2.84 (s, 3H), 1.97-1.94 (m, 2H), 1.81-1.75 (m, 2H), 1.45 (s, 9H), 0.88 (s, 9H), 0.11 (s, 6H).

**Preparation of Compound 108**

Compound 14 (76 mg, 0.44 mmol) and Compound 107 (250 mg, 0.53 mmol) were dissolved in *N,N*-dimethylformamide (10 mL), then 1-ethyl-3-(3-dimethylaminopropyl)carbodiimide (171 mg, 0.88 mmol) and 4-dimethylaminopyridine (136 mg, 1.11 mmol) were added at 0°C under a nitrogen atmosphere, and the mixture was stirred at room temperature for 16 hours. After addition of ethyl acetate (10 mL), the reaction mixture was washed with saturated aqueous ammonium chloride solution (10 mL), saturated aqueous sodium hydrogen carbonate solution (10 mL) and distilled water (10 mL). The collected organic layers were dried over anhydrous sodium sulfate, filtered, and then purified by column chromatography, thereby obtaining Compound 108 (197 mg, 72%). $^1$H-NMR (400 MHz, $CDCl_3$) δ 7.60 (s, 1H), 7.19 (br s, 2H), 6.54 (br s, 1H), 6.37 (br s, 1H), 4.29 (s, 2H), 4.03 (s, 3H), 3.78-3.75 (m, 2H), 3.50-3.46 (m, 2H), 3.26 (s, 2H), 2.84 (s, 3H), 2.04-1.99 (m, 2H), 1.82-1.76 (m, 2H), 1.45 (s, 9H), 0.98 (s, 9H), 0.08 (s, 6H). EI-MS m/z : $[M+H]^+$621.80.

**Preparation of Compound 109**

Compound 108 (197 mg, 0.317 mmol) was dissolved in methanol (20 mL), then palladium/charcoal (10% w/w, 80 mg) was added under a hydrogen atmosphere, and the mixture was stirred at room temperature for 5 hours. The reaction mixture was filtered through Celite and then concentrated under reduced pressure, thereby obtaining Compound 109 (185 mg, 99%). $^1$H-NMR (400 MHz, $CDCl_3$) δ 7.29 (br s, 1H), 7.19 (br s, 1H), 6.54 (br s, 1H), 6.33 (br s, 2H), 6.15 (s, 1H), 4.30-4.26 (m, 2H), 3.88 (s, 3H), 3.77-3.74 (m, 2H), 3.48-3.44 (m, 2H), 3.29 (s, 2H), 2.84 (s, 3H), 2.05-1.98 (m, 2H), 1.79-1.76 (m, 2H), 1.40 (s, 9H), 0.90 (s, 9H), 0.08 (s, 6H).

**<Example 39> Preparation of Compound 110**

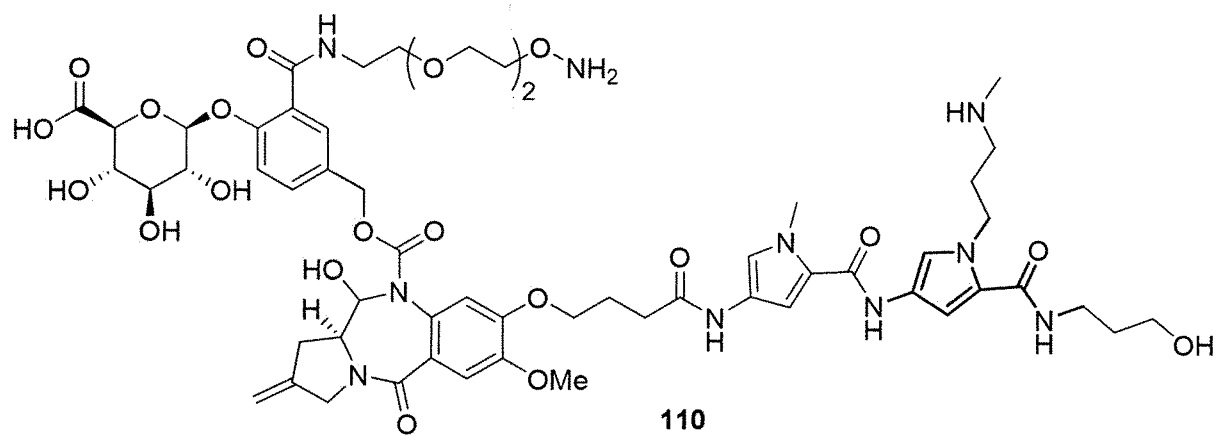

110

Compound 110 was synthesized from Compound 10 and Compound 109 in a similar manner to the synthesis of Compound 29. EI-MS m/z : $[M+H]^+$1238.03, $[M/2+H]^+$619.81.

**<Example 40> Preparation of Compound 117**

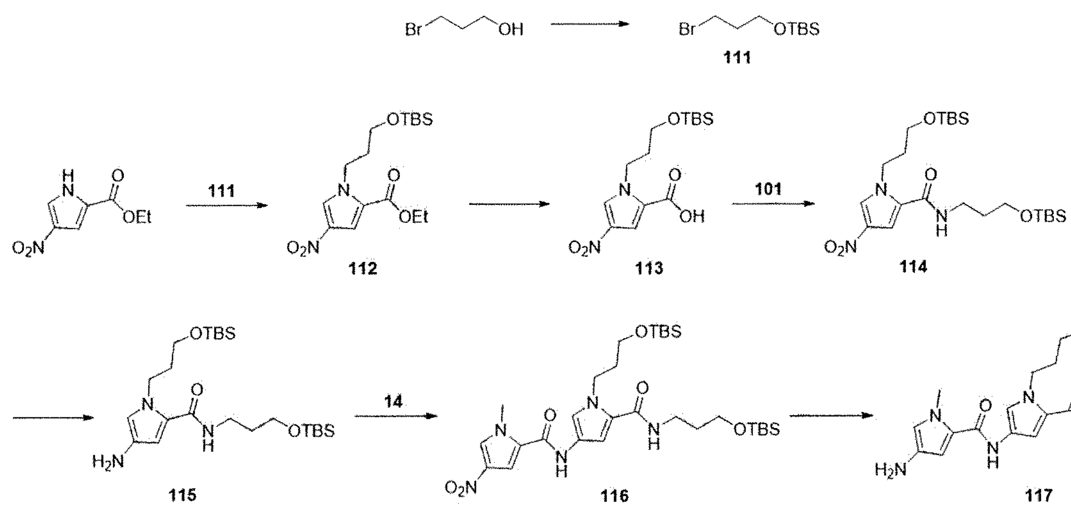


111 112 113 114 115 116 117

__Preparation of Compound 111__

In dichloromethane (20 mL) was dissolved 3-bromo-1-propanol (1.0 g, 7.19 mmol), *t*-butyldimethylsilyl chloride (1.08 g, 7.19 mmol) and imidazole (0.49 g, 7.19 mmol) were added at 0°C, and the mixture was stirred at room temperature for 16 hours. Concentration under reduced pressure and purification by column chromatography were performed, thereby obtaining Compound 111 (1.47 g, 81%). $^1$H-NMR (400 MHz, $CDCl_3$) $\delta$ 3.72 (t, *J* = 5.6 Hz, 2H), 3.51 (t, *J* = 5.62 Hz, 2H), 2.06-2.00 (m, 2H), 0.89 (s, 9H), 0.06 (s, 6H).

__Preparation of Compound 112__

In acetone (30 mL) was dissolved ethyl 4-nitro-1*H*-pyrrole-2-carboxylate (500 mg, 2.71 mmol), Compound 111 (1.03 g, 4.07 mmol) and potassium carbonate (1.12 g, 8.14 mmol) were added, and the mixture was stirred at 60°C for 12 hours under a nitrogen atmosphere. The reaction mixture was concentrated under reduced pressure, diluted with dichloromethane (20 mL), and washed with distilled water (20 mL). The collected organic layers were dried over anhydrous sodium sulfate, filtered, concentrated, and then purified by column chromatography, thereby obtaining Compound 112 (894 mg, 92%). $^1$H-NMR (400 MHz, $CDCl_3$) $\delta$ 7.67 (s, 1H), 7.45 (s, 1H), 4.50-4.47 (m, 2H), 4.35-4.29 (m, 2H), 3.59-3.56 (m, 2H), 2.02-1.96 (m, 2H), 1.38-1.36 (m, 2H), 0.93 (s, 9H), 0.07 (s, 6H).

**Preparation of Compound 113**

Compound 112 (890 mg, 2.49 mmol) was dissolved in ethanol (20 mL), 1 M aqueous sodium hydroxide solution (10 mL)) was added under a nitrogen atmosphere, and the mixture was stirred at 60°C for 2 hours. After concentration of ethanol, the pH was adjusted to about 2 with 1 N aqueous hydrochloric acid solution, extraction with ethyl acetate (20 mL) was performed, and the organic layer was concentrated to dryness over anhydrous sodium sulfate, thereby obtaining Compound 113 (724 mg, 88%). $^1$H-NMR (400 MHz, $CDCl_3$) δ 7.70 (br s, 1H), 7.55 (s, 1H), 4.48 (br s, 2H), 3.56 (s, 2H), 1.99 (s, 2H), 0.91 (s, 9H), 0.10 (s, 6H).

**Preparation of Compound 114**

Compound 113 (548 mg, 1.66 mmol) and Compound 101 (537 mg, 2.83 mmol) were dissolved in *N,N*-dimethylformamide (20 mL), *N,N,N',N'*-tetramethyl-*O*-(1*H*-benzotriazol-1-yl)uronium hexafluorophosphate (HBTU, 999 mg, 2.50 mmol) and *N,N'*-diisopropylethylamine (0.58 mL, 3.30 mmol) were sequentially added at 0°C under a nitrogen atmosphere, and the mixture was stirred at room temperature for 12 hours. After addition of ethyl acetate (20 mL), the reaction mixture was washed with distilled water (20 mL) and brine (20 mL), and the organic layer was dried over anhydrous sodium sulfate. Filtration, concentration under reduced pressure, and purification by column chromatography were performed, thereby obtaining Compound 114 (556 mg, 67%). $^1$H-NMR (400 MHz, $CDCl_3$) δ 7.60 (s, 1H), 7.01 (s, 1H), 6.87 (br s, 1H), 4.49-4.45 (m, 2H), 3.83-3.81 (m, 2H), 3.56-3.49 (m, 4H), 2.01-1.98 (m, 2H), 1.81-1.76 (m, 2H), 0.91 (s, 18H), 0.10 (s, 6H), 0.05 (s, 6H).

**Preparation of Compound 115**

Compound 114 (300 mg, 0.60 mmol) was dissolved in methanol (30 mL), then palladium/charcoal (10% w/w, 100 mg) was added under a hydrogen atmosphere, and the mixture was stirred at room temperature for 2 hours. The reaction mixture was filtered through Celite and then concentrated under reduced pressure, thereby obtaining Compound 115 (276 mg, 98%). $^1$H-NMR (400 MHz, $CDCl_3$) δ 6.35 (s, 1H), 6.27 (br s, 1H), 6.05 (s, 1H), 4.30-4.27 (m, 2H), 3.77-3.74 (m, 2H), 3.58-3.56 (m, 2H), 3.46-3.43 (m, 2H), 1.96-1.89 (m, 2H), 1.80-1.76 (m, 2H), 1.72 (br s, 2H), 0.91 (s, 18H), 0.08 (s, 6H), 0.05 (s, 6H). EI-MS m/z : $[M+H]^+$470.5.

**Preparation of Compound 116**

Compound 14 (83 mg, 0.48 mmol) and Compound 115 (276 mg, 0.58 mmol) were dissolved in *N,N*-dimethylformamide (10 mL), *N,N,N',N'*-tetramethyl-*O*-(1*H*-benzotriazol-1-yl)uronium hexafluorophosphate (HBTU, 350 mg, 0.73 mmol) and *N,N'*-diisopropylethylamine (0.20 mL, 0.97 mmol) were sequentially added at 0°C under a nitrogen atmosphere, and the mixture was stirred at room temperature for 12 hours. Concentration under reduced pressure and purification by column chromatography were performed, thereby obtaining Compound 116 (208 mg, 57%). $^1$H-NMR (400 MHz, $CDCl_3$) δ 7.71 (s, 1H), 7.60 (s, 1H), 7.20 (br s, 1H), 6.55 (s, 1H), 6.36 (br s, 1H), 4.40-4.36 (m, 2H), 4.02 (s, 3H), 3.77-3.74 (m, 2H), 3.61-3.58 (m, 2H), 3.49-3.45 (m, 2H), 2.07-1.94 (m, 2H), 1.82-1.80 (m, 2H), 0.91 (s, 18H), 0.08 (s, 6H), 0.05 (s, 6H). EI-MS m/z : $[M+H]^+$622.7.

**Preparation of Compound 117**

Compound 116 (100 mg, 0.16 mmol) was dissolved in methanol (20 mL), then palladium/charcoal (10% w/w, 30 mg) was added under a hydrogen atmosphere, and the mixture was stirred at room temperature for 2 hours. The reaction mixture was filtered through Celite and then concentrated under reduced pressure, thereby obtaining Compound 117 (88 mg, 93%). EI-MS m/z : $[M+H]^+$592.5.

**<Example 41> Preparation of Compound 118**

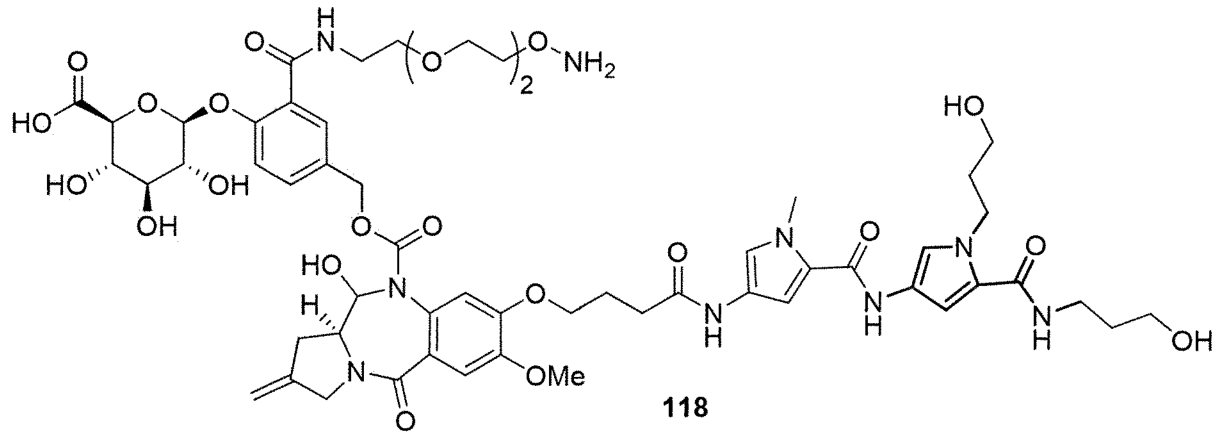

118

Compound 118 was synthesized from Compound 10 and Compound 117 in a similar manner to the synthesis of Compound 29. EI-MS m/z : $[M+H]^+$1224.9, $[M/2+H]^+$623.08.

**<Example 42> Preparation of Compound 124**

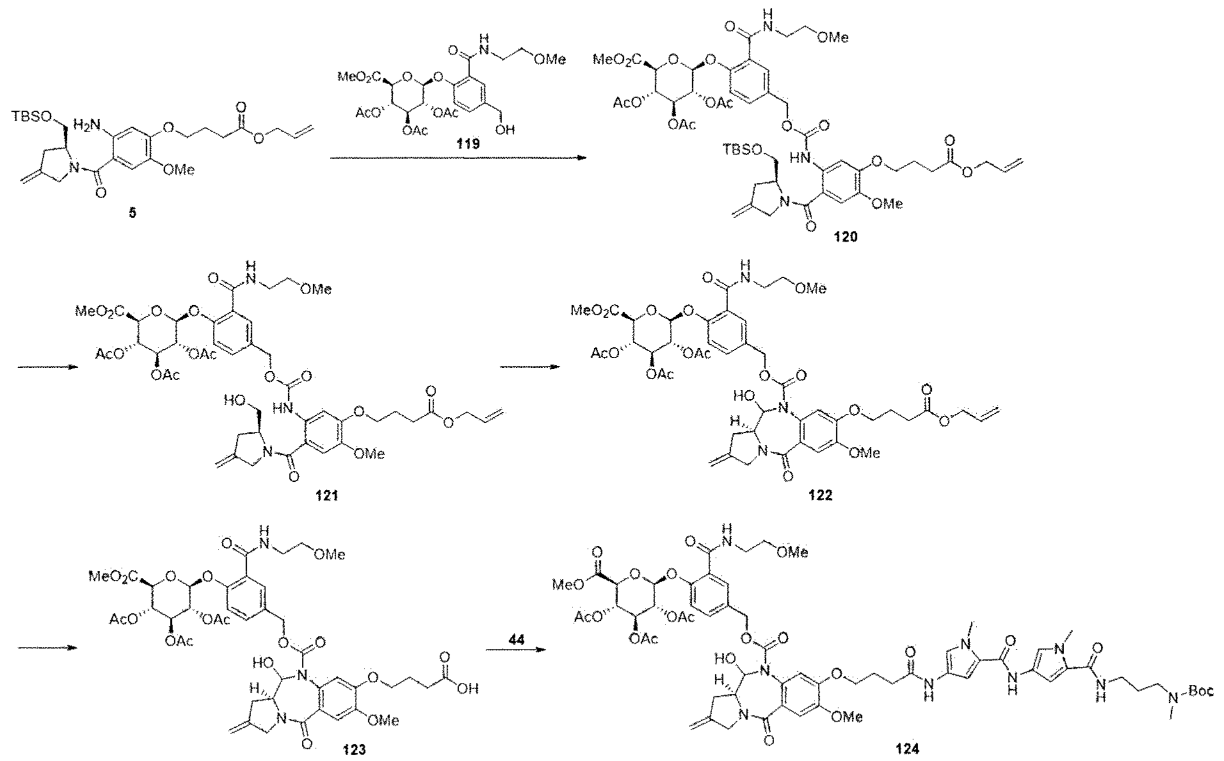


5, 119, 120, 121, 122, 123, 124

__Preparation of Compound 120__

Compound 5 (1.84 g, 3.6 mmol) was dissolved in dichloromethane (10 mL), then triphosgene (767 mg, 2.59 mmol) and *N,N'*-diisopropylethylamine (1.69 mL, 9.72 mmol) were added at 0°C, and the mixture was stirred for 10 minutes. To the reaction solution were added dibutyltin dilaurate (227 mg, 0.36 mmol) and *N,N'*-diisopropylethylamine (1.25 mL, 7.2 mmol), and the mixture was stirred for 5 minutes. A solution of Compound 119 (16.4 g, 21.0 mmol, Compound 119 was prepared by the method described in Korean Patent Publication No. 10-2018-0110645) in dichloromethane (5 mL) was added, then the temperature was gradually raised, the mixture was stirred at room temperature for 1.5 hours, and then the reaction mixture was concentrated under reduced pressure. Saturated aqueous ammonium chloride solution (15 mL) was added to the reaction mixture, followed by extraction with ethyl acetate (30 mL × 3),

washing with distilled water (50 mL) and aqueous sodium chloride solution (50 mL), and drying over anhydrous sodium sulfate. After filtration and concentration under reduced pressure, the product was purified by column chromatography, thereby obtaining Compound 120 (2.84 g, 72%). $^1H$-NMR(400 MHz, $CDCl_3$) δ 8.93 (br s, 1H), 8.04 (d, J =2.4 Hz, 1H), 7.83 (br s, 1H), 7.52-7.44 (m, 2H), 7.05 (d, J =8.8 Hz, 1H), 6.81 (s, 1H), 5.93 (m, 1H), 5.41-5.22 (m, 7H), 5.13 (s, 2H), 4.97 (br s, 1H), 4.89 (br s, 1H), 4.59 (d, *J* =5.6 Hz, 2H), 4.18 (d, J =8.4 Hz, 2H), 4.12 (m, 3H), 3.80 (s, 3H), 3.73 (s, 3H), 3.69 (m, 1H), 3.62-3.53 (m, 4H), 3.41 (s, 3H), 2.66 (m, 2H), 2.58 (t, J =7.6 Hz, 2H), 2.19 (m, 2H), 2.05-2.04 (m, 9H), 0.87 (s, 9H), 0.02 (br s, 6H), EI-MS m/z : $[M+H]^+$1087.49, $[M/2+H]^+$544.13.

**Preparation of Compound 121**

Compound 120 (2.84 g, 2.6 mmol) was dissolved in tetrahydrofuran/distilled water (4 mL/4 mL), and acetic acid (8 mL) was added at 0°C, and the mixture was stirred at room temperature for 15 hours. After concentration under reduced pressure, saturated aqueous sodium hydrogen carbonate solution (50 mL) was added, followed by extraction with ethyl acetate (50 mL × 3). The extracted solution was washed with distilled water (30 mL) and aqueous sodium chloride solution (30 mL), and dried over anhydrous sodium sulfate. After filtration and concentration under reduced pressure, the product was purified by column chromatography, thereby obtaining Compound 121 (2.34 g, 92%). $^1H$-NMR(400 MHz, $CDCl_3$) δ 8.37 (br s, 1H), 8.06 (s, 1H), 7.74 (br s, 1H), 7.52 (m, 1H), 7.44 (dd, J =8.8 Hz, 2 Hz, 1H), 7.05 (d, J =8.4 Hz, 1H), 6.81 (s, 1H), 5.93 (m, 1H), 5.42-5.22 (m, 7H), 5.14 (br s, 2H), 5.00 (br s, 1H), 4.91 (br s, 1H), 4.59 (m, 3H), 4.18 (d, J =8.4 Hz, 2H), 4.13-4.04 (m, 5H), 3.83 (s, 3H), 3.72-3.67 (m, 6H), 3.62-3.53 (m, 4H), 3.41 (s, 3H), 2.81 (m, 1H), 2.59 (t, J =7.6 Hz, 2H), 2.52 (m, 1H), 2.22 (m, 2H), 2.05-2.04 (m, 9H), EI-MS m/z : $[M+H]^+$972.48, $[M/2+H]^+$486.99.

**Preparation of Compound 122**

Compound 121 (2.34 g, 2.4 mmol) was dissolved in dichloromethane (24 mL), then Dess-Matin periodinane (1.23 g, 2.9 mmol) was added at 0°C, the temperature was raised to room temperature, and the mixture was stirred for 1 hour under a nitrogen atmosphere. The reaction mixture was diluted with saturated aqueous sodium thiosulfate solution/saturated aqueous sodium hydrogen carbonate solution (v/v=1/1, 30 mL) and stirred for 10 minutes. After extraction with dichloromethane (30 mL × 3), washing with distilled water (30 mL) and brine (30 mL) was performed. The collected organic layers were dried over anhydrous sodium sulfate, filtered, concentrated, and then purified by column chromatography, thereby obtaining Compound 122 (1.75 g, 75%). $^1H$-NMR(400 MHz, $CDCl_3$) δ 7.99 (br s, 1H), 7.50 (br s, 1H), 7.23-7.19 (m, 2H), 7.01 (d, J =7.2 Hz, 1H), 6.60 (s, 1H), 5.92 (m, 1H), 5.59 (m, 1H), 5.43-5.22 (m, 7H), 5.14 (m, 2H), 4.89 (d, J =11.6 Hz, 1H), 4.59 (d, J =5.6 Hz, 2H), 4.30-4.23 (m, 2H), 4.15-4.09 (m, 2H), 3.95 (m, 1H), 3.88 (s, 3H), 3.72-3.69 (m, 4H), 3.63-3.52 (m, 5H), 3.41 (s, 3H), 2.89 (m, 1H), 2.70 (d, *J* =12 Hz, 1H), 2.56 (t, J =7.2 Hz, 2H), 2.10 (m, 2H), 2.05-2.04 (m, 9H), EI-MS m/z : $[M+H]^+$970.46, $[M/2+H]^+$485.97.

**Preparation of Compound 123**

Compound 122 (250 mg, 0.25 mmol) was dissolved in N,N-dimethylformamide (2.5 mL), tetrakis(triphenylphosphine)palladium(0) (119 mg, 0.10 mmol) and pyrrolidine (0.02 mL, 0.28 mmol) were gradually added at 0°C under a nitrogen atmosphere, and the mixture was stirred at room temperature for 1 hour. The reaction mixture was acidified (pH ~4) with 1 N aqueous hydrochloric acid solution, followed by extraction with dichloromethane (20 mL × 3) and washing with sodium chloride aqueous solution (20 mL). Drying over anhydrous sodium sulfate, filtration, and concentration under reduced pressure were performed, thereby obtaining Compound 123 (239 mg). EI-MS m/z : $[M+H]^+$930.40, $[M/2+H]^+$466.13.

**Preparation of Compound 124**

Compound 123 (239 mg, 0.25 mmol) was dissolved in *N,N*-dimethylformamide (2 mL), and then *N,N,N',N'*-tetramethyl-*O*-(1*H*-benzotriazol-1-yl)uronium hexafluorophosphate (HBTU, 146 mg, 0.38 mmol) and *N,N'*-diisopropylethylamine (0.09 mL, 0.51 mmol) were added at 0°C under a nitrogen atmosphere. A solution of Compound 44 (133 mg, 0.30 mmol) in N,N-dimethylformamide (1 mL) was added to the reaction solution, and the mixture was stirred at room temperature for 1 hour. Saturated aqueous ammonium chloride solution (10 mL) was added to the reaction mixture, followed by extraction with ethyl acetate (20 mL × 3), and washing with saturated sodium hydrogen carbonate solution (10 mL) and distilled water (10 mL) and drying over anhydrous sodium sulfate were performed. After filtration and concentration, the product was purified by column chromatography, thereby obtaining Compound 124 (267 mg, 77%). EI-MS m/z : $[M+H]^+$1344.69, $[M/2+H]^+$673.20.

**<Example 43> Preparation of Compound 129**

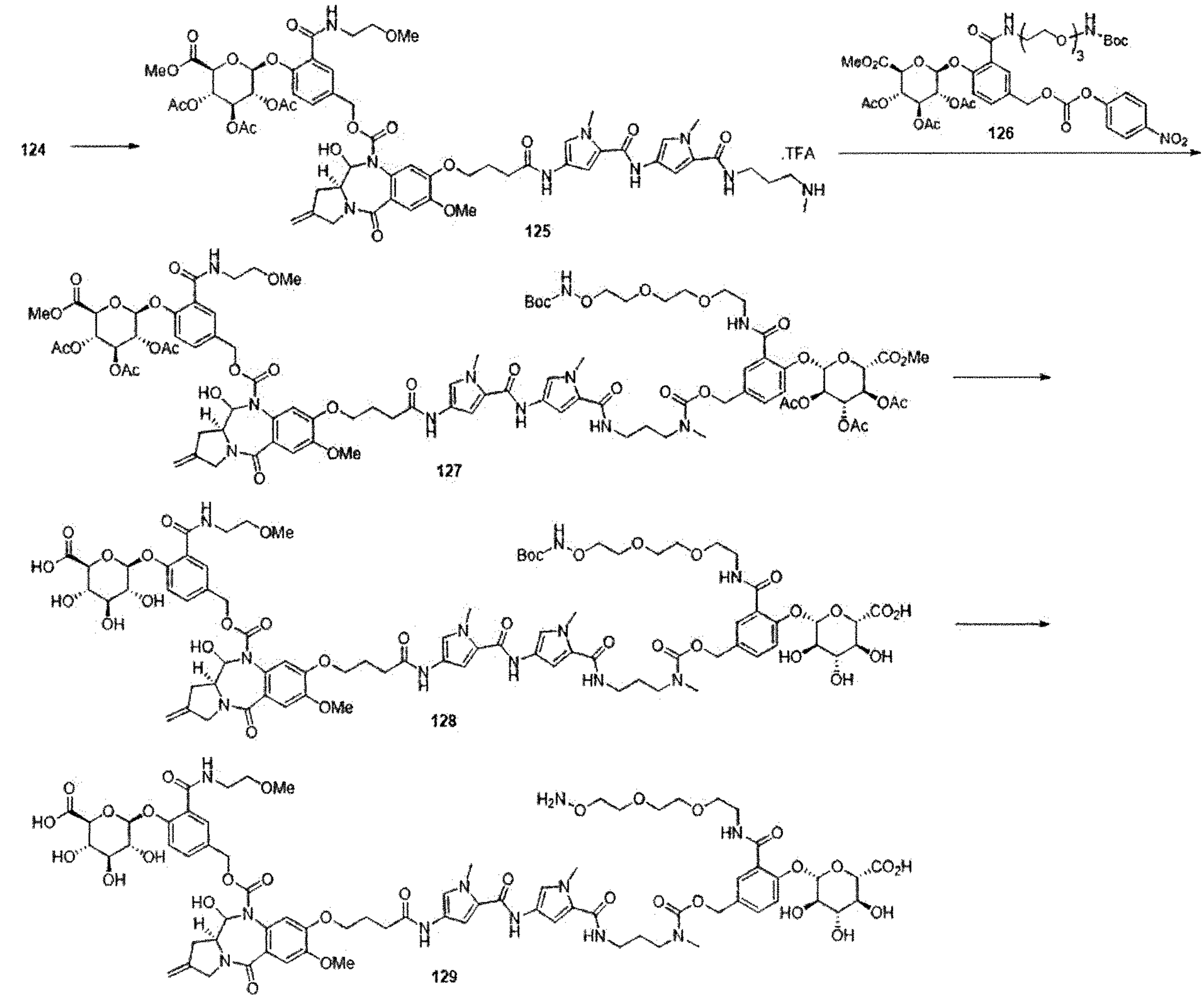

124 125 126 127 128 129

<u>**Preparation of Compound 125**</u>

Compound 124 (170 mg, 0.12 mmol) was diluted with dichloromethane (1.6 mL), then trifluoroacetic acid (0.4 mL) was added at 0°C under a nitrogen atmosphere, and the mixture was stirred for 1 hour. The reaction mixture was concentrated under nitrogen gas, thereby obtaining Compound 125 (156 mg). EI-MS m/z : [M+H]+ 1244.67, [M/2+H]+ 623.13.

<u>**Preparation of Compound 127**</u>

Compound 125 (156 mg, 0.25 mmol) was dissolved in N,N-dimethylformamide (3 mL), and then *N,N'*-diisopropylethylamine (0.06 mL, 0.37 mmol) was added at 0°C under a nitrogen atmosphere. Compound 126 (123 mg, 0.13 mmol, Compound 126 was prepared by the method described in Patent Publication No. WO 2017089890), 1-hydroxy-7-azabenzotriazole (3.4 mg, 0.38 mmol) and *N,N'*-diisopropylethylamine (0.04 mL, 0.19 mmol) were sequentially added, the temperature was gradually raised, and the mixture was stirred at room temperature for 17 hours. Saturated aqueous ammonium chloride solution (10 mL) was added to the reaction mixture, followed by extraction with ethyl acetate (20 mL × 3), washing with distilled water (10 mL) and aqueous sodium chloride solution (10 mL), and drying over anhydrous sodium sulfate. After filtration and concentration, the product was purified by column chromatography, thereby obtaining Compound 127 (143 mg, 56%). EI-MS m/z : [M+H]+ 2001.40, [M/2+H]+ 1001.52.

## Preparation of Compound 128

Compound 127 (142 mg, 0.07 mmol) was dissolved in methanol/tetrahydrofuran (2 mL/2 mL), and then a solution of lithium hydroxide (18 mg, 0.42 mmol) in distilled water (3 mL) was gradually added at -40°C under a nitrogen atmosphere. The mixture was stirred for 4 hours while the reaction temperature was gradually raised to 0°C. The reaction mixture was acidified with acetic acid (pH of about 4), concentrated under reduced pressure, purified by HPLC, and freeze-dried, thereby obtaining Compound 128 (70.4 mg, 57%). EI-MS m/z : [M+H]+ 1721.15, [M/2+H]+ 861.25.

## Preparation of Compound 129

Compound 128 (70.4 mg, 0.04 mmol) was diluted with dichloromethane (1.6 mL), then trifluoroacetic acid (0.4 mL) was added at 0°C under a nitrogen atmosphere, and the mixture was stirred for 1 hour. The reaction mixture was concentrated under nitrogen gas, purified by HPLC, and freeze-dried, thereby obtaining Compound 129 (53.8 mg, 81%). EI-MS m/z : [M+H]+ 1621.35, [M/2+H]+ 811.21.

## <Example 44> Preparation of Compound 134

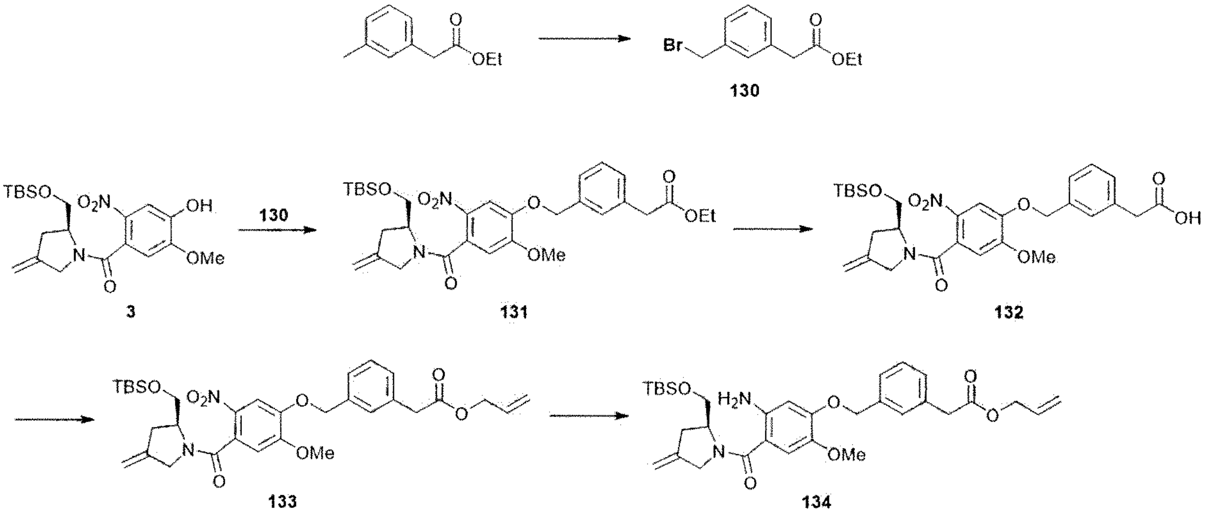


130

3 131 132

133 134

## Preparation of Compound 130

In carbon tetrachloride (20 mL) was dissolved ethyl m-tolyl acetate (3 g, 16.83 mmol), then N-bromosuccinimide (NBS, 3.6 g, 20.19 mm) and 2,2'-azobis(isobutyronitrile) (AIBN, 111 mg, 0.67 mmol) were added, and the mixture was stirred under reflux for 18 hours. After cooling to room temperature, the produced solid was filtered and washed with hexane (20 mL), and the filtrate was concentrated under reduced pressure and purified by column chromatography, thereby obtaining Compound 130 (2.63 g, 60%). $^1$H-NMR(400 MHz, $CDCl_3$) δ 7.35-7.28 (m, 3H), 7.24-7.20 (m, 1H), 4.49 (s, 2H), 4.16 (q, 2H), 3.61 (s, 2H), 1.26 (t, 3H).

## Preparation of Compound 131

Compound 3 (3.5 g, 8.28 mmol) and Compound 130 (2.4 g, 9.33 mmol) were dissolved in N,N-dimethylformamide (20 mL), then potassium carbonate (1.72 g, 12.42 mmol) and sodium iodide (0.25 g, 1.65 mmol) were added, and the mixture was stirred at room temperature for 12 hours under a nitrogen atmosphere. The reaction mixture was diluted with ethyl acetate (100 mL) and washed with distilled water (2 × 60 mL). The washed organic layer was washed with brine (60 mL) and dried over anhydrous sodium sulfate. After filtration and concentration under reduced pressure, the product was purified by column chromatography, thereby obtaining Compound 131 (4.7 g, 94%). $^1$H-NMR(400 MHz, $CDCl_3$) (rotamers) δ 7.67 (s, 1H), 7.32-7.24 (m, 3H), 7.19 (m, 1H), 6.68 (s, 1H), 5.10 (s, 2H), 5.01 (d, 1H), 4.89 (s, 1H), 4.75 (s, 1H), 4.10-4.01 (m, 2H), 3.87 (s, 3H), 3.79 (m, 1H), 3.70-3.60 (m, 2H), 3.55 (s, 2H), 2.76-2.58 (m, 2H), 1.16 (t, 3H), 0.80 (s, 6H), 0.70 (s, 3H), -0.09 (s, 4H), -0.21 (d, 2H).

**Preparation of Compound 132**

Compound 131 (4.7 g, 7.85 mmol) was dissolved in 1,4-dioxane (20 mL), 1 N aqueous sodium hydroxide solution (9.4 mL) was added at 0°C, and the mixture was stirred at room temperature for 2 hours. The reaction solvent was concentrated under reduced pressure, the pH was adjusted to about 3 with 1 N aqueous hydrochloric acid solution, extraction with dichloromethane (3 × 40 mL) was performed, and the collected organic layers were dried over anhydrous sodium sulfate. Filtration and concentration were performed, thereby obtaining Compound 132 (4.56 g, 100%). $^1$H-NMR(400 MHz, $CDCl_3$)(rotamers) δ 7.73 (s, 1H), 7.37-7.34 (m, 2H), 7.25 (t, 1H), 7.18 (s, 1H), 6.75 (s, 1H), 5.22 9s, 2H), 5.09 (d, 1H), 4.99 (s, 1H), 4.84 9s, 1H), 4.59 (m, 1H), 3.89 (s, 4H), 3.75 (s, 2H), 3.71 (s, 8H), 3.58 (s, 3H), 2.82-2.64 (m, 2H), 0.89 (s, 6H), 0.79 (s, 3H), 0.09 (s, 4H), -0.11 (d, 3H).

**Preparation of Compound 133**

Compound 132 (4.56 g, 7.85 mmol) was dissolved in N,N-dimethylformamide (40 mL), then potassium carbonate (1.63 g, 11.77 mmol) and allyl bromide (0.81 mL, 9.42 mmol) were added at 0°C, and the mixture was stirred at room temperature for 4 hours. The reaction mixture was diluted with ethyl acetate (70 mL) and washed with saturated aqueous ammonium chloride solution (50 mL) and distilled water (2 × 60 mL). The washed organic layer was washed with brine (60 mL) and dried over anhydrous sodium sulfate. After filtration and concentration under reduced pressure, the product was purified by column chromatography, thereby obtaining Compound 133 (4.36 g, 91%). $^1$H-NMR(400 MHz, $CDCl_3$)(rotamers) δ 7.76 (s, 1H), 7.39-7.36 (m, 3H), 7.28 (t, 1H), 6.77 (s, 1H), 5.95-5.86 (m, 1H), 5.30-5.19 (m, 5H), 5.09 (m, 1H), 4.99 (s, 1H), 4.84 (s, 1H), 4.60 (d, 3H), 4.03-3.95 (m, 4H), 3.93-3.85 (m, 1H), 3.81-3.72 (m, 2H), 3.68 (s, 3H), 3.32 (m, 1H), 2.84-2.65 (m, 2H), 0.90 (s, 9H), 0.09 (s, 6H).

**Preparation of Compound 134**

Compound 133 (4.36 g, 7.13 mmol) was dissolved in ethyl acetate (30 mL) and ethanol (60 mL), then zinc dust (14 g, 214 mmol) was added at 0°C, and formic acid (5.38 mL, 143 mmol) was added. After stirring at room temperature for 4 hours, the reaction mixture was diluted with ethyl acetate (30 mL) and filtered through Celite, and ethyl acetate (70 mL) was added. The organic layer was washed with saturated aqueous sodium hydrogen carbonate solution (2 × 100 mL) and brine (50 mL) in that order, and then dried over anhydrous sodium sulfate. Filtration, concentration, and purification by column chromatography were performed, thereby obtaining Compound 134 (2.52 g, 61%). $^1$H-NMR(400 MHz, $CDCl_3$) δ 7.38-7.30 (m, 3H), 7.24 (s, 1H), 6.77 (s, 1H), 6.26 (s, 1H), 5.95-5.85 (m, 1H), 5.30-5.10 (m, 2H), 5.10 (s, 2H), 5.10-4.85 (m, 2H), 4.59 (d, 2H), 4.40-4.18 (m, 3H), 4.20-4.05 (m, 2H), 3.80 (s, 3H), 3.66 (s, 2H), 3.61 (m, 1H), 2.68 (m, 2H), 0.87 (s, 9H), 0.02 (s, 6H). EI-MS m/z : $[M+H]^+$581.42.

**<Example 45> Preparation of Compound 138**

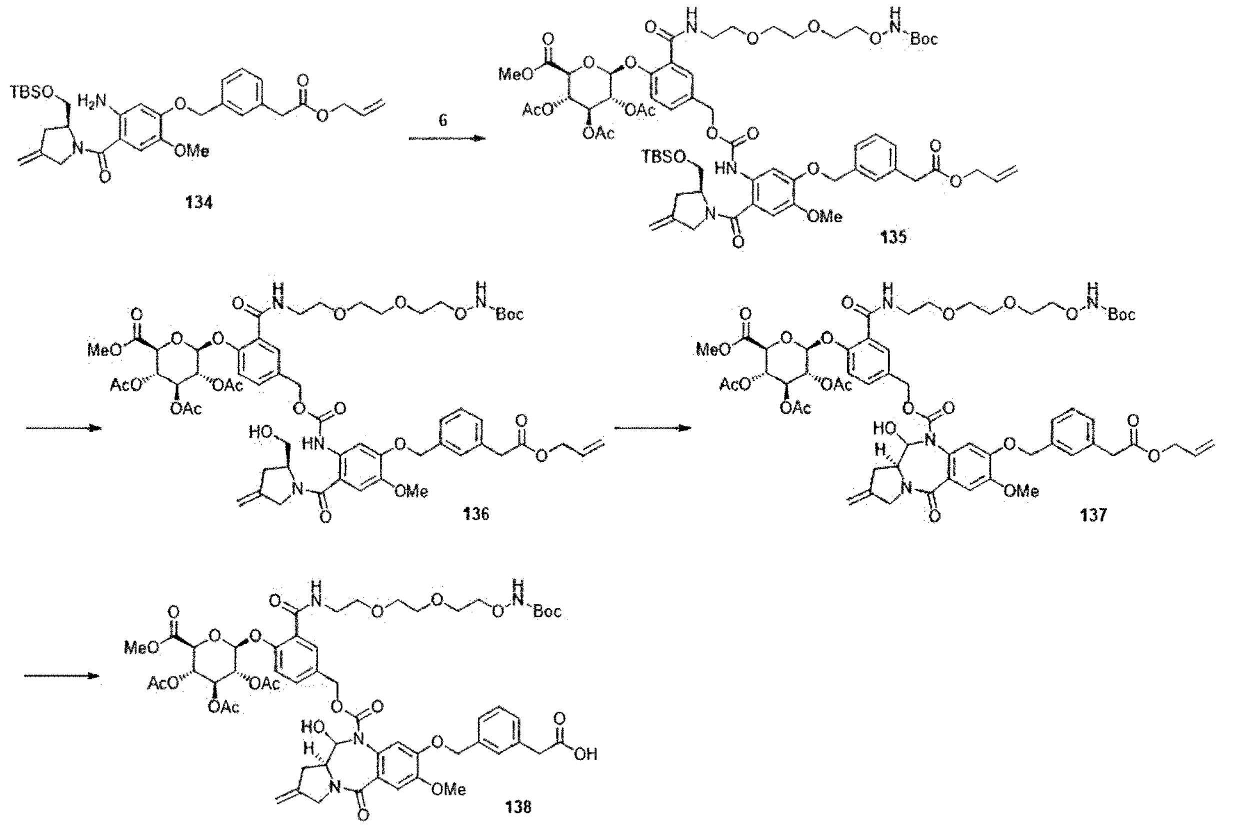


134

135

136

137

138

## Preparation of Compound 135

Compound 134 (2.4 g, 4.13 mmol) was dissolved in dry tetrahydrofuran (40 mL), then triphosgene (0.44 g, 1.48 mmol) and triethylamine (2.32 mL, 16.5 mmol) were added at -10°C, and Compound 6 (3.32 g, 4.54 mmol) dissolved in dry tetrahydrofuran (20 mL) was added. After stirring at room temperature for 17 hours, the reaction mixture was diluted with ethyl acetate (100 mL), washed with distilled water (50 mL), and then dried over anhydrous sodium sulfate. After filtration and concentration under reduced pressure, the product was purified by column chromatography, thereby obtaining Compound 135 (4.2 g, 76%). EI-MS m/z : $[M+H]^+$1337.94.

## Preparation of Compound 136

Compound 135 (4.21 g, 3.14 mmol) was dissolved in tetrahydrofuran/distilled water (20 mL/20 mL), acetic acid (20 mL) was added at 0°C, and the mixture was stirred at room temperature for 17 hours. The reaction mixture was diluted with ethyl acetate (100 mL), washed with distilled water (50 mL) and saturated sodium hydrogen carbonate solution (2 × 70 mL), and then dried over anhydrous sodium sulfate. After filtration and concentration under reduced pressure, the product was purified by column chromatography, thereby obtaining Compound 136 (3.5 g, 91%). EI-MS m/z : $[M+H]^+$1223.61.

## Preparation of Compound 137

Compound 136 (3.5 g, 2.68 mmol) was dissolved in dichloromethane (140 mL), Dess-Martin periodinane (1.33 g, 3.14 mmol) was added, and the mixture was stirred at room temperature for 5 hours under a nitrogen atmosphere. The reaction mixture was diluted with dichloromethane (200 mL), washed with distilled water (80 mL), and then dried over anhydrous sodium sulfate. After filtration and concentration under reduced pressure, the product was purified by column chromatography, thereby obtaining Compound 137 (2.03 g, 58%). $^1$H-NMR(400 MHz, $CDCl_3$) δ 7.97 (s, 1H), 7.80 (s, 1H), 7.45 (s, 1H), 7.23 (d, 1H), 7.19 (s, 1H), 7.00 (d, 1H), 6.61 (s, 1H), 5.92 (m, 1H), 5.58 (d, 1H), 5.41-5.22 (m, 8H), 5.13 (s, 2H), 4.83 (d, 1H), 4.59 (d, 2H), 4.27 (m, 2H), 4.11 (m, 2H), 3.96 (m, 2H), 3.88 (s, 3H), 3.74-3.65 (m, 15H), 3.52 (m, 1H), 2.90 (m, 1H), 2.70 (d, 1H), 2.57 (t, 2H), 2.13 (q, 2H) 2.05 (s, 9H), 1.46 (s, 9H). EI-MS m/z : $[M+H]^+$1121.65.

**Preparation of Compound 138**

Compound 137 (350 mg, 0.286 mmol) was dissolved in N,N-dimethylformamide (5 mL), then tetrakis(triphenylphosphine)palladium(0) (132 mg, 0.114 mmol) and pyrrolidine (0.026 mL, 0.314 mmol) were added, and the mixture was stirred at room temperature for 1.5 hours under a nitrogen atmosphere. The solvent was concentrated under reduced pressure, and then purification by column chromatography was performed, thereby obtaining Compound 138 (279 mg, 83%). EI-MS m/z : $[M+H]^+$1181.52.

**<Example 46> Preparation of Compound 139**

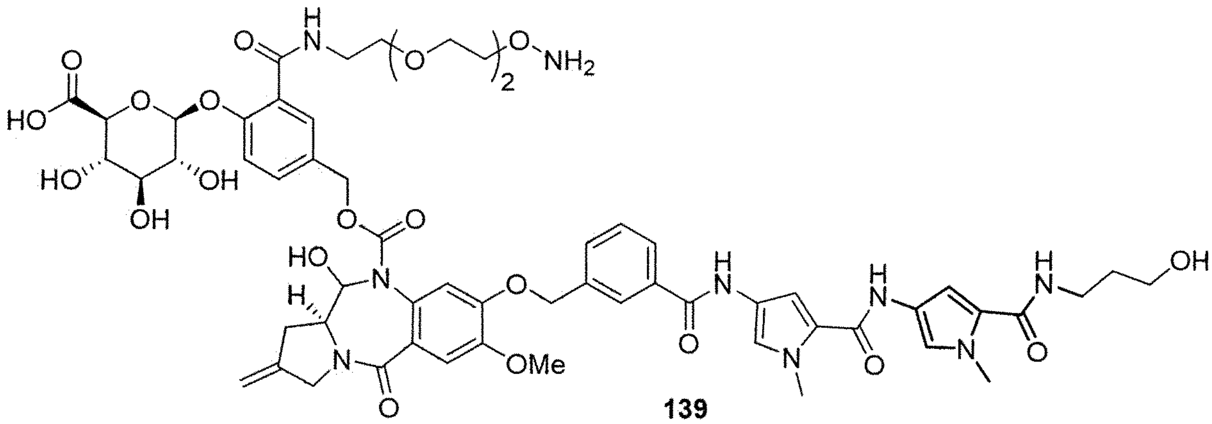

**139**

Compound 139 was synthesized from Compound 138 and Compound 26 in a similar manner to the synthesis of Compound 29. EI-MS m/z : $[M+H]^+$1228.7.

**<Example 47> Preparation of Compound 147**

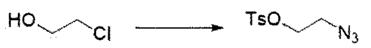

**140**

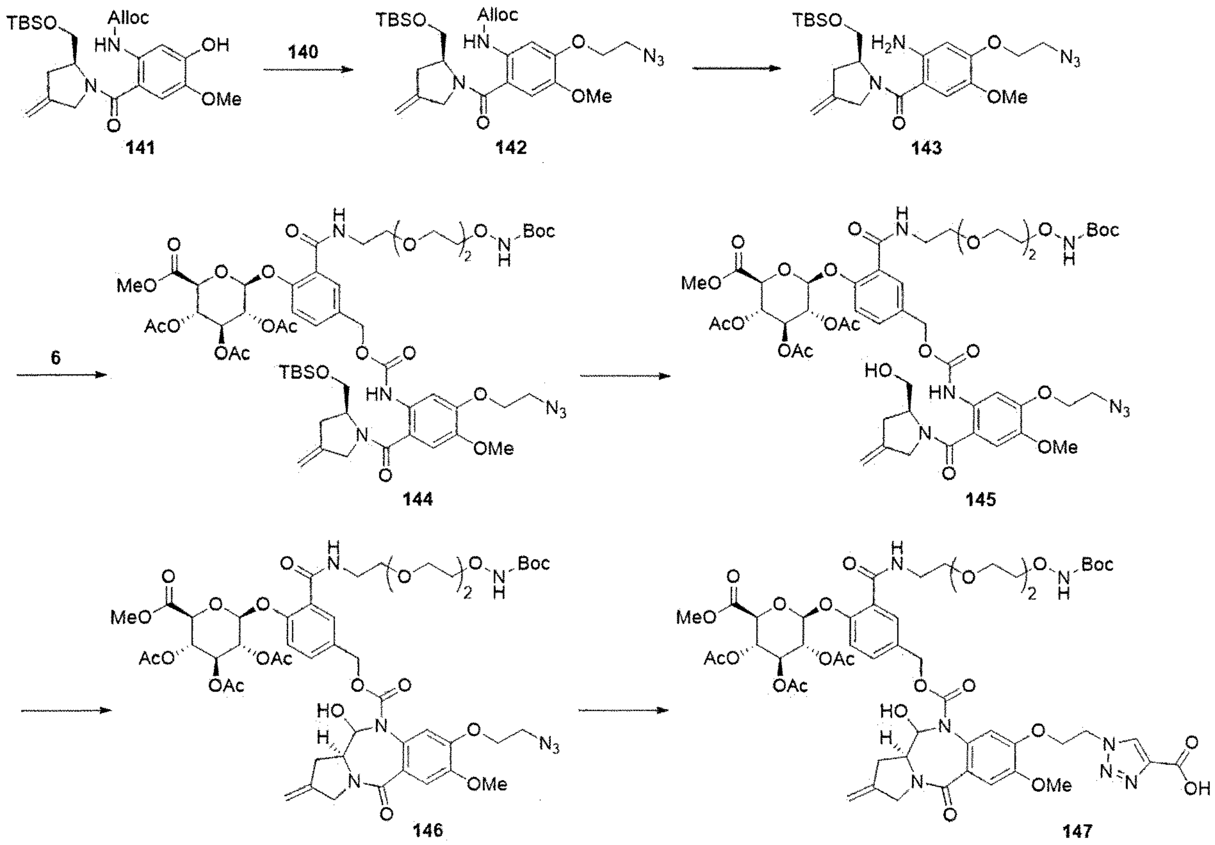


141 142 143

144 145

146 147

### Preparation of Compound 140

After 2-chloroethanol (5.0 g, 62.1 mmol) and sodium azide (5.6 g, 86.9 mmol) were dissolved in *N,N*-dimethylformamide (15 mL), and the solution was stirred at 100°C for 18 hours under a nitrogen atmosphere. After the reaction mixture was cooled to room temperature, 4-toluenesulfonyl chloride (13.0 g, 68.3 mmol) and triethylamine (13.0 mL, 93.1 mmol) were added, and the mixture was stirred at room temperature for 18 hours. The reaction mixture was diluted with diethyl ether (200 mL), washed with distilled water (150 mL), and then dried over anhydrous sodium sulfate. After filtration and concentration under reduced pressure, the product was purified by column chromatography, thereby obtaining Compound 140 (8.8 g, 58%). $^1$H-NMR(400 MHz, $CDCl_3$) δ 7.82 (d, 2H), 7.37 (d, 2H), 4.16 (t, 2H), 3.48 (t, 2H), 2.46 (s, 3H).

### Preparation of Compound 142

Compound 141 (2.26 g, 4.73 mmol, Compound 141 was prepared by the method described in Korean Patent Publication No. 10-2020-0084802) and Compound 140 (1.7 g, 7.10 mmol) were dissolved in N,N-dimethylformamide (30 mL), then potassium carbonate (1.3 g, 9.46 mmol) was added, and the mixture was stirred at 100°C for 2 hours under a nitrogen atmosphere. The reaction mixture was diluted with ethyl acetate (200 mL) and washed with distilled water (2 × 100 mL) and brine (100 mL). The collected organic layers were dried over anhydrous sodium sulfate, filtered, concentrated under reduced pressure, and then purified by column chromatography, thereby obtaining Compound 142 (2.3 g, 89%). $^1$H-NMR(400 MHz, $CDCl_3$) (rotamers) δ 7.86 (s, 1H), 6.84 (s, 1H), 5.98-5.91 (m, 1H), 5.35 (d, 1H), 5.24 (d, 1H), 4.99 (br s, 1H), 4.92 (br s, 1H), 4.25 (t, 2H), 4.17 (br s, 1H), 3.82 (s, 3H), 3.66 (t, 2H), 2.70 (br s, 2H), 0.91 (s, 9H), 0.09 (s, 6H).

### Preparation of Compound 143

Compound 142 (2.3 g, 4.21 mmol) was dissolved in 1,2-dichloroethane (50 mL), and then trimethyltin hydroxide (7.6 g, 42.1 mmol) was added. The mixture was heated under reflux and stirred for 18 hours under a nitrogen atmosphere. The reaction mixture was diluted with dichloromethane (200 mL), washed with 0.01 N aqueous potassium hydrogen sulfate (100 mL) and brine (100 mL), and then dried over anhydrous sodium sulfate. After filtration and concentration under reduced pressure, the product was purified by column chromatography, thereby obtaining Compound 143 (1.7 g,

88%). EI-MS m/z : $[M+H]^+$462.25.

**Preparation of Compound 144**

Compound 143 (1.7 g, 3.69 mmol) was dissolved in dry tetrahydrofuran (40 mL), then triphosgene (393 mg, 1.33 mmol) and triethylamine (2.1 mL, 14.8 mmol) were added at 0°C, and a solution of Compound 6 (3.2 g, 4.43 mmol) in dry tetrahydrofuran (20 mL) was added. After stirring at room temperature for 2 hours, the reaction mixture was diluted with ethyl acetate (200 mL), washed with distilled water (100 mL), and then dried over anhydrous sodium sulfate. After filtration and concentration under reduced pressure, the product was purified by column chromatography, thereby obtaining Compound 144 (3.5 g, 78%). EI-MS m/z : $[M+H]^+$1218.70, 1/2$[M-BOC+H]^+$559.82.

**Preparation of Compound 145**

Compound 144 (3.5 g, 2.87 mmol) was dissolved in tetrahydrofuran/distilled water (10 mL/10 mL), acetic acid (10 mL) was added at 0°C, and the mixture was stirred at room temperature for 18 hours. The reaction mixture was diluted with ethyl acetate (100 mL), washed with distilled water (50 mL) and saturated sodium hydrogen carbonate solution (2 × 100 mL), and then dried over anhydrous sodium sulfate. After filtration and concentration under reduced pressure, the product was purified by column chromatography, thereby obtaining Compound 145 (2.9 g, 92%). EI-MS m/z : $[M+H]^+$1104.53, 1/2$[M-BOC+H]^+$502.74.

**Preparation of Compound 146**

Compound 145 (2.9 g, 2.63 mmol) was dissolved in dichloromethane (60 mL), Dess-Martin periodinane (1.3 g, 3.15 mmol) was added, and the mixture was stirred at room temperature for 6 hours under a nitrogen atmosphere. The reaction mixture was diluted with dichloromethane (200 mL), washed with saturated aqueous sodium hydrogen carbonate solution (150 mL), and then dried over anhydrous sodium sulfate. After filtration and concentration under reduced pressure, the product was purified by column chromatography, thereby obtaining Compound 146 (1.37 g, 47%). EI-MS m/z : $[M+H]^+$1102.45, 1/2$[M+H]^+$501.98.

**Preparation of Compound 147**

Compound 146 (150 mg, 0.14 mmol) and propiolic acid (0.01 mL, 0.16 mmol) were dissolved in ethanol (4 mL) and distilled water (2 mL), then copper sulfate pentahydrate (14 mg, 0.05 mmol) and sodium L-ascorbate (22 mg, 0.11 mmol) were added, and the mixture was stirred at room temperature for 2 hours under a nitrogen atmosphere. The reaction mixture was diluted with dichloromethane (50 mL), washed with distilled water (50 mL), and then dried over anhydrous sodium sulfate. After filtration and concentration under reduced pressure, the product was purified by column chromatography, thereby obtaining Compound 147 (154 mg, 96%). EI-MS m/z : $[M+H]^+$1172.26.

**<Example 48> Preparation of Compound 148**

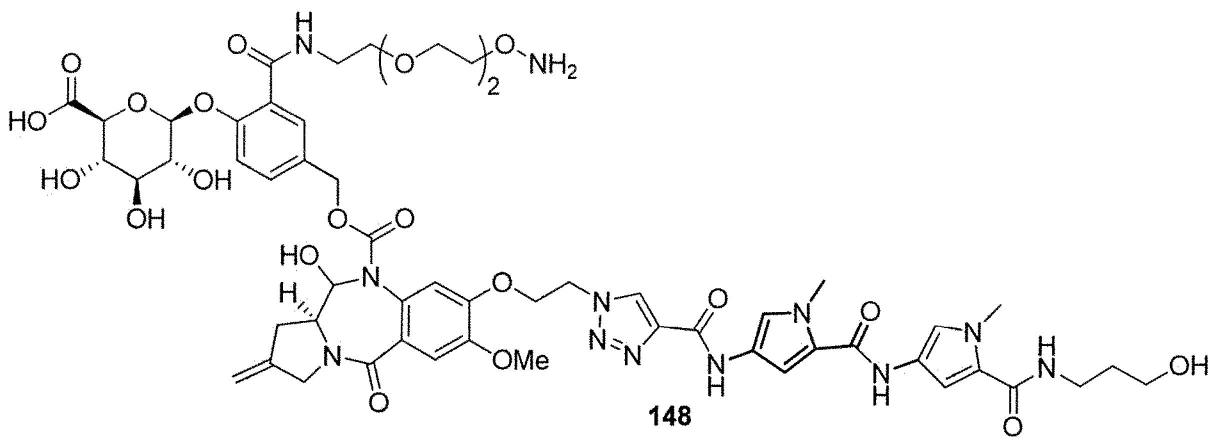

148

Compound 148 was synthesized from Compound 147 and Compound 26 in a similar manner to the synthesis

of Compound 29. EI-MS m/z : $[M+H]^+$1233.49, $[M/2+H]^+$617.29.

**<Example 49> Preparation of Compound 151**

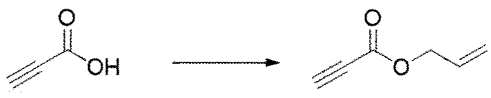

149

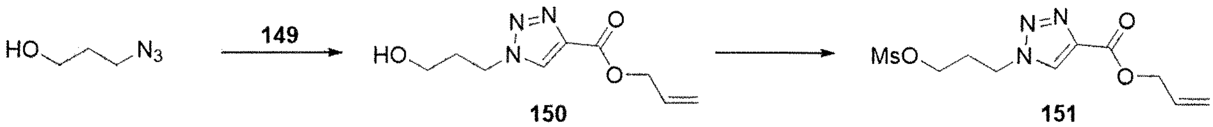


150 151

**Preparation of Compound 149**

After propiolic acid (4 ml, 64.52 mmol) was dissolved in N,N-dimethylformamide (25 mL), sodium hydrogen carbonate (10.9 g, 129.1 mmol) was added, and the mixture was stirred at room temperature for 1 hour. To the reaction solution was added allyl bromide (8.4 mL, 96.8 mmol), and the mixture was stirred at room temperature for 12 hours. The reaction mixture was diluted with diethyl ether (25 mL), washed with distilled water (50 × 3 mL) and brine (50 mL), and then dried over anhydrous sodium sulfate. After filtration and concentration under reduced pressure, the product was purified by column chromatography, thereby obtaining Compound 149 (6.9 g, 97%). $^1$H-NMR(400 MHz, $CDCl_3$) δ 5.98-5.41 (m, 1H), 5.39 (dd, 1H), 5.31 (dd, 1H), 4.69 (d, 2H), 2.89 (s, 1H).

**Preparation of Compound 150**

Compound 149 (800 mg, 7.26 mmol) and 3-azido-1-propanol (734 mg, 7.26 mmol) were dissolved in t-butyl alcohol/distilled water (8 mL/2 mL) at 0°C under a nitrogen atmosphere, then a solution of copper sulfate pentahydrate (180 mg, 0.72 mmol) and sodium L-ascorbate (718 mg, 3.62 mmol) in distilled water (1 mL) was added to the reaction solution, and the mixture was stirred at room temperature for 5 hours. The reaction product was filtered through Celite, and then concentrated under reduced pressure. The filtered solution was subjected to extraction with dichloromethane (20 mL × 3), followed by drying over anhydrous sodium sulfate, filtration, and concentration under reduced pressure. The product was purified by column chromatography, thereby obtaining Compound 150 (720 mg, 46%). $^1$H-NMR(400 MHz, $CDCl_3$) δ 8.15 (s, 1H), 6.09-5.99 (m, 1H), 5.44 (dd, J = 17.2 Hz, 1.6 Hz, 1H), 5.31 (dd, J = 10.8 Hz, 1.2 Hz, 1H), 4.86 (d, J =6 Hz, 2H), 4.61 (t, *J* =6.8 Hz, 2H), 3.67 (q, *J* =4.8 Hz, 2H), 2.17 (quin, 2H), 1.91 (t, 1H).

**Preparation of Compound 151**

Compound 150 (265 mg, 1.25 mmol) was dissolved in dichloromethane (10 mL), then methanesulfonyl chloride (0.11 mL, 1.38 mmol) and triethylamine (0.34 mL, 2.51 mmol) were added at 0°C under a nitrogen atmosphere, and the mixture was stirred at 0°C for 2 hours. Saturated aqueous ammonium chloride solution (10 mL) was added to the reaction mixture, followed by extraction with dichloromethane (20 mL × 2), washing with distilled water (10 mL) and brine (10 mL), and drying over anhydrous sodium sulfate. Compound 151 obtained after filtration and concentration was used in the next reaction without further purification. $^1$H-NMR(400 MHz, $CDCl_3$) δ 8.18 (s, 1H), 6.09-5.99 (m, 1H), 5.42 (dd, *J* = 17.2 Hz, 1.2 Hz, 1H), 5.31 (dd, *J* = 10.8 Hz, 1.2 Hz, 1H), 4.86 (d, *J* =6 Hz, 2H), 4.61 (t, *J* =7.2 Hz, 2H), 4.25 (t, J =6 Hz, 2H), 3.02 (s, 3H), 2.43 (m, 2H).

**<Example 50> Preparation of Compound 157**

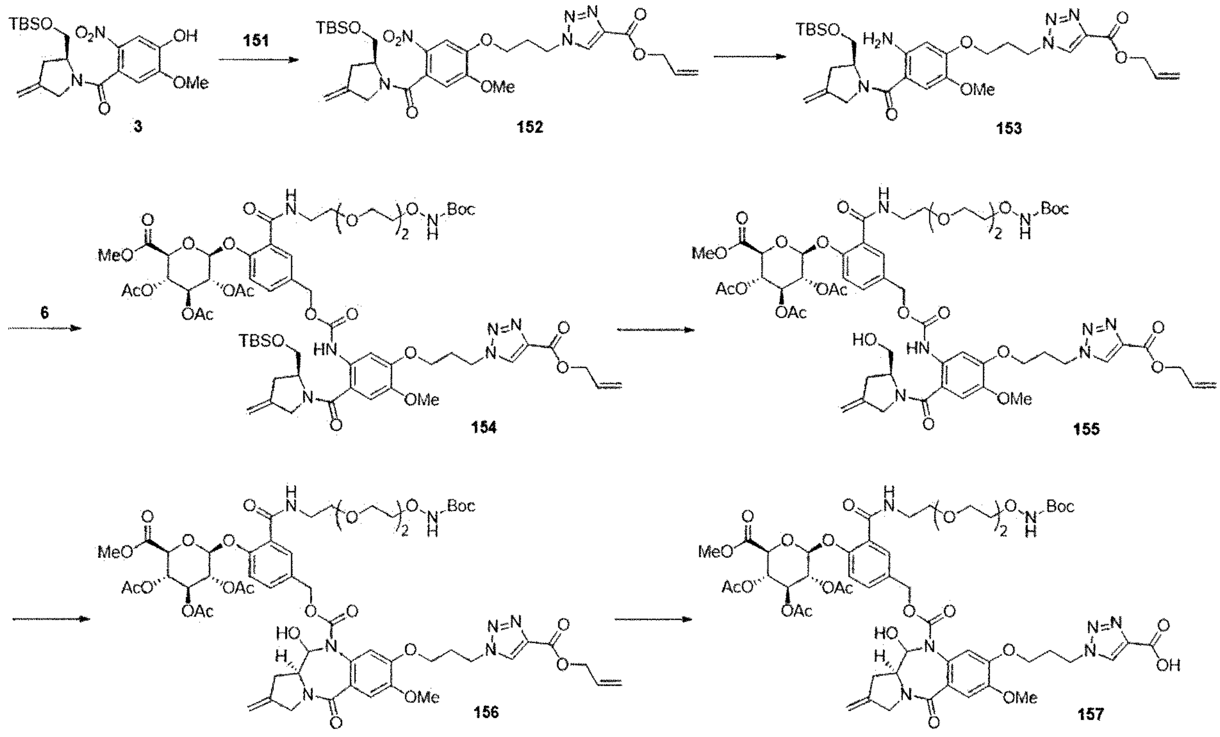

3 152 153 154 155 156 157

## Preparation of Compound 152

Compound 3 (528 mg, 1.25 mmol) and Compound 151 (361 mg, 1.25 mmol) were dissolved in N,N-dimethylformamide (12 mL), then cesium carbonate (488 mg, 1.49 mmol) was added at 0°C under a nitrogen atmosphere, and the mixture was stirred at 60°C for 2 hours. Saturated aqueous ammonium chloride solution (10 mL) was added to the reaction mixture, followed by extraction with ethyl acetate (40 mL), washing with distilled water (10 mL × 2) and brine (10 mL), and drying over anhydrous sodium sulfate. After filtration and concentration, the product was purified by column chromatography, thereby obtaining Compound 152 (455 mg, 60%). $^1$H-NMR (400 MHz, $CDCl_3$) (rotamers) δ 8.25 (s, 1H), 7.69 (d, 2H), 6.81 (d, 1H), 6.09-6.00 (m, 1H), 5.45 (d, 1H), 5.32 (d, 1H), 5.11-5.00 (m, 1H), 4.87 (d, 2H), 4.71 (t, 2H), 4.61 (t, 1H), 4.16-4.15 (m, 2H), 3.97 (s, 3H), 3.79-3.71 (m, 2H), 2.79-2.69 (m, 2H), 2.58-2.52 (m, 2H). EI-MS m/z : $[M+H]^+$616.4.

## Preparation of Compound 153

Compound 152 (455 mg, 0.73 mmol) was dissolved in ethyl acetate (6 mL), then zinc dust (1.5 g, 22.2 mmol) and formic acid solution (0.56 mL, 14.8 mmol) diluted with ethanol (6 mL) were sequentially added at 0°C under a nitrogen atmosphere, and the mixture was stirred at room temperature for 3 hours. The reaction mixture was filtered through Celite and then concentrated under reduced pressure. The filtered solution was basified with saturated aqueous sodium hydrogen carbonate solution (pH of about 8) and subjected to extraction with ethyl acetate, followed by drying over anhydrous sodium sulfate. After filtration and concentration under reduced pressure, the product was purified by column chromatography, thereby obtaining Compound 153 (406 mg, 94%). $^1$H-NMR (400 MHz, $CDCl_3$) (rotamers) δ 8.26 (s, 1H), 6.76 (s, 1H), 6.21 (s, 1H), 6.07-5.98 (m, 1H), 5.41 (dd, J = 17.2 Hz, 1.6 Hz, 1H), 5.29 (dd, J = 10.4 Hz, 1.6 Hz, 1H), 4.98 (bs, 1H), 4.91 (bs, 1H), 4.86 (d, *J* = 6 Hz, 2H), 4.59 (t, 2H), 4.26-4.19 (m, 2H), 3.96 (t, 2H), 3.79 (s, 3H), 2.71 (m, 2H), 2.48-2.42 (m, 2H), 0.87 (s, 9H), 0.03 (s, 6H). EI-MS m/z : $[M+H]^+$587.5.

## Preparation of Compound 154

Compound 153 (406 mg, 0.69 mmol) was dissolved in dichloromethane (12 mL), then triphosgene (74 mg, 0.25 mmol) and triethylamine (0.29 mL, 2.07 mmol) were added at 0°C, and the mixture was stirred for 10 minutes. A solution of Compound 6 (607 mg, 0.83 mmol) and triethylamine (0.19 mL, 1.38 mmol) in dichloromethane (8 mL) was added to the reaction solution, the temperature was gradually raised, and the mixture was stirred at room temperature for 15 hours. The reaction mixture was diluted with dichloromethane (20 mL), distilled water (20 mL) was added, and then extraction was performed. The aqueous layer was extracted with dichloromethane (20 mL × 2), and the collected

organic layers were washed with brine (20 mL) and dried over anhydrous sodium sulfate. After filtration and concentration under reduced pressure, the product was purified by column chromatography, thereby obtaining Compound 154 (780 mg, 84%). $^1$H-NMR (400 MHz, $CDCl_3$) δ 8.46 (s, 1H), 8.04-8.03 (m, 1H), 7.84 (s, 1H), 7.51-7.42 (m, 2H), 7.04-7.02 (m, 1H), 6.82 (s, 1H), 6.07-6.01 (m, 1H), 5.44-5.25 (m, 6H), 5.12 (s, 2H), 4.98 (m, 1H), 4.87-4.86 (m, 3H), 4.59 (t, J =6.8 Hz, 2H), 4.20-4.11 (m, 5H), 4.01-3.97 (m, 3H), 3.83 (s, 3H), 3.74-3.53 (m, 18H), 2.69 (m, 2H), 2.47 (m, 2H), 2.05-2.03 (m, 9H), 1.85-1.83 (m, 2H), 1.47-1.46 (m, 9H), 0.87 (s, 9H), 0.03 (s, 6H). EI-MS m/z : $[M+H]^+$1343.3.

**Preparation of Compound 155**

Compound 154 (780 mg, 0.58 mmol) was dissolved in tetrahydrofuran/distilled water (3 mL/3 mL), acetic acid (6 mL) was added at 0°C, and the mixture was stirred at room temperature for 15 hours. After concentration under reduced pressure, the reaction mixture was diluted with saturated sodium hydrogen carbonate solution (50 mL), followed by extraction with ethyl acetate (50 mL × 3). The extracted solution was dried over anhydrous sodium sulfate, then filtered, concentrated under reduced pressure, and purified by column chromatography, thereby obtaining Compound 155 (460 mg, 63%). EI-MS m/z : $[M+H]^+$1229.1.

**Preparation of Compound 156**

Compound 155 (460 mg, 0.37 mmol) was dissolved in dichloromethane (5 mL), then Dess-Matin periodinane (190 mg, 0.45 mmol) was added at 0°C, the temperature was raised to room temperature, and the mixture was stirred for 3 hours under a nitrogen atmosphere. The reaction mixture was diluted with saturated aqueous sodium thiosulfate solution/saturated aqueous sodium hydrogen carbonate solution (v/v=1/1, 30 mL) and stirred for 10 minutes. After extraction with dichloromethane (20 mL × 3), washing with distilled water (20 mL) and aqueous sodium chloride solution (20 mL) was performed. Drying over anhydrous sodium sulfate, filtration, concentration, and purification by column chromatography were performed, thereby obtaining Compound 156 (340 mg, 72%). EI-MS m/z : $[M+H]^+$1226.9.

**Preparation of Compound 157**

Compound 156 (340 mg, 0.27 mmol) was dissolved in N,N-dimethylformamide (3.5 mL), then tetrakis(triphenylphosphine)palladium(0) (128 mg, 0.11 mmol) and pyrrolidine (0.04 mL, 0.54 mmol) were added at 0°C under a nitrogen atmosphere, and the mixture was stirred at room temperature for 1 hour. Saturated aqueous ammonium chloride solution (10 mL) was added to the reaction mixture, followed by extraction with ethyl acetate (20 mL × 3) and drying over anhydrous sodium sulfate. Compound 157 (165 mg) obtained after filtration and concentration under reduced pressure was used in the next reaction without further purification. EI-MS m/z : $[M+H]^+$1187.1.

**<Example 51> Preparation of Compound 158**

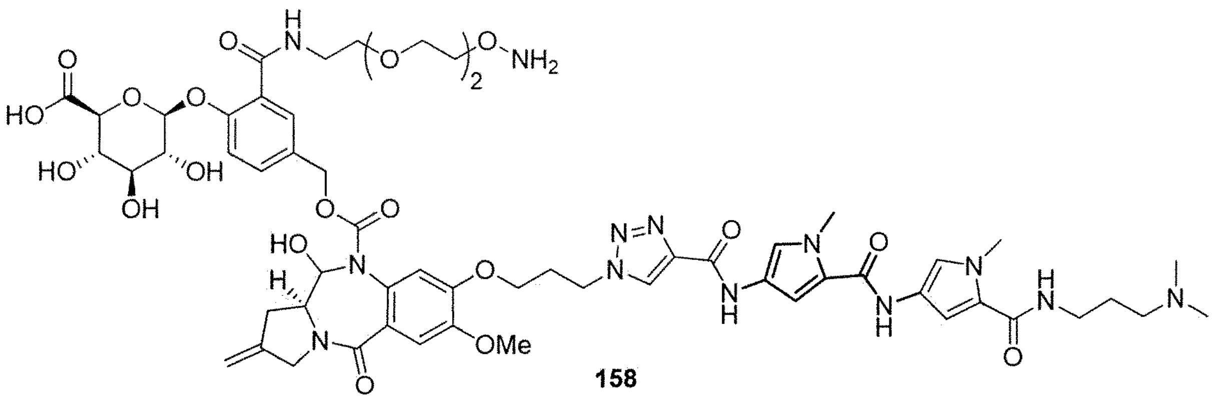

**158**

Compound 158 was synthesized from Compound 157 and Compound 20 in a similar manner to the synthesis of Compound 23. EI-MS m/z : $[M+H]^+$1375.3.

**<Example 52> Preparation of Compound 161**

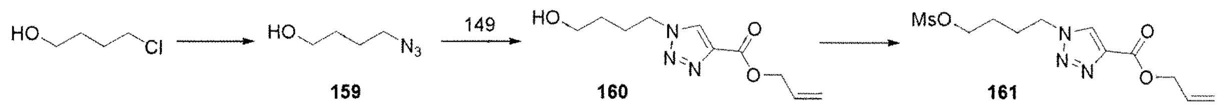


159 160 161

**Preparation of Compound 159**

After 4-chloro-1-butanol (3 g, 27.6 mmol) was dissolved in N,N-dimethylformamide (30 mL), then sodium azide (3.58 g, 55.2 mmol) was added at 0°C under a nitrogen atmosphere, and the mixture was heated to 70°C and stirred for 17 hours. The reaction mixture was diluted with diethyl ether (30 mL), washed with distilled water (50 mL) and brine (50 mL), and then dried over anhydrous sodium sulfate. Filtration and concentration under reduced pressure were performed, thereby obtaining Compound 159 (2.21 g, 69%). $^1$H-NMR(400 MHz, $CDCl_3$) $\delta$ 3.70 (m, 2H), 3.33 (t, *J* =6.4 Hz, 2H), 1.73-1.64 (m, 4H).

**Preparation of Compound 160**

Compound 149 (1 g, 9.1 mmol) and Compound 159 (1.04 g, 9.1 mmol) were dissolved in tertiary butyl alcohol/distilled water (12 mL/3 mL) at 0°C under a nitrogen atmosphere, followed by stirring. Copper sulfate pentahydrate (227 mg, 0.91 mmol) and sodium L-ascorbate (901 mg, 4.55 mmol) were dissolved in distilled water (3 mL) and then added to the reaction solution, and the mixture was stirred at room temperature for 17 hours. The reaction product was filtered through Celite, and then concentrated under reduced pressure. The filtered solution was subjected to extraction with dichloromethane (20 mL $\times$ 3), followed by drying over anhydrous sodium sulfate, filtration, and concentration under reduced pressure. The product was purified by column chromatography, thereby obtaining Compound 160 (997 mg, 48%). $^1$H-NMR (400 MHz, $CDCl_3$) $\delta$ 8.11 (s, 1H), 6.09-5.99 (m, 1H), 5.42 (dd, J = 17.2 Hz, 1.6 Hz, 1H), 5.31 (dd, J = 10.8 Hz, 1.2 Hz, 1H), 4.86 (d, J =6 Hz, 2H), 4.49 (t, J =7.2 Hz, 2H), 3.71 (q, J =4.8 Hz, 2H), 2.07 (m, 2H), 1.60 (m, 2H), 1.35 (br, 1H). EI-MS m/z : $[M+H]^+$226.20, $[M/2+H]^+$113.82.

**Preparation of Compound 161**

Compound 160 (450 mg, 1.99 mmol) was dissolved in dichloromethane (10 mL), then methanesulfonyl chloride (251 mg, 0.17 mL) and triethylamine (404 mg, 3.99 mmol) were sequentially added at 0°C under a nitrogen atmosphere, and the mixture was stirred at 0°C for 1 hour. Saturated aqueous ammonium chloride solution (10 mL) was added to the reaction mixture, followed by extraction with ethyl acetate (20 mL $\times$ 3), and the collected organic layers were washed with distilled water (10 mL) and brine (10 mL) and then dried over anhydrous sodium sulfate. Filtration and concentration were performed, thereby obtaining Compound 161 (606 mg). $^1$H-NMR (400 MHz, $CDCl_3$) $\delta$ 8.11 (s, 1H), 6.09-5.99 (m, 1H), 5.42 (dd, J = 17.2 Hz, 1.2 Hz, 1H), 5.31 (dd, *J* = 10.8 Hz, 1.2 Hz, 1H), 4.86 (d, *J* =6 Hz, 2H), 4.49 (t, J =7.2 Hz, 2H), 4.27 (t, J =6 Hz, 2H), 3.02 (s, 3H), 2.12 (m, 2H), 1.81 (m, 2H).

**<Example 53> Preparation of Compound 162**

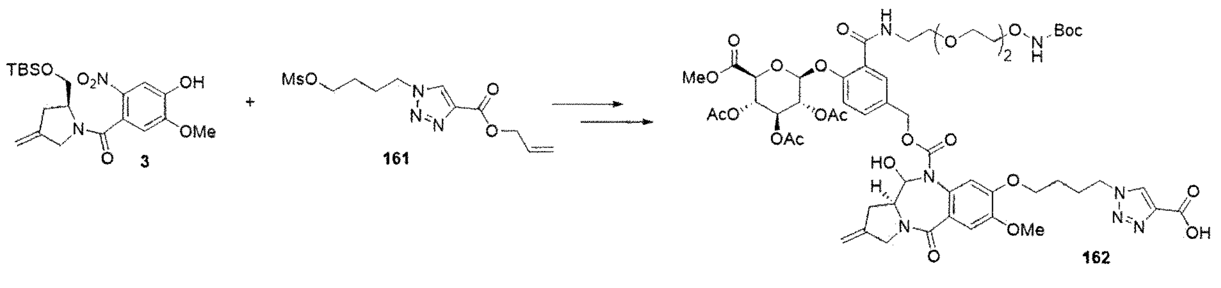

3 + 161 162

Compound 162 was synthesized from Compound 3 and Compound 161 in a similar manner to the synthesis of Compound 157. EI-MS m/z : $[M+H]^+$1200.91.

**<Example 54> Preparation of Compound 163**

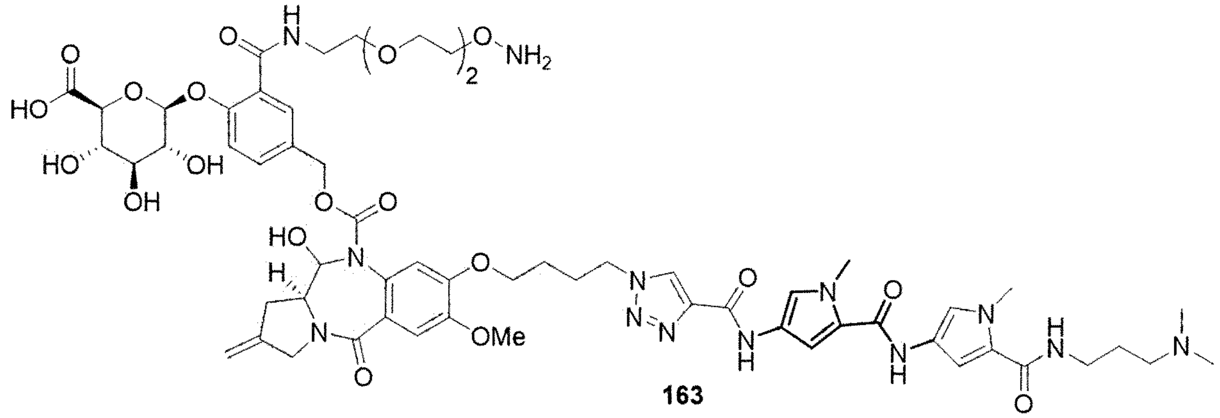

163

Compound 163 was synthesized from Compound 158 and Compound 20 in a similar manner to the synthesis of Compound 23. $^1$H-NMR (400 MHz, Methanol-$d_4$) $\delta$ 8.53 (s, 1H), 7.75 (s, 1H), 7.35 (m, 1H), 7.29-7.27 (m, 2H), 7.18-7.15 (m, 2H), 7.01 (s, 1H), 6.90 (s, 1H), 6.73 (s, 1H), 5.57 (d, J =7.2 Hz, 1H), 5.28-5.24 (m, 1H), 5.17-5.11 (m, 3H), 4.57 (m, 2H), 4.23-4.06 (m, 6H), 3.92-3.89 (m, 11H), 3.78 (m, 3H), 3.67-3.54 (m, 15H), 3.39 (t, J =6.4 Hz, 2H), 3.17 (t, J =7.6 Hz, 2H), 2.91 (s, 8H), 2.10 (m, 2H), 2.02-1.97 (m, 3H), 1.75 (m, 2H). EI-MS m/z : $[M+H]^+$1289.09, $[M/2+H]^+$645.13.

**<Example 55> Preparation of Compound 166**

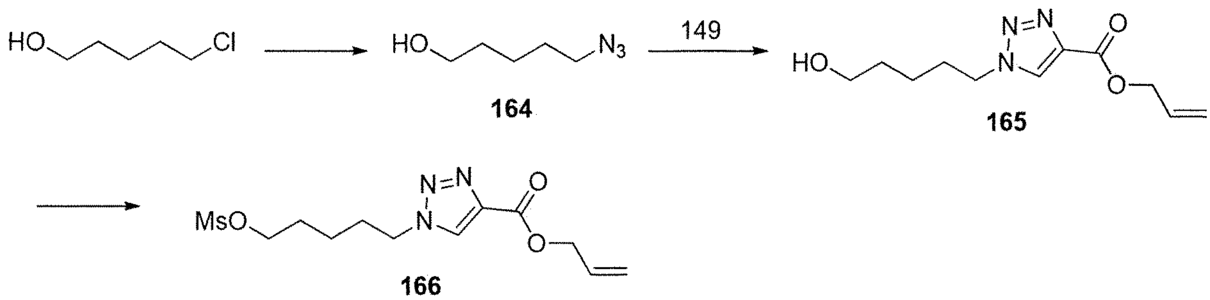


164

165

166

## Preparation of Compound 164

After 1-chloro-5-hydroxypentane (1 g, 8.15 mmol) was dissolved in N,N-dimethylformamide (30 mL), then sodium azide (2.1 g, 32.6 mmol) was added at 0°C under a nitrogen atmosphere, and the mixture was heated to 80°C and stirred for 15 hours. The reaction mixture was diluted with diethyl ether (30 mL), washed with distilled water (50 mL) and brine (50 mL), and then dried over anhydrous sodium sulfate. Filtration and concentration under reduced pressure were performed, thereby obtaining Compound 164 (980 mg, 93%). $^1$H-NMR (400 MHz, $CDCl_3$) $\delta$ 3.66 (t, J = 6 Hz 2H), 3.29 (t, J = 6.8 Hz, 2H), 1.68-1.53 (m, 4H), 1.54-1.42 (m, 2H).

## Preparation of Compound 165

Compound 149 (919 mg, 8.34 mmol) and Compound 164 (980 mg, 7.58 mmol) were dissolved in tertiary butyl alcohol/distilled water (16 mL/4 mL) at 0°C under a nitrogen atmosphere, followed by stirring. Copper sulfate pentahydrate (189 mg, 0.75 mmol) and sodium L-ascorbate (751 mg, 3.79 mmol) were dissolved in distilled water (1 mL) and then added to the reaction solution, and the mixture was stirred at room temperature for 5 hours. The reaction product was filtered through Celite, and then concentrated under reduced pressure. The filtered solution was subjected to extraction with dichloromethane (20 mL $\times$ 3), followed by drying over anhydrous sodium sulfate, filtration, and concentration under reduced pressure. The product was purified by column chromatography, thereby obtaining Compound 165 (920 mg,

50%). $^1$H-NMR(400 MHz, $CDCl_3$) δ 8.09 (s, 1H), 6.09-5.99 (m, 1H), 5.42 (dd, *J* = 17.2 Hz, 1.6 Hz, 1H), 5.31 (dd, J = 10.8 Hz, 1.2 Hz, 1H), 4.86 (d, J = 6 Hz, 2H), 4.43 (t, *J* = 7.2 Hz, 2H), 3.65 (bs, 2H), 1.98 (quin, 2H), 1.61 (quin, 2H), 1.41 (quin, 2H).

## Preparation of Compound 166

Compound 165 (920 mg, 3.84 mmol) was dissolved in dichloromethane (40 mL), then methanesulfonyl chloride (0.32 ml, 4.22 mmol) and triethylamine (1.07 ml, 7.68 mmol) were added at 0°C under a nitrogen atmosphere, and the mixture was stirred at 0°C for 2 hours. Saturated aqueous ammonium chloride solution (40 mL) was added to the reaction mixture, followed by extraction with dichloromethane (20 mL × 2), washing with distilled water (10 mL) and brine (10 mL), and drying over anhydrous sodium sulfate. The product was filtered and concentrated, and used in the next reaction without purification. $^1$H-NMR(400 MHz, $CDCl_3$) δ 8.11 (s, 1H), 6.09-5.99 (m, 1H), 5.42 (dd, J = 17.2 Hz, 1.2 Hz, 1H), 5.31 (dd, *J* = 10.8 Hz, 1.2 Hz, 1H), 4.86 (d, *J* = 6 Hz, 2H), 4.44 (t, *J* = 7.2 Hz, 2H), 4.22 (t, J = 6 Hz, 2H), 3.01 (s, 3H), 2.01 (quin, J = 7.6 Hz, 2H), 1.81 (quin, J = 7.6 Hz, 2H), 1.512-1.452 (m, 2H).

**<Example 56> Preparation of Compound 167**

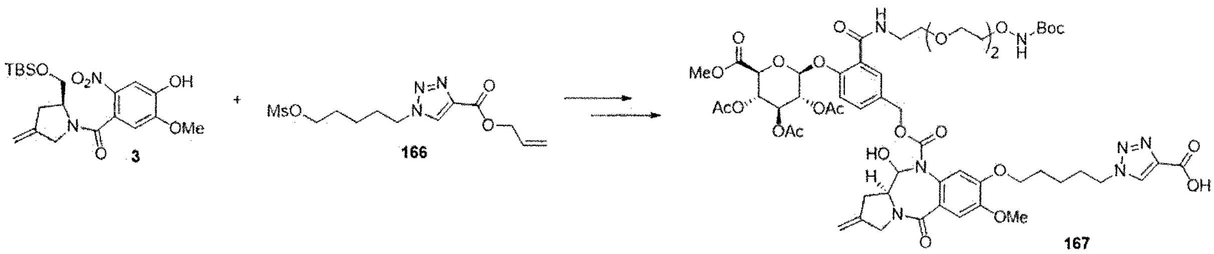

3 166 167

Compound 167 was synthesized from Compound 3 and Compound 166 in a similar manner to the synthesis of Compound 157. EI-MS m/z : $[M+H]^+$1215.2.

**<Example 57> Preparation of Compound 168**

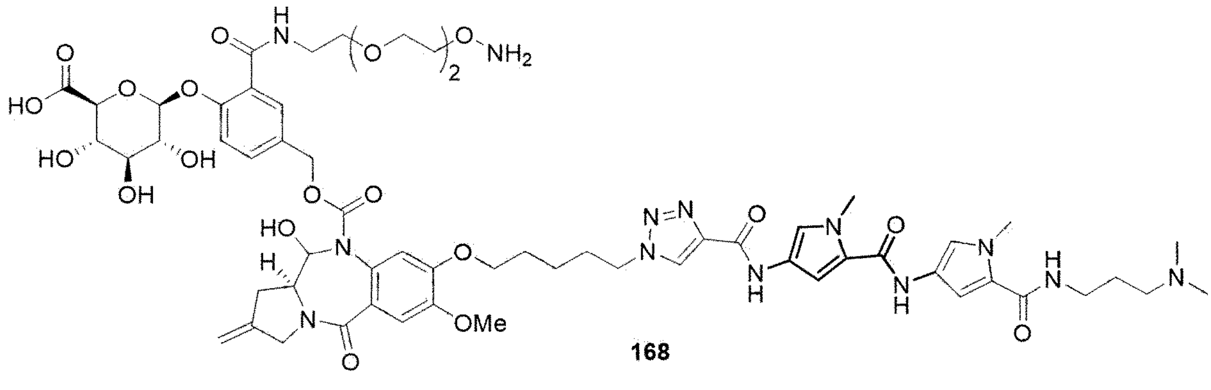

168

Compound 168 was synthesized from Compound 167 and Compound 20 in a similar manner to the synthesis of Compound 23. EI-MS m/z : $[M+H]^+$1303.4, $[M+Na]^+$ 652.1

**<Example 58> Preparation of Compound 171**

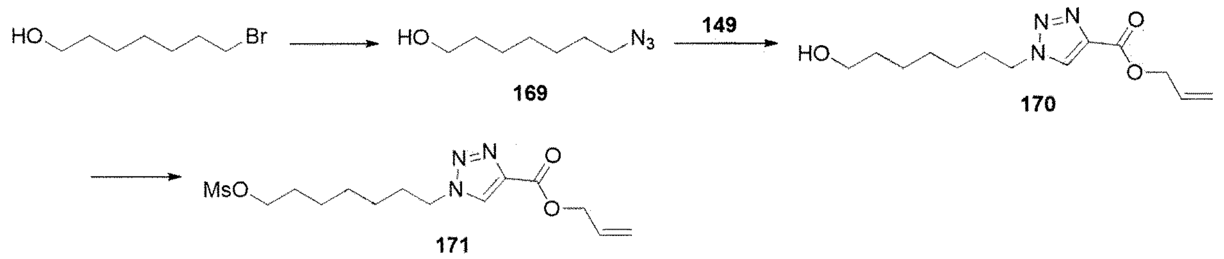


## Preparation of Compound 169

After 1-bromo-7-hydroxyheptane (1 g, 5.12 mmol) was dissolved in N,N-dimethylformamide (30 mL), then sodium azide (666 mg, 10.2 mmol) was added at 0°C under a nitrogen atmosphere, and the mixture was heated to 80°C and stirred for 15 hours. The reaction mixture was diluted with ethyl acetate (30 mL), washed with distilled water (50 mL) and brine (50 mL), and then dried over anhydrous sodium sulfate. Filtration and concentration under reduced pressure were performed, thereby obtaining Compound 169 (800 mg, 99%). $^1$H-NMR (400 MHz, $CDCl_3$) δ 3.64 (t, *J* = 6 Hz, 2H), 3.26 (t, *J* = 6.8 Hz, 2H), 1.61-1.54 (m, 4H), 1.42-1.36 (m, 6H).

## Preparation of Compound 170

Compound 149 (564 mg, 5.12 mmol) and Compound 169 (800 mg, 5.12 mmol) were dissolved in tertiary butyl alcohol/distilled water (8 mL/2 mL) at 0°C under a nitrogen atmosphere, followed by stirring. Copper sulfate pentahydrate (127 mg, 0.51 mmol) and sodium L-ascorbate (507 mg, 2.5 mmol) were dissolved in distilled water (1 mL) and then added to the reaction solution, and the mixture was stirred at room temperature for 5 hours. The reaction product was filtered through Celite, and then concentrated under reduced pressure. The filtered solution was subjected to extraction with dichloromethane (20 mL × 3), followed by drying over anhydrous sodium sulfate, filtration, and concentration under reduced pressure. The product was purified by column chromatography, thereby obtaining Compound 170 (566 mg, 41%). $^1$H-NMR(400 MHz, $CDCl_3$) δ 8.09 (s, 1H), 6.09-5.99 (m, 1H), 5.42 (dd, *J* = 17.2 Hz, 1.6 Hz, 1H), 5.31 (dd, J = 10.8 Hz, 1.2 Hz, 1H), 4.86 (d, J = 6 Hz, 2H), 4.41 (t, *J* = 7.2 Hz, 2H), 3.63 (bs, 2H), 1.98 (quin, 2H), 1.61-1.54 (m, 4H), 1.42-1.36 (m, 6H).

## Preparation of Compound 171

Compound 170 (566 mg, 2.11 mmol) was dissolved in dichloromethane (20 mL), then methanesulfonyl chloride (0.19 mL, 2.54 mol) and triethylamine (0.59 ml, 4.23 mmol) were added at 0°C under a nitrogen atmosphere, and the mixture was stirred at 0°C for 2 hours. Saturated aqueous ammonium chloride solution (20 mL) was added to the reaction mixture, followed by extraction with dichloromethane (20 mL × 2), washing with distilled water (10 mL) and brine (10 mL), and drying over anhydrous sodium sulfate. The product was filtered and concentrated, and used in the next reaction without purification. $^1$H-NMR(400 MHz, $CDCl_3$) δ 8.09 (s, 1H), 6.09-5.99 (m, 1H), 5.42 (dd, J = 17.2 Hz, 1.6 Hz, 1H), 5.31 (dd, *J* = 10.8 Hz, 1.2 Hz, 1H), 4.86 (d, J =6 Hz, 2H), 4.41 (t, J =7.2 Hz, 2H), 3.63 (bs, 2H), 3.01 (s, 3H), 1.98 (quin, 2H), 1.61-1.54 (m, 4H), 1.42-1.36 (m, 6H) .

## <Example 59> Preparation of Compound 172

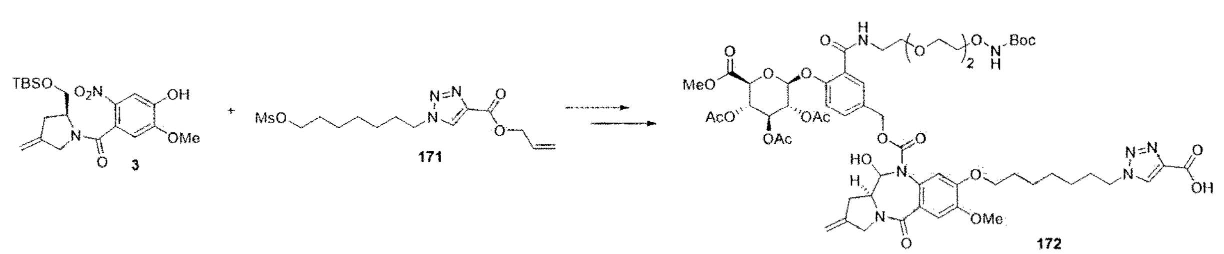


Compound 172 was synthesized from Compound 3 and Compound 171 in a similar manner to the synthesis of Compound 157. EI-MS m/z : [M+H]$^+$1243.2.

**<Example 60> Preparation of Compound 173**

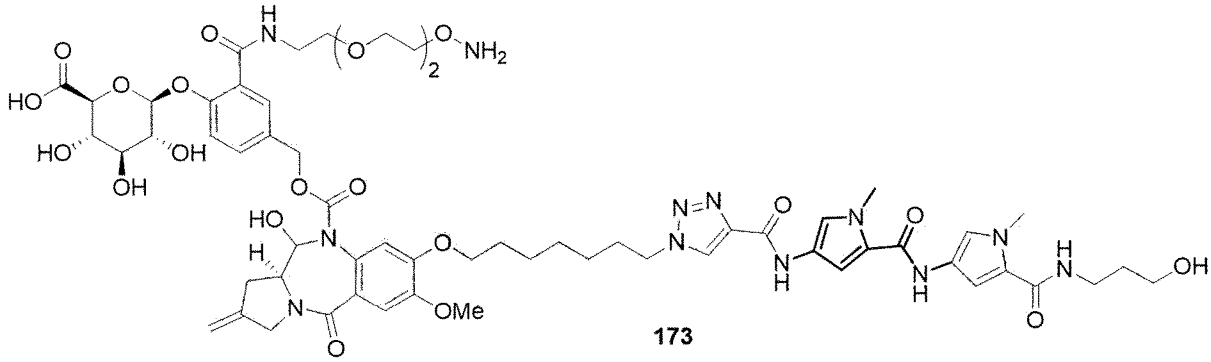

173

Compound 173 was synthesized from Compound 167 and Compound 26 in a similar manner to the synthesis of Compound 29. EI-MS m/z : $[M+H]^+$1467.8.

**<Example 61> Preparation of Compound 175**

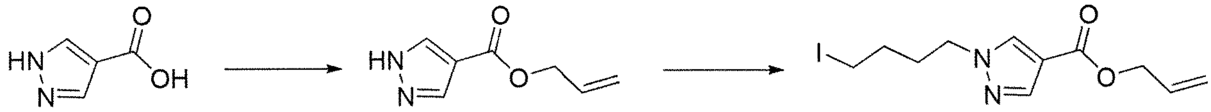

174 175

**Preparation of Compound 174**

After 4-pyrazolecarboxylic acid (500 mg, 4.46 mmol) was dissolved in N,N-dimethylformamide (5 mL) and dichloromethane (5 mL), then allyl alcohol (0.37 mL, 5.35 mmol), 4-dimethylaminopyridine (54 mg, 0.45 mmol), and 1,3-diisopropylcarbodiimide (0.83 mL, 5.35 mmol) were added, and the mixture was stirred at room temperature for 17 hours, heated to 60°C, and further stirred for 3 hours. The reaction mixture was diluted with ethyl acetate (100 mL), washed with distilled water (2 × 100 mL), and then dried over anhydrous sodium sulfate. After filtration and concentration under reduced pressure, the product was purified by column chromatography, thereby obtaining Compound 174 (328 mg, 48%). $^1$H-NMR(400 MHz, $CDCl_3$) δ 8.07 (s, 1H), 6.04-5.96 (m, 1H), 5.38 (d, 1H), 5.27 (d, 1H), 4.76 (d, 2H).

**Preparation of Compound 175**

Compound 174 (328 mg, 2.16 mmol) and 1,4-diodobutane (0.71 mL, 5.39 mmol) were dissolved in *N,N*-dimethylformamide (20 mL), then cesium carbonate (913 mg, 2.80 mmol) was added, and the mixture was stirred at room temperature for 2 hours. The reaction mixture was diluted with ethyl acetate (100 mL), washed with distilled water (2 × 100 mL), and then dried over anhydrous sodium sulfate. After filtration and concentration under reduced pressure, the product was purified by column chromatography, thereby obtaining Compound 175 (535 mg, 74%). $^1$H-NMR(400 MHz, $CDCl_3$) δ 7.93 (s, 1H), 7.90 (s, 1H), 6.03-5.96 (m, 1H), 5.37 (d, 1H), 5.27 (d, 1H), 4.74 (d, 2H), 4.16 (t, 2H), 3.18 (t, 2H), 2.05-1.98 (m, 2H), 1.84-1.79 (m, 2H).

**<Example 62> Preparation of Compound 176**

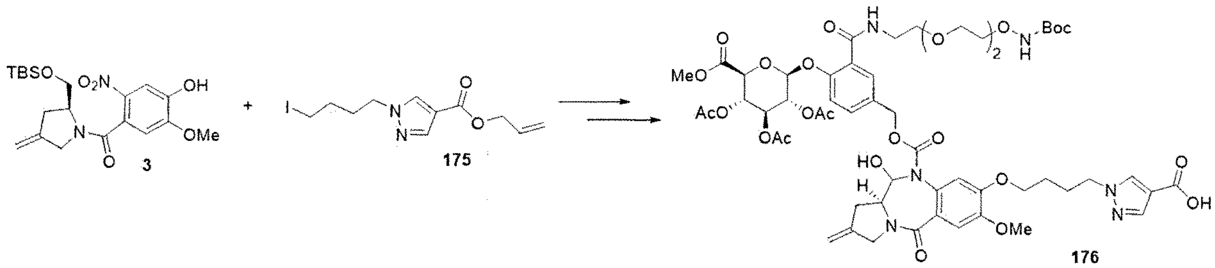


Compound 176 was synthesized from Compound 3 and Compound 175 in a similar manner to the synthesis of Compound 157. EI-MS m/z : $[M+H]^+$1200.21.

**<Example 63> Preparation of Compound 177**

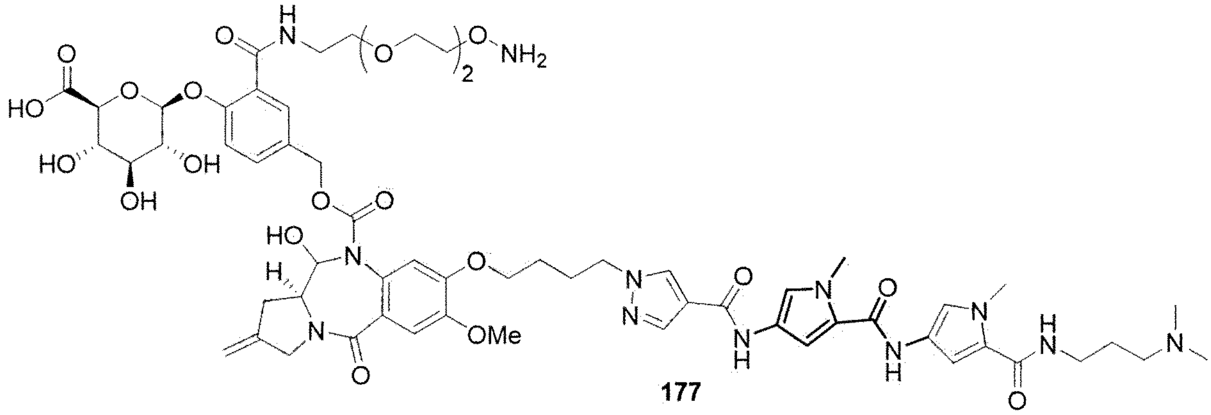

177

Compound 177 was synthesized from Compound 176 and Compound 20 in a similar manner to the synthesis of Compound 23. EI-MS m/z : $[M+H]^+$1288.24, 1/2$[M+H]^+$644.73.

**<Example 64> Preparation of Compound 181**

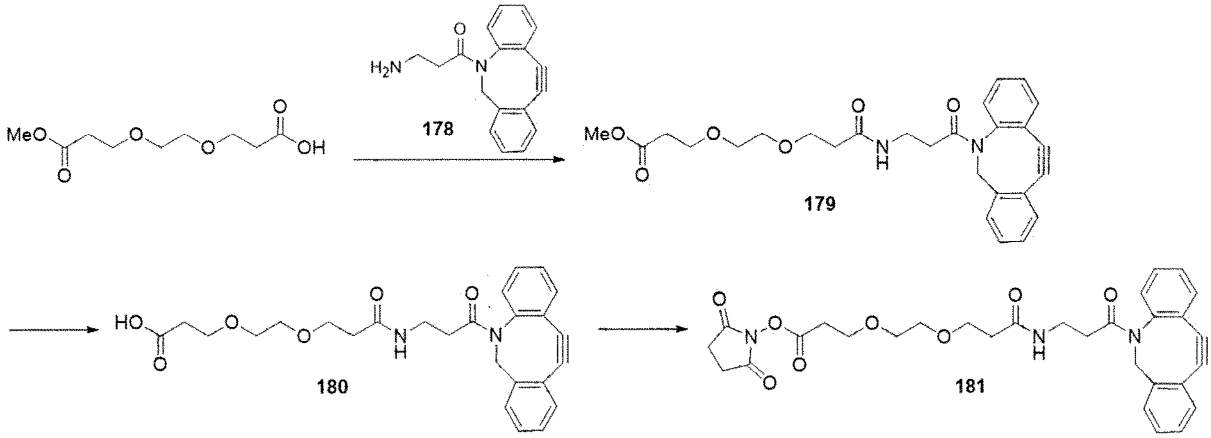


<u>**Preparation of Compound 179**</u>

After 3-(2-(3-methoxy-3-oxopropoxy)ethoxy)propanoic acid (200 mg, 0.85 mmol) was dissolved in dichloromethane (4 mL), then *N,N,N',N'*-tetramethyl-*O*-(1*H*-benzotriazol-1-yl)uronium hexafluorophosphate (HBTU, 440 mg, 1.11

mmol), *N,N'*-diisopropylethylamine (0.22 mL, 1.28 mmol), and Compound 178 (259 mg, 0.94 mmol) were sequentially added at 0°C under a nitrogen atmosphere, and the mixture was stirred at room temperature for 5 hours. Saturated aqueous sodium hydrogen carbonate solution (10 mL) was added to the reaction mixture, followed by extraction with dichloromethane (20 mL × 3), washing with distilled water (10 mL) and brine (10 mL), and drying over anhydrous sodium sulfate. After filtration and concentration, the product was purified by column chromatography, thereby obtaining Compound 179 (391 mg, 93%). $^1$H-NMR(400 MHz, $CDCl_3$) δ 7.68 (d, 1H), 7.44-7.24 (m, 7H), 6.53 (t, 1H), 5.14 (d, 1H), 4.15 (q, 2H), 3.79-3.44 (m, 9H), 3.42-3.30 (m, 1H), 3.28-3.14 (m, 2H), 2.59 (t, 2H), 2.56-2.46 (m, 1H), 2.33 (t, 2H), 2.02-1.92 (m, 2H), 1.25 (t, 3H).

**Preparation of Compound 180**

Compound 179 (391 mg, 0.79 mmol) was dissolved in methanol/tetrahydrofuran (5 mL/5 mL), then a solution of lithium hydroxide (50 mg, 1.19 mmol) in distilled water (5 mL) was gradually added at 0°C under a nitrogen atmosphere, and the mixture was stirred for 2 hours. The pH was adjusted to about 4 with 0.1 N aqueous hydrochloric acid solution, followed by extraction with ethyl acetate (20 mL × 3), and the collected organic layers were dried over anhydrous sodium sulfate. Filtration and concentration were performed, thereby obtaining Compound 180 (380 mg, 100%). $^1$H-NMR(400 MHz, $CDCl_3$) δ 7.66 (d, 1H), 7.46-7.22 (m, 7H), 6.83 (t, 1H), 5.15 (d, 1H), 3.79-3.44 (m, 9H), 3.42-3.32 (m, 1H), 3.28-3.12 (m, 2H), 2.56 (t, 2H), 2.51 (t, 1H), 2.42-2.32 (m, 2H), 2.12 (t, 1H).

**Preparation of Compound 181**

Compound 180 (200 mg, 0.36 mmol) was dissolved in dichloromethane (5 mL), then N-hydroxysuccinimide (50 mg, 0.43 mmol) and 1-ethyl-3-(3-dimethylaminopropyl)carbodiimamide (84 mg, 0.43 mmol) were added at 0°C under a nitrogen atmosphere, and the mixture was stirred at room temperature for 2 hours. The reaction was terminated with distilled water (10 mL), followed by extraction with dichloromethane (10 mL × 2), washing with distilled water (10 mL) and brine (10 mL), and then drying over anhydrous sodium sulfate. After filtration and concentration, Compound 181 (220 mg, crude) was used in the next reaction without purification. EI-MS m/z : $[M+H]^+$562.5.

**<Example 65> Preparation of Compound 186**

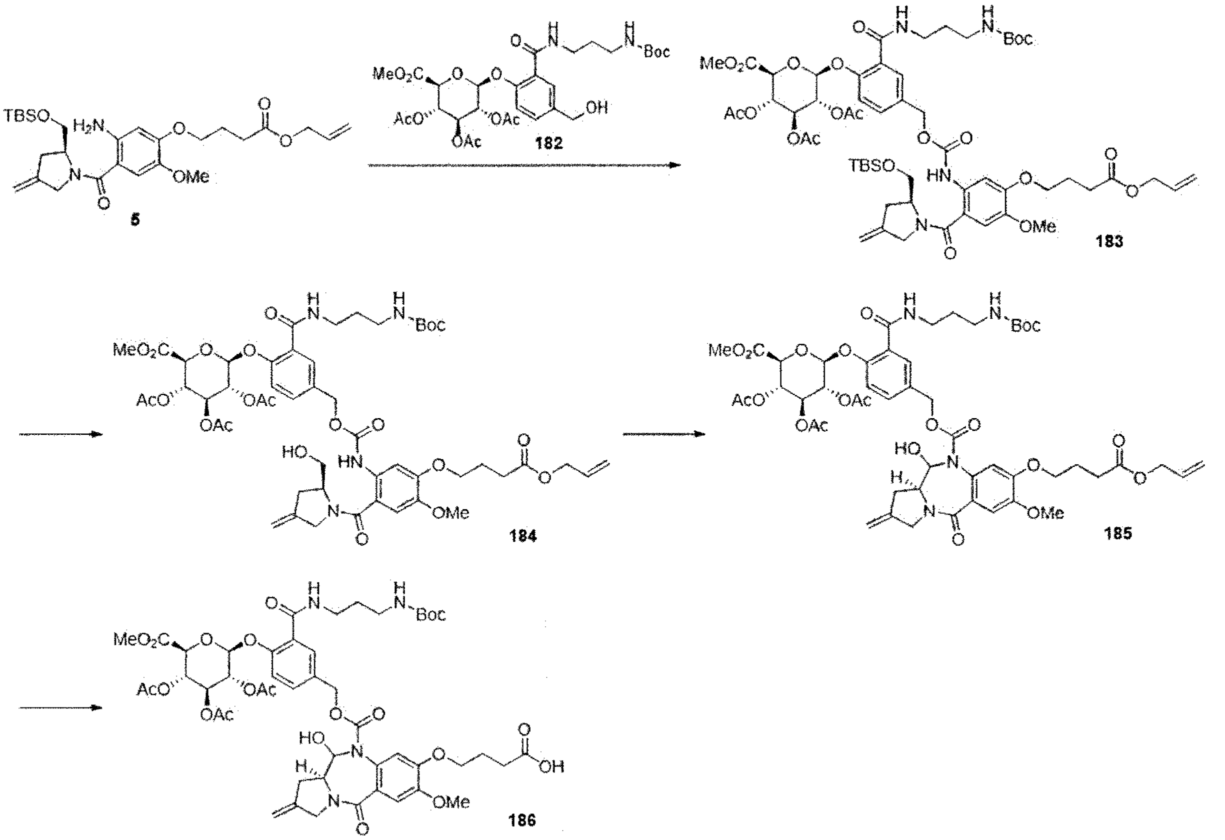

5 182 183 184 185 186

### Preparation of Compound 183

Compound 5 (500 mg, 0.96 mmol) was dissolved in dry dichloromethane (20 mL), then triphosgene (206 mg, 0.69 mmol) and N,N-diisopropylethylamine (0.45 mL, 2.60 mmol) were added at 0°C, and Compound 182 (679 mg, 1.06 mmol, Compound 182 was prepared by the method described in Korean Patent Publication No. 10-2018-0110645) dissolved in dry dichloromethane (3 mL) was added. After stirring at room temperature for 17 hours, the reaction mixture was diluted with dichloromethane (30 mL), washed with distilled water (50 mL), and dried over anhydrous sodium sulfate. After filtration and concentration under reduced pressure, the product was purified by column chromatography, thereby obtaining Compound 183 (1.14 g, 79%). EI-MS m/z : $[M+H]^+$1185.8.

### Preparation of Compound 184

Compound 183 (899 mg, 0.76 mmol) was dissolved in tetrahydrofuran/distilled water (5 mL/5 mL), acetic acid (5 mL) was added at 0°C, and then the mixture was stirred at room temperature for 17 hours. The reaction mixture was diluted with ethyl acetate (10 mL), washed with distilled water (5 mL) and saturated sodium hydrogen carbonate solution (2 × 10 mL) in that order, and then dried over anhydrous sodium sulfate. After filtration and concentration under reduced pressure, the product was purified by column chromatography, thereby obtaining Compound 184 (812 mg, 86%). EI-MS m/z : $[M+H]^+$1071.7.

### Preparation of Compound 185

Compound 184 (700 mg, 0.65 mmol) was dissolved in dichloromethane (60 mL), Dess-Martin periodinane (333 mg, 0.78 mmol) was added, and the mixture was stirred at room temperature for 5 hours under a nitrogen atmosphere. The reaction mixture was diluted with dichloromethane (40 mL), washed with saturated aqueous sodium hydrogen carbonate solution (150 mL) and aqueous sodium thiosulfate solution (100 mL), and then dried over anhydrous sodium sulfate. After filtration and concentration under reduced pressure, the product was purified by column chromatography, thereby obtaining Compound 185 (460 mg, 66%). EI-MS m/z : $[M+H]^+$1069.9.

**Preparation of Compound 186**

Compound 185 (450 mg, 0.42 mmol) was dissolved in N,N-dimethylformamide (10 mL), then tetrakis(triphenylphosphine)palladium(0) (97 mg, 0.08 mmol) and pyrrolidine (0.04 mL, 0.51 mmol) were added, and the mixture was stirred at room temperature for 1 hour under a nitrogen atmosphere. The reaction mixture was diluted with ethyl acetate (50 mL), washed with saturated aqueous ammonium chloride solution (50 mL) and distilled water (50 mL), and dried over anhydrous sodium sulfate. Filtration and concentration under reduced pressure were performed, thereby obtaining Compound 186 (354 mg, 82%). EI-MS m/z : $[M+H]^+$1029.7.

**<Example 66> Preparation of Compound 190**

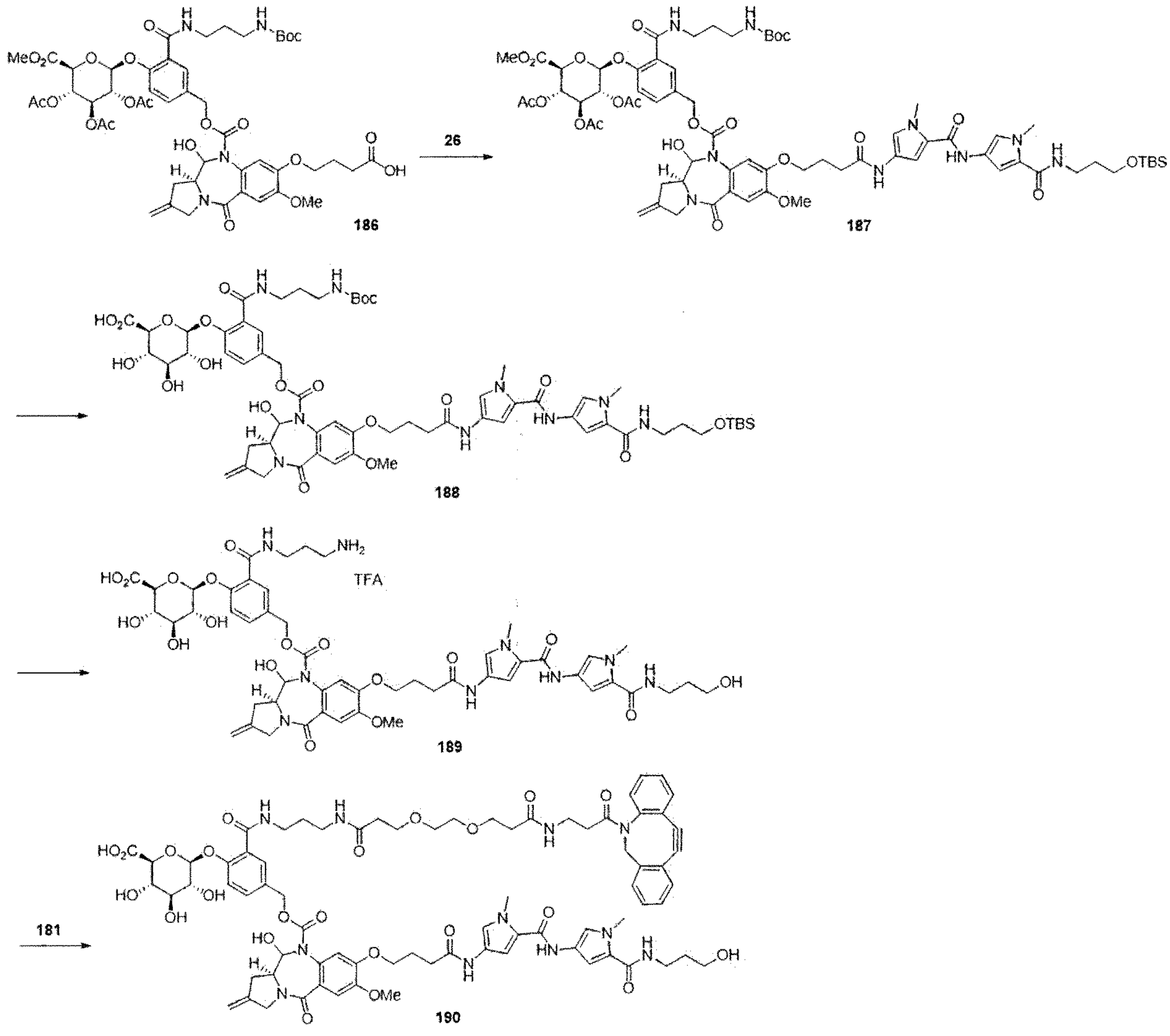


186 187 188 189 190

**Preparation of Compound 187**

Compound 186 (354 mg, 0.35 mmol) and Compound 26 (164 mg, 0.38 mmol) were dissolved in *N,N*-dimethylformamide (8 mL), *N,N,N',N'*-tetramethyl-*O*-(1*H*-benzotriazol-1-yl)uronium hexafluorophosphate (HBTU, 196 mg, 0.52 mmol) and *N,N'*-diisopropylethylamine (0.09 mL, 0.52 mmol) were sequentially added at 0°C under a nitrogen atmosphere, and the mixture was stirred at room temperature for 16 hours. Saturated aqueous ammonium chloride solution (10 mL) was added to the reaction mixture, followed by extraction with ethyl acetate (20 mL), washing with distilled water ($10 \times 2$ mL) and brine (10 mL), and drying over anhydrous sodium sulfate. After filtration and concentration, the product was purified by column chromatography, thereby obtaining Compound 187 (497 mg, 70%). EI-MS m/z : $[M+H]^+$1145.4

**Preparation of Compound 188**

Compound 187 (325 mg, 0.24 mmol) was dissolved in methanol (4 mL), and then a solution of sodium hydroxide (41 mg, 0.97 mmol) in distilled water (4 mL) was gradually added at -40°C under a nitrogen atmosphere. The mixture was stirred for 2 hours while the reaction temperature was gradually raised to 0°C. The reaction mixture was neutralized with acetic acid, then concentrated under reduced pressure, and freeze-dried, thereby obtaining Compound 188 (297 mg). EI-MS m/z : $[M+H]^+$1305.1.

**Preparation of Compound 189**

Compound 188 (297 mg, 0.11 mmol) was diluted with dichloromethane (5 mL), then trifluoroacetic acid (1 mL) was added at 0°C under a nitrogen atmosphere, and the mixture was stirred for 2 hours. The reaction mixture was concentrated under reduced pressure, then purified by HPLC, and freeze-dried, thereby obtaining Compound 189 as a white solid (51 mg, 41%). EI-MS m/z : $[M/2+H]^+$1090.9, $[M/2+H]^+$546.1.

**Preparation of Compound 190**

Compound 189 (51 mg, 0.047 mmol) and Compound 181 (29 mg, 0.052 mmol) were dissolved in *N,N*-dimethylformamide (2.7 mL), then N,*N*-diisopropylethylamine (0.02 mL, 0.12 mmol) was added sequentially at 0°C under a nitrogen atmosphere, and the mixture was stirred at room temperature for 4 hours. The reaction mixture was concentrated under reduced pressure, then purified by HPLC, and freeze-dried, thereby obtaining Compound 190 as a white solid (38 mg, 52%). EI-MS m/z : $[M/2+H]^+$769.4

**<Example 67> Preparation of Compound 192**

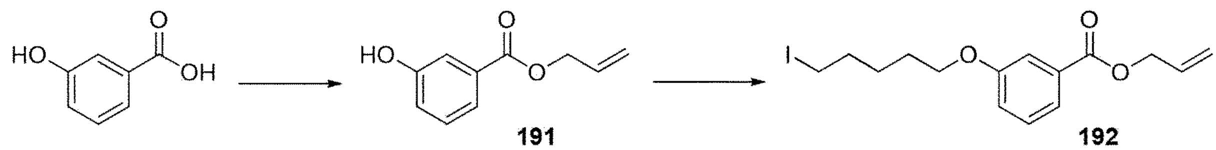

**Preparation of Compound 191**

After 3-hydroxybenzoic acid (2.0 g, 14.5 mmol) was dissolved in distilled water (15 mL), potassium hydroxide (893 mg, 15.9 mmol) was added, the mixture was stirred at room temperature for 10 minutes under a nitrogen atmosphere, and the reaction mixture was concentrated. The concentrated reaction product was diluted with *N,N*-dimethylformamide (30 mL), then allyl bromide (1.4 mL, 15.9 mmol) was added, and the mixture was stirred at room temperature for 17 hours under a nitrogen atmosphere. The reaction mixture was diluted with ethyl acetate (300 mL), then washed with distilled water (2 × 100 mL) and brine (100 mL), and dried over anhydrous sodium sulfate. After filtration and concentration under reduced pressure, the product was purified by column chromatography, thereby obtaining Compound 191 (2.5 g, 96%). $^1$H-NMR(400 MHz, $CDCl_3$) δ 7.65 (d, 1H), 7.57 (s, 1H), 7.32 (t, 1H), 7.07-7.05 (m, 1H), 6.06-5.98 (m, 1H), 5.41 (d, 1H), 5.36 (s, 1H), 5.29 (d, 1H), 4.82 (d, 2H).

**Preparation of Compound 192**

Compound 191 (2.5 g, 14.0 mmol) and 1,5-diodopentane (6.5 mL, 43.5 mmol) were dissolved in acetone (100 mL), then potassium carbonate (2.2 g, 15.95 mmol)was added, and the mixture was heated under reflux and stirred for 18 hours under a nitrogen atmosphere. The reaction mixture was concentrated under reduced pressure and purified by column chromatography, thereby obtaining Compound 192 (3.8 g, 70%). $^1$H-NMR(400 MHz, $CDCl_3$) δ 7.65 (d, 1H), 7.56 (s, 1H), 7.34 (t, 1H), 7.10-7.08 (m, 1H), 6.09-5.99 (m, 1H), 5.41 (d, 1H), 5.29 (d, 1H), 4.82 (d, 2H), 4.01 (t, 2H), 3.22 (t, 2H), 1.94-1.87 (m, 2H), 1.86-1.79 (m, 2H), 1.64-1.5 (m, 2H).

**<Example 68> Preparation of Compound 193**

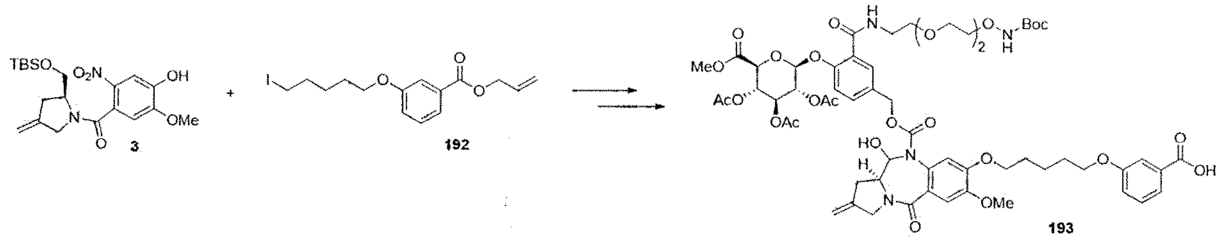

3 192 193

Compound 193 was synthesized from Compound 3 and Compound 192 in a similar manner to the synthesis of Compound 157. EI-MS m/z : $[M+H]^+$1240.09, $[M/2+H]^+$570.48.

**<Example 69> Preparation of Compound 194**

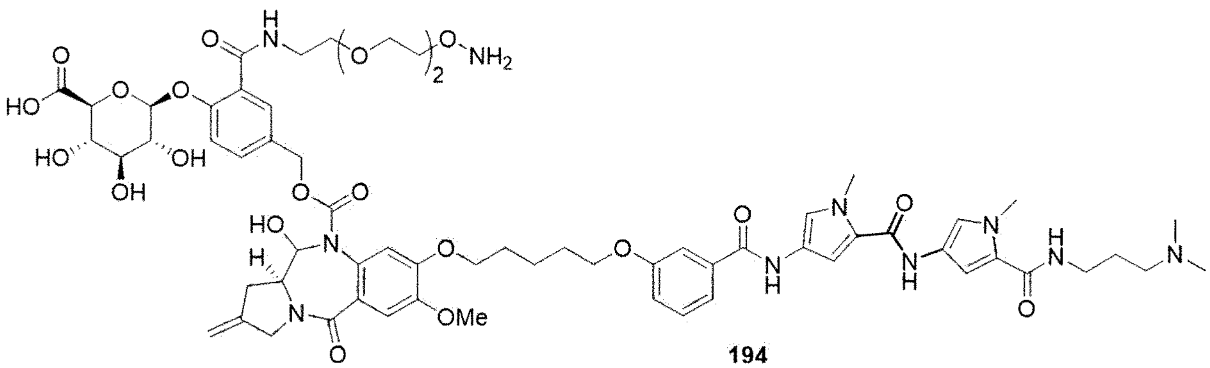

194

Compound 194 was synthesized from Compound 193 and Compound 20 in a similar manner to the synthesis of Compound 23. EI-MS m/z : $[M+H]^+$1328.38, $[M/2+H]^+$664.82.

**<Example 70> Preparation of Compound 200**

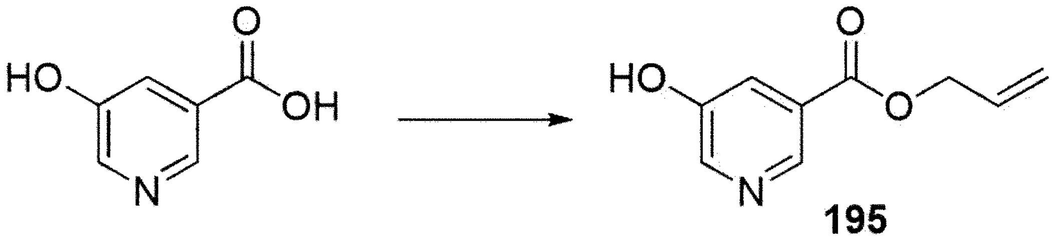

195

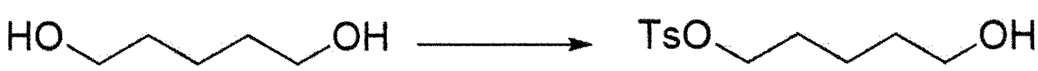

196

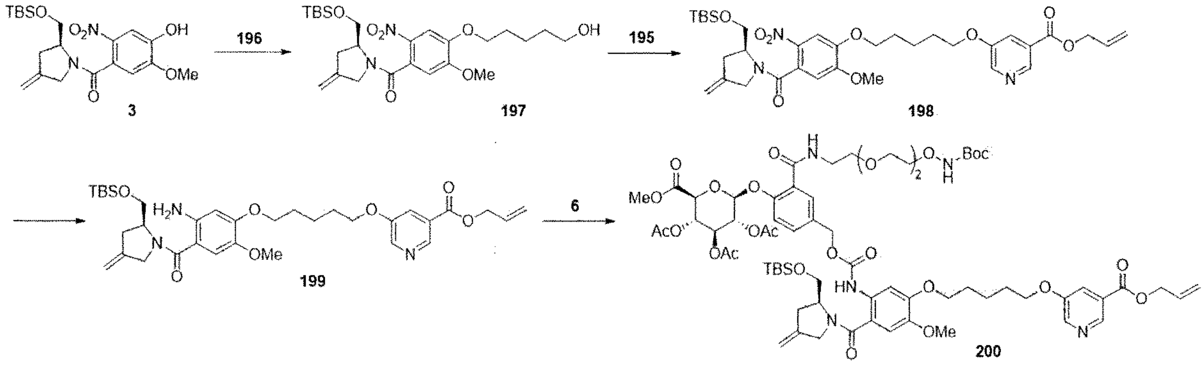


## Preparation of Compound 195

After 5-hydroxypyridine-3-carboxylic acid (500 mg, 3.6 mmol) was dissolved in N,N-dimethylformamide (10 mL), then allyl alcohol (0.3 mL, 4.31 mmol), 4-dimethylaminopyridine (44 mg, 0.36 mmol), and 1,3-diisopropylcarbodiimide (0.67 mL, 4.31 mmol) were added, and the mixture was stirred at room temperature for 17 hours. The reaction mixture was diluted with ethyl acetate (100 mL), washed with distilled water (2 $\times$ 100 mL), and then dried over anhydrous sodium sulfate. After filtration and concentration under reduced pressure, the product was purified by column chromatography, thereby obtaining Compound 195 (347 mg, 54%). $^1$H-NMR(400 MHz, $CDCl_3$) $\delta$ 8.81 (s, 1H), 8.45 (s, 1H), 7.83 (s, 1H), 6.06-5.98 (m, 1H), 5.42 (d, 1H), 5.32 (d, 1H), 4.85 (d, 2H).

## Preparation of Compound 196

After 1,5-pentanediol (2.0 g, 19.2 mmol) was dissolved in dichloromethane (3 mL), 4-toluenesulfonyl chloride (4.4 g, 23.04 mmol), 4-dimethylaminopyridine (234 mg, 1.92 mmol), and triethylamine (8.0 mL, 57.6 mmol) were added, and the mixture was stirred at room temperature for 3 hours. The reaction mixture was diluted with dichloromethane (100 mL), then washed with distilled water (2 $\times$ 100 mL), and dried over anhydrous sodium sulfate. After filtration and concentration under reduced pressure, the product was purified by column chromatography, thereby obtaining Compound 196 (2.6 g, 52%). $^1$H-NMR(400 MHz, $CDCl_3$) $\delta$ 7.79 (d, 2H), 7.35 (d, 2H), 4.03 (t, 2H), 3.60 (t, 2H), 2.45 (s, 3H), 1.72-1.65 (m, 2H), 1.54-1.48 (m, 2H), 1.42-1.38 (m, 2H).

## Preparation of Compound 197

Compound 3 (1.78 g, 4.19 mmol) and Compound 196 (1.3 g, 5.03 mmol) were dissolved in N,N-dimethylformamide (40 mL), then cesium carbonate (1.78 g, 5.45 mmol) was added, and the mixture was stirred at room temperature for 18 hours. The reaction mixture was diluted with ethyl acetate (100 mL), washed with distilled water (2 $\times$ 100 mL), and then dried over anhydrous sodium sulfate. After filtration and concentration under reduced pressure, the product was purified by column chromatography, thereby obtaining Compound 197 (1.42 g, 67%). $^1$H-NMR(400 MHz, $CDCl_3$) (rotamers) $\delta$ 7.71(s, 1H), 6.75 (s, 1H), 4.98 (br s, 1H), 4.83 (br s, 1H), 4.59 (br s, 1H), 4.11 (t, 2H), 3.95 (s, 3H), 3.73-3.69 (m, 4H), 2.82-2.67 (m, 2H), 1.94-1.91 (m, 2H), 1.69-1.56 (m, 4H), 0.91 (s, 9H), 0.09 (s, 6H).

## Preparation of Compound 198

Compound 197 (1.1 g, 2.61 mmol) and Compound 195 (342 mg, 1.90 mmol) were dissolved in tetrahydrofuran (25 mL), then diethyl azodicarboxylate (40% toluene solution, 1.4 mL, 2.85 mmol) and triphenylphosphine (750 mg, 2.85 mmol) were added, and the mixture was stirred at room temperature for 3 hours. The reaction mixture was concentrated under reduced pressure and purified by column chromatography, thereby obtaining Compound 198 (915 mg). EI-MS m/z : $[M+H]^+$671.85, $[M/2+H]^+$336.07.

## Preparation of Compound 199

Compound 198 (915 mg) was dissolved in ethyl acetate (10 mL) and ethanol (20 mL), then zinc dust (2.2 g, 33.5 mmol) was added at 0°C, and formic acid (1.3 mL, 33.48 mmol) was added. After stirring at room temperature for 3 hours, the reaction mixture was diluted with ethyl acetate (30 mL) and filtered through Celite, and ethyl acetate (70 mL) was added. The organic layer was washed with saturated aqueous sodium hydrogen carbonate solution (2 $\times$ 100 mL) and brine (50 mL) in that order, and then dried over anhydrous sodium sulfate. Filtration, concentration, and purification by column chromatography were performed, thereby obtaining Compound 199 (582 mg). EI-MS m/z : $[M+H]^+$640.74, $[M/2+H]^+$320.99.

**Preparation of Compound 200**

Compound 199 (582 mg) was dissolved in dry tetrahydrofuran (10 mL), then triphosgene (61.4 mg, 0.24 mmol) and triethylamine (0.4 mL, 2.69 mmol) were added at 0°C, and Compound 6 (589 mg, 0.81 mmol) dissolved in dry tetrahydrofuran (5 mL) was added. After stirring at room temperature for 4 hours, the reaction mixture was diluted with ethyl acetate (100 mL), washed with distilled water (50 mL), and then dried over anhydrous sodium sulfate. After filtration and concentration under reduced pressure, the product was purified by column chromatography, thereby obtaining Compound 200 (330 mg, 35%). EI-MS m/z : $[M+H]^+$1397.36, 1/2$[M-BOC+H]^+$649.30.

**<Example 71> Preparation of Compound 203**

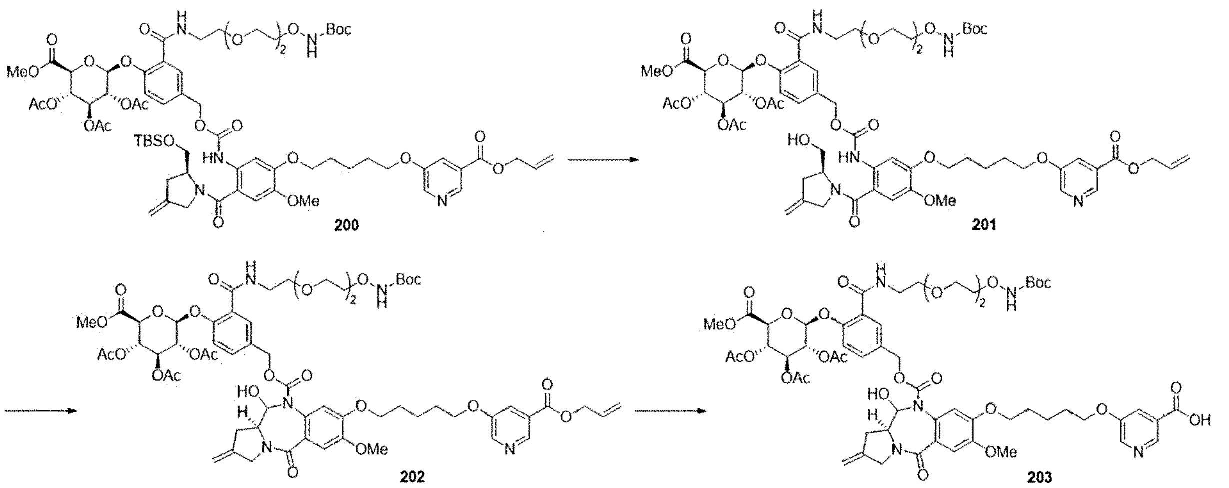

200 201 202 203

**Preparation of Compound 201**

Compound 200 (330 mg, 0.24 mmol) was dissolved in tetrahydrofuran/distilled water (2 mL/2 mL), acetic acid (6 mL) was added at 0°C, and then the mixture was stirred at room temperature for 17 hours. The reaction mixture was diluted with ethyl acetate (100 mL), washed with distilled water (50 mL) and saturated sodium hydrogen carbonate solution (2 × 100 mL), and then dried over anhydrous sodium sulfate. After filtration and concentration under reduced pressure, the product was purified by column chromatography, thereby obtaining Compound 201 (254 mg, 84%). EI-MS m/z : $[M+H]^+$1283.09, 1/2$[M-BOC+H]^+$592.09.

**Preparation of Compound 202**

Compound 201 (254 mg, 0.20 mmol) was dissolved in dichloromethane (8 mL), Dess-Martin periodinane (100 mg, 0.24 mmol) was added, and the mixture was stirred at room temperature for 22 hours under a nitrogen atmosphere. The reaction mixture was diluted with dichloromethane (100 mL), washed with saturated aqueous sodium hydrogen carbonate solution (50 mL), and then dried over anhydrous sodium sulfate. After filtration and concentration under reduced pressure, the product was purified by column chromatography, thereby obtaining Compound 202 (139 mg, 55%). EI-MS m/z : $[M+H]^+$1281.20, 1/2$[M-BOC+H]^+$591.08.

**Preparation of Compound 203**

Compound 202 (139 mg, 0.11 mmol) was dissolved in N,N-dimethylformamide (3 mL), then tetrakis(triphenylphosphine)palladium(0) (50 mg, 0.04 mmol) and pyrrolidine (0.01 mL, 0.13 mmol) were added, and the mixture was stirred at room temperature for 1.5 hours under a nitrogen atmosphere. The reaction mixture was diluted with ethyl acetate (50 mL), washed with saturated aqueous ammonium chloride solution (50 mL) and distilled water (50 mL), and dried over anhydrous sodium sulfate. Filtration and concentration under reduced pressure were performed, thereby

obtaining Compound 203 (144 mg). EI-MS m/z : $[M+H]^+$1241.18, 1/2$[M-BOC+H]^+$571.21.

**<Example 72> Preparation of Compound 204**

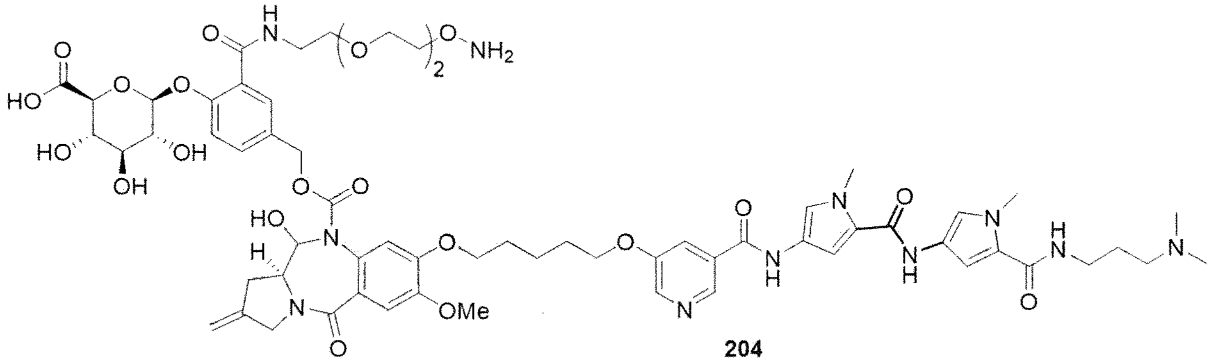

204

Compound 204 was synthesized from Compound 203 and Compound 20 in a similar manner to the synthesis of Compound 23. EI-MS m/z : $[M+H]^+$1329.71, $[M/2+H]^+$665.39.

**<Example 73> Preparation of Compound 209**

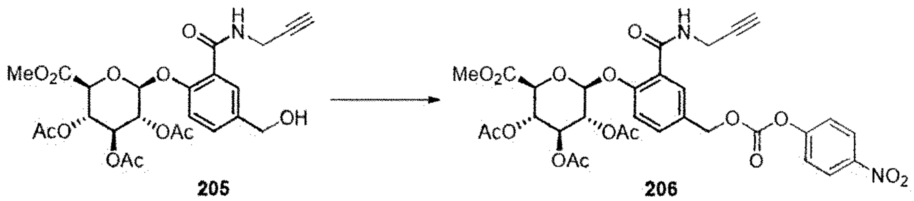

205 206

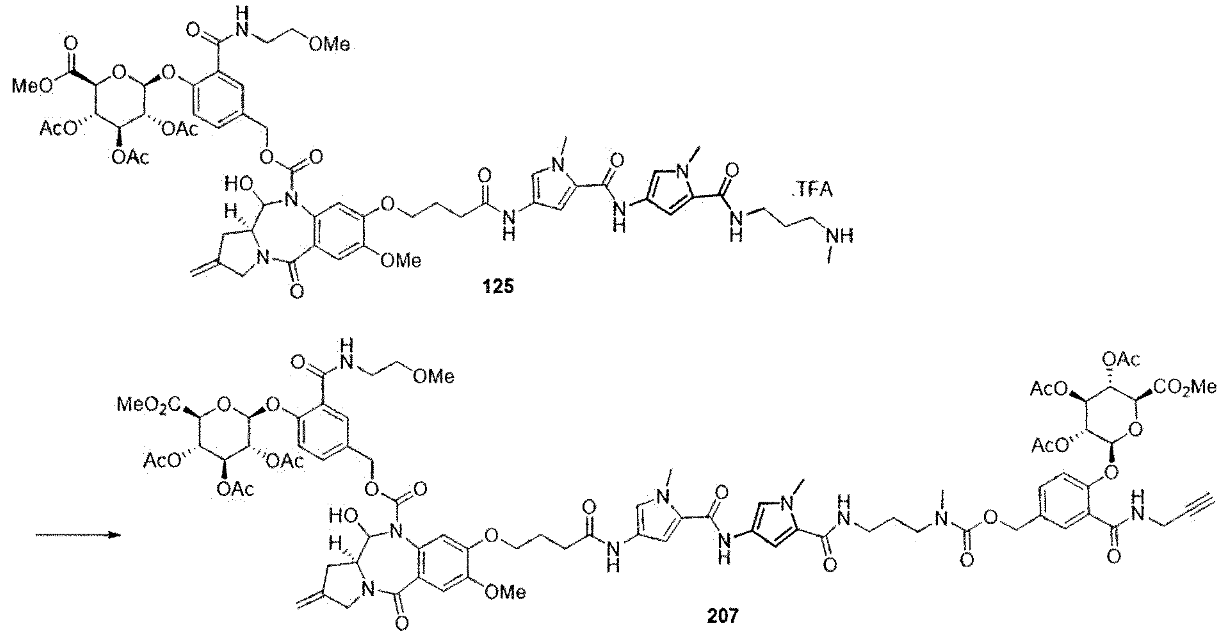

125

207

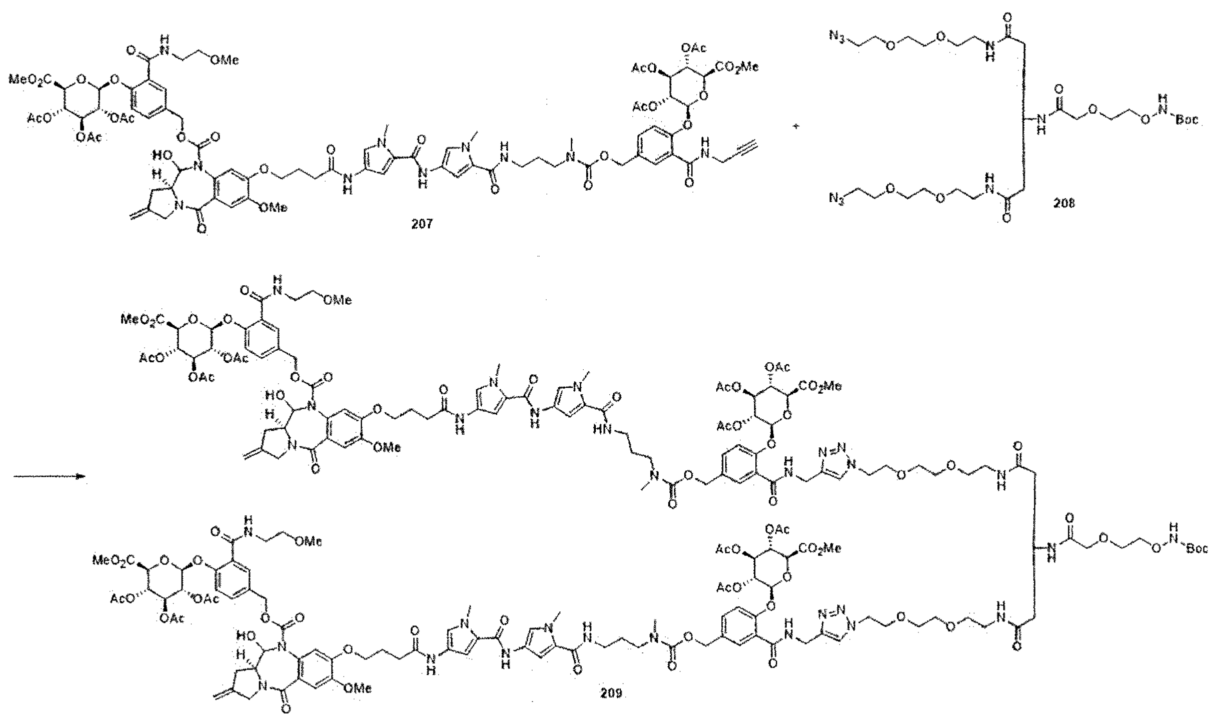

207

208

209

## Preparation of Compound 206

Compound 205 (500 mg, 0.97 mmol, Compound 205 was prepared by the method described in Korean Patent Publication No. 10-2018-0110645) and bis(4-nitrophenyl)carbonate (356 mg, 1.17 mmol) were dissolved in N,N-dimethylformamide (10 mL), then *N,N*-diisopropylethylamine (0.25 mL, 1.46 mmol) was added at 0°C, and the mixture was stirred at room temperature for 15 hours under a nitrogen atmosphere. To the reaction mixture was added ethyl acetate (30 mL), followed by extraction with distilled water (30 × 3 mL). The collected organic layers were washed with brine (50 mL) and dried over anhydrous sodium sulfate. After filtration and concentration under reduced pressure, the product was purified by column chromatography, thereby obtaining Compound 206 (528 mg, 79%). EI-MS m/z : $[M+H]^+$687.5.

## Preparation of Compound 207

Compound 125 (687 mg, 0.51 mmol) was dissolved in N,N-dimethylformamide (10 mL), and then *N,N'*-diisopropylethylamine (0.26 mL, 1.51 mmol) was added at 0°C under a nitrogen atmosphere. Compound 206 (451 mg, 0.66 mmol), 1-hydroxy-7-azabenzotriazole (30 mg, 0.22 mmol), and N,N-diisopropylethylamine (0.26 mL, 1.51 mmol) were sequentially added, the temperature was gradually raised, and the mixture was stirred at room temperature for 17 hours. Saturated aqueous ammonium chloride solution (20 mL) was added to the reaction mixture, followed by extraction with ethyl acetate (40 mL), washing with distilled water (20 × 3 mL) and brine (10 mL), and drying over anhydrous sodium sulfate. Filtration, concentration, and purification by column chromatography were performed, thereby obtaining Compound 207 (629 mg, 70%). EI-MS m/z : $[M+H]^+$1792.8.

**Preparation of Compound 209**

Compound 207 (273 mg, 0.15 mmol) and Compound 208 (50 mg, 0.069 mmol, Compound 208 was prepared by the method described in Korean Patent Publication No. 10-2020-0127891) were dissolved in tertiary butyl alcohol/distilled water (2.3 mL/0.8 mL) at 0°C under a nitrogen atmosphere, followed by stirring. Copper sulfate pentahydrate (3.46 mg, 0.013 mmol) and sodium L-ascorbate (13.7 mg, 0.069 mmol) were dissolved in distilled water (1 mL) and then added to the reaction solution, then 3,3',3"-(4,4',4"-(nitrotris(methylene))tris(1*H*-1,2,3-triazole-4,1-diyl))tris( propane-1-ol) (THPTA, 12 mg, 0.027 mmol) was added, and the mixture was stirred at room temperature for 2.5 hours. The reaction product was allowed to pass through Celite to filter inorganic substances, followed by concentration under reduced pressure. The filtered solution was subjected to extraction with dichloromethane (20 mL × 3), followed by drying over anhydrous sodium sulfate, filtration, and concentration under reduced pressure. The product was purified by column chromatography, thereby obtaining Compound 209 (210 mg, 69%). EI-MS m/z : $[M/3+H]^+$1435.4.

**<Example 74> Preparation of Compound 211**

209

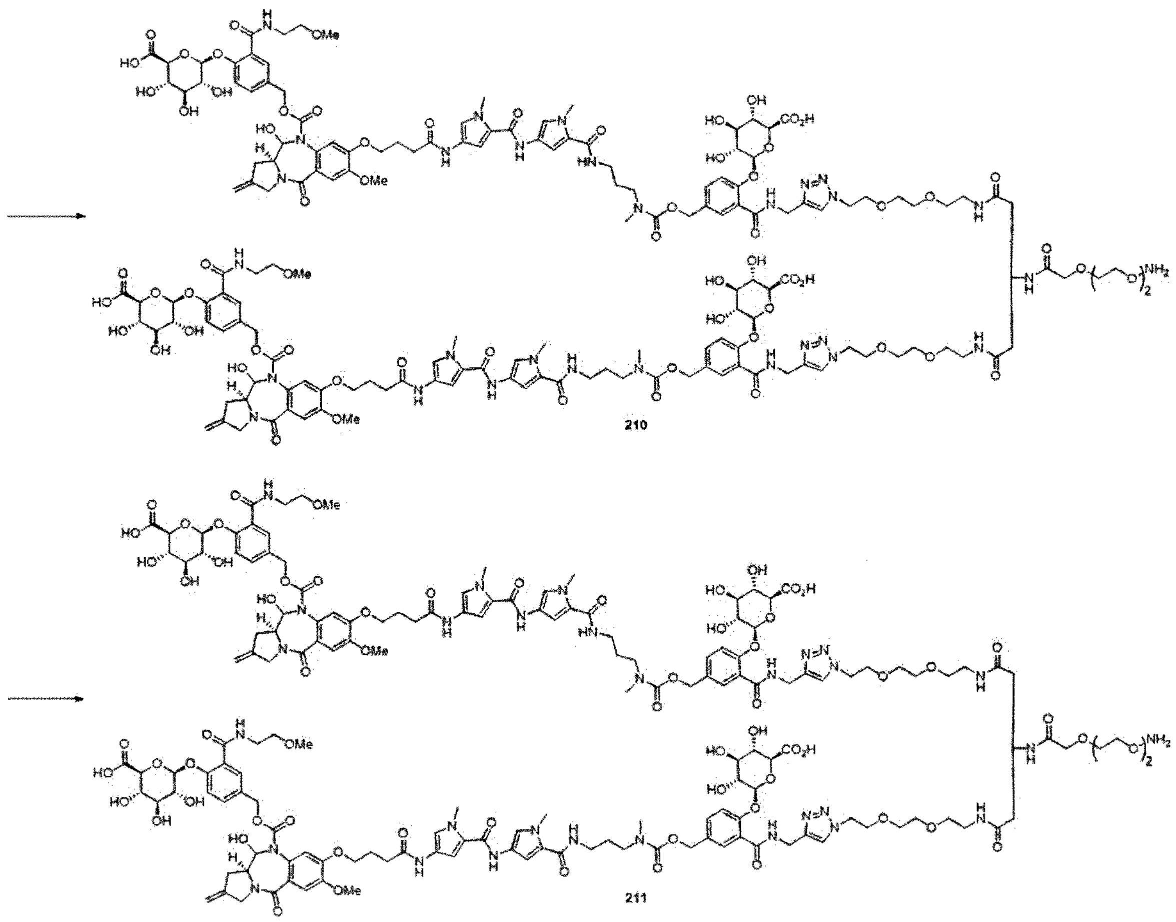

210

211

**Preparation of Compound 210**

Compound 209 (210 mg, 0.05 mmol) was dissolved in methanol/tetrahydrofuran (1 mL/1 mL), and then a solution of lithium hydroxide (8.2 mg, 0.19 mmol) in distilled water (1 mL) was gradually added at -40°C under a nitrogen atmosphere. The mixture was stirred for 2 hours while the reaction temperature was gradually raised to 0°C. The reaction mixture was neutralized with acetic acid, then concentrated under reduced pressure, and freeze-dried, thereby obtaining Compound 210 (220 mg). EI-MS m/z : $[M/2+H]^+$1872.6, $[M/3+H]^+$1248.6.

**Preparation of Compound 211**

Compound 210 (220 mg, 0.05 mmol) was diluted with dichloromethane (4 mL), then trifluoroacetic acid (1 mL) was added at 0°C under a nitrogen atmosphere, and the mixture was stirred for 2 hours. The reaction mixture was concentrated under reduced pressure, then purified by HPLC, and freeze-dried, thereby obtaining Compound 211 as a white solid (45 mg, 21%). EI-MS m/z : $[M/2+H]^+$1822.4, $[M/3+H]^+$1215.4.

**<Example 75> Preparation of Compound 214**

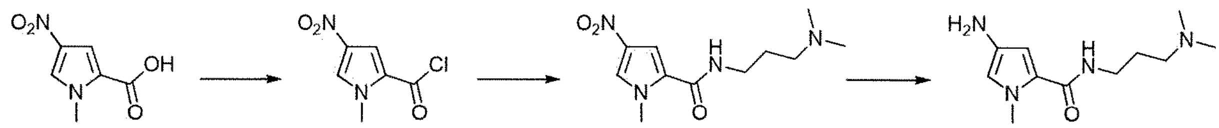

14 212 213 214

**Preparation of Compound 213**

To Compound 14 (300 mg, 1.72 mmol) was added thionyl chloride (2 mL), and the mixture was stirred for 30 minutes under reflux. The solvent was concentrated under reduced pressure, thereby obtaining Compound 212.

After 3-(dimethylamino)-1-propylamine (466 mg, 5.29 mmol) was dissolved in dichloromethane (5 mL), triethylamine (0.74 mL, 5.29 mmol) was added at -50°C under a nitrogen atmosphere. Synthesized Compound 212 was dissolved in dichloromethane (8 mL) and added, and the mixture was stirred for 2 hours while the reaction temperature was gradually raised to 0°C. The reaction mixture was diluted with dichloromethane (30 mL), washed with distilled water (20 mL), and dried over anhydrous sodium sulfate. Filtration and concentration were performed, thereby obtaining Compound 213 (396 mg, 94%). $^1$H-NMR(400 MHz, DMSO-$d_6$) $\delta$ 8.40 (t, 1H), 8.11 (d, 1H), 7.40 (d, 1H), 3.90 (s, 3H), 3.20 (m, 2H), 2.23 (t, 2H), 1.61 (m, 2H).

**Preparation of Compound 214**

Compound 213 (150 mg, 0.59 mmol) was dissolved in methanol (6 mL), and then palladium/charcoal (10% w/w, 80 mg) was added. The reaction solution was stirred at room temperature for 2 hours under a hydrogen atmosphere. The reaction mixture was filtered through Celite and then concentrated, thereby obtaining Compound 214 (132 mg, 100%) .

**<Example 76> Preparation of Compound 215**

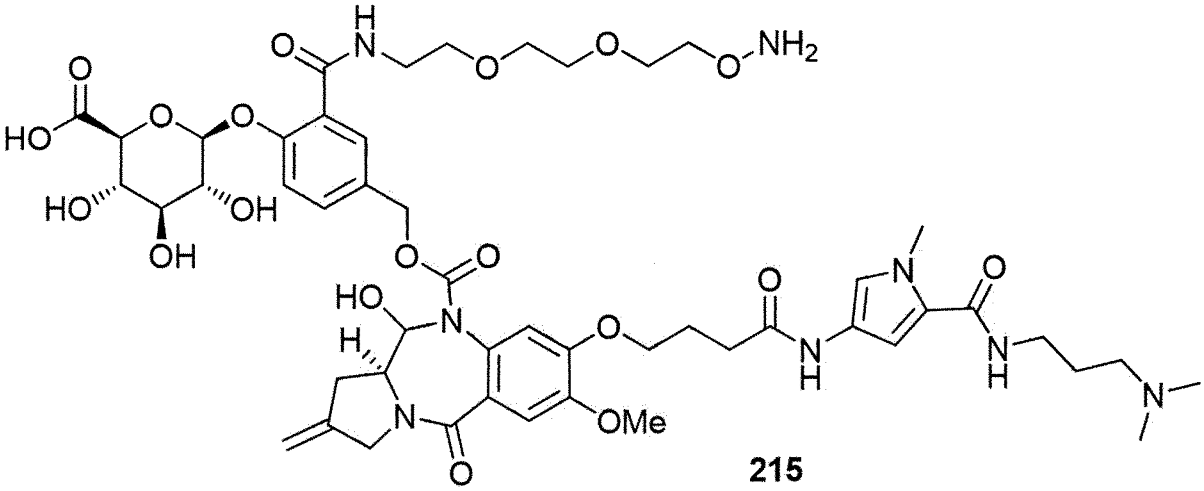

215

Compound 215 was synthesized from Compound 10 and Compound 214 in a similar manner to the synthesis of Compound 23. EI-MS m/z : $[M+H]^+$1085.68, $[M/2+H]^+$543.46.

**<Example 77> Preparation of Compound 217**

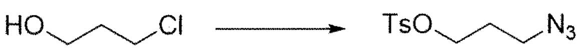

216

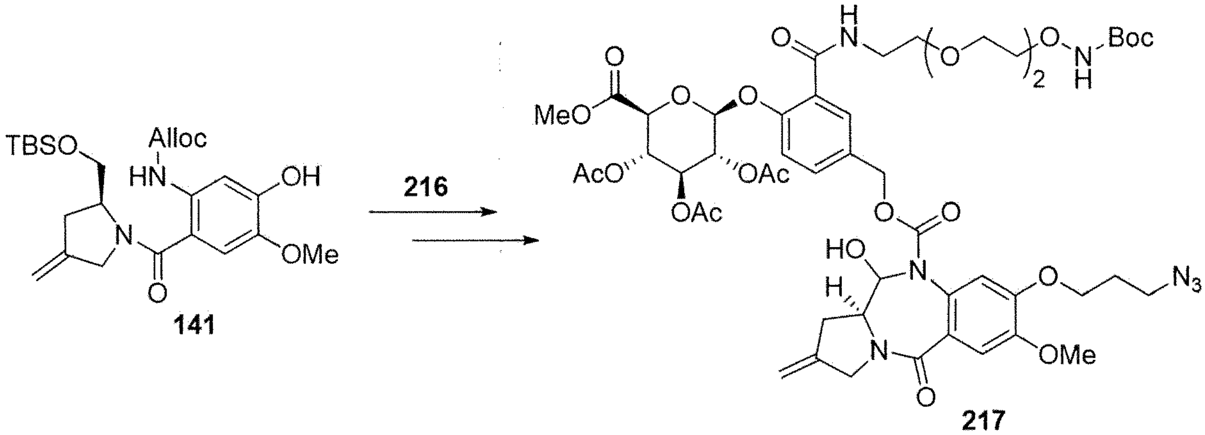


141

217

__Preparation of Compound 216__

After 3-chloropropanol (3.0 g, 31.7 mmol) and sodium azide (3.1 g, 47.6 mmol) were dissolved in *N,N*-dimethylformamide (15 mL), and the solution was stirred at 100°C for 18 hours under a nitrogen atmosphere. After the reaction mixture was cooled to room temperature, 4-toluenesulfonyl chloride (6.6 g, 34.9 mmol) and triethylamine (6.7 mL, 47.6 mmol) were added, and the mixture was stirred at room temperature for 18 hours. The reaction mixture was diluted with diethyl ether (200 mL), washed with distilled water (150 mL), and then dried over anhydrous sodium sulfate. After filtration and concentration under reduced pressure, the product was purified by column chromatography, thereby obtaining Compound 216 (1.9 g, 23%). $^1$H-NMR(400 MHz, $CDCl_3$) δ 7.80 (d, 2H), 7.36 (d, 2H), 4.11 (t, 2H), 3.38 (t, 2H), 2.45 (s, 3H), 1.92-1.86 (m, 2H).

__Preparation of Compound 217__

Compound 217 was synthesized from Compound 141 and Compound 216 in a similar manner to the synthesis of Compound 146. EI-MS m/z : $[M+H]^+$1116.42.

**<Example 78> Preparation of Compound 223**

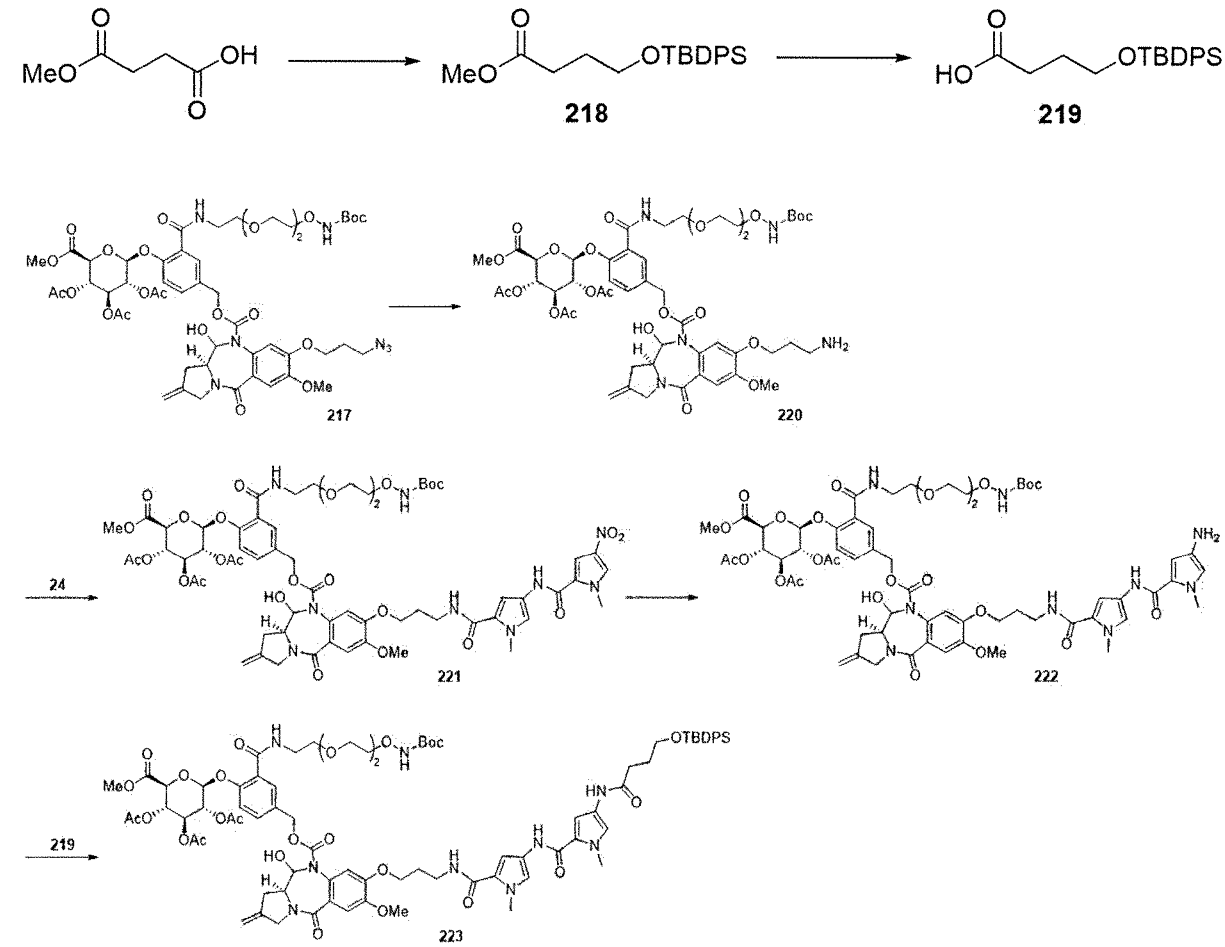


218 219 217 220 221 222 223

**Preparation of Compound 218**

After 4-methoxy-4-oxobutanoic acid (2.0 g, 15.13 mmol) was dissolved in tetrahydrofuran (30 mL), then borane dimethylsulfide (1.0 M, 19.6 mL, 19.6 mmol) was added at 0°C under a nitrogen atmosphere, and the mixture was stirred for 48 hours. Distilled water and calcium carbonate were gradually added to the reaction mixture, and then the mixture was filtered through Celite and concentrated. The concentrated compound was dissolved in dichloromethane (30 mL), then t-butylchlorodiphenylsilane (TBDPS-Cl, 4.3 mL, 16.65 mmol), 4-dimethylaminopyridine (184 mg, 1.51 mmol), and triethylamine (4.2 mL, 30.27 mmol) were sequentially added, and the mixture was stirred at room temperature for 18 hours. The reaction mixture was diluted with dichloromethane (200 mL), washed with saturated aqueous ammonium chloride solution (150 mL), and then dried over anhydrous sodium sulfate. After filtration and concentration under reduced pressure, the product was purified by column chromatography, thereby obtaining Compound 218 (2.3 g, 42%). $^1$H-NMR(400 MHz, $CDCl_3$) $\delta$ 7.65-7.63 (m, 4H), 7.42-7.35 (m, 6H), 3.68 (t, 2H), 3.65 (s, 3H), 2.46 (t, 2H), 1.92-1.84 (m, 2H), 1.04 (s, 9H).

**Preparation of Compound 219**

Compound 218 (500 mg, 1.40 mmol) was dissolved in 1,2-dichloroethane (10 mL), and then trimethyltin hydroxide (1.2 g, 7.01 mmol) was added. The reaction solution was heated under reflux and stirred for 8 hours under a nitrogen atmosphere. The reaction mixture was diluted with dichloromethane (100 mL), washed with 0.01 N aqueous potassium hydrogen sulfate (50 mL) and brine (50 mL), and then dried over anhydrous sodium sulfate. Filtration and concentration under reduced pressure were performed, thereby obtaining Compound 219 (451 mg, 94%). $^1$H-NMR (400 MHz, $CDCl_3$) $\delta$ 7.66-7.64 (m, 4H), 7.44-7.38 (m, 6H), 3.70 (t, 2H), 2.52 (t, 2H), 1.92-1.89 (m, 2H), 1.07 (s, 9H) .

**Preparation of Compound 220**

Compound 217 (472 mg, 0.42 mmol) was dissolved in ethyl acetate (4 mL) and ethanol (4 mL), then zinc dust (1.1 g, 16.91 mmol) was added at 0°C, and acetic acid (1.0 mL, 16.91 mmol) was added. After stirring at room temperature for 5 hours, the reaction mixture was diluted with ethyl acetate (30 mL) and filtered through Celite, and ethyl acetate (70 mL) was added. The organic layer was washed with saturated aqueous sodium hydrogen carbonate solution (2 × 100 mL) and brine (50 mL) in that order, and then dried over anhydrous sodium sulfate. Filtration and concentration were performed, thereby obtaining Compound 220 (423 mg, 92%). EI-MS m/z : $[M+H]^+$1090.85, 1/2$[M-BOC+H]^+$496.13.

**Preparation of Compound 221**

Compound 220 (423 mg, 0.42 mmol) was dissolved in *N*,*N*-dimethylformamide (5 mL), then Compound 24 (185 mg, 0.50 mmol), *N,N,N',N'*-tetramethyl-O-(1*H*-benzotriazol-1-yl)uronium hexafluorophosphate (HBTU, 252 mg, 0.63 mmol) and diisopropylethylamine (0.2 mL, 1.26 mmol) were sequentially added at 0°C under a nitrogen atmosphere, and the mixture was stirred at room temperature for 17 hours. The reaction mixture was concentrated and purified by column chromatography, thereby obtaining Compound 221 (321 mg, 56%). EI-MS m/z : $[M+H]^+$1364.84, 1/2$[M-BOC+H]^+$633.20.

**Preparation of Compound 222**

Compound 221 (321 mg, 0.24 mmol) was dissolved in ethyl acetate (4 mL) and ethanol (4 mL), then zinc dust (615 mg, 9.41 mmol) was added at 0°C, and acetic acid (0.5 mL, 9.41 mmol) was added. After stirring at room temperature for 5 hours, the reaction mixture was diluted with ethyl acetate (30 mL) and filtered through Celite, and ethyl acetate (70 mL) was added. The organic layer was washed with saturated aqueous sodium hydrogen carbonate solution (2 × 100 mL) and brine (50 mL) in that order, and then dried over anhydrous sodium sulfate. Filtration and concentration were performed, thereby obtaining Compound 222 (266 mg, 85%). EI-MS m/z : $[M+H]^+$1334.92, $[M/2+H]^+$668.29, 1/2$[M-BOC+H]^+$618.19.

**Preparation of Compound 223**

Compound 222 (266 mg, 0.11 mmol) was dissolved in N,N-dimethylformamide (5 mL), then Compound 219 (75 mg, 0.22 mmol), *N,N,N',N'*-tetramethyl-*O*-(1*H*-benzotriazol-1-yl)uronium hexafluorophosphate (HBTU, 96 mg, 0.24 mmol) and diisopropylethylamine (0.06 mL, 0.33 mmol) were sequentially added at 0°C under a nitrogen atmosphere, and the mixture was stirred at room temperature for 3 hours. The reaction mixture was concentrated and purified by column chromatography, thereby obtaining Compound 223 (122 mg, 66%). EI-MS m/z : $[M+H]^+$1660.02, $[M/2+H]^+$830.52, 1/2$[M-BOC+H]^+$780.43.

**<Example 79> Preparation of Compound 225**

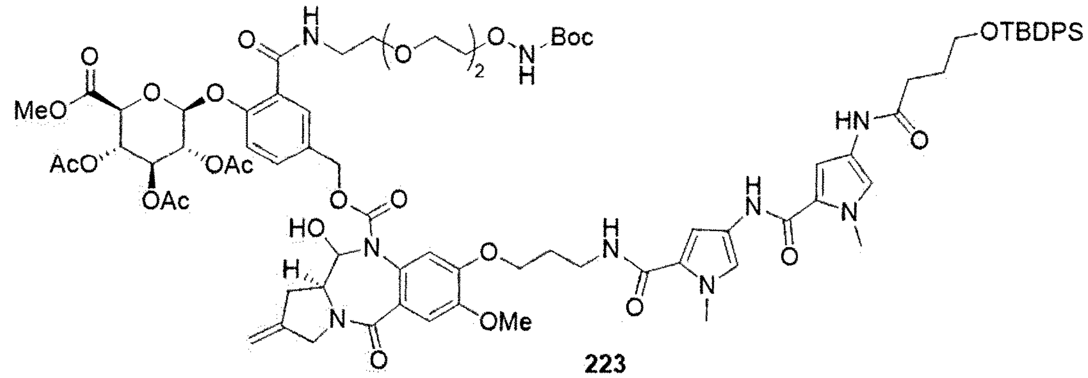

223

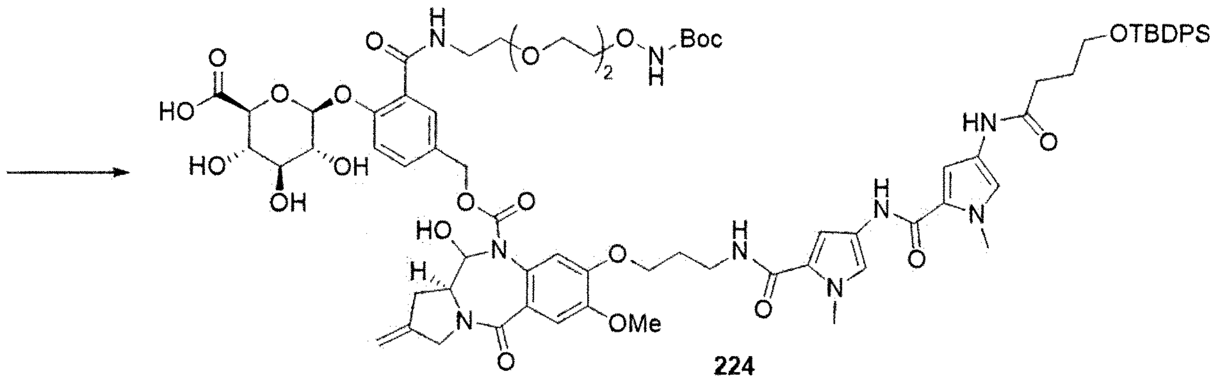

224

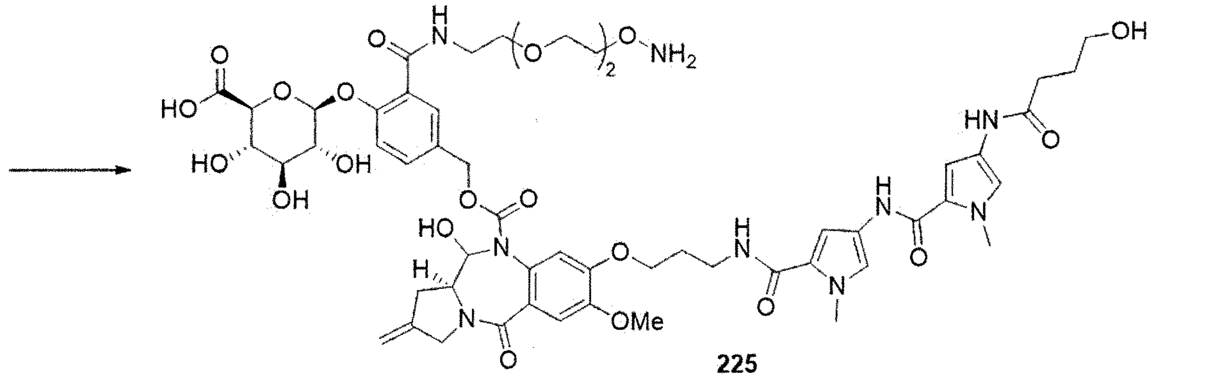

225

## Preparation of Compound 224

Compound 223 (122 mg, 0.07 mmol) was dissolved in methanol/tetrahydrofuran (1.0 mL/1.0 mL), and then a solution of lithium hydroxide (9.2 mg, 0.22 mmol) in distilled water (2.0 mL) was gradually added at -40°C under a nitrogen atmosphere. The mixture was stirred for 4 hours while the reaction temperature was gradually raised to 0°C. The reaction mixture was neutralized with acetic acid, concentrated under reduced pressure, purified by HPLC, and freeze-dried, thereby obtaining Compound 224 (22 mg) . EI-MS m/z : $[M+H]^+$1519.97, $[M/2+H]^+$760.30, 1/2$[M\text{-}BOC+H]^+$710.32.

## Preparation of Compound 225

Compound 224 (22 mg) was diluted with dichloromethane (1.5 mL), then trifluoroacetic acid (0.5 mL) was added at 0°C under a nitrogen atmosphere, and the mixture was stirred for 2 hours. The reaction mixture was concentrated under reduced pressure, then purified by HPLC, and freeze-dried, thereby obtaining Compound 225 (4.5 mg). EI-MS m/z : $[M+H]^+$1182.18, $[M/2+H]^+$591.16.

**<Example 80> Preparation of Compound 230**

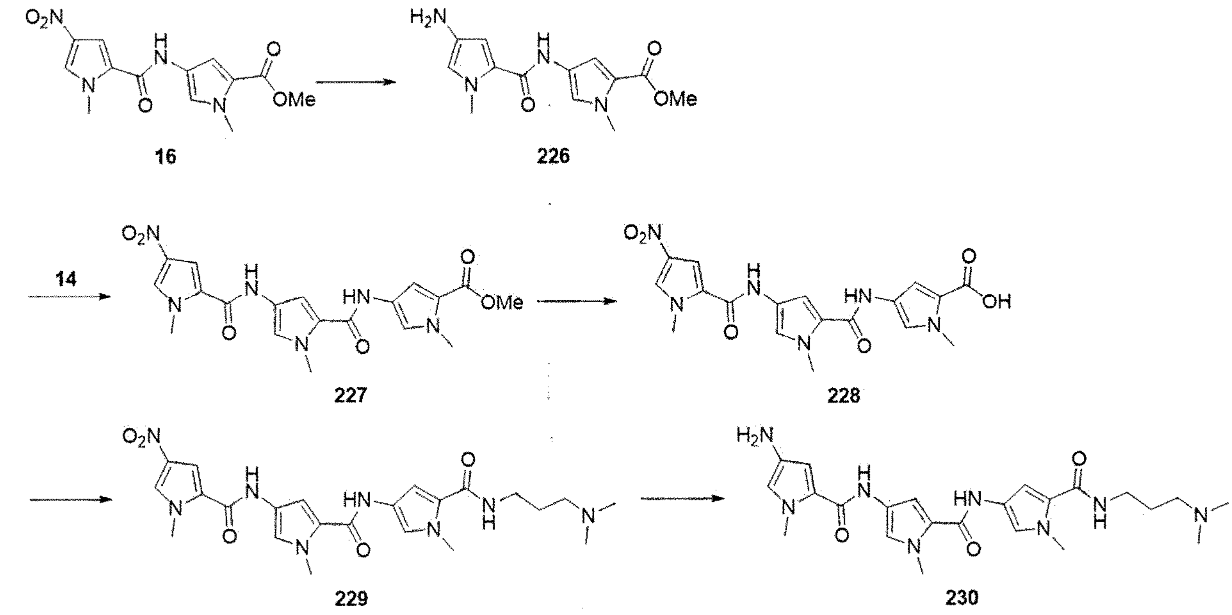


16 226 227 228 229 230

**Preparation of Compound 226**

Compound 16 (4.6 g, 15.0 mmol) was dissolved in methanol (70 mL) and N,N-dimethylformamide (70 mL), and then palladium/charcoal (10% w/w, 0.9 g) was added at 0°C under a nitrogen atmosphere. After sodium borohydride (1.7 g, 45.0 mmol) dissolved in distilled water (35 mL) was gradually added using a dropping funnel, the mixture was stirred at room temperature for 1 hour. The reaction mixture was filtered through Celite, then diluted with ethyl acetate (100 mL), and washed with distilled water (50 mL). The collected organic layers were dried over anhydrous sodium sulfate, then filtered, concentrated, and purified by column chromatography, thereby obtaining Compound 226 (4.08 g, 99%). $^1$H-NMR (400 MHz, DMSO-$d_6$) $\delta$9.59 (s,1H), 7.42 (d, *J* = 1.6 Hz, 1H), 6.85 (d, *J* = 1.6 Hz, 1H), 6.33 (d, *J* = 2 Hz, 1H), 6.26 (d, *J* = 2 Hz, 1H), 3.82 (s, 3H), 3.73 (s, 3H), 3.72 (s, 3H) .

**Preparation of Compound 227**

Compound 14 (2.3 g, 13.5 mmol) was dissolved in *N,N*-dimethylformamide (40 mL), then *N,N,N',N'*-tetramethyl-*O*-(1*H*-benzotriazol-1-yl)uronium hexafluorophosphate (HBTU, 8.0 g, 20.2 mmol) and *N,N'*-diisopropylethylamine (7.0 mL, 40.5 mmol) were added sequentially at 0°C under a nitrogen atmosphere, and the mixture was stirred at room temperature for 30 minutes. Compound 226 (4.1 g, 14.8 mmol) dissolved in *N,N*-dimethylformamide (20 mL) was added to the reaction solution, and the mixture was stirred at room temperature for 12 hours. The reaction mixture was concentrated, then chloroform (50 mL) was added, and the produced solid was filtered, thereby obtaining Compound 227 (5.65 g, 99%). $^1$H-NMR (400 MHz, DMSO-$d_6$) $\delta$10.28 (s, 1H), 9.97 (s, 1H), 8.18 (d, J = 1.6 Hz, 1H), 7.58 (d, *J* = 2 Hz, 1H), 7.46 (d, *J* = 1.6 Hz, 1H), 7.26 (d, *J* = 1.6 Hz, 1H), 7.05 (d, *J* = 1.6 Hz, 1H), 6.90 (d, *J* = 2 Hz, 1H), 3.96 (s, 3H), 3.85 (s, 3H), 3.83 (s, 3H), 3.73 (s, 3H) .

**Preparation of Compound 228**

After methanol (120 mL) was added to Compound 227 (3.7 g, 8.6 mmol), sodium hydroxide (1.72 g, 43.1 mmol) dissolved in distilled water (90 mL) was added at 0°C under a nitrogen atmosphere. The reaction solution was heated to 90°C and stirred for 12 hours. After concentration of methanol, the pH was adjusted to about 2 with 6 N aqueous hydrochloric acid solution, and the produced solid was filtered, thereby obtaining Compound 228 (3.5 g, 99%). $^1$H-NMR (400 MHz, DMSO-$d_6$) $\delta$ 12.15 (bs, 1H), 10.30 (s, 1H), 9.95 (s, 1H), 8.18 (d, *J* = 1.2 Hz, 1H), 7.59 (d, *J* = 2 Hz, 1H), 7.42 (d, *J* = 1.6 Hz, 1H), 7.26 (d, *J* = 1.6 Hz, 1H), 7.05 (d, *J* = 1.2 Hz, 1H), 6.85 (d, *J* = 1.6 Hz, 1H), 3.96 (s, 3H), 3.85 (s, 3H), 3.82 (s, 3H) .

**Preparation of Compound 229**

Compound 228 (500 mg, 1.2 mmol) was dissolved in *N*,*N*-dimethylformamide (10 mL), then 3-(dimethylamino)-1-propylamine (0.18 mL, 1.4 mmol), *N,N,N',N'*-tetramethyl-*O*-(1*H*-benzotriazol-1-yl)uronium hexafluorophosphate (HBTU, 623 mg, 1.5 mmol) and diisopropylethylamine (0.63 mL, 3.6 mmol) were sequentially added at 0°C under a nitrogen atmosphere, and the mixture was stirred at room temperature for 12 hours. The reaction mixture was concentrated, then dichloromethane (20 mL) was added, the mixture was stirred, and then the produced solid was filtered, thereby obtaining Compound 229 (600 mg, 99%). $^1$H-NMR (400 MHz, DMSO-$d_6$) δ 10.31 (s, 1H), 9.96 (s, 1H), 8.19 (s, 1H), 7.60 (s, 1H), 7.27 (s, 1H), 7.19 (s, 1H), 7.04 (s, 1H), 6.90 (s, 1H), 3.96 (s, 3H), 3.85 (s, 3H), 3.81 (s, 3H), 3.25-3.22 (m, 2H), 2.68-2.64 (m, 2H), 1.77-1.76 (m, 2H).

**Preparation of Compound 230**

Compound 229 (300 mg, 0.6 mmol) was dissolved in methanol (30 mL), and then palladium/charcoal (10% w/w, 240 mg) was added. The reaction solution was stirred at room temperature for 5 hours under a hydrogen atmosphere. The reaction mixture was filtered through Celite and then concentrated, thereby obtaining Compound 230 (275 mg, 98%). EI-MS m/z : $[M+H]^+$469.20.

**<Example 81> Preparation of Compound 231**

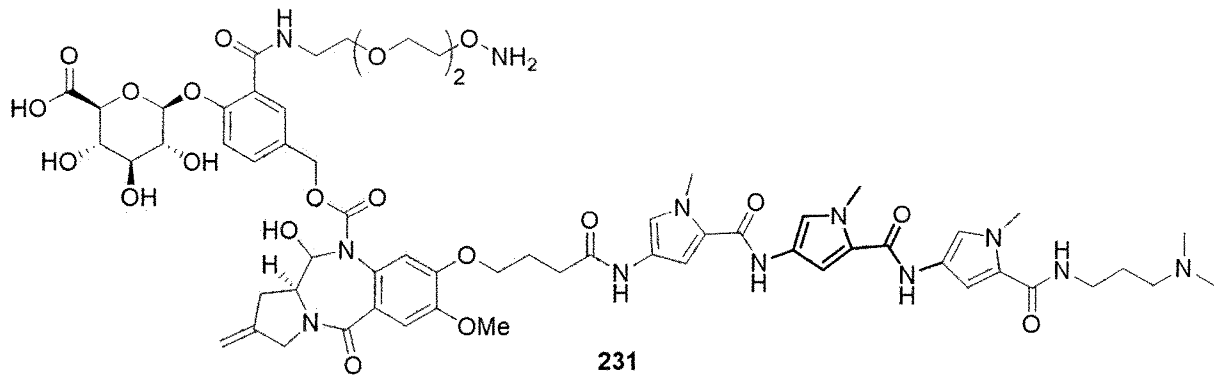

**231**

Compound 231 was synthesized from Compound 10 and Compound 230 in a similar manner to the synthesis of Compound 23. EI-MS m/z : $[M+H]^+$1330.07, $[M/2+H]^+$665.5.

**<Example 82> Preparation of Compound 234**

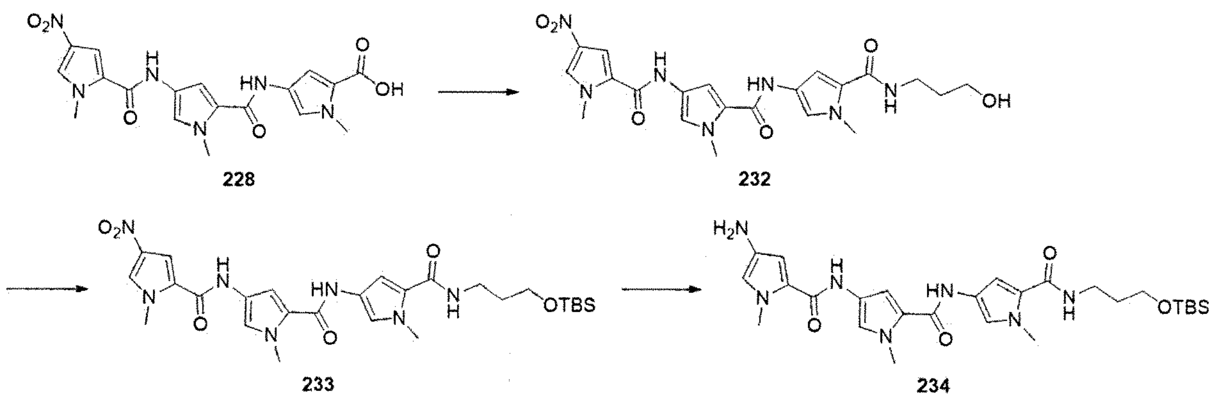

228 232 233 234

**Preparation of Compound 232**

Compound 228 (980 mg, 2.36 mmol) and 3-amino-1-propanol (0.26 mL, 3.54 mmol) were dissolved in *N,N*-dimethylformamide (12 mL), then *N,N,N',N'*-tetramethyl-*O*-(1*H*-benzotriazol-1-yl)uronium hexafluorophosphate (HBTU, 1.4 g, 3.54 mmol), and *N,N'*-diisopropylethylamine (0.82 mL, 4.72 mmol) were sequentially added at 0°C under a nitrogen atmosphere, and the mixture was stirred at room temperature for 4 hours. The reaction mixture was diluted with ethyl acetate (100 mL) and washed with distilled water (60 mL × 3). The collected organic layers were dried over anhydrous sodium sulfate, filtered and concentrated under reduced pressure, thereby obtaining Compound 232 (3.93 g). EI-MS m/z : $[M+H]^+$1491.80, 1/2$[M+H]^+$746.41.

**Preparation of Compound 233**

Compound 232 (3.93 g, 2.36 mmol) was dissolved in dichloromethane (200 mL) and N,N-dimethylformamide (10 mL), then imidazole (321 mg, 4.72 mmol) and t-butyldimethylsilyl chloride (534 mg, 3.54 mmol) were added at 0°C under a nitrogen atmosphere, and the mixture was stirred at room temperature for 6 hours. The reaction mixture was concentrated under reduced pressure, diluted with ethyl acetate (80 mL), washed with saturated aqueous ammonium chloride solution (50 mL) and distilled water (50 mL) in that order, and then dried over anhydrous sodium sulfate. After filtration and concentration under reduced pressure, the product was purified by column chromatography, thereby obtaining Compound 233 (1.01 g, 74%). $^1$H-NMR (400 MHz, DMSO-$d_6$) δ 10.30 (s, 1H), 9.96 (s, 1H), 8.02 (s, 1H), 7.99 (t, 1H), 7.60 (d, 1H), 7.28 (d, 1H), 7.19 (d, 1H), 7.03 (d, 1H), 6.84 (d, 1H), 3.96 (s, 3H), 3.86 (s, 3H), 3.79 (s, 3H), 3.63 (t, 2H), 3.22 (m, 2H), 1.69 (m, 2H), 0.87 (s, 9H), 0.04 (s, 6H).

**Preparation of Compound 234**

Compound 233 (750 mg, 1.28 mmol) was dissolved in methanol (20 mL) and ethyl acetate (10 mL), and then palladium/charcoal (10% w/w, 440 mg) was added. The reaction solution was stirred at room temperature for 3 hours under a hydrogen atmosphere. The reaction mixture was filtered through Celite and then concentrated, thereby obtaining Compound 234 (704 mg, 99%). $^1$H-NMR (400 MHz, DMSO-$d_6$) δ 9.88 (s, 1H), 9.63 (s, 1H), 7.96 (t, 1H), 7.21 (d, 1H), 7.17 (d, 1H), 7.00 (d, 1H), 6.84 (d, 1H), 6.39 (d, 1H), 6.28 (d, 1H), 3.83 (s, 3H), 3.79 (s, 3H), 3.74 (s, 3H), 3.63 (t, 2H), 3.22 (m, 2H), 1.69 (m, 2H), 0.87 (s, 9H), 0.04 (s, 6H) EI-MS m/z : $[M+H]^+$556.30.

**<Example 83> Preparation of Compound 235**

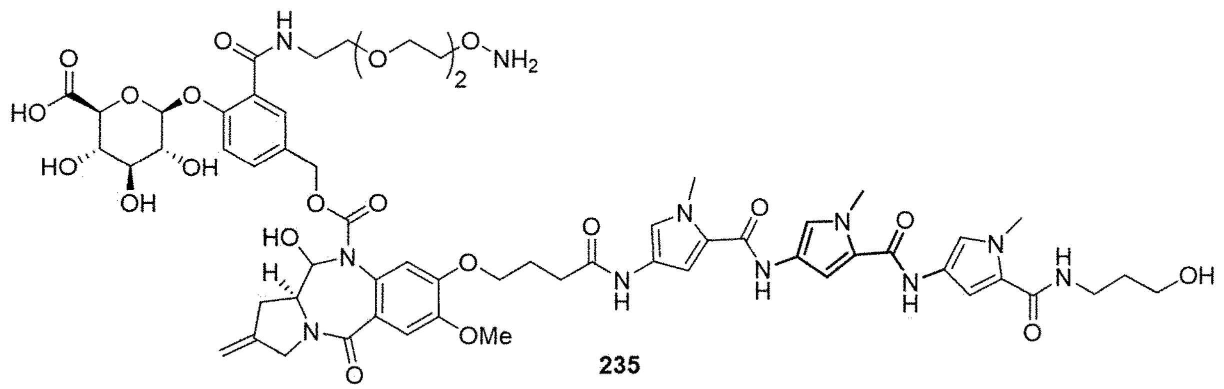

**235**

Compound 235 was synthesized from Compound 10 and Compound 234 in a similar manner to the synthesis of Compound 29. EI-MS m/z : $[M+H]^+$1303.22, $[M/2+H]^+$652.07.

**<Example 84> Preparation of Compound 239**

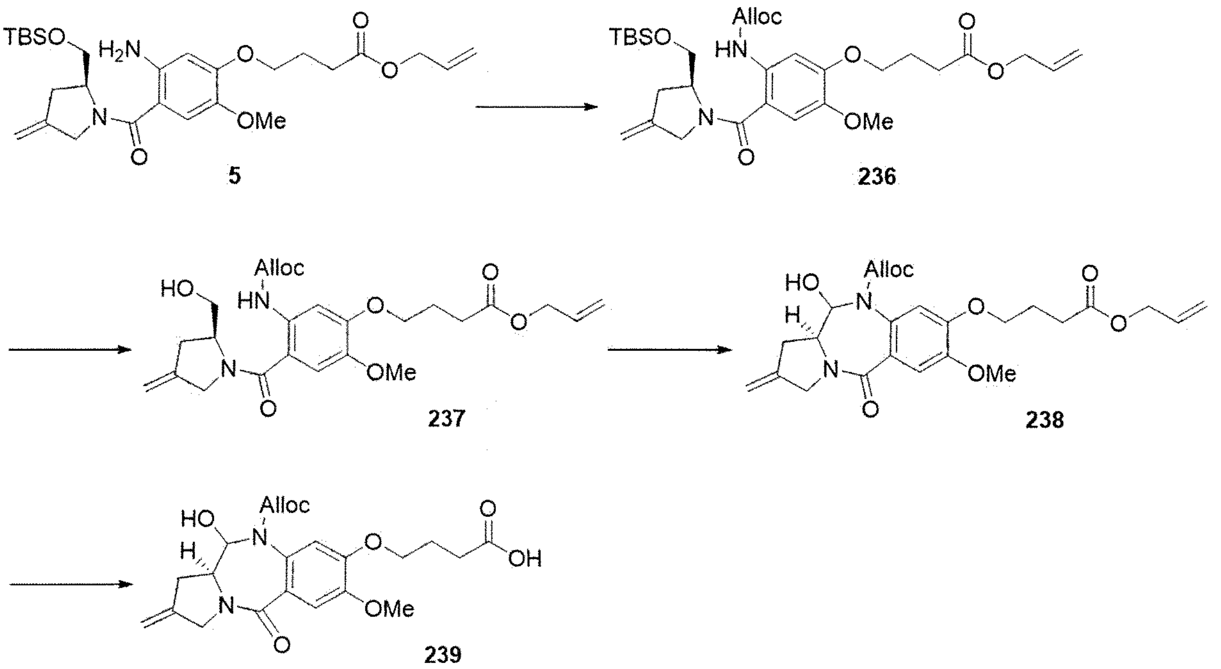

5

236

237

238

239

**Preparation of Compound 236**

Compound 5 (2.49 g, 4.8 mmol) was dissolved in dichloromethane (25 mL), then pyridine (0.85 mL, 10.56 mmol) and allyl chloroformate (0.56 mL, 5.28 mmol) were sequentially added at -78°C under a nitrogen atmosphere, the temperature was raised to room temperature, and the mixture was stirred for 0.5 hour. The reaction mixture was concentrated under reduced pressure, and the concentrated solution was washed with distilled water (20 mL) and brine (20 mL) and then dried over anhydrous sodium sulfate. Filtration and concentration under reduced pressure were performed, thereby obtaining Compound 236 (2.86 g). $^1$H-NMR (400 MHz, $CDCl_3$) δ7.82 (s, 1H), 6.81 (s, 1H), 5.94 (m, 1H), 5.36-5.32 (m, 2H), 5.27 (m, 2H), 4.98 (brs, 1H), 4.90 (brs, 1H), 4.64-4.59 (m, 5H), 4.20 (m. 2H), 4.12 (t, *J* =6.4 Hz, 2H), 3.81 (s, 3H), 2.69 (m, 2H), 2.57 (t, J =7.6 Hz, 2H), 2.19 (m, 2H), 0.87 (s, 9H), 0.03 (s, 6H), EI-MS m/z : $[M+H]^+$603.54.

**Preparation of Compound 237**

Compound 236 (2.86 g, 4.7 mmol) was dissolved in tetrahydrofuran/distilled water (5 mL/5 mL), and acetic acid (15 mL) was added at 0°C, and the mixture was stirred at room temperature for 17 hours. After further addition of acetic acid (1.5 mL), the mixture was stirred at room temperature for 6 hours, and the reaction was terminated. After concentration under reduced pressure, the reaction mixture was subjected to extraction with ethyl acetate (50 mL × 3), followed by washing with saturated aqueous sodium hydrogen carbonate solution (50 mL), distilled water (30 mL), and brine (30 mL) in that order, and then drying over anhydrous sodium sulfate. Filtration, concentration under reduced pressure, and purification by column chromatography were performed, thereby obtaining Compound 237 (1.96 g, 85%). $^1$H-NMR (400 MHz, $CDCl_3$) δ8.50 (brs, 1H), 7.75 (brs, 1H), 6.82 (s, 1H), 5.96 (m, 1H), 5.37-5.30 (m, 2H), 5.26-5.22 (m, 2H), 5.02 (brs, 1H), 4.94 (brs, 1H), 4.64-4.59 (m, 5H), 4.18(m, 2H), 4.12(t,J = 6.4 Hz, 2H), 3.83 (s, 3H), 3.70 (br s, 1H), 2.79 (m, 1H), 2.57 (t, J =7.2 Hz, 2H), 2.47 (m, 2H), 2.19 (m, 2H), EI-MS m/z : $[M+H]^+$490.39.

**Preparation of Compound 238**

Compound 237 (1.95 g, 4 mmol) was dissolved in dichloromethane (40 mL), then Dess-Matin periodinane (2.03 g, 4.8 mmol) was added at 0°C, the temperature was raised to room temperature, and the mixture was stirred for 2 hours under a nitrogen atmosphere. The reaction mixture was diluted with saturated aqueous sodium thiosulfate solution/saturated aqueous sodium hydrogen carbonate solution (20 mL/20 mL) and stirred for 10 minutes. After extraction with dichloromethane (30 mL × 3), washing was performed with saturated aqueous sodium thiosulfate solution/saturated aqueous sodium hydrogen carbonate solution (15 mL/15 mL), distilled water (20 mL), and brine (20 mL). Drying over anhydrous sodium sulfate, filtration, concentration, and purification by column chromatography were performed, thereby obtaining Compound 238 (899 mg, 46%). $^1$H-NMR(400 MHz, $CDCl_3$) δ7.21 (s, 1H), 6.86 (brs, 1H), 5.94 (m, 1H), 5.80 (m, 1H), 5.56 (m, 1H), 5.32 (m, 1H), 5.30 (m, 1H), 5.13 (m, 4H), 4.67 (dd, J =13.2 Hz, 5.2 Hz, 1H), 4.66 (d, J = 5.6 Hz,

2H), 4.45 (m, 1H), 4.32 (d, J = 15.6 Hz, 1H), 4.15 (d, J = 15.6 Hz, 1H), 4.09 (m, 2H), 3.90 (s, 3H), 3.63 (m, 1H), 3.48 (m, 1H), 2.91 (m, 1H), 2.71 (m, 1H), 2.57 (t, J = 7.2 Hz, 2H), 2.17 (m, 2H), EI-MS m/z : $[M+H]^+$487.39.

## Preparation of Compound 239

Compound 238 (100 mg, 0.20 mmol) was dissolved in tetrahydrofuran (3 mL), and then a solution of lithium hydroxide (17.2 mg, 0.41 mmol) in distilled water (3 mL) was gradually added at 0°C under a nitrogen atmosphere. The mixture was stirred for 6 hours while the reaction temperature was maintained at 0°C. The reaction mixture was washed with dichloromethane (5 mL), and then the aqueous layer was acidified (pH of about 4) with 1 N aqueous hydrochloric acid solution. Extraction with ethyl acetate (20 mL × 3), drying over anhydrous sodium sulfate, and concentration under reduced pressure were performed, thereby obtaining Compound 239 (91.5 mg). $^1$H-NMR(400 MHz, $CDCl_3$) δ 7.22 (s, 1H), 6.75 (brs, 1H), 5.77 (m, 1H), 5.58 (d, *J* = 10 Hz, 1H), 5.14 (m, 4H), 4.66 (dd, *J* = 13.2 Hz, 5.2 Hz, 1H), 4.43 (m, 1H), 4.32 (d, *J* = 16 Hz, 1H), 4.16-4.06 (m, 5H), 3.90 (s, 3H), 3.63 (t, *J* = 8 Hz, 1H), 2.90 (m, 1H), 2.70 (m, 1H), 2.58 (t, *J* = 6.4 Hz, 2H), 2.16 (m, 2H), EI-MS m/z : $[M+H]^+$447.24.

## <Example 85> Preparation of Compound 242

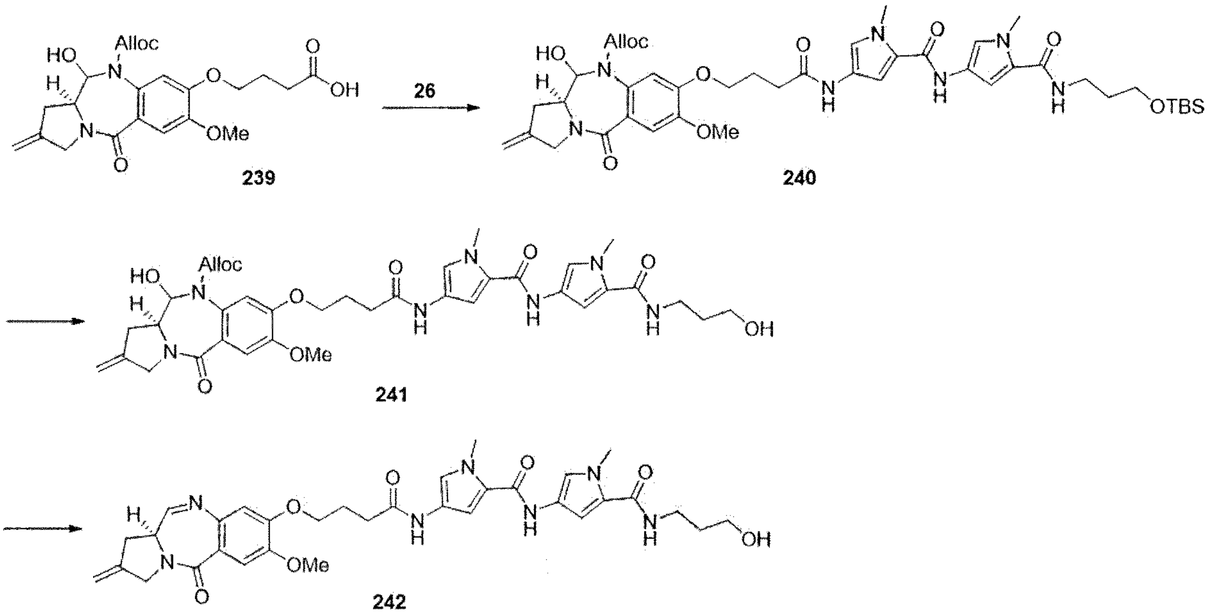


239

240

241

242

## Preparation of Compound 240

Compound 239 (141 mg, 0.32 mmol) was dissolved in N,N-dimethylformamide (3 mL), then Compound 26 (150 mg, 0.35 mmol), *N,N,N',N'*-tetramethyl-*O*-(1*H*-benzotriazol-1-yl)uronium hexafluorophosphate (HBTU, 163 mg, 0.41 mmol) and diisopropylethylamine (0.11 mL, 0.63 mmol) were sequentially added at 0°C under a nitrogen atmosphere, and the mixture was stirred at room temperature for 2 hours. The reaction mixture was diluted with ethyl acetate (50 mL), then washed with saturated aqueous ammonium chloride solution (50 mL), saturated sodium hydrogen carbonate solution (50 mL) and distilled water (50 mL), and dried over anhydrous sodium sulfate. After filtration and concentration, the product was purified by column chromatography, thereby obtaining Compound 240 (178 mg, 66%). EI-MS m/z : $[M+H]^+$862.44, $[M-TBS+H]^+$748.36, $[M/2+H]^+$423.04, 1/2$[M-TBS+H]^+$374.92.

## Preparation of Compound 241

Compound 240 (178 mg, 0.20 mmol) was dissolved in tetrahydrofuran (5 mL), then tetra-n-butylammonium fluoride (1.0 M tetrahydrofuran solution, 1.2 mL, 1.2 mmol) was gradually added at 0°C under a nitrogen atmosphere, and the mixture was stirred at room temperature for 5 hours. The reaction mixture was washed with saturated aqueous ammonium chloride solution (50 mL) and distilled water (50 mL), and then dried over anhydrous sodium sulfate. After filtration and concentration under reduced pressure, the product was purified by column chromatography, thereby obtaining Compound 241 (115 mg, 74%). EI-MS m/z : [M+H]+748.42, [M/2+H]+374.96.

**Preparation of Compound 242**

Compound 241 (115 mg, 0.15 mmol) was dissolved in dichloromethane (4 mL), then tetrakis(triphenylphosphine)palladium(0) (8.8 mg, 0.01 mmol) and pyrrolidine (0.05 mL, 0.62 mmol) were added, and the mixture was stirred at room temperature for 0.5 hour under a nitrogen atmosphere. After concentration under reduced pressure, the product was purified by column chromatography, thereby obtaining Compound 242 (74 mg, 75%). EI-MS m/z : [M+H]+646.18.

**<Example 86> Preparation of Compound 244**

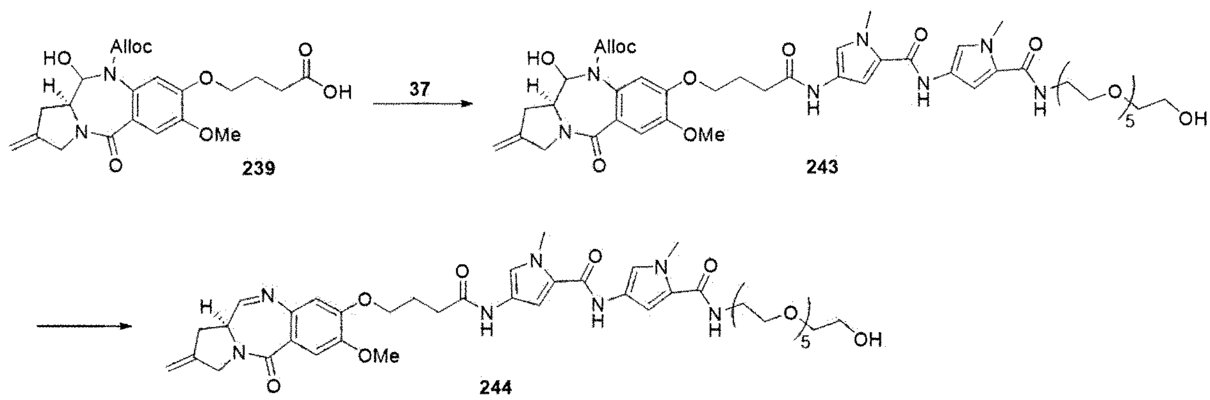

239
243
244

**Preparation of Compound 243**

Compound 239 (155 mg, 0.35 mmol) was dissolved in N,N-dimethylformamide (3 mL), then Compound 37 (200 mg, 0.38 mmol), *N,N,N',N'*-tetramethyl-*O*-(1*H*-benzotriazol-1-yl)uronium hexafluorophosphate (HBTU, 179 mg, 0.45 mmol) and diisopropylethylamine (0.18 mL, 1.04 mmol) were sequentially added at 0°C under a nitrogen atmosphere, and the mixture was stirred at room temperature for 3 hours. The reaction mixture was diluted with ethyl acetate (50 mL), then washed with saturated aqueous ammonium chloride solution (50 mL), saturated sodium hydrogen carbonate solution (50 mL) and distilled water (50 mL) in that order, and dried over anhydrous sodium sulfate. After filtration and concentration, the product was purified by column chromatography, thereby obtaining Compound 243 (281 mg, 85%). EI-MS m/z : [M+H]+954.54, [M/2+H]+478.04.

**Preparation of Compound 244**

Compound 243 (281 mg, 0.15 mmol) was dissolved in dichloromethane (6 mL), then tetrakis(triphenylphosphine)palladium(0) (17 mg, 0.01 mmol) and pyrrolidine (0.1 mL, 1.18 mmol) were added, and the mixture was stirred at room temperature for 1 hour under a nitrogen atmosphere. After concentration under reduced pressure, the product was purified by column chromatography, thereby obtaining Compound 244 (28 mg). EI-MS m/z : [M+H]+852.57, [M/2+H]+427.06.

**<Example 87> Preparation of Compound 245**

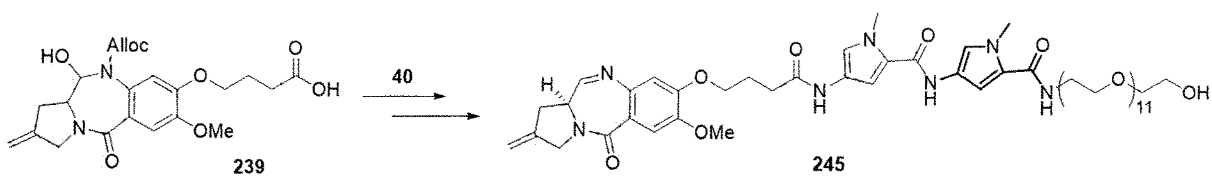

239
245

Compound 245 was synthesized from Compound 239 and Compound 40 in a similar manner to the synthesis

of Compound 244. EI-MS m/z : $[M+OH]^+$1138.69, $[M+H]^+$1116.70, $[M/2+H]^+$559.15.

**<Example 88> Preparation of Compound 248**

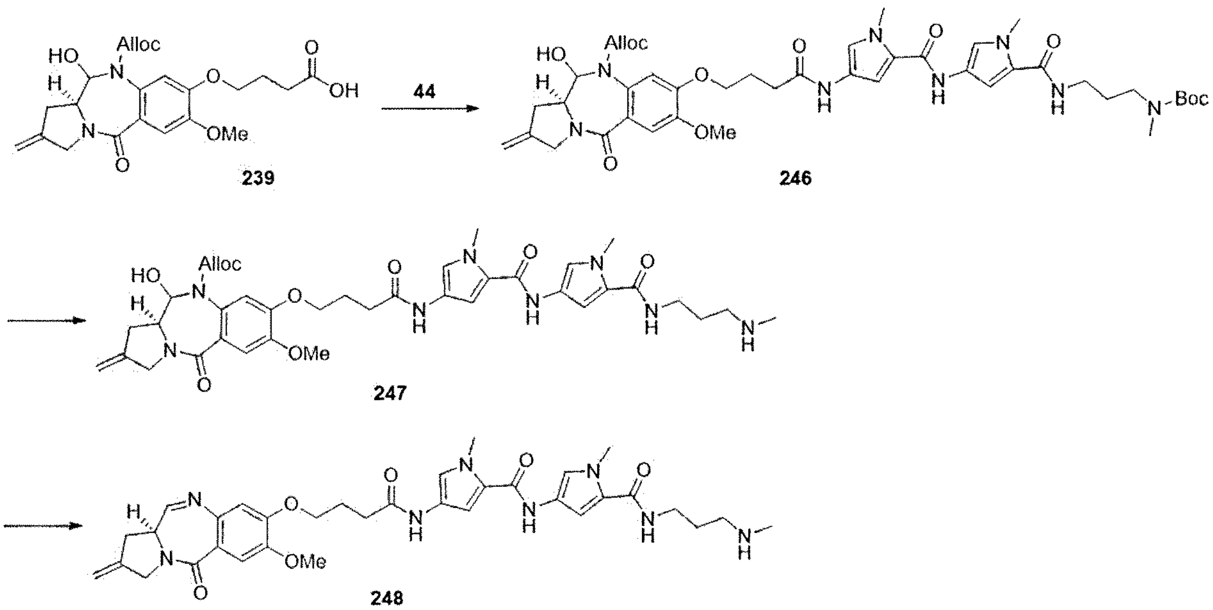


239 246 247 248

<u>**Preparation of Compound 246**</u>

Compound 239 (137 mg, 0.30 mmol) was dissolved in *N,N*-dimethylformamide (2.5 mL), and then *N,N,N',N'*-tetramethyl-*O*-(1*H*-benzotriazol-1-yl)uronium hexafluorophosphate (HBTU, 175 mg, 0.46 mmol) and diisopropylethylamine (0.1 mL, 0.61 mmol) were gradually added at 0°C under a nitrogen atmosphere. A solution of Compound 44 (160 mg, 0.36 mmol) in *N,N*-dimethylformamide (1 mL) was added to the reaction solution, and the mixture was stirred at room temperature for 1.5 hours. Saturated aqueous ammonium chloride solution (10 mL) was added to the reaction mixture, followed by extraction with ethyl acetate (20 mL × 3), washing with saturated sodium hydrogen carbonate solution (10 mL) and distilled water (10 mL), and then drying over anhydrous sodium sulfate. After filtration and concentration, the product was purified by column chromatography, thereby obtaining Compound 246 (210 mg, 79%). $^1$H-NMR (400 MHz, $CDCl_3$) δ 8.89 (br s, 0.5H), 8.02 (m, 0.5H), 7.73 (br s, 0.5H), 7.32-7.27 (m, 1H), 7.25-7.22 (m, 2H), 7.05 (br s, 1H), 6.81 (m, 1.5H), 6.66 (br s, 0.5H), 6.39 (br s, 0.5H), 5.97 (br, 0.5H), 5.75 (br s, 0.5H), 5.57 (m, 1H), 5.37 (m, 0.5H), 5.13 (m, 3H), 4.67 (m, 1H), 4.43 (m, 1H), 4.30 (m, 1H), 4.13 (m, 2H), 3.92-3.90 (m, 7H), 3.69-3.62 (m, 3H), 3.36 (m, 4H), 2.88 (s, 3H), 2.70 (m, 1H), 2.50 (m, 2H), 2.21 (m, 2H), 1.72 (m, 2H), 1.46 (s, 9H), EI-MS m/z : $[M+H]^+$861.49

<u>**Preparation of Compound 247**</u>

Compound 246 (140 mg, 0.16 mmol) was diluted with dichloromethane (1.6 mL), then trifluoroacetic acid (0.4 mL) was added at 0°C under a nitrogen atmosphere, and the mixture was stirred for 1 hour. The reaction mixture was concentrated under nitrogen gas, thereby obtaining Compound 247 (123 mg). EI-MS m/z : $[M+H]^+$761.56, $[M/2+H]^+$381.5

<u>**Preparation of Compound 248**</u>

Compound 247 (123 mg, 0.16 mmol) was dissolved in dichloromethane (2 mL), then tetrakis(triphenylphosphine)palladium(0) (3.5 mg, 0.003 mmol) and pyrrolidine (0.06 mL, 0.81 mmol) were added at 0°C under a nitrogen atmosphere, and the mixture was stirred at room temperature for 1 hour. The reaction mixture was concentrated under nitrogen gas, purified by HPLC, and freeze-dried, thereby obtaining Compound 248 (20.3 mg, 38%). EI-MS m/z : $[M+H]^+$659.55, $[M/2+H]^+$330.45

**<Example 89> Preparation of Compound 250**

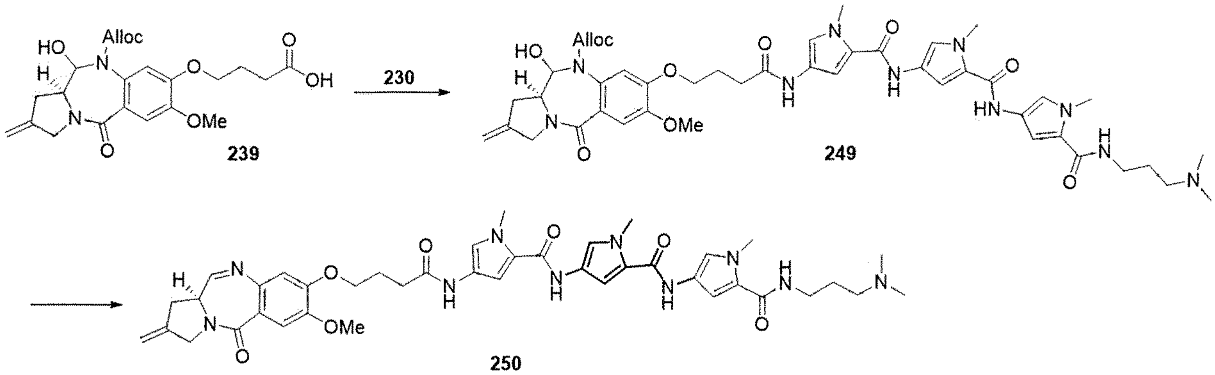


239

249

250

**Preparation of Compound 249**

Compound 239 (215 mg, 0.439 mmol) was dissolved in N,N-dimethylformamide (5 mL), then Compound 230 (180 mg, 0.337 mmol), *N,N,N',N'*-tetramethyl-*O*-(1*H*-benzotriazol-1-yl)uronium hexafluorophosphate (HBTU, 189 mg, 0.498 mmol) and diisopropylethylamine (0.29 mL, 1.68 mmol) were sequentially added at 0°C under a nitrogen atmosphere, and the mixture was stirred at room temperature for 12 hours. After addition of ethyl acetate (50 mL), the reaction mixture was washed with saturated aqueous ammonium chloride solution (50 mL), saturated aqueous sodium hydrogen carbonate solution (50 mL) and distilled water (50 mL). The collected organic layers were dried over anhydrous sodium sulfate, filtered and then concentrated. The product was purified by column chromatography, thereby obtaining Compound 249 (233 mg, 77%). EI-MS m/z : $[M+H]^+$897.59, 1/2$[M+H]^+$440.35.

**Preparation of Compound 250**

Compound 249 (233 mg, 0.126 mmol) was dissolved in dichloromethane (3 mL), tetrakis(triphenylphosphine)palladium(0) (6.0 mg, 0.005mmol) and pyrrolidine (0.021 mL, 1.298 mmol) were added under a nitrogen atmosphere, and then the mixture was stirred at room temperature for 3 hours. The reaction mixture was concentrated, purified by HPLC, and freeze-dried, thereby obtaining Compound 250 as a white solid (24 mg). EI-MS m/z : $[M+H]^+$795.50, $[M/2+H]^+$398.34.

**<Example 90> Preparation of Compound 251**

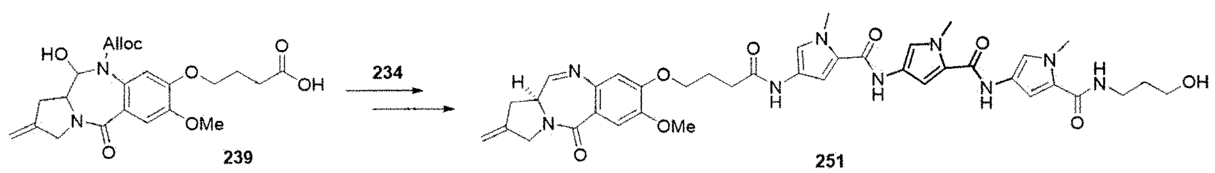


239

251

Compound 251 was synthesized from Compound 239 and Compound 234 in a similar manner to the synthesis of Compound 242. $^1$H-NMR (400 MHz, DMSO-$d_6$) δ 9.87 (m, 3H), 7.94 (t, *J* =5.6 Hz, 1H), 7.20 (m, 2H), 7.15 (s, 2H), 7.03 (m, 1H), 6.84 (dd, *J* =11.2 Hz, 1.2 Hz, 2H), 6.57 (s, 0.5H), 6.34 (s, 1H), 6.10 (s, 0.5H), 5.08 (m, 1H), 4.94 (m, 1H), 4.50-4.44 (m, 2H), 4.24-4.18 (m, 1H), 4.13-4.11 (m, 2H), 4.03-3.95 (m, 2H), 3.86-3.77 (m, 10H), 3.70-3.65 (m, 4H), 3.45 (m, 2H), 2.42 (m, 2H), 2.05 (m, 2H), 1.61 (m, 2H), EI-MS m/z : $[M+H]^+$768.32, $[M/2+H]^+$384.97.

**<Example 91> Preparation of Compound 255**

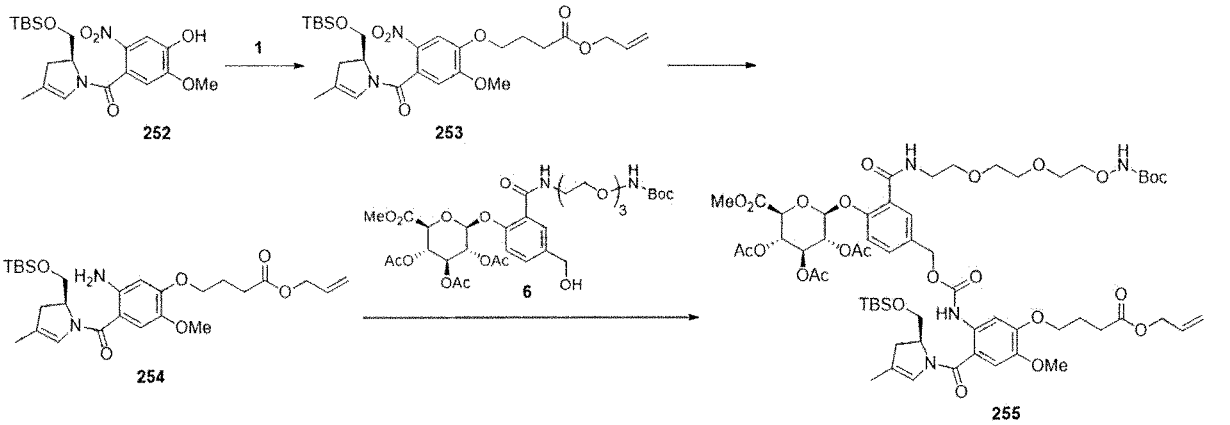


252

253

254

255

**Preparation of Compound 253**

Compound 252 (1.0 g, 2.36 mmol, Compound 252 was prepared by the method described in Korean Patent Publication No. 10-2020-0084802) and Compound 1 (735 mg, 3.54 mmol) were dissolved in *N*,*N*-dimethylformamide (20 mL), then potassium carbonate (654 mg, 4.73 mmol) and sodium iodide (177 mg, 1.18 mmol) were added, and the mixture was stirred at 90°C for 2 hours under a nitrogen atmosphere. The reaction mixture was diluted with ethyl acetate (300 mL), washed with distilled water (2 × 100 mL), and then dried over anhydrous sodium sulfate. After filtration and concentration under reduced pressure, the product was purified by column chromatography, thereby obtaining Compound 253 (1.12 g, 86%). $^1$H-NMR (400 MHz, $CDCl_3$) δ 7.70 (s, 1H), 6.78 (s, 1H), 5.91-5.88 (m, 1H), 5.53 (s, 1H), 5.33 (d, 1H), 5.25 (d, 1H), 4.65 (br s, 1H), 4.61 (d, 2H), 4.15 (t, 2H), 3.93 (s, 3H), 2.82-2.75 (m, 1H), 2.61-2.57 (m, 3H), 2.23-2.20 (m, 2H), 1.62 (s, 3H), 0.90 (s, 9H), 0.10 (s, 6H).

**Preparation of Compound 254**

Compound 253 (2.4 g, 20.0 mmol) was dissolved in ethyl acetate (20 mL) and ethanol (20 mL), then zinc dust (5.7 g, 87.4 mmol) was added at 0°C, and formic acid (3.3 mL, 87.4 mmol) was added. After stirring at room temperature for 2 hours, the reaction mixture was diluted with ethyl acetate (30 mL) and filtered through Celite, and ethyl acetate (70 mL) was added. The organic layer was washed with saturated aqueous sodium hydrogen carbonate solution (2 × 100 mL) and brine (50 mL) in that order, and then dried over anhydrous sodium sulfate. Filtration, concentration under reduced pressure, and purification by column chromatography were performed, thereby obtaining Compound 254 (2.2 g, 97%). EI-MS m/z : $[M+H]^+$519.38.

**Preparation of Compound 255**

Compound 254 (2.2 g, 4.24 mmol) was dissolved in dry tetrahydrofuran (50 mL), then triphosgene (452 mg, 1.53 mmol) and triethylamine (2.4 mL, 16.9 mmol) were added at 0°C, and Compound 6 (3.4 g, 4.66 mmol) was added. After stirring at room temperature for 3 hours, the reaction mixture was diluted with ethyl acetate (200 mL), washed with distilled water (150 mL), and then dried over anhydrous sodium sulfate. After filtration and concentration under reduced pressure, the product was purified by column chromatography, thereby obtaining Compound 255 (3.2 g, 60%). EI-MS m/z : $[M+H]^+$1275.53, $[M/2+H]^+$638.53.

**<Example 92> Preparation of Compound 258**

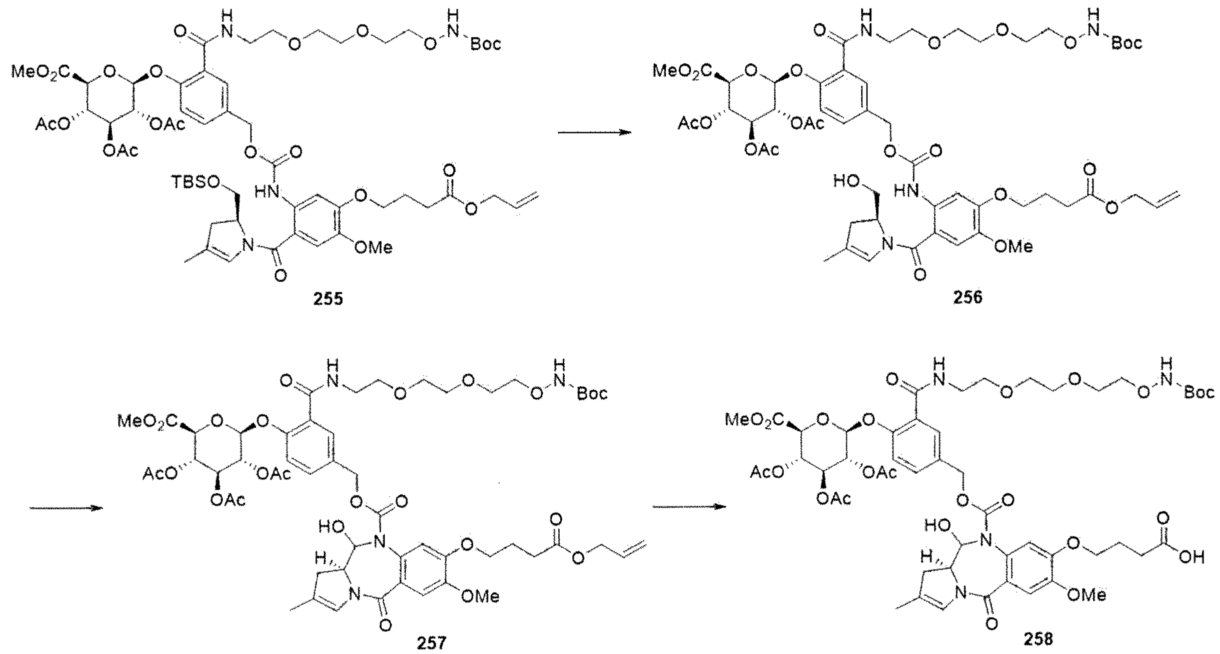

255 256 257 258

## Preparation of Compound 256

Compound 255 (3.2 g, 2.51 mmol) was dissolved in tetrahydrofuran/distilled water (10 mL/10 mL), acetic acid (10 mL) was added at 0°C, and the mixture was stirred at room temperature for 17 hours. The reaction mixture was diluted with ethyl acetate (100 mL), washed with distilled water (50 mL) and saturated sodium hydrogen carbonate solution (2 × 100 mL), and then dried over anhydrous sodium sulfate. After filtration and concentration under reduced pressure, the product was purified by column chromatography, thereby obtaining Compound 256 (2.6 g, 89%). EI-MS m/z : $[M+H]^+$1161.45, $[(M-BOC)/2+H]^+$531.47.

## Preparation of Compound 257

Compound 256 (2.0 g, 1.72 mmol) was dissolved in dichloromethane (50 mL), Dess-Martin periodinane (876 mg, 2.0 mmol) was added, and the mixture was stirred at room temperature for 2 hours under a nitrogen atmosphere. The reaction mixture was diluted with dichloromethane (200 mL), washed with saturated aqueous sodium hydrogen carbonate solution (150 mL) and aqueous sodium thiosulfate solution (100 mL), and then dried over anhydrous sodium sulfate. After filtration and concentration under reduced pressure, the product was purified by column chromatography, thereby obtaining Compound 257 (1.4 g, 71%). EI-MS m/z : $[M+H]^+$1159.40, $[(M-BOC)/2+H]^+$530.46.

## Preparation of Compound 258

Compound 257 (200 mg, 0.17 mmol) was dissolved in *N*,*N*-dimethylformamide (2 mL), then tetrakis(triphenylphosphine)palladium(0) (60 mg, 0.05 mmol) and sodium 2-ethylhexanoate (91 mg, 0.61 mmol) were added, and the mixture was stirred at room temperature for 19 hours under a nitrogen atmosphere. The reaction mixture was diluted with ethyl acetate (50 mL), washed with saturated aqueous ammonium chloride solution (50 mL) and distilled water (50 mL), and then dried over anhydrous sodium sulfate. Filtration and concentration under reduced pressure were performed, thereby obtaining Compound 258 (196 mg).EI-MS m/z : $[M+H]^+$1119.37, $[M/2+H]^+$510.44.

## <Example 93> Preparation of Compound 259

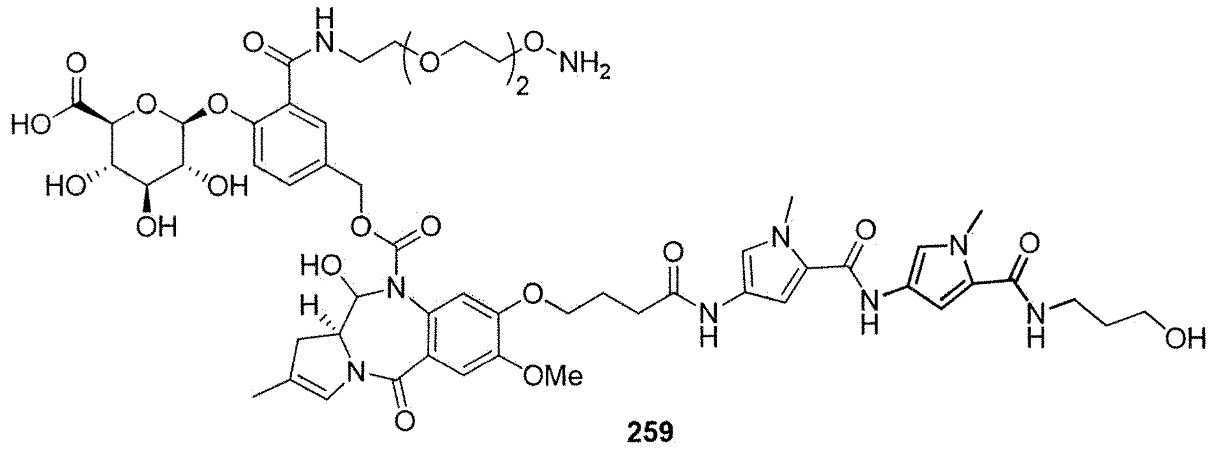

259

Compound 259 was synthesized from Compound 258 and Compound 26 in a similar manner to the synthesis of Compound 29. EI-MS m/z : $[M+H]^+$1180.48, $[M/2+H]^+$591.67.

**<Example 94> Preparation of Compound 264**

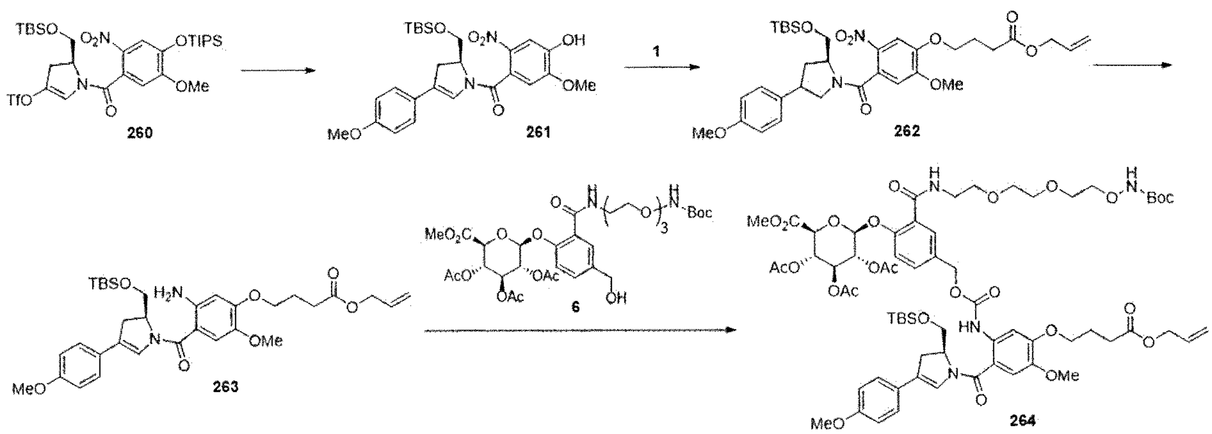

## Preparation of Compound 261

Compound 260 (4.6 g, 6.45 mmol, Compound 260 was prepared by the method described in ACS. Med. Chem. 2016, 7, 11, 983-987) was dissolved in ethanol/toluene/distilled water (10 mL/20 mL/10 mL), and then 4-methoxyphenylboronic acid (1.27 g, 8.39 mmol), sodium carbonate (2.0 g, 19.36 mmol), and tetrakis(triphenylphosphine)palladium(0) (745 mg, 0.65 mmol) were added at room temperature under a nitrogen atmosphere. After the reaction solution was stirred at 85°C for 1 hour, the reaction mixture was diluted with ethyl acetate (100 mL), and the organic layer was washed with brine (100 mL) and distilled water (100 mL). The collected organic layers were dried over anhydrous sodium sulfate, filtered and then concentrated under reduced pressure. The obtained compound was dissolved in *N,N*-dimethylformamide/distilled water (50 mL/1.0 mL), sodium acetate (688 mg, 8.38 mmol) was added, and then the mixture was stirred at room temperature for 1 hour under a nitrogen atmosphere. The reaction mixture was diluted with ethyl acetate (200 mL) and washed with distilled water (2 × 100 mL), and then the organic layer was dried over anhydrous sodium sulfate. After filtration and concentration under reduced pressure, the product was purified by column chromatography, thereby obtaining Compound 261 (2.75 g, 83%). $^1$H-NMR (400 MHz, $CDCl_3$) δ 7.81 (s, 1H), 7.14 (d, 2H), 6.85 (s, 1H), 6.80 (d, 2H), 6.15 (s, 1H), 6.05 (br s, 1H), 4.79 (br s, 1H), 4.00 (s, 3H), 3.78 (s, 3H), 3.18 (br, 1H), 3.02-2.98 (m, 1H), 0.90 (s, 9H), 0.10 (s, 6H).

## Preparation of Compound 262

Compound 261 (3.7 g, 7.19 mmol) and Compound 1 (2.2 g, 10.78 mmol) were dissolved in acetone/*N,N*-

dimethylformamide (40 mL/10 mL), then potassium carbonate (2.0 mg, 14.4 mmol) and sodium iodide (538 mg, 3.59 mmol) were added, and the mixture was stirred for 19 hours under a nitrogen atmosphere while being heated under reflux. The reaction mixture was concentrated, then diluted with ethyl acetate (300 mL), washed with distilled water (2 × 100 mL), and then dried over anhydrous sodium sulfate. After filtration and concentration under reduced pressure, the product was purified by column chromatography, thereby obtaining Compound 262 (4.2 g, 91%). $^1$H-NMR (400 MHz, $CDCl_3$) δ 7.73 (s, 1H), 7.13 (d, 2H) , 6.82 (s, 1H), 6.80 (d, 2H), 6.13 (s, 1H), 5.97-5.90 (m, 1H), 5.33 (d, 1H), 5.25 (d, 1H), 4.79 (brs, 1H), 4.62 (d, 2H), 4.18 (t, 2H), 3.95 (s, 3H), 3.78 (s, 3H), 3.18 (br , 1H), 3.02-2.98 (m, 1H), 2.61 (t, 2H), 2.27-2.20 (m, 2H), 0.90 (s, 9H), 0.10 (s, 6H) .

**Preparation of Compound 263**

Compound 262 (2.0 g, 3.12 mmol) was dissolved in ethyl acetate (20 mL) and ethanol (30 mL), then zinc dust (5.1 g, 78.0 mmol) was added at 0°C, and formic acid (2.9 mL, 78.03 mmol) was added. After stirring at room temperature for 3 hours, the reaction mixture was diluted with ethyl acetate (30 mL) and filtered through Celite, and ethyl acetate (70 mL) was added. The organic layer was washed with saturated aqueous sodium hydrogen carbonate solution (2 × 100 mL) and brine (50 mL) in that order, and then dried over anhydrous sodium sulfate. Filtration, concentration under reduced pressure, and purification by column chromatography were performed, thereby obtaining Compound 263 (1.9 g, 96%). EI-MS m/z : $[M+H]^+$612.83.

**Preparation of Compound 264**

Compound 263 (1.9 g, 3.08 mmol) was dissolved in dry tetrahydrofuran (20 mL), then triphosgene (328 mg, 1.11 mmol) and triethylamine (1.7 mL, 12.33 mmol) were added at 0°C, and Compound 6 (2.5 g, 3.39 mmol) was added. After stirring at room temperature for 17 hours, the reaction mixture was diluted with ethyl acetate (200 mL), washed with distilled water (150 mL), and then dried over anhydrous sodium sulfate. After filtration and concentration under reduced pressure, the product was purified by column chromatography, thereby obtaining Compound 264 (2.9 g, 70%). EI-MS m/z : $[M+H]^+$1368.79, $[(M-BOC)/2+H]^+$634.80.

**<Example 95> Preparation of Compound 267**

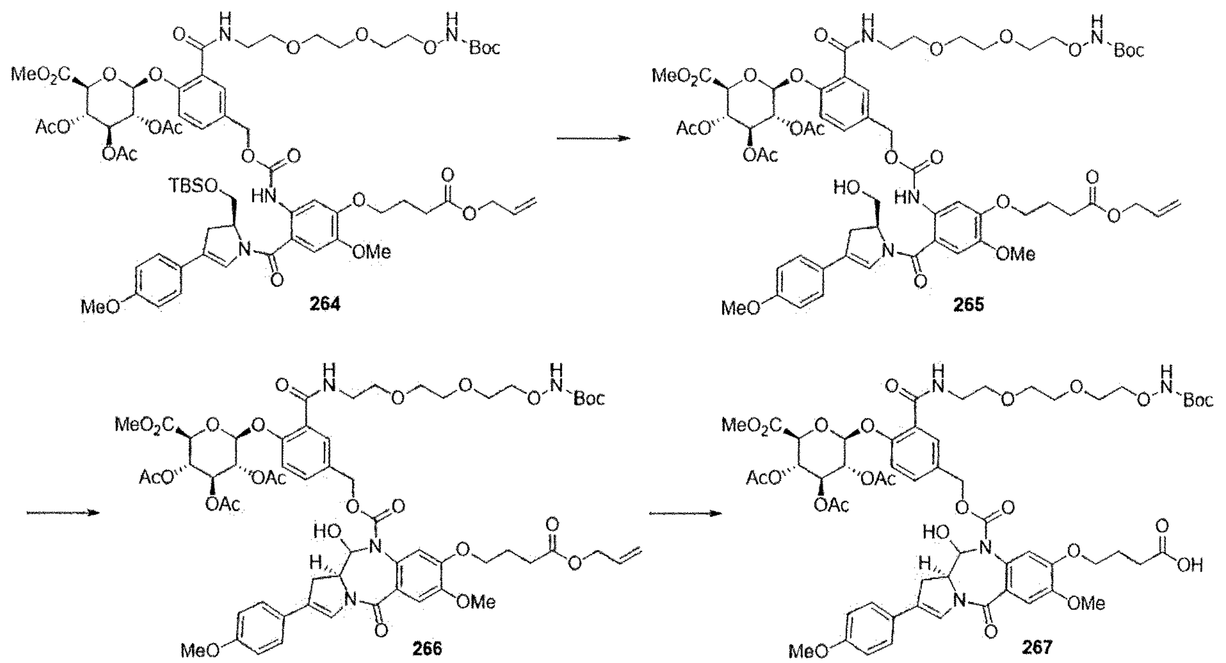

264

265

266

267

**Preparation of Compound 265**

Compound 264 (2.9 g, 2.15 mmol) was dissolved in tetrahydrofuran/distilled water (5 mL/5 mL), acetic acid (5 mL) was added at 0°C, and then the mixture was stirred at room temperature for 17 hours. The reaction mixture was

diluted with ethyl acetate (100 mL), washed with distilled water (50 mL) and saturated sodium hydrogen carbonate solution (2 × 100 mL), and then dried over anhydrous sodium sulfate. After filtration and concentration under reduced pressure, the product was purified by column chromatography, thereby obtaining Compound 265 (2.2 g, 81%). EI-MS m/z : $[M+H]^+$1254.45, $[(M-BOC)/2+H]^+$577.73.

## Preparation of Compound 266

Compound 265 (507 mg, 0.40 mmol) was dissolved in dichloromethane (8 mL), then Dess-Martin periodinane (206 mg, 0.49 mmol) was added, and the mixture was stirred at room temperature for 1.5 hours under a nitrogen atmosphere. The reaction mixture was diluted with dichloromethane (200 mL), washed with saturated aqueous sodium hydrogen carbonate solution (150 mL) and aqueous sodium thiosulfate solution (100 mL), and then dried over anhydrous sodium sulfate. After filtration and concentration under reduced pressure, the product was purified by column chromatography, thereby obtaining Compound 266 (483 mg, 48%). EI-MS m/z : $[M+H]^+$1252.34, $[(M-BOC)/2+H]^+$576.42.

## Preparation of Compound 267

Compound 266 (300 mg, 0.24 mmol) was dissolved in *N*,*N*-dimethylformamide (6 mL), then tetrakis(triphenylphosphine)palladium(0) (14 mg, 0.01 mmol) and pyrrolidine (0.04 mL, 0.48 mmol) were added, and the mixture was stirred at room temperature for 19 hours under a nitrogen atmosphere. The reaction mixture was diluted with ethyl acetate (50 mL), washed with saturated aqueous ammonium chloride solution (50 mL) and distilled water (50 mL), and then dried over anhydrous sodium sulfate. After filtration and concentration under reduced pressure, the product was purified by column chromatography, thereby obtaining Compound 267 (232 mg, 80%). EI-MS m/z : $[M+H]^+$1211.80, $[(M-BOC)/2+H]^+$556.42.

## <Example 96> Preparation of Compound 268

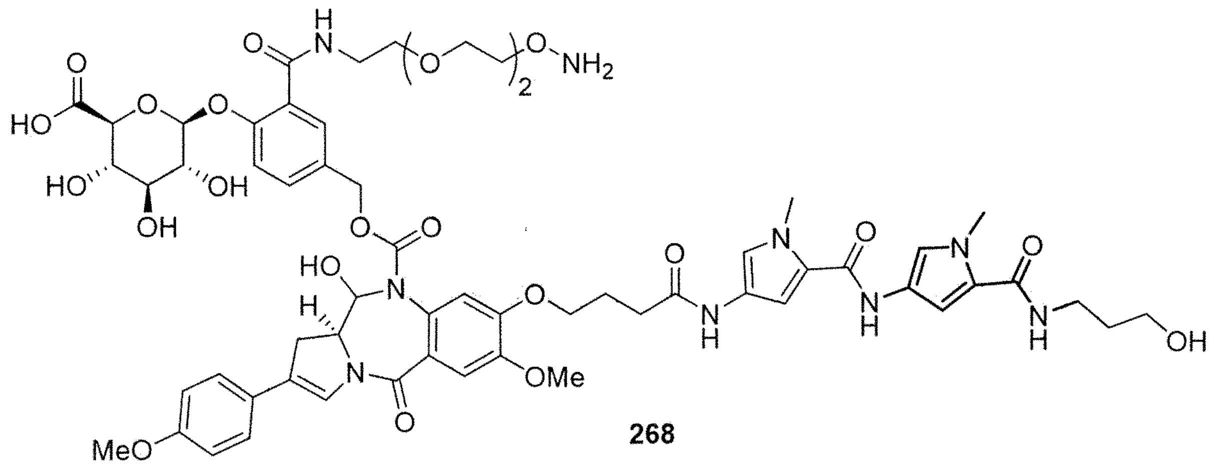

268

Compound 268 was synthesized from Compound 267 and Compound 26 in a similar manner to the synthesis of Compound 29. EI-MS m/z : $[M+H]^+$1272.65, $[M/2+H]^+$637.12.

## <Example 97> Preparation of ADC

### Example 97-1. Method for preparing ADC

ADCs were prepared through the following two steps, and commonly used isoprenoids LCB14-0511, 0512 and 0606 were prepared by the method described in Korean Patent Application Laid-Open No. 10-2014-0035393. The structural formulas of LCB14-0511, 0512 and 0606 are as follows.

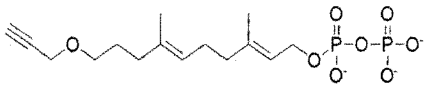

$3(NH_4^+)$

LCB14-0511

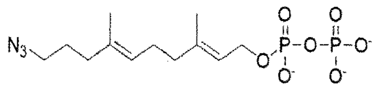

$3(NH_4^+)$

LCB14-0512

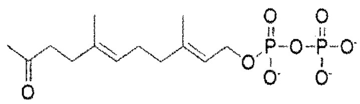

$3(NH_4^+)$

LCB14-0606

**Step 1: Preparation of prenylated antibody**

For the prenylation reaction of the antibody in the present invention, a reaction mixture of a buffer solution {50 mM tris hydrochloride (Tris-HCl, pH 7.4), 5 mM magnesium chloride ($MgCl_2$), 10 μM zinc chloride ($ZnCl_2$), 0.250 mM dithiothreitol (DTT)} containing 24 μM antibody, 200 nM FTase and 0.144 mM or 0.250 mM isoprenoid (LCB14-0511, 0512 or 0606) was used. After completion of the reaction, the prenylated antibody was desalted using a G25 Sepharose column (AKTA purifier, GE healthcare) equilibrated with PBS buffer.

**Step 2: Drug-conjugation reaction**

In order to prepare ADCs using oxime binding reaction, a reaction mixture was prepared by mixing 100 mM sodium acetate (NaOAc) buffer (pH 5.2), 10% dimethyl sulfoxide (DMSO), 24 μM prenylated antibody and 240 μM linker-drug together, and gently stirred at 30°C. After 2 hours or 24 hours of reaction, excess low molecular weight compounds were removed using a G25 Sepharose column, and protein fractions were collected and concentrated.

In order to prepare ADCs using copper-free click binding reaction, a reaction mixture was prepared by mixing PBS buffer (pH 7.4), 1% dimethyl sulfoxide (DMSO), 10 μM prenylation antibody and 100 μM linker-drug together, and reacted at 25°C for 6 hours, then excess low molecular weight compounds were removed using a G25 Sepharose column, and protein fractions were collected and concentrated.

In order to prepare ADCs using a click binding reaction using copper, a reaction mixture was prepared by mixing 20 μM prenylation antibody, 200 μM linker-drug, 10% dimethyl sulfoxide (DMSO), 1 mM copper sulfate pentahydrate, 2 mM (BimC4A)3 (Sigma-Aldrich), 10 mM sodium ascorbate and 10 mM aminoguanidine hydrochloride together, reacted at 25°C for 3 hours, then treated with 20 mM EDTA, and reacted for 30 minutes. After completion of the reaction, excess low molecular weight compounds were removed using a G25 Sepharose column, and protein fractions were collected and concentrated.

**Example 97-2. Preparation of ADC using antibody HER2**

ADCs were prepared using the linker-drug prepared in Examples including Examples 23 and 29 above and antibody HER2. The antibody HER2 was prenylated by step 1 of the method disclosed in Example 97-1, and then ADCs were prepared by using the oxime binding reaction and copper-free click binding reaction or click binding reaction using copper in step 2 of the method disclosed in Example 97-1. At this time, the compounds used and the ADCs prepared are as presented in Table 1 below. (Table 1. List of ADCs prepared)

[Table 1]

| ADC number | Compound number |
|---|---|
| ADC1 | 23 |
| ADC2 | 29 |
| ADC3 | 35 |
| ADC4 | 38 |
| ADC5 | 41 |
| ADC 6 | 45 |
| ADC7 | 54 |
| ADC8 | 57 |
| ADC9 | 61 |
| ADC10 | 65 |

(continued)

| ADC number | Compound number |
|---|---|
| ADC11 | 68 |
| ADC12 | 76 |
| ADC13 | 85 |
| ADC14 | 88 |
| ADC15 | 94 |
| ADC16 | 100 |
| ADC17 | 110 |
| ADC18 | 118 |
| ADC19 | 129 |
| ADC20 | 139 |
| ADC21 | 148 |
| ADC22 | 158 |
| ADC23 | 163 |
| ADC24 | 168 |
| ADC25 | 173 |
| ADC26 | 177 |
| ADC27 | 190 |
| ADC28 | 194 |
| ADC29 | 204 |
| ADC30 | 211 |
| Comparative Example 1 | 231 |
| (Example 81) | |
| Comparative Example 2 (Example 83) | 235 |

**<Example 98> In vitro cytotoxicity evaluation**

The cell proliferation inhibitory activity of the ADCs prepared in Example 91 against cancer cells was measured. For this purpose, commercially available cancer cell lines (SK-BR3, JIMT-1, and MDA-MB-468 cell lines) were used. Each cancer cell line was inoculated into a 96-well plate by 1500 to 5000 cells per well, incubated for 24 hours, and then serially treated with the diluted ADC samples. After 144 hours, the number of viable cells was quantified by SRB assay. The results of cytotoxicity against cancer cells are presented in Table 2. (Table 2. Comparison of cytotoxicity of ADC samples in antigen-expressing cancer cell lines)

[Table 2]

| ADC number | $IC_{50}$(pM) | |
|---|---|---|
| | SK-BR3 | JIMT-1 |
| ADC1 | 13.6 | 52.8 |
| ADC2 | 22.5 | 74.4 |
| ADC3 | 13.5 | 30.7 |
| ADC4 | 22.4 | 307.0 |
| ADC5 | 29.2 | 285.3 |

(continued)

| ADC number | $IC_{50}$(pM) | |
|---|---|---|
| | SK-BR3 | JIMT-1 |
| ADC6 | 11.5 | 79.9 |
| ADC7 | 31.1 | 406.2 |
| ADC8 | 31.6 | ND |
| ADC9 | 85.1 | ND |
| ADC10 | 13.9 | 115.1 |
| ADC11 | 29.2 | ND |
| ADC12 | 43.3 | ND |
| ADC13 | 63.3 | ND |
| ADC14 | 27.9 | 224.1 |
| ADC15 | 33.5 | ND |
| ADC16 | 33.2 | ND |
| ADC17 | 12.0 | 83.1 |
| ADC18 | 10.9 | 57.0 |
| ADC19 | 44.5 | 65.8 |
| ADC21 | 150.7 | ND |
| ADC22 | 94.9 | ND |
| ADC23 | 71.5 | ND |
| ADC24 | 44.0 | ND |
| ADC25 | 513.5 | ND |
| ADC26 | 121.7 | ND |
| ADC27 | 20.8 | 270.0 |
| ADC28 | 50.9 | ND |
| ADC30 | 18.6 | 108.5 |
| Comparative Example 1 (Example 81) | 19.1 | 188.5 |
| Comparative Example 2 (Example 83) | 8.6 | 55.8 |
| * ND : Not determined. | | |

It was confirmed that ADCs 1 to 7, 10, 14, 17 to 19, 27, and 30 exhibited cytotoxicity similar or superior to that of Comparative Example 1 or Comparative Example 2 in the antigen-overexpressed cancer cell lines. Among others, ADC2 exhibited cytotoxicity superior to that of Comparative Example 1 and similar to that of Comparative Example 2.

The cytotoxicity was evaluated in the MDA-MB-468 cell line, which does not express an antigen, by treating the cell line with ADCs at a high concentration. (Table 3. Comparison of cytotoxicity of ADC samples in cancer cell lines not expressing antigens)

[Table 3]

| ADC number | $IC_{50}$ (nM) |
|---|---|
| | MDA-MB-468 |
| ADC2 | 238.9 |
| Comparative Example 2 (Example 83) | 57.3 |

In the MDA-MB-468 cancer cell line in which an antigen was not expressed, ADC2 exhibited cytotoxicity weaker than that of Comparative Example 2 by four times or more.

From the results above, it can be seen that ADC2 according to the present invention has a much wider therapeutic window since the efficacy of ADC2 in antigen-expressing cell lines is similar to that of Comparative Examples and ADC2 exhibits weak toxicity in cells that do not express antigens.

**<Example 99> Rat preliminary toxicity evaluation**

The ADCs prepared in Example 91 were administered intravenously to 8-week-old SD rats once, and the weight changes and death were observed for 70 days. The lethal dose and maximum tolerable dose (MTD) are summarized as follows based on the death according to each ADC and dose. (Table 4. Comparison of toxicity of ADC samples in SD rats)

[Table 4]

| | MTD | Lethal dose |
|---|---|---|
| ADC2 | 9 mg/kg | 12 mg/kg |
| Comparative Example 1 | NA | 2 mg/kg |
| Comparative Example 2 | NA | 4 mg/kg |
| (* NA : not available (MTD cannot be confirmed since all died at the experimental dose) | | |

It was confirmed that the lethal dose of ADC2 according to the present invention in the rat toxicity test was significantly higher than those of Comparative Examples 1 and 2.

From the results above, it can be seen that ADC2 according to the present invention exhibits relatively weak toxicity and excellent safety since the lethal dose of ADC2 in SD rats is significantly higher than those of Comparative Examples 1 and 2.

[Industrial Applicability]

The present invention has application in the pharmaceutical industry related to the treatment of diseases, including the treatment of proliferative disease.

## Claims

1. A pyrrolobenzodiazepine derivative represented by Chemical Formula I, or a pharmaceutically acceptable salt or solvate of the derivative:

$$P\text{-}(B)_a\text{-}(B')_b\text{-}D \qquad (I)$$

where,
P is represented by the following Chemical Formula II, where P is bonded or not bonded to Linker I;

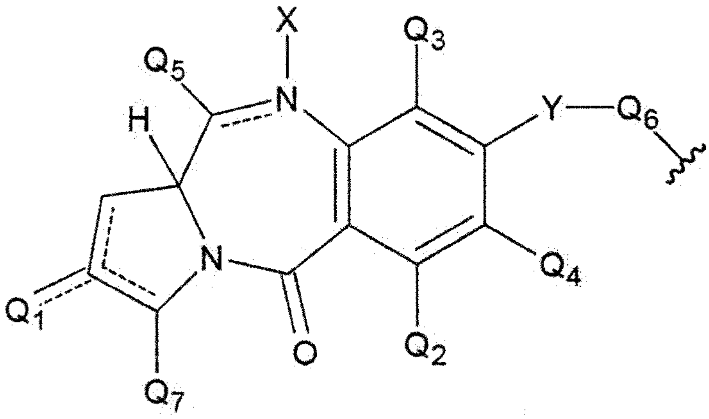

(II)

where,

a dotted line indicates arbitrary presence of a double bond between C1 and C2 or C2 and C3 and arbitrary presence of a double bond between N10 and C11;

$Q_1$ is selected from the group consisting of H, OH, O, $CH_2$, CN, $R^m$, $OR^m$, CH-$R^{m'}$, $C(R^{m'})_2$, O-$SO_2$-$R^m$, $CO_2R^m$, $COR^m$, halo and dihalo,

where $R^{m'}$ is selected from the group consisting of $R^m$, $CO_2R^m$, $COR^m$, CHO, $CO_2H$, and halo,

where $R^m$ is selected from the group consisting of substituted or unsubstituted $C_{1-12}$ alkyl, substituted or unsubstituted $C_{2-12}$ alkenyl, substituted or unsubstituted $C_{2-12}$ alkynyl, substituted or unsubstituted $C_{5-20}$ aryl, substituted or unsubstituted $C_{5-20}$ heteroaryl, substituted or unsubstituted $C_{3-6}$ cycloalkyl, substituted or unsubstituted 3- to 7-membered heterocyclyl, substituted or unsubstituted 3- to 7-membered heterocycloalkyl, and substituted or unsubstituted 5- to 7-membered heteroaryl,

wherein, when substituted, respective hydrogen atoms of $C_{1-12}$ alkyl, $C_{1-12}$ alkoxy, $C_{2-12}$ alkenyl, $C_{2-12}$ alkynyl, $C_{5-20}$ aryl, $C_{5-20}$ heteroaryl, $C_{3-6}$ cycloalkyl, 3- to 7-membered heterocyclyl, 3- to 7-membered heterocycloalkyl, or 5- to 7-membered heteroaryl are each independently substituted with any one or more selected from the group consisting of $C_{1-12}$ alkyl, $C_{2-12}$ alkenyl, $C_{2-12}$ alkynyl, $C_{5-20}$ aryl, $C_{5-20}$ heteroaryl, $C_{3-6}$ cycloalkyl, 3- to 7-membered heterocyclyl, 3- to 7-membered heterocycloalkyl, and 5-to 7-membered heteroaryl;

$Q_2$, $Q_3$, $Q_5$, and $Q_7$ are each independently selected from the group consisting of -H, -$R^m$, -OH, -$OR^m$, -SH, -$SR^m$, -$NH_2$, -$NHR^m$, -$NR^mR^{m'}$, -$NO_2$, and halo,

where $R^m$ and $R^{m'}$ are as defined above;

$Q_4$ is selected from the group consisting of -H, -$R^m$, -OH, -$OR^m$, -SH, -$SR^m$, -$NH_2$, -$NHR^m$, -$NR^mR^{m'}$, -$NO_2$, halo, substituted or unsubstituted $C_{1-6}$ alkyl, substituted or unsubstituted $C_{1-6}$ alkoxy, substituted or unsubstituted $C_{2-6}$ alkenyl, substituted or unsubstituted $C_{2-6}$ alkynyl, substituted or unsubstituted $C_{3-6}$ cycloalkyl, substituted or unsubstituted 3- to 7-membered heterocycloalkyl, substituted or unsubstituted $C_{5-12}$ aryl, substituted or unsubstituted 5- to 7-membered heteroaryl, -CN, -NCO, - $OR^n$, -OC(=O)$R^n$, -OC(=O)$NR^nR^{n'}$, -OS(=O)$R^n$, -$OS(=O)_2R^n$, -$SR^n$, -S(=O)$R^n$, -$S(=O)_2R^n$, -S(=O)$NR^nR^{n'}$, -$S(=O)_2NR^nR^{n'}$,-OS(=O)$NR^nR^{n'}$, -$OS(=O)_2NR^nR^{n'}$, -$NR^nR^{n'}$, -$NR^nC(=O)R^o$,-$NR^nC(=O)OR^o$, -$NR^nC(=O)NR^oR^{o'}$, -$NR^nS(=O)R^o$, -$NR^nS(=O)_2R^o$,-$NR^nS(=O)NR^oR^{o'}$, -$NR^nS(=O)_2NR^oR^{o'}$, -C(=O)$R^n$, -C(=O)$OR^n$ and-C(=O)$NR^nR^{n'}$,

wherein, when substituted, one or more hydrogen atoms may be each independently substituted with $C_{1-6}$ alkyl, $C_{1-6}$ alkoxy, $C_{2-6}$ alkenyl, $C_{2-6}$ alkynyl, $C_{3-6}$ cycloalkyl, 3- to 7-membered heterocycloalkyl, $C_{5-10}$ aryl, 5- to 7-membered heteroaryl, -ORP, -OC(=O)$R^p$, - OC(=O)$NR^pR^{p'}$, -OS(=O)$R^p$, -$OS(=O)_2R^p$, -SRP, -S(=O)$R^p$, -$S(=O)_2$RP, -S (=O) NRPRP', -S (=O) $_2NR^pR^{p'}$, -OS (=O) NRPRP',-$OS(=O)_2NR^pR^{p'}$, -NRPRP', -$NR^pC(=O)R^q$, -$NR^pC(=O)OR^q$,-$NR^pC(=O)NR^qR^{q'}$, -$NR^pS(=O)R^q$, -$NR^pS(=O)_2R^q$, -$NR^pS(=O)NR^qR^{q'}$,-$NR^pS(=O)_2NR^qR^{q'}$, -C(=O)$R^p$, -C(=O)$OR^p$ or -C(=O)$NR^pR^p$,

where, $R^n$, $R^{n'}$, $R^o$, $R^{o'}$, RP, $R^{p'}$, $R^q$ and $R^{q'}$ are each independently selected from the group consisting of hydrogen, $C_{1-7}$ alkyl, $C_{2-7}$ alkenyl, $C_{2-7}$ alkynyl, $C_{3-13}$ cycloalkyl, 3- to 7-membered heterocycloalkyl, $C_{6-10}$ aryl, and 5- to 7-membered heteroaryl;

X is a moiety bonded to Linker I, and is absent when not bonded to Linker I,

Y is selected from the group consisting of -O-, -S-, and -NH-; and

$Q_6$ is absent, or is a substituted or unsubstituted and saturated or unsaturated $C_{3-12}$ hydrocarbon chain,

B and B' are each independently a bond, -O-, -NH-, - S-, -C(=O)-, -S(=O)-, -O-$(CH_2)_n$-, -$(CH_2)$n-, -$(CH_2)_n$(C=O)-, -$(CH_2)_n$(C=O)NH-, -$C_{6-10}$ arylene-, -O-(-$C_{6-10}$ arylene) -, - $(CH_2)_n$-$C_{6-10}$ arylene-, 5- to 20-membered heteroarylene containing a heteroatom independently selected from N, O or S, -0-(5- to 20-membered heteroarylene

containing a heteroatom independently selected from N, O or S)- or - $(CH_2CH_2O)n$-, where n is each independently an integer of 1 or more and 10 or less, and a and b are each independently an integer of 0 or 1, and D is represented by the following Chemical Formula III,

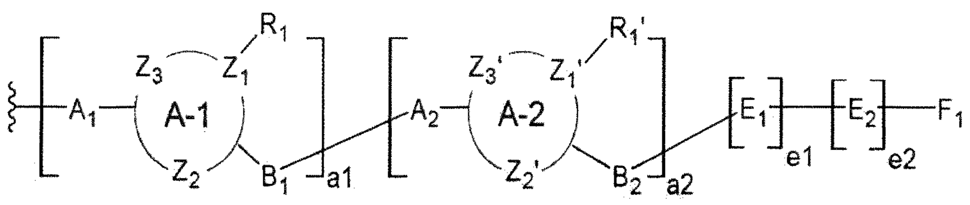

(III)

where,

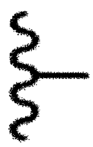

means a site bonded to P, B or B',
$A_1$ and $A_2$ are each independently a bond, -NH-, -O-, -C(=O)-, -C(=O)NH-, -NHC(=O)-, $C_{6-10}$ arylene or 6- to 10-membered heteroarylene containing a heteroatom independently selected from N, O or S;
A-1 and A-2 are each independently selected from 5-to 20-membered heterocycloalkylene, 5- to 20-membered heterocycloalkenylene, or 5- to 20-membered heteroarylene, in which one or more ring atoms are heteroatoms independently selected from N, O or S,
$Z_1$, $Z_2$, $Z_3$, $Z_1$', $Z_2$' and $Z_3$' are each independently C, N or S,
$R_1$ and $R_1$' are each independently $C_{1-10}$ heteroalkyl in which one or more atoms include heteroatoms independently selected from N, O or S, $C_{1-10}$ alkyl, $-(CH_2)_mOH$, $-(CH_2)_mNH_2$, $-(CH_2)_mNHCH_3$, hydrogen, halo, or -C(=O)OH,
m is an integer from 0 to 10,
$B_1$ and $B_2$ are each independently a bond, -NH-, -O-, -C(=O)-, or -S-,
$a_1$ and $a_2$ are each independently an integer from 0 to 2; wherein $a_1 + a_2$ is an integer of 2 or less,
$E_1$ is a bond, -NH-, -C(=O)-, -C(=O)O-, -0-, -S-,$-OCH_2$-, $C_{1-20}$ alkylene, or $-((CH_2)_p(CH_2E_{11}))_q$-, and $e_1$ is an integer from 0 to 10;
$E_2$ is a bond, $C_{1-20}$ alkylene, or $-((CH_2)_p(CH_2E_{11}))_q$-, and $e_2$ is an integer from 0 to 20,
where $E_{11}$ is a bond, O, or S, p is an integer from 0 to 10, and q is an integer from 1 to 20; and
$F_1$ is hydrogen, -OH, $-OCH_3$, -SH, $-SCH_3$,

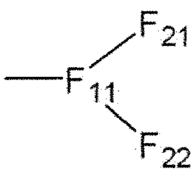
,
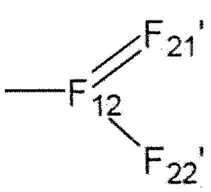
, or
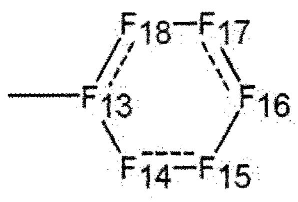
,

where,
$F_{11}$, $F_{12}$, $F_{13}$, $F_{14}$, $F_{15}$, $F_{16}$, $F_{17}$, and $F_{18}$ are each independently C, N, O or S,
a dotted line means a double bond or a single bond,
$F_{21}$, $F_{22}$ and $F_{22}$' are each independently selected from -H, $-NH_2$, $-CH_3$, $-(CH_2)_rCH_3$, $-(CH_2)_rOH$, $-(CH_2)rNH_2$,$-F_{33}(CH_2)_rCH_3$, $-F_{33}(CH_2)_rOH$, $-F_{33}(CH_2)_rNH_2$, or Linker II, where $F_{33}$ is selected from -O-, -S-, $-CH_2$-, -C(=O)-, -NH-, - $C(NH_2)$ - or -C(=NH) -, and
$F_{21}$' is selected from $=CH_2$, =NH, $=F_{33}' (CH_2)_rCH_3$, $=F_{33}'(CH_2)_rOH$, or $=F_{33}'(CH_2)rNH_2$, where $F_{33}$' is selected from =N-, =CH-, =C(OH)- or $=C(NH_2)$- and r is an integer from 0 to 10;
wherein, Linker I and Linker II are represented by the following Chemical Formula IV, and are each independently

selected,

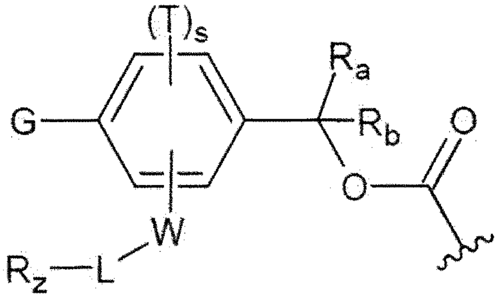

(IV)

where,
G is a glucuronic acid) moiety or

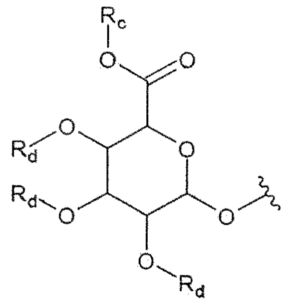

;

where $R_c$ is hydrogen or a carboxyl protecting group,
each $R_d$ is independently hydrogen or a hydroxyl protecting group;
$R_a$ and $R_b$ are each independently hydrogen, $C_{1-8}$ alkyl or $C_{3-8}$ cycloalkyl;
W is -C(=O)-, -C(=O)NR'-, -C(=O)O-, $-S(=O)_2NR'-$, - P(=O)R"NR'-, -S(=O)NR'- or $-P(=O)_2NR'-$, and C, S or P is directly bonded to a phenyl ring,
where R' and R" are each independently hydrogen, $C_{1-8}$ alkyl, $C_{3-8}$-membered cycloalkyl, $C_{1-8}$ alkoxy, $C_{1-8}$ alkylthio, mono- or di- $C_{1-8}$ alkylamino, 3- to 20-membered heteroaryl or a compound of $C_{6-20}$-membered aryl;
T is independently $C_{1-8}$ alkyl, halogen, cyano or nitro;
s is an integer from 0 to 3;
L is one or more units selected from the group consisting of a connection unit and a branching unit, or a combination of these units,
wherein the connection unit connects W and $R_z$, W and a branching unit, or a branching unit and another branching unit, wherein the branching unit connects a connection unit and W or a connection unit and another connection unit,
the connection unit is

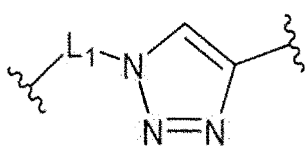

,

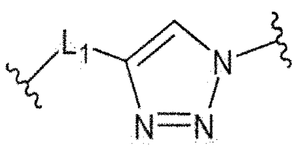, 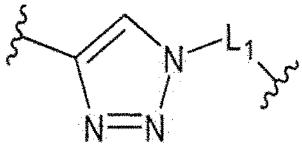, 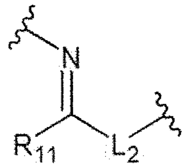, -

$(CH_2CH_2V)_t$-, $-(CH_2)_t(V(CH_2)_u)_v$-, or a combination thereof;
where $L_1$ is a single bond or $C_{2-30}$ alkenyl, $R_{11}$ is H or $C_{1-10}$ alkyl, and $L_2$ is $C_{2-30}$ alkenyl;
where t is an integer from 0 to 10, u is an integer from 0 to 12, v is an integer from 1 to 20, V is a single bond, -O-, or -S-,
the branching unit is selected from the group consisting of $C_{2-100}$ alkenyl, a hydrophilic amino acid, - C(=O)-, -C(=O)$NR^v$-, -C(=O)O-, $-(CH_2)_{n1}$-NHC(=O)-$(CH_2)_{n2}$-, - $(CH_2)_{n3}$-C(=O)NH-$(CH_2)_{n4}$-, $-(CH_2)_{n1}$-NHC(=O)-$(CH_2)_{n2}$-C(=O)-, - $(CH_2)_{n3}$-C(=O)NH-$(CH_2)_{n4}$-C(=O)-, -S(=O)$_2NR^v$-, -P(=O)$R^wNR^v$-, - S(=O)$NR^v$-, and -P(=O)$_2NR^v$-,
wherein, when the branching unit is $C_{2-100}$ alkenyl, a carbon atom of the alkenyl may be substituted with one or more heteroatoms selected from the group consisting of N, O and S, and the alkenyl may be further substituted with one or more $C_{1-20}$ alkyl,
where $R^v$ and $R^w$ are each independently H, $C_{1-8}$ alkyl, $C_{3-8}$ cycloalkyl, $C_{1-8}$ alkoxy, $C_{1-8}$ alkylthio, mono- or di-$C_{1-8}$ alkylamino, $C_{3-20}$ heteroaryl or $C_{5-20}$ aryl,
n1, n2, n3 and n4 are each independently an integer from 0 to 10, and
$R_z$ is -O-$NH_2$, -$NH_2$, $N_3$, substituted or unsubstituted $C_{1-12}$ alkyl, $C_{1-12}$ alkynyl, $C_{1-3}$ alkoxy, substituted or unsubstituted 3- to 20-membered heteroaryl, 3- to 20-membered heterocyclyl, substituted or unsubstituted $C_{5-20}$ aryl, or

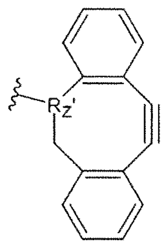,

where $R_z$' is N or C,
wherein, when substituted, one or more hydrogen atoms are each independently substituted with OH, =O, halo, $C_{1-6}$ alkyl, $C_{1-6}$ alkoxy, $C_{2-6}$ alkenyl, oxy, carboxy, $C_{1-6}$ alkoxycarbonyl, $C_{1-6}$ alkylcarbonyl, formyl, $C_{3-8}$ aryl, $C_{5-12}$ aryloxy, $C_{5-12}$ arylcarbonyl or $C_{3-6}$ heteroaryl.

**2.** The pyrrolobenzodiazepine derivative or a pharmaceutically acceptable salt or solvate of the derivative according to claim 1,
wherein the dotted line indicates presence of a double bond between C2 and C3.

**3.** The pyrrolobenzodiazepine derivative or a pharmaceutically acceptable salt or solvate of the derivative according to claim 1,
wherein $Q_1$ is selected from the group consisting of substituted or unsubstituted $C_{1-6}$ alkyl, substituted or unsubstituted $C_{2-6}$ alkenyl, substituted or unsubstituted $C_{5-7}$ aryl and substituted or unsubstituted $C_{3-6}$ heteroaryl.

**4.** The pyrrolobenzodiazepine derivative or a pharmaceutically acceptable salt or solvate of the derivative according to claim 1,
wherein $Q_2$, $Q_3$, $Q_5$, and $Q_7$ are each independently -H or -OH.

**5.** The pyrrolobenzodiazepine derivative or a pharmaceutically acceptable salt or solvate of the derivative according to claim 1,
wherein $Q_4$ is methoxy, ethoxy or butoxy.

6. The pyrrolobenzodiazepine derivative or a pharmaceutically acceptable salt or solvate of the derivative according to claim 1,
wherein Y is -O-.

7. The pyrrolobenzodiazepine derivative or a pharmaceutically acceptable salt or solvate of the derivative according to claim 1,
wherein $Q_6$ is a substituted or unsubstituted and saturated or unsaturated $C_{3-8}$ hydrocarbon chain.

8. The pyrrolobenzodiazepine derivative or a pharmaceutically acceptable salt or solvate of the derivative according to claim 1,

wherein G is

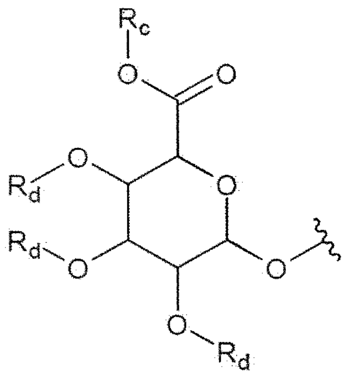

,

where $R_c$ is hydrogen or a carboxyl protecting group, and
each $R_d$ is independently hydrogen or a hydroxyl protecting group.

9. The pyrrolobenzodiazepine derivative or a pharmaceutically acceptable salt or solvate of the derivative according to claim 1,
wherein $R_c$ is hydrogen and each $R_d$ is hydrogen.

10. The pyrrolobenzodiazepine derivative or a pharmaceutically acceptable salt or solvate of the derivative according to claim 1,
wherein W is -C(=O)-, -C(=O)NR'- or -C(=O)O-.

11. The pyrrolobenzodiazepine derivative or a pharmaceutically acceptable salt or solvate of the derivative according to claim 1,
wherein W is -C(=O)NR'-, where C(=O) is bonded to a phenyl ring and NR' is bonded to L.

12. The pyrrolobenzodiazepine derivative or a pharmaceutically acceptable salt or solvate of the derivative according to claim 1,

wherein G is

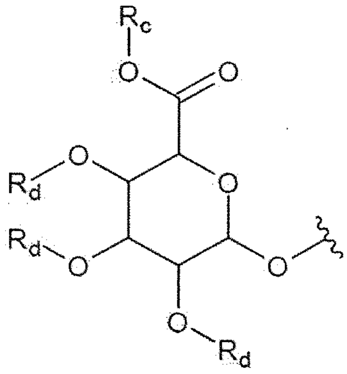

, and

W is -C(O)NR'-, where C(O) is bonded to a phenyl ring and NR' is bonded to L, and $R_c$ and $R_d$ are hydrogen.

**13.** The pyrrolobenzodiazepine derivative or a pharmaceutically acceptable salt or solvate of the derivative according to claim 1,

wherein the branching unit is selected from the group consisting of $C_{2-8}$ alkenyl, a hydrophilic amino acid, -C(O)-, -C(O)NR$^v$-, -C(O)O-, $-(CH_2)_{n1}-NHC(=O)-(CH_2)_{n2}-$, $-(CH_2)_{n3}-C(O)NH-(CH_2)_{n4}-$, $-(CH_2)_{n1}-NHC(O)-(CH_2)_{n2}-C(O)-$, and $-(CH_2)_{n3}-C(O)NH-(CH_2)_{n4}-C(O)-$,

wherein, when the branching unit is $C_{2-8}$ alkenyl, a carbon atom of the alkenyl may be substituted with one or more heteroatoms selected from the group consisting of N, O and S, and the alkenyl may be further substituted with one or more $C_{1-20}$ alkyl,

where $R^v$ is H, $C_{1-8}$ alkyl, $C_{3-8}$ cycloalkyl, $C_{1-8}$ alkoxy, $C_{1-8}$ alkylthio, mono- or di- $C_{1-8}$ alkylamino, $C_{3-20}$ heteroaryl or $C_{5-20}$ aryl, and

n1, n2, n3 and n4 are each independently an integer from 0 to 5.

**14.** The pyrrolobenzodiazepine derivative or a pharmaceutically acceptable salt or solvate of the derivative according to claim 1,
wherein the connection unit is $-(CH_2)_t(V(CH_2)_u)_v-$, where t is an integer from 0 to 8, u is an integer from 0 to 12, v is an integer from 1 to 10, and V is a single bond or -O-.

**15.** The pyrrolobenzodiazepine derivative or a pharmaceutically acceptable salt or solvate of the derivative according to claim 1,
wherein the connection unit is $-(CH_2)_t(V(CH_2)_u)_v-$, where t is an integer of 2, u is an integer of 2, v is an integer of 2, and V is -O-.

**16.** The pyrrolobenzodiazepine derivative or a pharmaceutically acceptable salt or solvate of the derivative according to claim 1,
wherein the connection unit includes

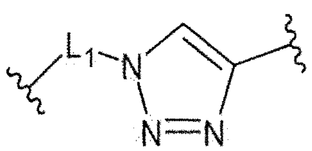 , 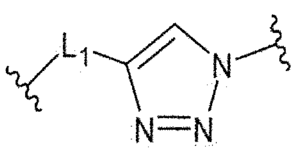 , 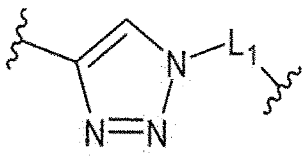

or

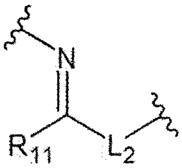

, where $L_1$ is a single bond or $C_{2-8}$ alkenyl, $R_{11}$ is H or $C_{1-6}$ alkyl, and $L_2$ is $C_{2-8}$ alkenyl.

**17.** The pyrrolobenzodiazepine derivative or a pharmaceutically acceptable salt or solvate of the derivative according to claim 1,
wherein $R_z$ is $-O-NH_2$, $-NH_2$, $N_3$, $-OCH_3$, $-OCH_2CH_3$, $-O(CH_2)_2CH_3$, or

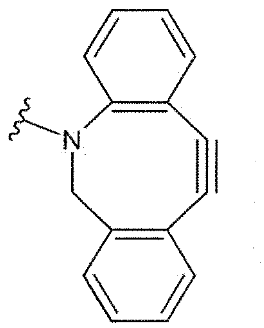

.

**18.** The pyrrolobenzodiazepine derivative or a pharmaceutically acceptable salt or solvate of the derivative according to claim 1,
wherein at least one or more of B or B' are -C(=O)-.

**19.** The pyrrolobenzodiazepine derivative or a pharmaceutically acceptable salt or solvate of the derivative according to claim 1,
wherein $A_1$ and $A_2$ are each independently -C(=O)- or -NH-.

**20.** The pyrrolobenzodiazepine derivative or a pharmaceutically acceptable salt or solvate of the derivative according to claim 1,
wherein at least one or more of $Z_1$, $Z_2$, $Z_3$, $Z_1'$, $Z_2'$, or $Z_3'$ are N.

**21.** The pyrrolobenzodiazepine derivative or a pharmaceutically acceptable salt or solvate of the derivative according to claim 1,
wherein A-1 and A-2 are selected from

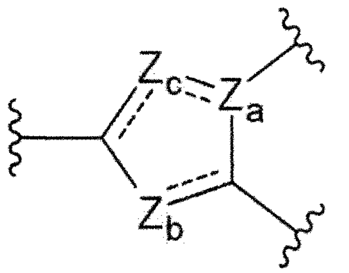

,

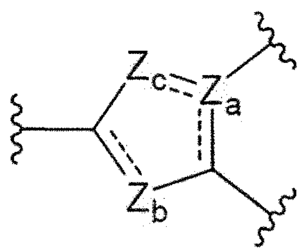

,

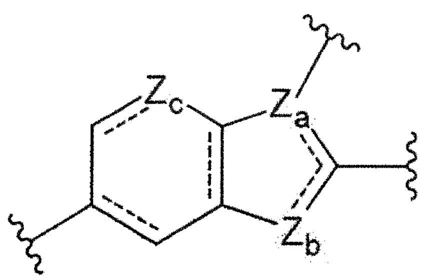

or

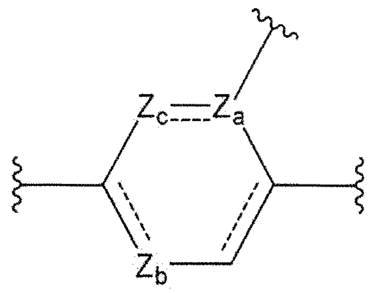

,

where a dotted line means a double bond or a single bond,

$Z_a$ is $Z_1$ or $Z_1$',
$Z_b$ is $Z_2$ or $Z_2$',
$Z_c$ is $Z_3$ or $Z_3$',
$Z_1$, $Z_2$, $Z_3$, $Z_1$', $Z_2$' and $Z_3$' are as defined above, and
$R_1$ or $R_1$' is connected to $Z_a$.

**22.** The pyrrolobenzodiazepine derivative or a pharmaceutically acceptable salt or solvate of the derivative according to claim 1,
wherein A-1 and A-2 are each independently

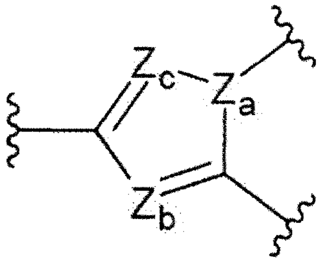

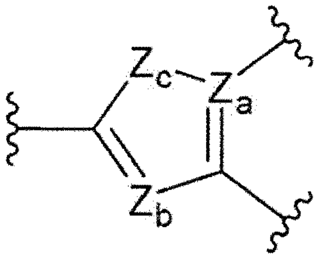

,

or

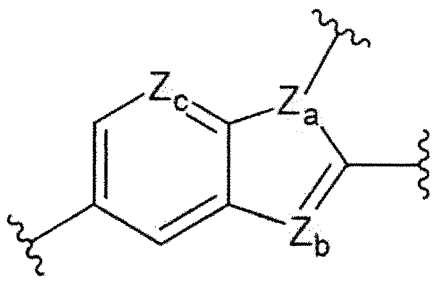

,

where a dotted line means a double bond or a single bond,
$Z_a$ is $Z_1$ or $Z_1$',
$Z_b$ is $Z_2$ or $Z_2$',
$Z_c$ is $Z_3$ or $Z_3$',
at least one or more of $Z_1$, $Z_2$, $Z_3$, $Z_1$', $Z_2$' or $Z_3$' are N or S, and
$R_1$ or $R_1$' is connected to $Z_a$.

**23.** The pyrrolobenzodiazepine derivative or a pharmaceutically acceptable salt or solvate of the derivative according to claim 1,
wherein $a_1$ and $a_2$ are an integer of 1.

**24.** The pyrrolobenzodiazepine derivative or a pharmaceutically acceptable salt or solvate of the derivative according to claim 1,
wherein $E_1$ is a bond, -NH- or -C(=O)-, and $e_1$ is an integer from 1 to 5.

**25.** The pyrrolobenzodiazepine derivative or a pharmaceutically acceptable salt or solvate of the derivative according to claim 1,

wherein $E_2$ is a bond, $C_{1-5}$ alkylene, or - $((CH_2)_p(CH_2E_{11}))_q$-,
when $E_2$ is -$((CH_2)_p(CH_2E_{11}))_q$-, $E_{11}$ is O,
p is an integer from 1 to 3,
q is an integer from 1 to 15, and
$e_2$ is an integer from 1 to 5.

**26.** The pyrrolobenzodiazepine derivative or a pharmaceutically acceptable salt or solvate of the derivative according to claim 1,
wherein $R_1$ and $R_1$' are each independently hydrogen, $C_{1-5}$ alkyl, $(CH_2)_mOH$ or $(CH_2)_mNH_2$, where m is an integer from 1 to 7.

**27.** The pyrrolobenzodiazepine derivative or a pharmaceutically acceptable salt or solvate of the derivative according to claim 1,

wherein $F_1$ is -OH, $-OCH_3$, -SH, $-SCH_3$,

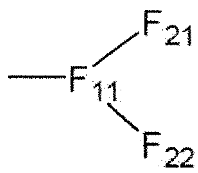

or

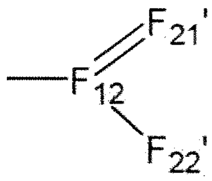

,

where $F_{11}$ and $F_{12}$ are each independently C or N,
$F_{21}$, $F_{22}$ and $F_{22}$' are each independently selected from H, $-(CH_2)_rCH_3$, $-(CH_2)_rOH$, $-(CH_2)_rNH_2$, $-F_{33}(CH_2)_rCH_3$,$-F_{33}(CH_2)_rOH$, $-F_{33}(CH_2)_rNH_2$ or Linker II, where $F_{33}$ is selected from --C(=O)-, -NH-, or -C(=NH)-,
$F_{21}$' is selected from $=CH_2$, =NH, $=F_{33}'(CH_2)_rCH_3$, $=F_{33}'(CH_2)_rOH$, $=F_{33}'(CH_2)_rNH_2$ or Linker II, where $F_{33}$' is selected from =N-, =CH-, =C(OH)- or $=C(NH_2)$-, and
at least one or more of $F_{21}$, $F_{22}$ or $F_{22}$' are $-CH_3$.

**28.** The pyrrolobenzodiazepine derivative or a pharmaceutically acceptable salt or solvate of the derivative according to claim 1,
wherein the pyrrolobenzodiazepine derivative further comprises the following General Formula V:

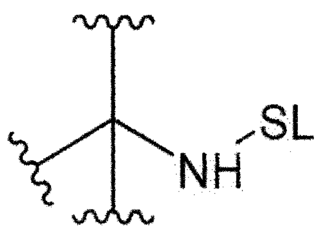

(V)

where,

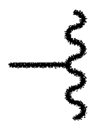

moieties are each independently bonded to a first linker or hydrogen, wherein the first linker is Linker I or Linker II; and SL means a second linker, wherein the second linker is represented by the following Formula VI,

L'-$R_{zz}$'          (VI)

where L' and $R_{zz}$' are the same as L and $R_z$ in Chemical Formula (IV), respectively.

**29.** The pyrrolobenzodiazepine derivative or a pharmaceutically acceptable salt or solvate of the derivative according to claim 28,

wherein the first linker contains at least one or more branching units, wherein the branching unit is selected from the group consisting of -C(O)-, -C(O)NR$^v$-, - C(O)O-, -$(CH_2)_{n1}$-NHC(O)-$(CH_2)_{n2}$-, -$(CH_2)_{n3}$-C(O)NH-$(CH_2)_{n4}$-, - $(CH_2)_{n1}$-NHC(O)-$(CH_2)_{n2}$C(O)-, -$(CH_2)_{n3}$-C(O)NH-$(CH_2)_{n4}$-C(O)-, -S(O)$_2$NR$^v$-, -P(O)R$^w$NR$^v$-, -S(O)NR$^v$-, and -PO$_2$NR$^v$-,
R$^v$ and R$^w$ are each independently H, $C_{1-8}$ alkyl, $C_{3-8}$ cycloalkyl, $C_{1-8}$ alkoxy, $C_{1-8}$ alkylthio, mono- or di- $C_{1-8}$ alkylamino, $C_{3-20}$ heteroaryl or $C_{5-20}$ aryl,
n1, n2, n3 and n4 are each independently an integer from 0 to 5.

**30.** The pyrrolobenzodiazepine derivative or a pharmaceutically acceptable salt or solvate of the derivative according to claim 28,

wherein L' includes one or more branching units and connection units,
wherein the branching unit is selected from the group consisting of $C_{2-8}$ alkenyl, a hydrophilic amino acid, -C(O)-, and -C(O)O-, and
the connection unit is -$(CH_2)_t(V(CH_2)_u)_v$-,
where t is an integer from 0 to 5, u is an integer of 2, v is an integer from 1 to 10, and V is -O-; and
$R_{zz}$' is -O-NH$_2$, -NH$_2$, N$_3$, -OCH$_3$, or -OCH$_2$CH$_3$.

**31.** The pyrrolobenzodiazepine derivative or a pharmaceutically acceptable salt or solvate of the derivative according to claim 1,
wherein the pyrrolobenzodiazepine derivative represented by Chemical Formula I is selected from the group consisting of

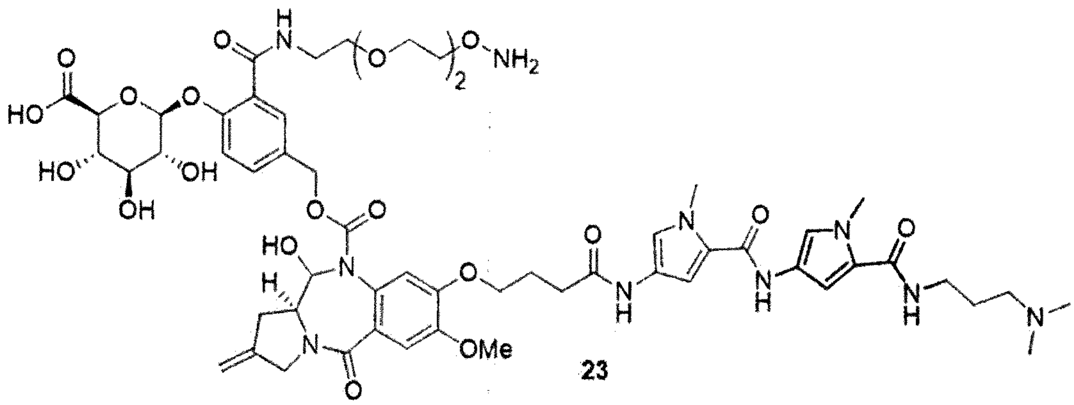

23

,

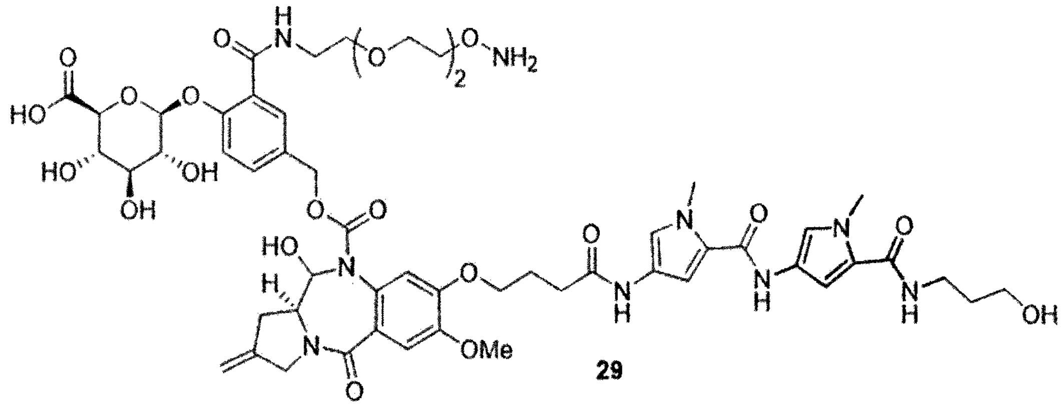

29

,

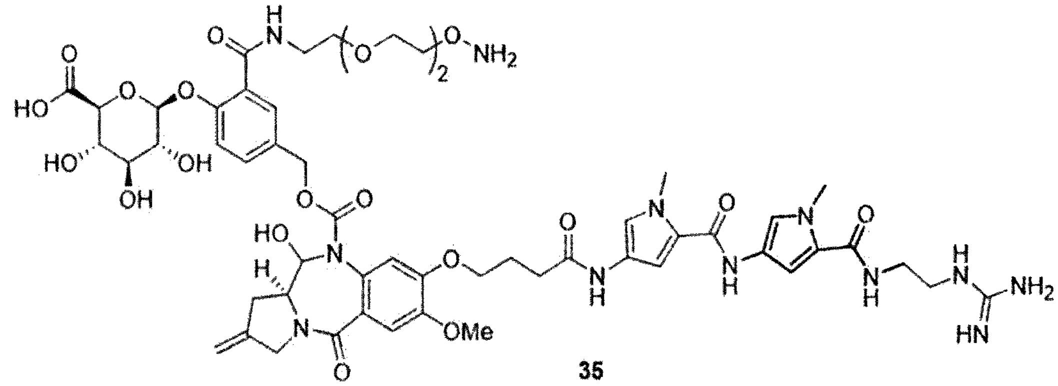

35

,

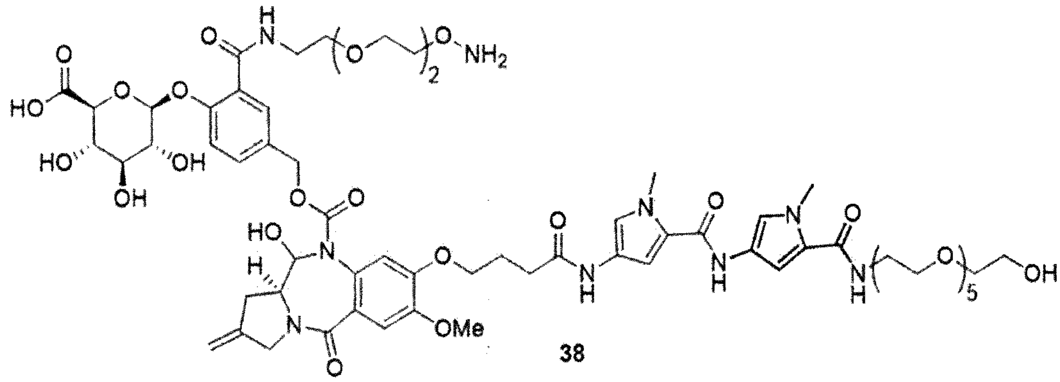

38

,

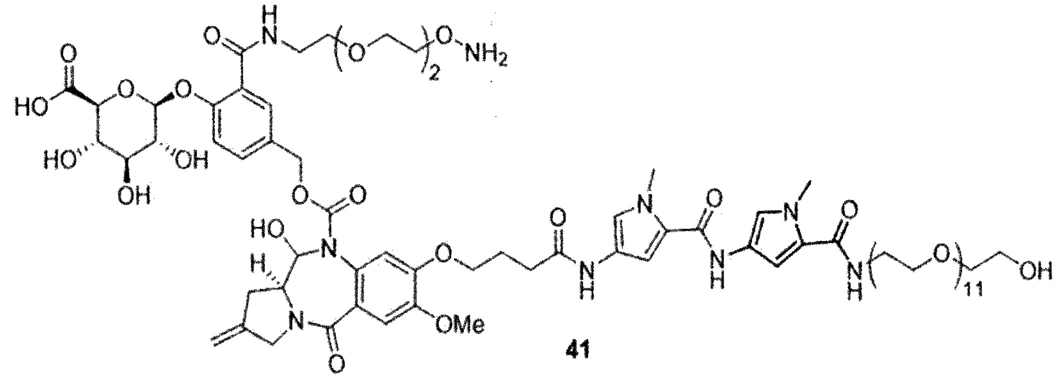

41

,

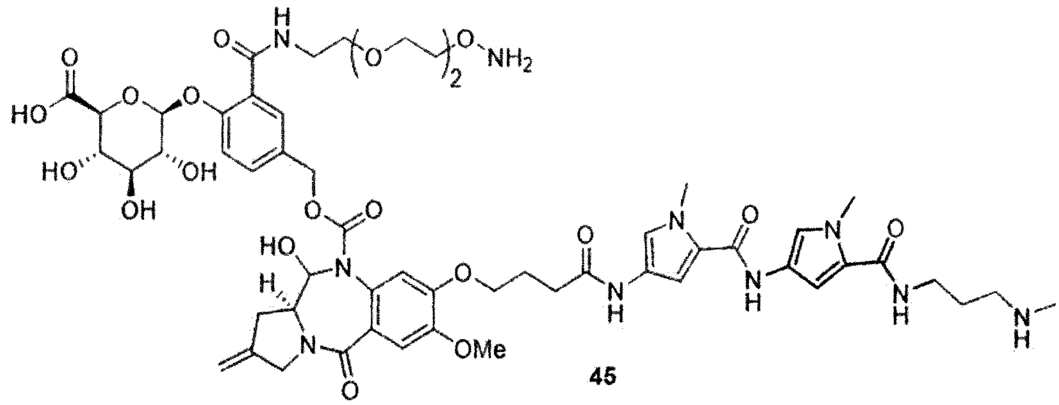

45

,

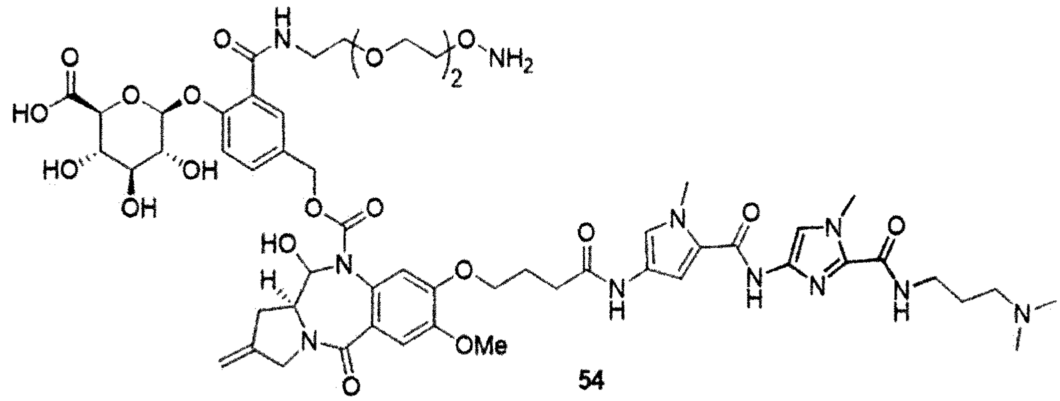

54

,

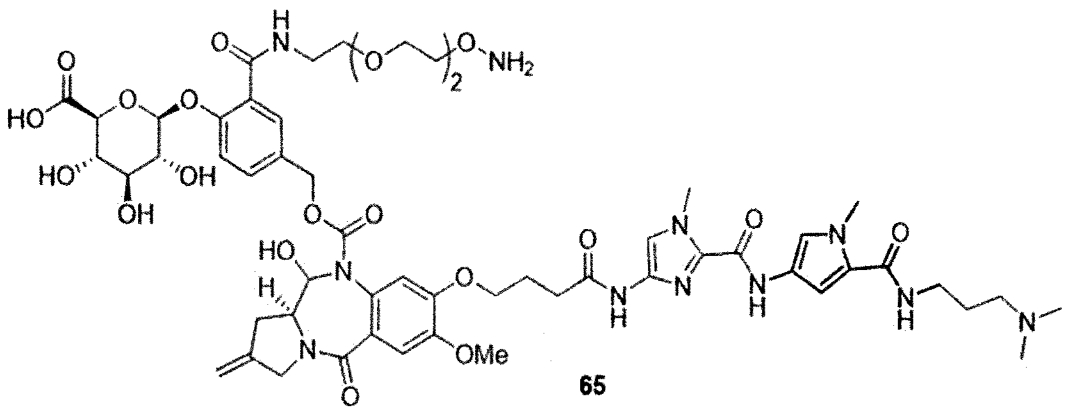

65

,

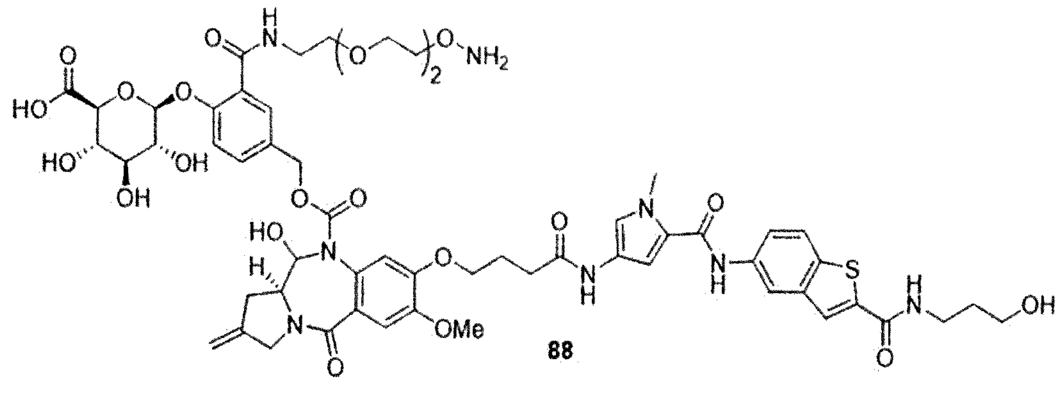

88

,

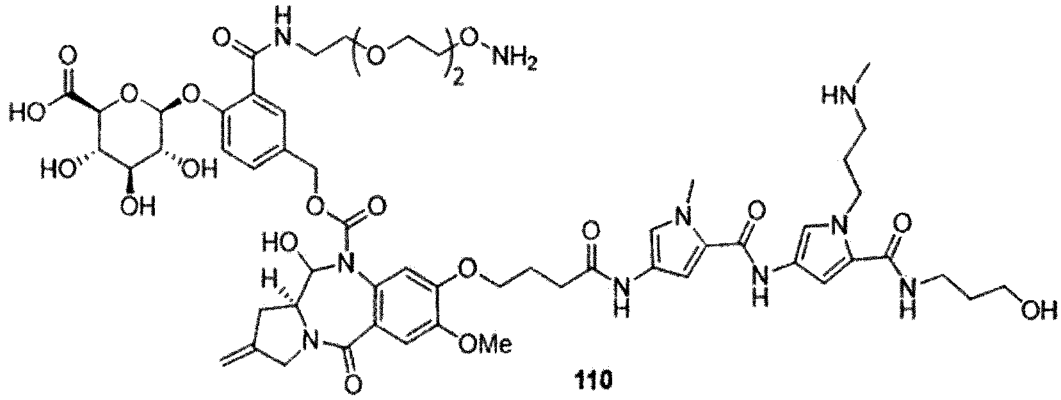

110

,

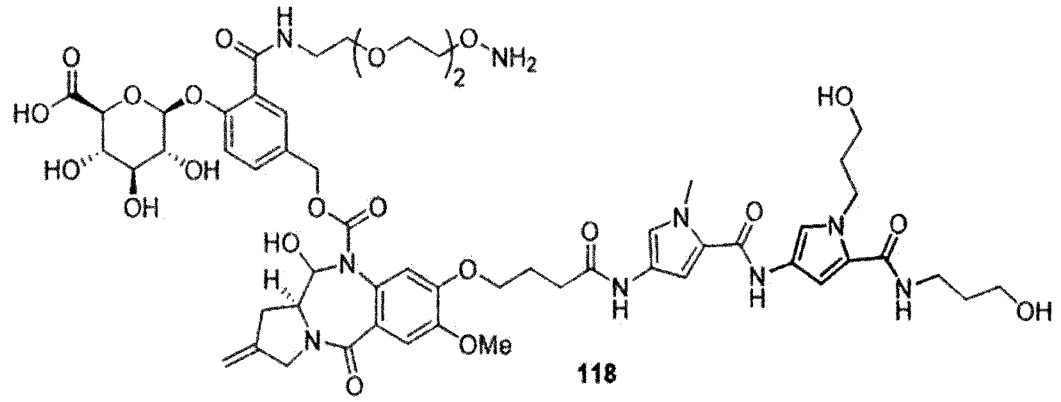

118

,

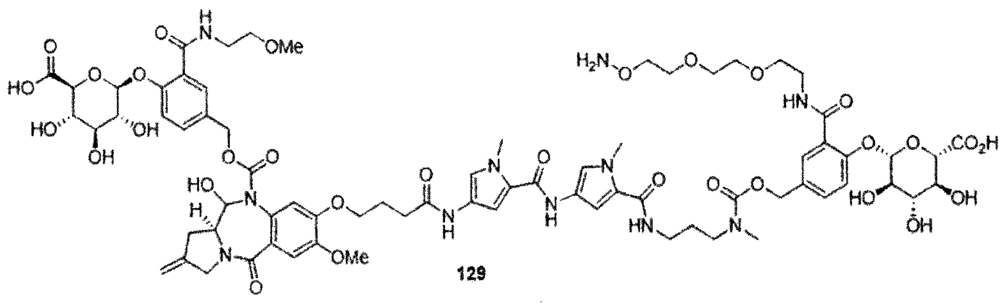

129

,

and

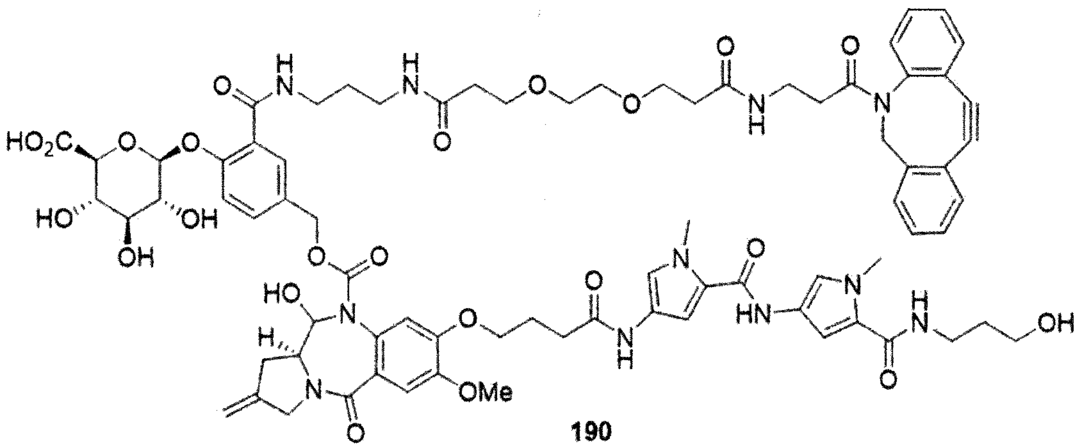

190

.

**32.** The pyrrolobenzodiazepine derivative or a pharmaceutically acceptable salt or solvate of the derivative according to claim 1,
wherein the pyrrolobenzodiazepine derivative represented by Chemical Formula I is selected from the group consisting of

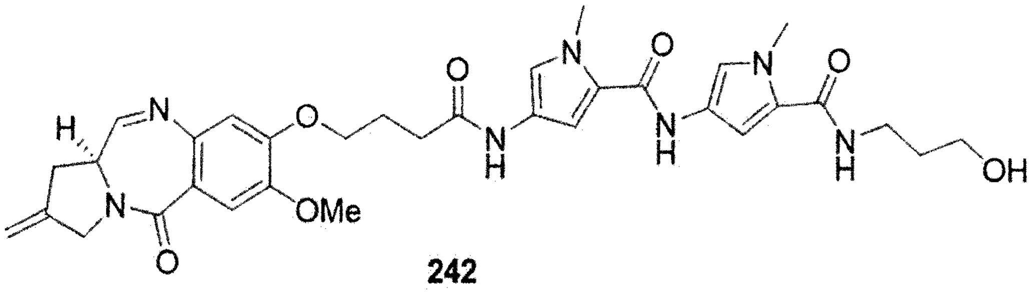

**242**

,

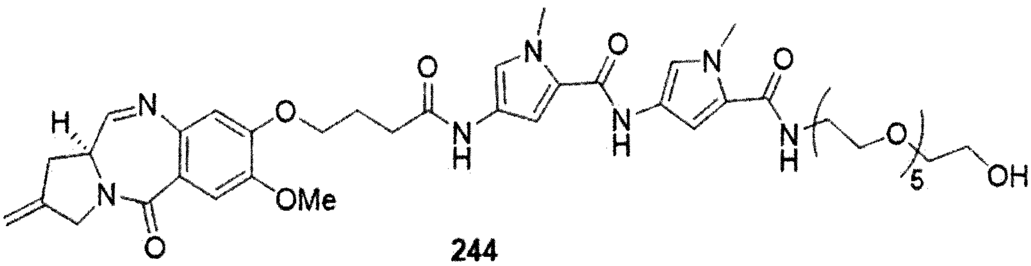

**244**

,

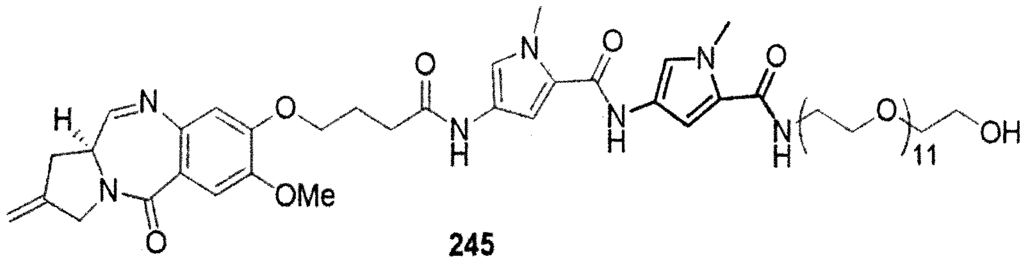

**245**

and

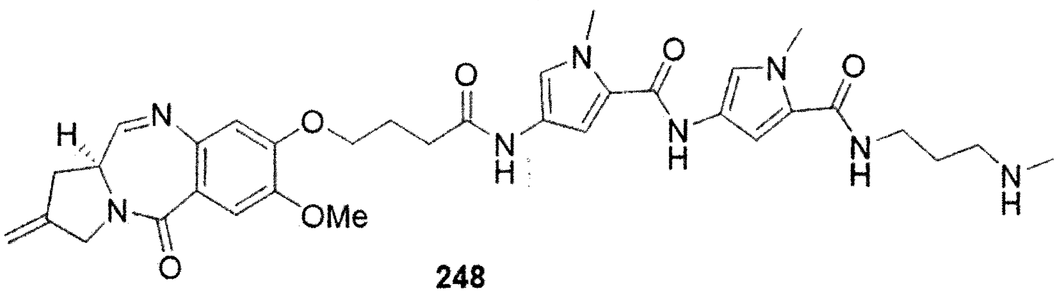

**248**

.

**33.** The pyrrolobenzodiazepine derivative or a pharmaceutically acceptable salt or solvate of the derivative according to claim 28,
wherein the pyrrolobenzodiazepine derivative is

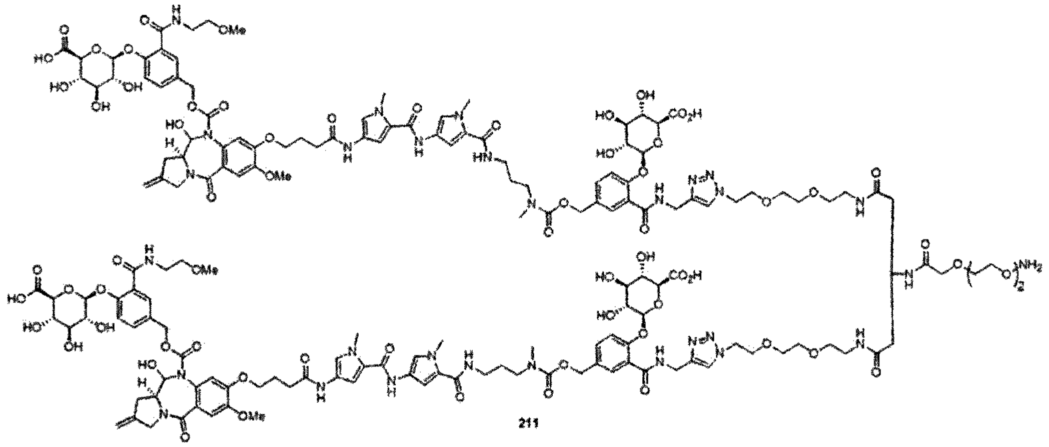

211

.

**34.** A pyrrolobenzodiazepine derivative represented by Chemical Formula VII, or a pharmaceutically acceptable salt or solvate of the derivative:

P'-B'-D' (VII)

where,

P' is the same as P described above;
B' is $B_a$'-$B_b$'-$B_c$',
where $B_a$' is -O-, -NH-, -S-, -C(=O)-, -S(=O)-, - $(CH_2)_{n'}(C=O)$-, $-(CH_2)_{n'}-$, $C_{6-10}$ arylene, 5 to 20-membered heteroarylene containing a heteroatom independently selected from N, O or S, or $-((CH_2)_{n'}B_1)_{m'}B_2-$, where $B_1$ and $B_2$ are each $CH_2$, NH, O, or S,
$B_b$' is a bond, $C_{5-20}$ arylene, $C_{5-20}$ cycloalkylene, or 5- to 20-membered heterocycloalkylene, 5- to 20-membered heterocycloalkenylene, or 5- to 20-membered heteroarylene, in which one or more ring atoms are heteroatoms independently selected from N, O or S, and
$B_c$' is a bond, -O-, -NH-, -S-, -C(=O)-, -S(=O)-, - $CH_2(C=O)$-, $-(CH_2)_{n'}-$ or $-(CH_2CH_2O)_{n'}-$,
where n' and m' are each independently an integer from 1 to 10, and
D' is the same as D described above.

**35.** The pyrrolobenzodiazepine derivative or a pharmaceutically acceptable salt or solvate of the derivative according to claim 34,
wherein $B_a$' is a bond, $-(CH_2)_{n'}-$ or $-(CH_2)_{n'}B_2-$, where $B_2$ is O and n' is an integer from 1 to 10.

**36.** The pyrrolobenzodiazepine derivative or a pharmaceutically acceptable salt or solvate of the derivative according to claim 34,
wherein $B_b$' is a bond,

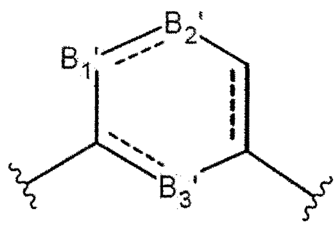

,

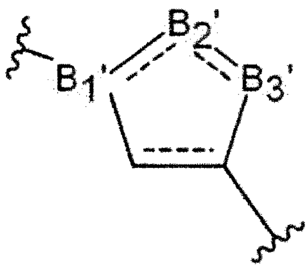

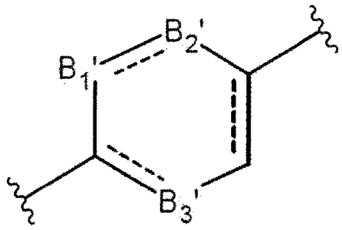

, ,

or

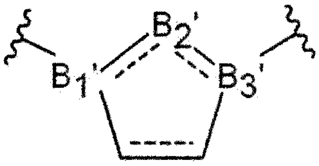

,

where the dotted line means a double bond or a single bond, and
$B_1$', $B_2$' and $B_3$' are each independently C, N, or S.

**37.** The pyrrolobenzodiazepine derivative or a pharmaceutically acceptable salt or solvate of the derivative according to claim 34,
wherein $B_c$' is a bond, -C(=O)-, -NH- or $-(CH_2CH_2O)_n-$, where n is an integer from 1 to 10.

**38.** The pyrrolobenzodiazepine derivative or a pharmaceutically acceptable salt or solvate of the derivative according to claim 34,
wherein D' is the same as D, and $a_1 + a_2$ is an integer from 1 to 10 except for 2.

**39.** The pyrrolobenzodiazepine derivative or a pharmaceutically acceptable salt or solvate of the derivative according to claim 34,
wherein the pyrrolobenzodiazepine derivative represented by Chemical Formula VII is selected from the group consisting of

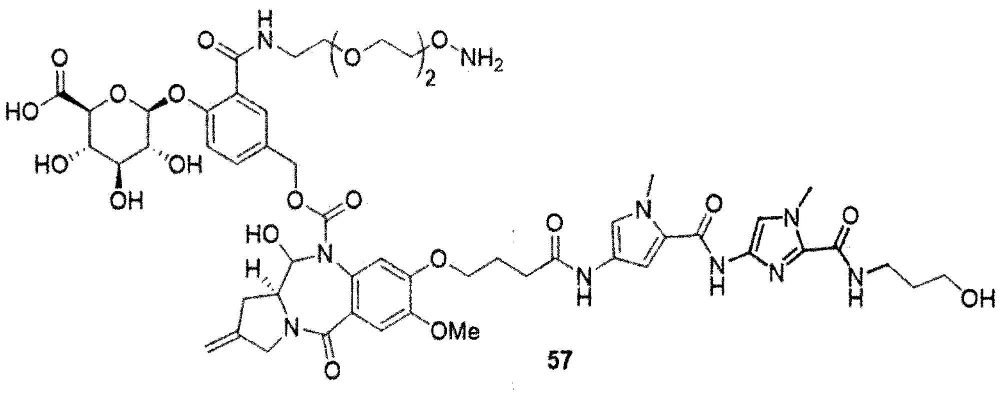

57

,

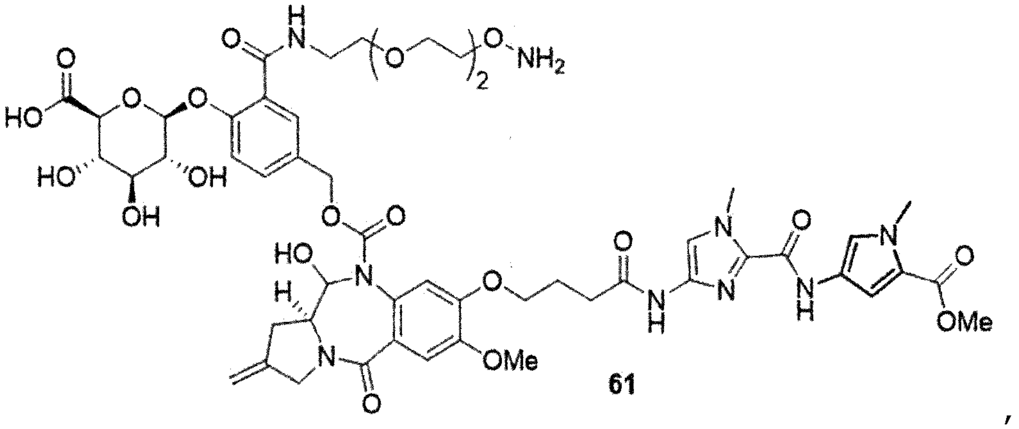

61

,

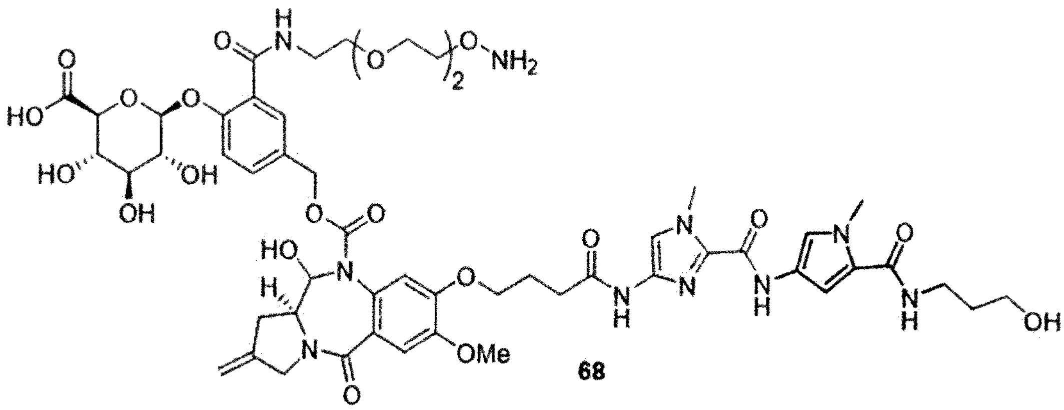

68

,

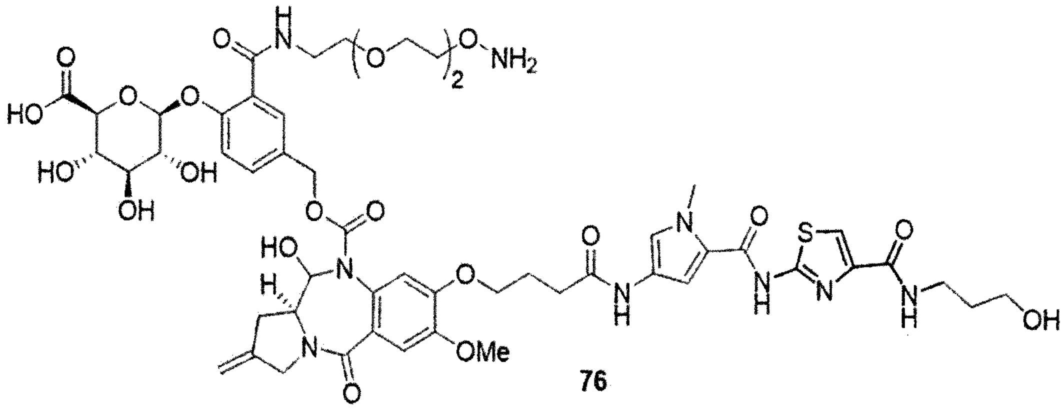

76

,

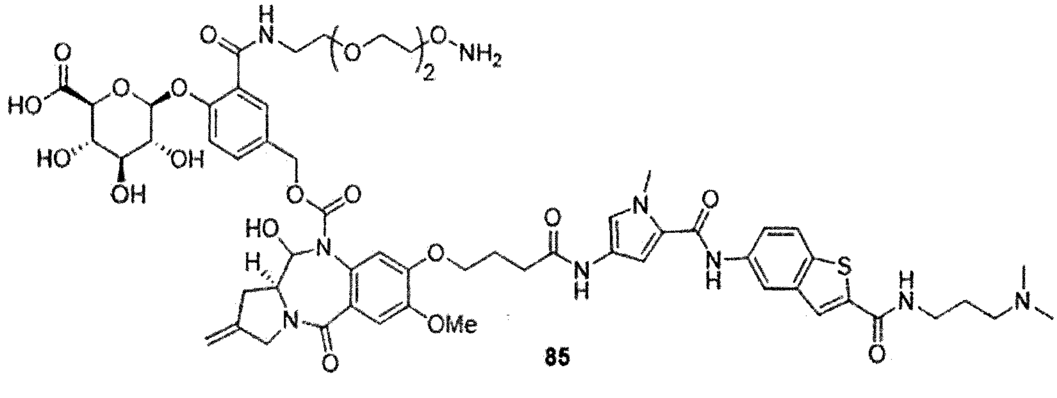

85

,

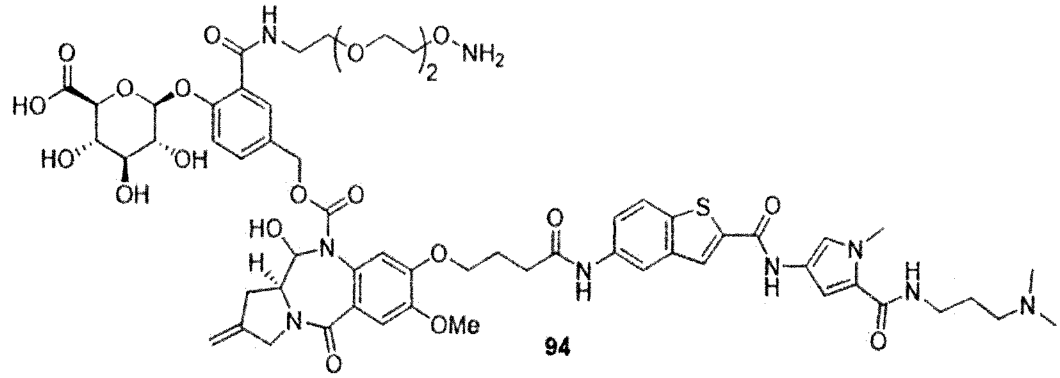

94

,

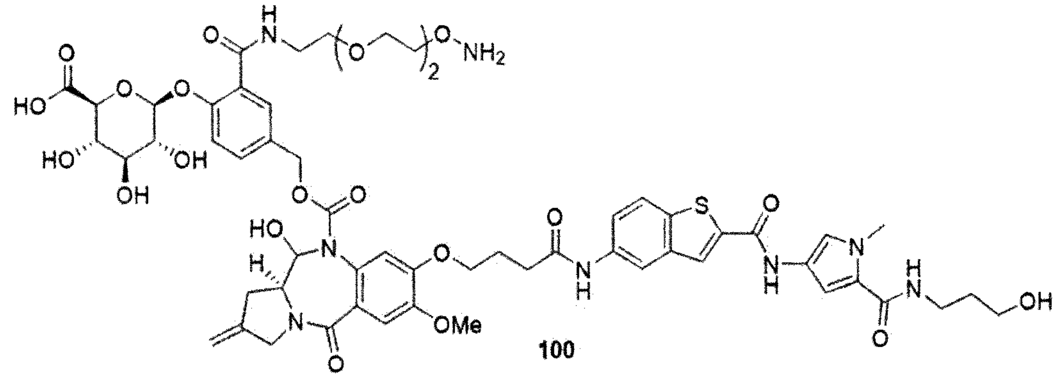

100

,

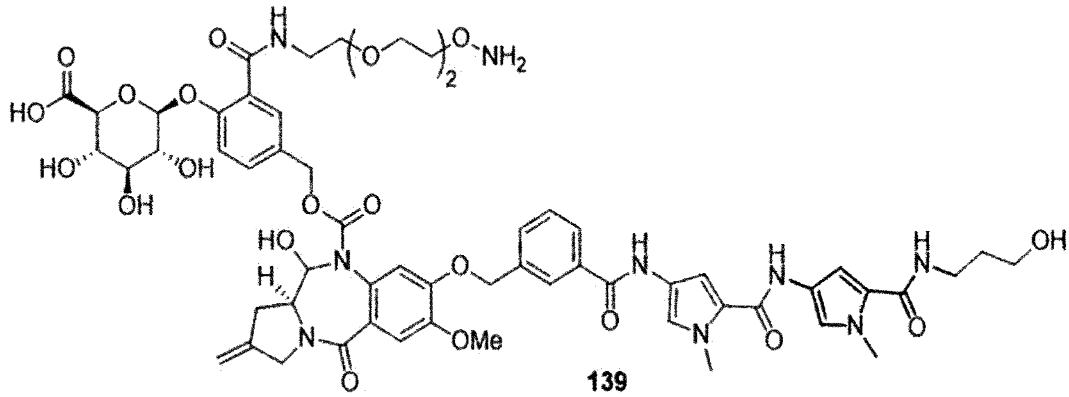

139

,

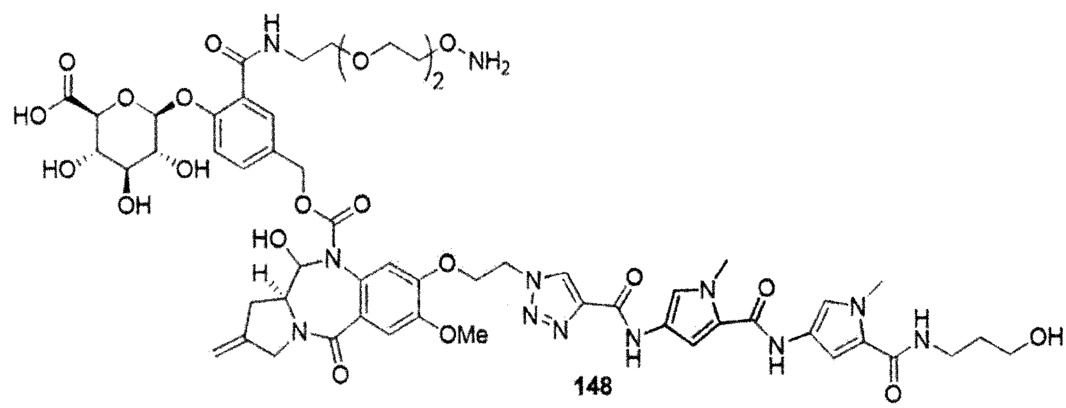

148

,

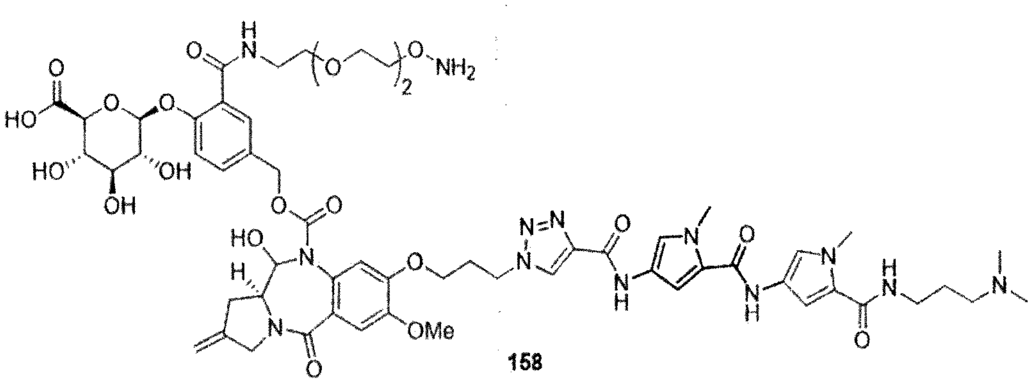

158

,

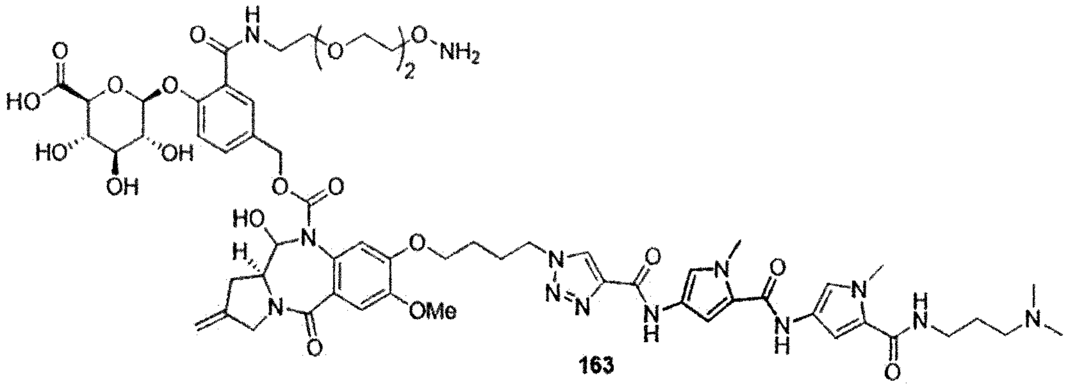

163

,

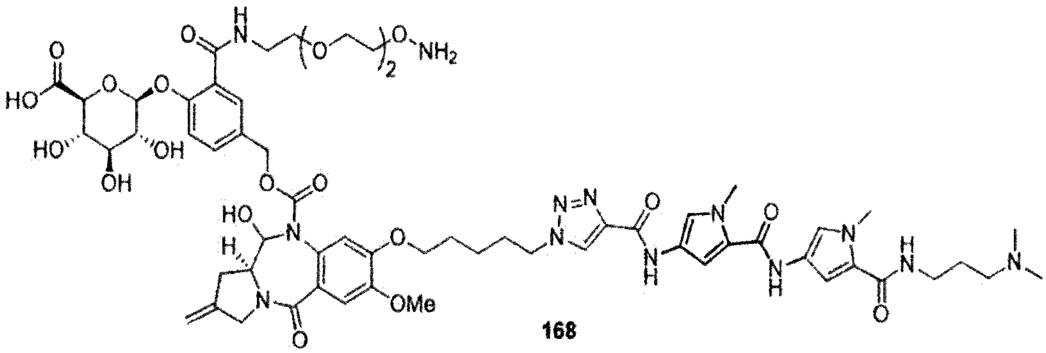

168

,

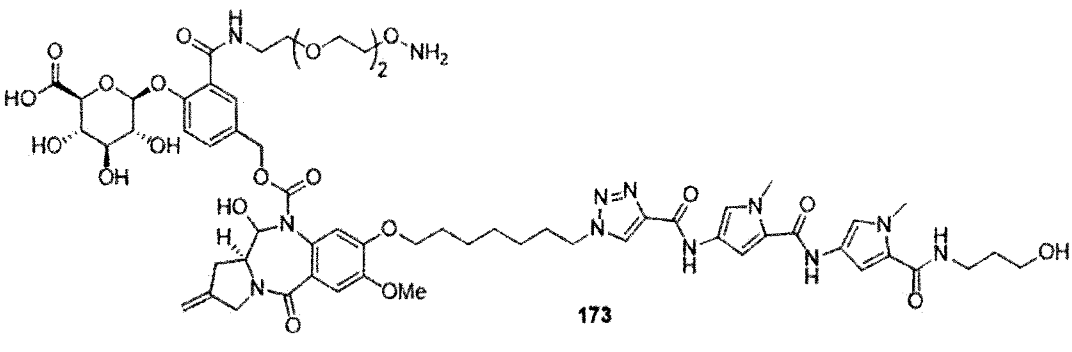

173

,

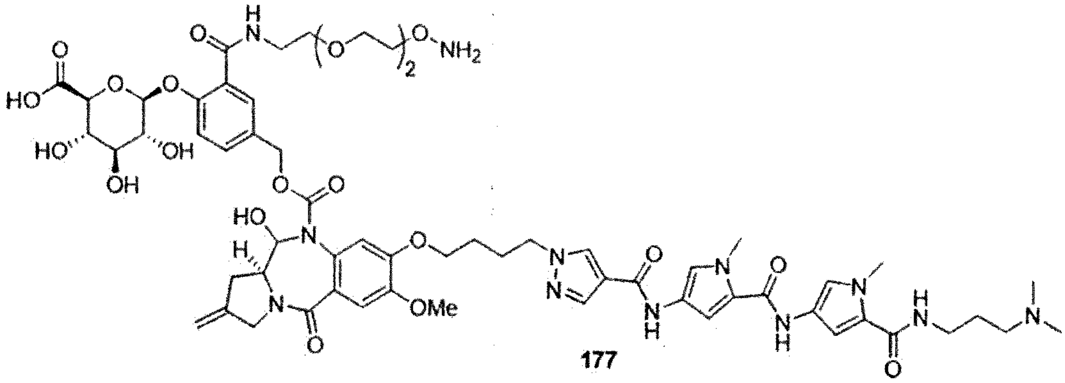

177

,

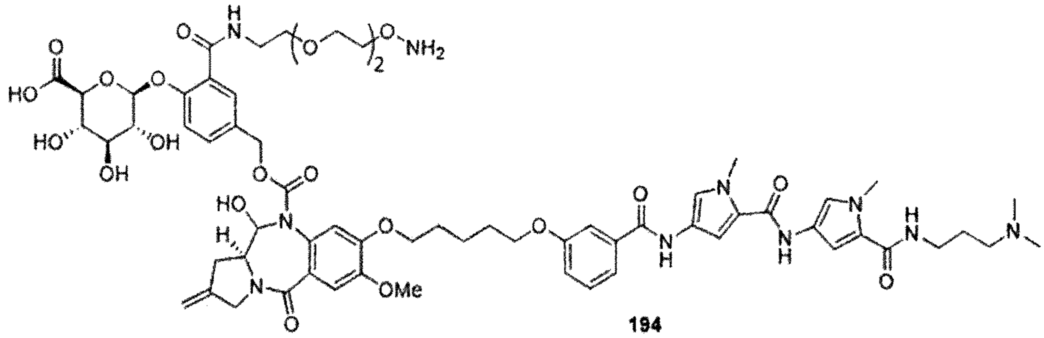

194

,

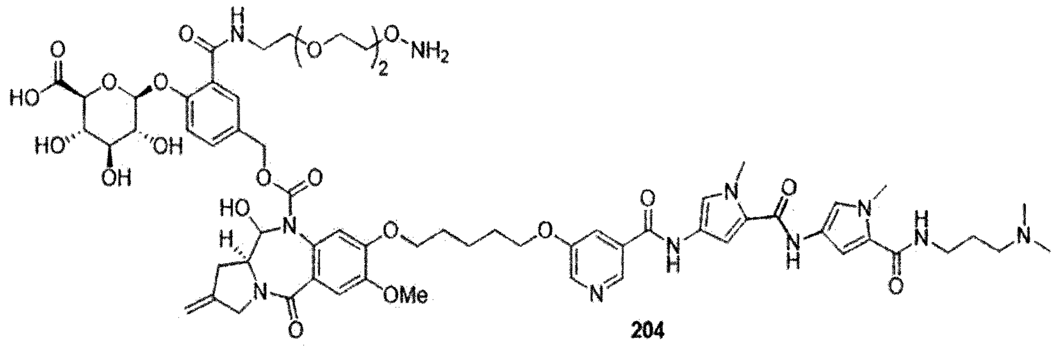

204

,

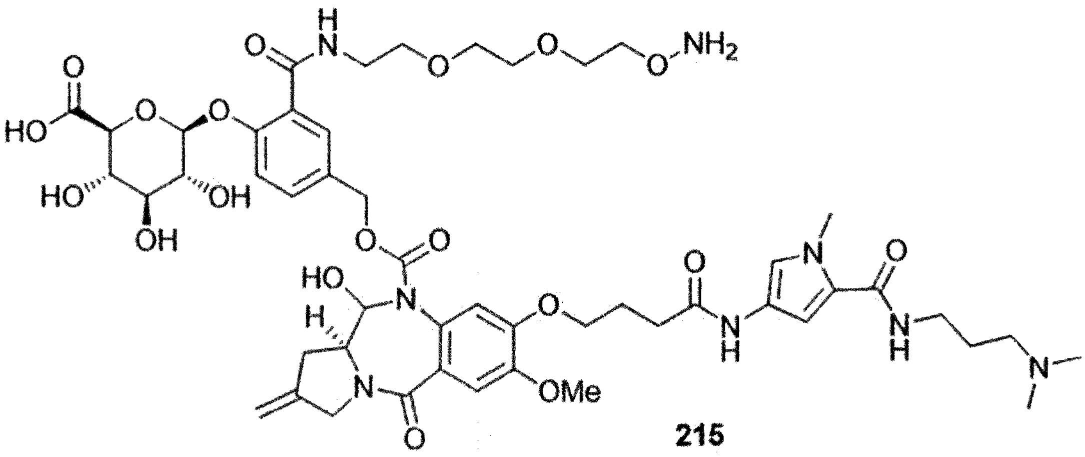

215

,

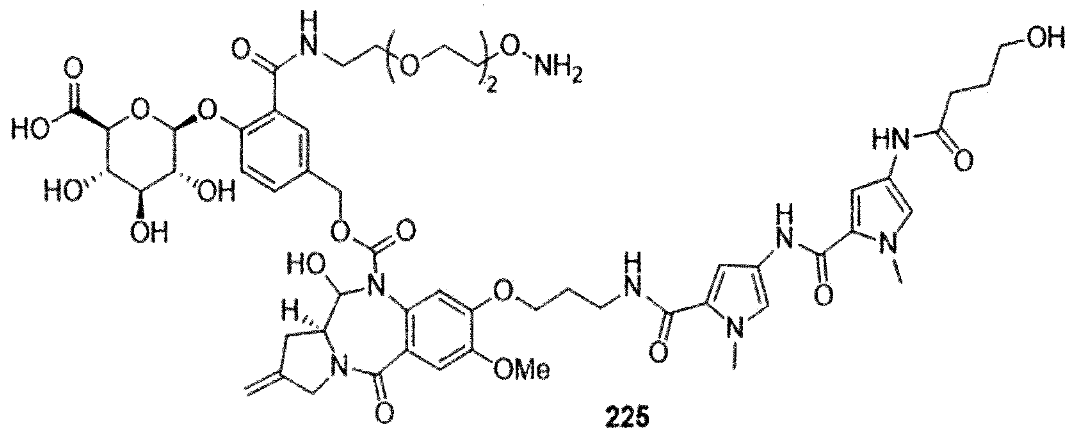

225

,

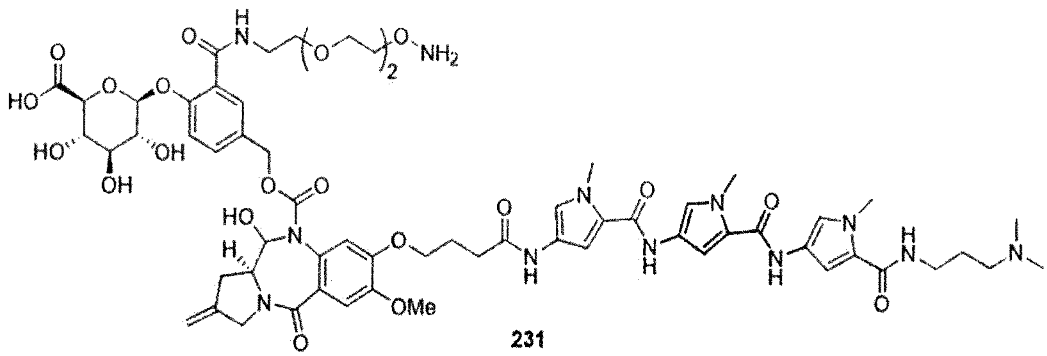

231

,

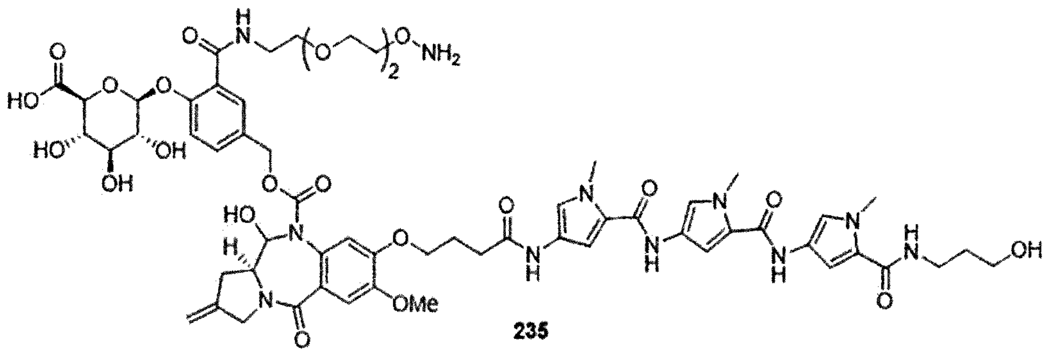

235

,

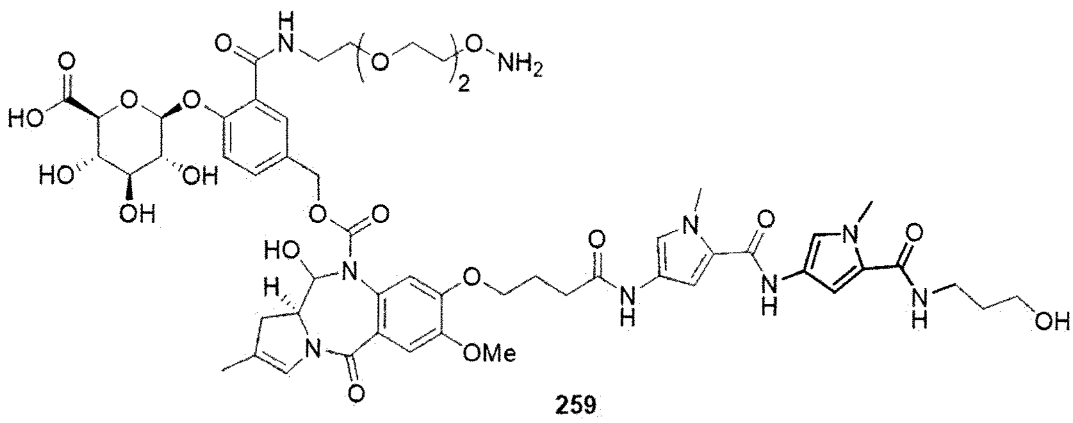

259

,

and

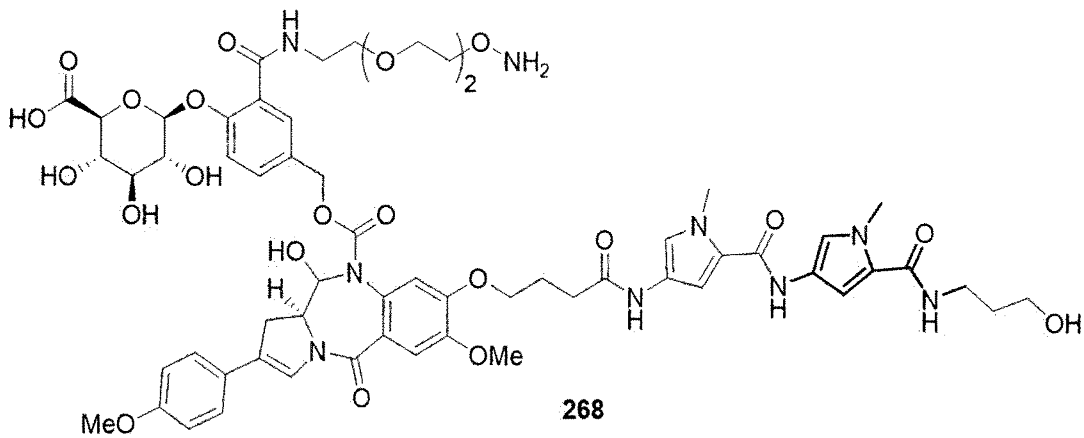

268

.

**40.** A pyrrolobenzodiazepine derivative-ligand conjugate comprising the pyrrolobenzodiazepine derivative according to any one of claims 1 to 33, or a pharmaceutically acceptable salt or solvate of the derivative-ligand conjugate.

**41.** The pyrrolobenzodiazepine derivative-ligand conjugate or a pharmaceutically acceptable salt or solvate of the derivative-ligand conjugate according to claim 40,
wherein the ligand is an antibody.

**42.** The pyrrolobenzodiazepine derivative-ligand conjugate or a pharmaceutically acceptable salt or solvate of the derivative-ligand conjugate according to claim 41,
wherein the antibody has one or more amino acid motifs that may be recognized by an isoprenoid transferase.

**43.** The pyrrolobenzodiazepine derivative-ligand conjugate or a pharmaceutically acceptable salt or solvate of the derivative-ligand conjugate according to claim 42,
wherein the isoprenoid transferase is FTase (farnesyl protein transferase) or GGTase (geranylgeranyl transferase).

**44.** The pyrrolobenzodiazepine derivative-ligand conjugate or a pharmaceutically acceptable salt or solvate of the derivative-ligand conjugate according to claim 42,

wherein the amino acid motif is CYYX, XXCC, XCXC or CXX,
where C is cysteine, Y is an aliphatic amino acid, and X is an amino acid that determines substrate specificity of an isoprenoid transferase.

**45.** A pharmaceutical composition for prevention or treatment of proliferative disease comprising the pyrrolobenzodiazepine derivative-ligand conjugate according to claim 40 or a pharmaceutically acceptable salt or solvate of the derivative-ligand conjugate.

**46.** A pharmaceutical composition for prevention or treatment of proliferative disease comprising the pyrrolobenzodiazepine derivative-ligand conjugate according to claim 40 or a pharmaceutically acceptable salt or solvate of the derivative-ligand conjugate; and a pharmaceutically acceptable excipient.

**47.** A pharmaceutical composition for prevention or treatment of proliferative disease comprising the pyrrolobenzodiazepine derivative-ligand conjugate according to claim 40 or a pharmaceutically acceptable salt or solvate of the derivative-ligand conjugate; one or more therapeutic co-agents; and a pharmaceutically acceptable excipient.

**48.** A method for preventing or treating proliferative disease, the method comprising administering a pyrrolobenzodiazepine derivative-ligand conjugate comprising the pyrrolobenzodiazepine derivative according to any one of claims 1 to 33, or a pharmaceutically acceptable salt or solvate of the derivative-ligand conjugate to an individual.

**49.** Use of a pyrrolobenzodiazepine derivative-ligand conjugate comprising the pyrrolobenzodiazepine derivative according to any one of claims 1 to 33, or a pharmaceutically acceptable salt or solvate of the derivative-ligand conjugate

in a pharmaceutical composition for prevention or treatment of proliferative disease.

【FIG. 1】

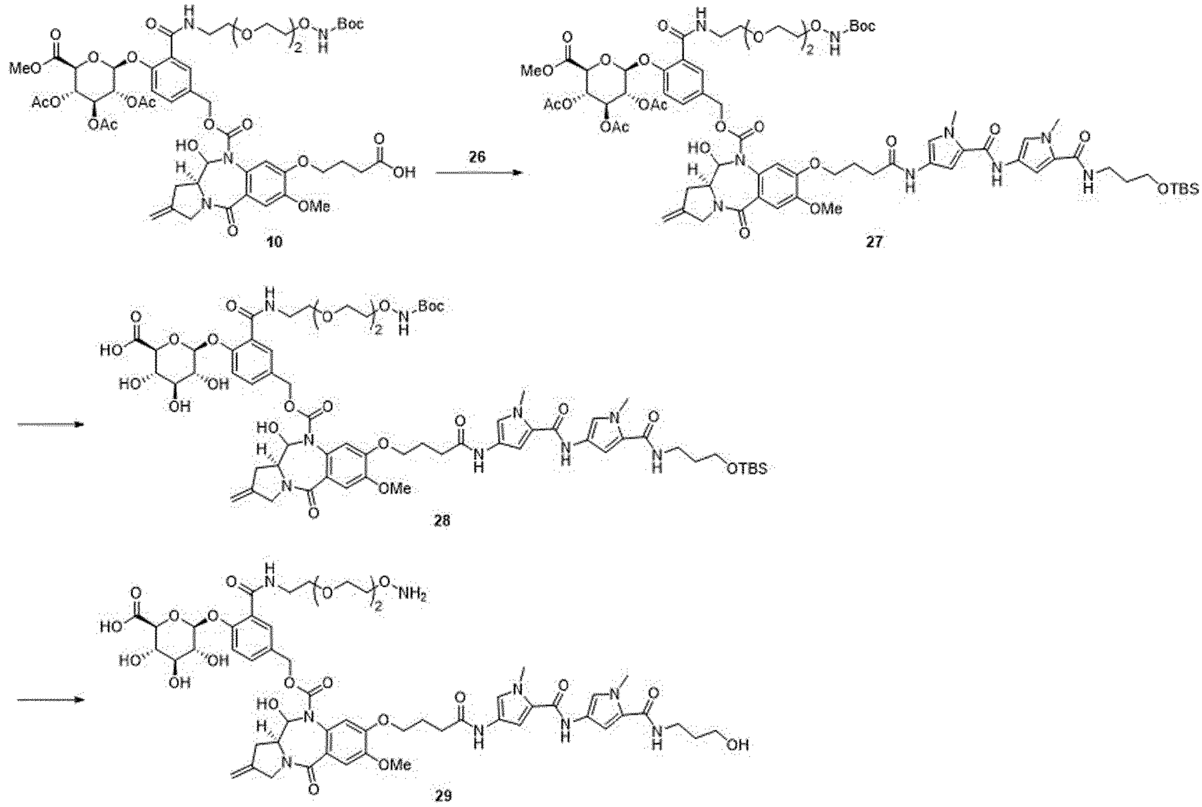


10

27

28

29

【FIG. 2】

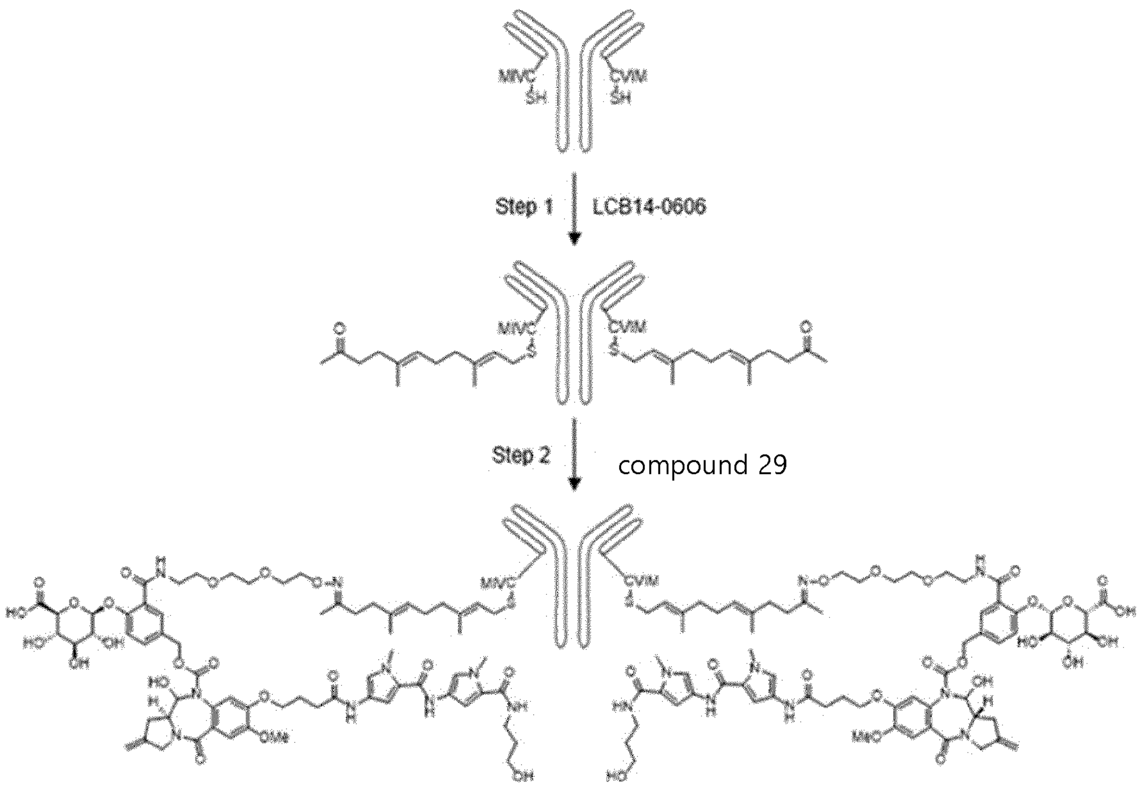

**INTERNATIONAL SEARCH REPORT**

International application No.
**PCT/KR2020/019466**

**A. CLASSIFICATION OF SUBJECT MATTER**

**C07H 15/26**(2006.01)i; **A61K 31/7056**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

C07H 15/26(2006.01); A61K 31/55(2006.01); A61K 31/5517(2006.01); C07D 403/14(2006.01); C07D 487/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 피롤로[1,4]벤조디아제핀(pyrrolo[1,4]benzodiazepin), 링커(linker), 리간드(ligand), 항체(antibody), 증식성 질환(proliferative disease)

**C. DOCUMENTS CONSIDERED TO BE RELEVANT**

| Category* | Citation of document, with indication, where appropriate, of the relevant passages | Relevant to claim No. |
|---|---|---|
| | WO 2007-039752 A1 (SPIROGEN LIMITED) 12 April 2007 (2007-04-12) | |
| X | See claims 1, 15, 17 and 18; and pages 43 and 45. | 1,4-7,18-27,34-38 |
| A | | 2,3,8-17,28-33,39-47,49 |
| | US 2017-0333442 A1 (MERSANA THERAPEUTICS, INC.) 23 November 2017 (2017-11-23) | |
| X | See claims 1, 31, 56 and 68; paragraphs [0417], [0420], [0421], [0436], [0437] and [0445]; and page 23. | 1-7,18-27,32,34-38,40,41,45-47,49 |
| Y | | 42-44 |
| | KR 10-2018-0110645 A (LEGOCHEM BIOSCIENCES, INC.) 10 October 2018 (2018-10-10) | |
| Y | See claims 1, 11, 20-23 and 26. | 42-44 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 April 2021** | **09 April 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2020/019466**

**C. DOCUMENTS CONSIDERED TO BE RELEVANT**

| Category* | Citation of document, with indication, where appropriate, of the relevant passages | Relevant to claim No. |
|---|---|---|
| X | BARALDI, P. G. et al. Synthesis, in Vitro Antiproliferative Activity, and DNA-Binding Properties of Hybrid Molecules Containing Pyrrolo[2,1-c][1,4]benzodiazepine and Minor-Groove-Binding Oligopyrrole Carriers. J. Med. Chem. 1999, vol. 42, pp. 5131-5141. See abstract; and formula 2, compounds 22-25. | 1,4-7,18-27,34-38 |
| X | KUMAR, R. et al. Design and Synthesis of Novel Pyrrolo[2,1-c][1,4]benzodiazepine-Imidazole Containing Polyamide Conjugates. Heterocyclic Communications. 2002, vol. 8, no. 1, pp. 19-26. See figure 1, compounds 1-5. | 1,4-7,18-27,34-38 |
| X | VARVOUNIS, G. An Update on the Synthesis of Pyrrolo[1,4]benzodiazepines. Molecules. 2016, vol. 21, no. 154, inner pp. 1-55. See formula 17, compound 102a-f; and formula 21, compound 124a-n. | 1,4-7,18-27,34-38 |
| X | DAMAYANTHI, Y. et al. Design and Synthesis of Novel Pyrrolo[2,1-c][1,4]benzodiazepine-Lexitropsin Conjugates. J. Org. Chem. 1999, vol. 64, pp. 290-292. See formula 2, compounds 5(a-c) and 6(a-c); and page 292. | 1,4-7,18-27,34-38 |

Form PCT/ISA/210 (second sheet) (July 2019)

**INTERNATIONAL SEARCH REPORT**

International application No.
**PCT/KR2020/019466**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **48**
because they relate to subject matter not required to be searched by this Authority, namely:
Claim 48 pertains to a method for treatment of the human body by surgery or therapy, as well as a diagnostic method (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2020/019466**

| Patent document cited in search report | Publication date (day/month/year) | Patent family member(s) | Publication date (day/month/year) |
|---|---|---|---|
| WO 2007-039752 A1 | 12 April 2007 | EP 1931671 A1 | 18 June 2008 |
| | | EP 1931671 B1 | 08 April 2009 |
| | | US 2007-0154906 A1 | 05 July 2007 |
| | | US 2008-0214525 A1 | 04 September 2008 |
| | | US 8637664 B2 | 28 January 2014 |
| US 2017-0333442 A1 | 23 November 2017 | EP 3458069 A2 | 27 March 2019 |
| | | US 10143695 B2 | 04 December 2018 |
| | | US 10660901 B2 | 26 May 2020 |
| | | US 2019-0070194 A1 | 07 March 2019 |
| | | WO 2017-201132 A2 | 23 November 2017 |
| | | WO 2017-201132 A3 | 22 February 2018 |
| KR 10-2018-0110645 A | 10 October 2018 | AU 2018-246806 A1 | 17 October 2019 |
| | | CN 109790171 A | 21 May 2019 |
| | | EP 3604311 A1 | 05 February 2020 |
| | | JP 2020-512270 A | 23 April 2020 |
| | | KR 10-2018-0110649 A | 10 October 2018 |
| | | WO 2018-182341 A1 | 04 October 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101960130
- KR 101891859
- KR 101059183
- KR 101995620
- KR 1020190100413
- US 20170369453 A
- WO 2016148674 A
- KR 1020200084802
- KR 1020180110645
- WO 2017089890 A
- KR 1020200127891
- KR 1020140035393


### Non-patent literature cited in the description


- **HU Y et al.** Benzodiazepine biosynthesis in Streptomyces refuineus. *Chem Biol.,* June 2007, vol. 14 (6), 691-701
- **LUKE A. et al.** Synthesis and biological evaluation of novel pyrrolo[2,1-c][1,4]benzodiazepine prodrugs for use in antibody directed enzyme prodrug therapy. *Bioorganic & Medicinal Chemistry Letters,* 2006, vol. 16 (2), 252-256
- **ZHANG, DONGLU et al.** Linker Immolation Determines Cell Killing Activity of Disulfide-Linked Pyrrolobenzodiazepine Antibody-Drug Conjugates. *ACS Medicinal Chemistry Letters,* 2016, vol. 7 (11), 988-993
- **BENJAMIN P. DUCKWORTH et al.** *ChemBioChem,* 2007, vol. 8, 98
- **UYEN T. T. NGUYEN et al.** *ChemBioChem,* 2007, vol. 8, 408
- **GUILLERMO R. LABADIE et al.** *J. Org. Chem.,* 2007, vol. 72 (24), 9291
- **JAMES W. WOLLACK et al.** *ChemBioChem,* 2009, vol. 10, 2934
- **IRAN M. BELL.** *J. Med. Chem.,* 2004, vol. 47 (8), 1869
- **HOLLINGER ; HUDSON.** *Nature Biotechnology,* 2005, vol. 23 (9), 1126-1136
- *ACS. Med. Chem.,* 2016, vol. 7 (11), 983-987